# EUROPEAN PATENT SPECIFICATION

(11) **EP 3 990 920 B1**
(45) Date of publication and mention of the grant of the patent: **12.02.2025**
(21) Application number: 20833334.4
(22) Date of filing: 25.06.2020
(51) Int. Cl.: G01N 33/542, C12N 15/10, C12Q 1/6876, C12Q 1/6804, G01N 33/58, G01N 33/536

(54) **METHODS AND COMPOSITIONS FOR PROXIMITY LIGATION**
VERFAHREN UND ZUSAMMENSETZUNGEN ZUR NÄHERUNGSLIGATION
PROCÉDÉS ET COMPOSITIONS POUR LIGATURE DE PROXIMITÉ

(30) Priority: 27.06.2019 US 201962867463 P; 03.07.2019 US 201962870297 P; 05.11.2019 US 201962931069 P; 17.04.2020 US 202063011490 P; 23.04.2020 US 202063014422 P
(43) Date of publication of application: 04.05.2022
(60) Divisional application: 24223881.4
(73) Proprietor: Dovetail Genomics, LLC, Scotts Valley, CA 95066 (US)
(72) Inventor: MUNDING, Elizabeth, Scotts Valley, California 95066 (US); BLANCHETTE, Marco, Scotts Valley, California 95066 (US)
(74) Representative: J A Kemp LLP
(86) International application number: PCT/US2020/039656
(87) International publication number: WO 2020/264185

(56) References cited:
- WO-A1-2016/089920
- WO-A1-2017/197300
- WO-A1-2019/152543
- US-A1- 2017 314 014
- JASON M RIZZO ET AL: "Standardized collection of MNase-seq experiments enables unbiased dataset comparisons", BMC MOLECULAR BIOLOGY, BIOMED CENTRAL LTD, GB, vol. 13, no. 1, 6 May 2012 (2012-05-06), pages 15, XP021115269, ISSN: 1471-2199, DOI: 10.1186/1471-2199-13-15
- VIVIANA I. RISCA ET AL: "Unraveling the 3D genome: genomics tools for multiscale exploration", TRENDS IN GENETICS., vol. 31, no. 7, 1 July 2015 (2015-07-01), NL, pages 357 - 372, XP055534599, ISSN: 0168-9525, DOI: 10.1016/j.tig.2015.03.010

## Description

### BACKGROUND

Obtaining high-quality, contiguous genome sequences is often difficult, especially in cases when limited source material is available for sequence analysis. While obtaining raw sequence data has become faster and available at a lower cost, suitable methods for analyzing and assembling the data efficiently and accurately remains a challenge. WO 2017/197300 discloses methods to isolate genome or chromosome level structural information from preserved samples. Risca et al. (2015) Trends Genet. 31(7):357-72. doi: 10.1016/j.tig.2015.03.010 discusses methods and tools for investigating 3D genomic architecture.

### SUMMARY

The invention is defined by the claims and any other aspects, configurations or embodiments set forth herein not falling within the scope of the claims are for information only. Any references in the description to methods of treatment refer to the compounds, pharmaceutical compositions and medicaments of the present invention for use in a method for treatment of the human (or animal) body by therapy (or for diagnosis).
In one aspect, the present invention relates to method comprising: (a) obtaining a cross-linked biological sample comprising a nucleic acid molecule complexed to at least one nucleic acid binding protein; (b) contacting the cross-linked biological sample to a non-specific endonuclease to cleave the nucleic acid molecule into a plurality of segments; (c) attaching a first segment and a second segment of the plurality of segments at a junction thereby obtaining a product; (d) reversing crosslinks in the product of (c) to obtain an additional product; and (e) subjecting the plurality of segments additional product of (d) to size selection to obtain a plurality of selected segments having a size from about 100 bp to about 600 bp, wherein attaching comprises contacting at least the first segment and the second segment to at least one bridge oligonucleotide.
In one aspect, there are provided methods of nucleic acid analysis. In some cases, methods may comprise: (a) obtaining a stabilized biological sample comprising a nucleic acid molecule complexed to at least one nucleic acid binding protein; (b) contacting the stabilized biological sample to a non-specific endonuclease to cleave the nucleic acid molecule into a plurality of segments; (c) attaching a first segment and a second segment of the plurality of segments at a junction; and (d) subjecting the plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the method further comprises, prior to step (d), preparing a sequencing library from the plurality of segments. In some cases, the method further comprises subjecting the sequencing library to a size selection to obtain a size-selected library. In some cases, the size-selected library is between about 350 bp and 1000 bp in size. In some cases, the size selection is conducted with gel electrophoresis, capillary electrophoresis, size selection beads, or a gel filtration column. In some cases, the method further comprises analyzing the plurality of selected segments to obtain a QC value. In some cases, the QC value is a chromatin digest efficiency (CDE) based on the proportion of segments between 100 and 2500 bp in size prior to step (d). In some cases, the method further comprises selecting a sample for further analysis when the CDE value is at least 65%. In some cases, the QC value is a chromatin digest index (CDI) based on the ratio of a number of mononucleosome-sized segments to a number of dinucleosome-sized segments prior to step (d). In some cases, the method further comprises selecting a sample for further analysis when the CDI value is greater than -1.5 and less than 1. In some cases, the method further comprises subsequent to the contacting the stabilized biological sample to a non-specific endonuclease, binding the plurality of segments to one or more surfaces. In some cases, the one or more surfaces comprise one or more beads. In some cases, the one or more beads are solid phase reversible immobilization (SPRI) beads. In some cases, the stabilized biological sample comprises a stabilized cell lysate. In some cases, the stabilized biological sample comprises a stabilized intact cell. In some cases, the stabilized biological sample comprises a stabilized intact nucleus. In some cases, step (b) is conducted prior to lysis of the intact cell or the intact nucleus. In some cases, the method further comprises prior to step (c), lysing cells and/or nuclei in the stabilized biological sample. In some cases, the stabilized biological sample comprises fewer than 3,000,000 cells. In some cases, the stabilized biological sample comprises fewer than 1,000,000 cells. In some cases, the stabilized biological sample comprises fewer than 100,000 cells. In some cases, the stabilized biological sample comprises less than 10 µg DNA. In some cases, the stabilized biological sample comprises less than 1 µg DNA. In some cases, the non-specific endonuclease is DNase. In some cases, the DNase is DNase I. In some cases, the DNase is DNase II. In some cases, the DNase is micrococcal nuclease. In some cases, the DNase is selected from one or more of DNase I, DNase II, and micrococcal nuclease. In some cases, the stabilized biological sample has been treated with a crosslinking agent. In some cases, the crosslinking agent is a chemical fixative. In some cases, the chemical fixative comprises formaldehyde. In some cases, the chemical fixative comprises psoralen. In some cases, the chemical fixative comprises disuccinimidyl glutarate (DSG). In some cases, the chemical fixative comprises ethylene glycol bis(succinimidyl succinate) (EGS). In some cases, the chemical fixative comprises disuccinimidyl glutarate (DSG) and ethylene glycol bis(succinimidyl succinate) (EGS). In some cases, the crosslinking agent is ultraviolet light. In some cases, the stabilized biological sample is a crosslinked paraffin-embedded tissue sample. In some cases, the method further comprises contacting the plurality of selected segments to an antibody. In some cases, the method further comprises conducting immunoprecipitation on the plurality of segments. In some cases, the immunoprecipitation is conducted subsequent to the attaching. In some cases, attaching comprises filling in sticky ends using biotin tagged nucleotides. In some cases, attaching comprises filling in sticky ends using untagged nucleotides. In some cases, attaching comprises ligating blunt ends. In some cases, attaching comprises adding overhangs. In some cases, adding overhangs comprises adenylation. In some cases, attaching comprises contacting at least the first segment and the second segment to at least one bridge oligonucleotide. In some cases, the bridge oligonucleotide is at least 10 bp in length. In some cases, the bridge oligonucleotide is at least 12 bp in length. In some cases, the bridge oligonucleotide is 12 bp in length. In some cases, the bridge oligonucleotide comprises a barcode sequence. In some cases, the bridge oligonucleotide comprises an affinity tag. In some cases, the affinity tag is biotin. In some cases, attaching comprises contacting at least the first segment and the second segment to multiple bridge oligonucleotides in series. In some cases, attaching results in samples, cells, nuclei, chromosomes, or nucleic acid molecules of the stabilized biological sample receiving a unique sequence of bridge oligonucleotides. In some cases, the at least one bridge oligonucleotide is coupled to an immunoglobulin binding protein or a fragment thereof. In some cases, the at least one bridge oligonucleotide is coupled or fused to two or more immunoglobulin binding proteins or fragments thereof. In some cases, the immunoglobulin binding protein is selected from a Protein A, a Protein G, a Protein A/G, and a Protein L. In some cases, attaching comprises contacting at least the first segment and the second segment to a barcode. In some cases, the method does not comprise a shearing step. In some cases, the method further comprises: (e) obtaining at least some sequence on each side of the junction to generate a first read pair. In some cases, the method further comprises (f) mapping the first read pair to a set of contigs; and (g) determining a path through the set of contigs that represents an order and/or orientation to a genome. Alternatively or in combination, the method further comprises (f) mapping the first read pair to a set of contigs; and (g) determining, from the set of contigs, a presence of a structural variant or loss of heterozygosity in the stabilized biological sample. Alternatively or in combination, the method further comprises (f) mapping the first read pair to a set of contigs; and (g) assigning a variant in the set of contigs to a phase. In some cases, the variant is a human leukocyte antigen (HLA) variant. In some cases, the variant is a killer-cell immunoglobulin-like receptor (KIR) variant. Alternatively or in combination, the method further comprises (f) mapping the first read pair to a set of contigs; (g) determining, from the set of contigs, a presence of a variant in the set of contigs; and (h) conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample. In some cases, the DNase is coupled or fused to an immunoglobulin binding protein or a fragment thereof. In some cases, the DNase is coupled to two or more immunoglobulin binding proteins or fragments thereof. In some cases, the immunoglobulin binding protein is selected from a Protein A, a Protein G, a Protein A/G, and a Protein L.

In another aspect, there are provided methods comprising: (a) obtaining a stabilized biological sample comprising a nucleic acid molecule complexed to at least one nucleic acid binding protein; (b) contacting the stabilized biological sample to a micrococcal nuclease (MNase) to cleave the nucleic acid molecule into a plurality of segments; and (c) attaching a first segment and a second segment of the plurality of segments at a junction. In some cases, the method further comprises (d) subjecting the plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the method further comprises prior to step (d), preparing a sequencing library from the plurality of segments. In some cases, the method further comprises subjecting the sequencing library to a size selection to obtain a size-selected library. In some cases, the size-selected library is between about 350 bp and 1000 bp in size. In some cases, the size selection is conducted with gel electrophoresis, capillary electrophoresis, size selection beads, or a gel filtration column. In some cases, the method further comprises analyzing the plurality of selected segments to obtain a QC value. In some cases, the QC value is a chromatin digest efficiency (CDE) based on the proportion of segments between 100 and 2500 bp in size prior to step (d). In some cases, the method further comprises selecting a sample for further analysis when the CDE value is at least 65%. In some cases, the QC value is a chromatin digest index (CDI) based on the ratio of a number of mononucleosome-sized segments to a number of dinucleosome-sized segments prior to step (d). In some cases, the method further comprises selecting a sample for further analysis when the CDI value is greater than -1.5 and less than 1. In some cases, the method further comprises subsequent to the contacting the stabilized biological sample to the MNase, binding the plurality of segments to one or more surfaces. In some cases, the one or more surfaces comprise one or more beads. In some cases, the one or more beads are solid phase reversible immobilization (SPRI) beads. In some cases, the stabilized biological sample comprises a stabilized cell lysate. In some cases, the stabilized biological sample comprises a stabilized intact cell. In some cases, the stabilized biological sample comprises a stabilized intact nucleus. In some cases, step (b) is conducted prior to lysis of the intact cell or the intact nucleus. In some cases, the method further comprises prior to step (c), lysing cells and/or nuclei in the stabilized biological sample. In some cases, the stabilized biological sample comprises fewer than 3,000,000 cells. In some cases, the stabilized biological sample comprises fewer than 1,000,000 cells. In some cases, the stabilized biological sample comprises fewer than 100,000 cells. In some cases, the stabilized biological sample comprises less than 10 µg DNA. In some cases, the stabilized biological sample comprises less than 1 µg DNA. In some cases, the stabilized biological sample is further treated with a DNase. In some cases, the DNase is DNase I. In some cases, the DNase is DNase II. In some cases, the DNase is selected from one or more of DNase I and DNase II. In some cases, the stabilized biological sample has been treated with a crosslinking agent. In some cases, the crosslinking agent is a chemical fixative. In some cases, the chemical fixative comprises formaldehyde. In some cases, the chemical fixative comprises psoralen. In some cases, the chemical fixative comprises disuccinimidyl glutarate (DSG). In some cases, the chemical fixative comprises ethylene glycol bis(succinimidyl succinate) (EGS). In some cases, the chemical fixative comprises disuccinimidyl glutarate (DSG) and ethylene glycol bis(succinimidyl succinate) (EGS). In some cases, the crosslinking agent is ultraviolet light. In some cases, the stabilized biological sample is a crosslinked paraffin-embedded tissue sample. In some cases, the method further comprises contacting the plurality of selected segments to an antibody. In some cases, the method further comprises conducting immunoprecipitation on the plurality of segments. In some cases, the immunoprecipitation is conducted subsequent to the attaching. In some cases, attaching comprises filling in sticky ends using biotin tagged nucleotides. In some cases, attaching comprises filling in sticky ends using untagged nucleotides. In some cases, attaching comprises ligating blunt ends. In some cases, attaching comprises adding overhangs. In some cases, the adding overhangs comprises adenylation. In some cases, attaching comprises contacting at least the first segment and the second segment to a bridge oligonucleotide. In some cases, the bridge oligonucleotide is at least 10 bp in length. In some cases, the bridge oligonucleotide is at least 12 bp in length. In some cases, the bridge oligonucleotide is 12 bp in length. In some cases, the bridge oligonucleotide comprises a barcode sequence. In some cases, the bridge oligonucleotide comprises an affinity tag. In some cases, the affinity tag is biotin. In some cases, attaching comprises contacting at least the first segment and the second segment to multiple bridge oligonucleotides in series. In some cases, the attaching results in samples, cells, nuclei, chromosomes, or nucleic acid molecules of the stabilized biological sample receiving a unique sequence of bridge oligonucleotides. In some cases, the at least one bridge oligonucleotide is coupled to an immunoglobulin binding protein or a fragment thereof. In some cases, the at least one bridge oligonucleotide is coupled to two or more immunoglobulin binding proteins or fragments thereof. In some cases, the immunoglobulin binding protein is selected from a Protein A, a Protein G, a Protein A/G, and a Protein L. In some cases, attaching comprises contacting at least the first segment and the second segment to a barcode. In some cases, the method does not comprise a shearing step. In some cases, the method further comprises (e) obtaining at least some sequence on each side of the junction to generate a first read pair. In some cases, the method further comprises (f) mapping the first read pair to a set of contigs; and (g) determining a path through the set of contigs that represents an order and/or orientation to a genome. Alternatively or in combination, the method further comprises (f) mapping the first read pair to a set of contigs; and (g) determining, from the set of contigs, a presence of a structural variant or loss of heterozygosity in the stabilized biological sample. Alternatively or in combination, the method further comprises (f) mapping the first read pair to a set of contigs; and (g) assigning a variant in the set of contigs to a phase. In some cases, the variant is a human leukocyte antigen (HLA) variant. In some cases, the variant is a killer-cell immunoglobulin-like receptor (KIR) variant. Alternatively or in combination, the method further comprises (f) mapping the first read pair to a set of contigs; (g) determining, from the set of contigs, a presence of a variant in the set of contigs; and (h) conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample. In some cases, the MNase is coupled or fused to an immunoglobulin binding protein. In some cases, the MNase is coupled or fused to two or more immunoglobulin binding proteins or fragments thereof. In some cases, the immunoglobulin binding protein is selected from a Protein A, a Protein G, a Protein A/G, and a Protein L.

In additional aspects, there are provided nucleic acid libraries comprising: (a) a first cell genome library component comprising a plurality of first cell genome fragment pairs, wherein at least one of said first cell genome fragment pairs comprises two first cell genome segments tethered via a nucleic acid segment comprising a first cell genome library-indicative tag; and (b) a second cell genome library component comprising a plurality of second cell genome fragment pairs, wherein at least one of said second cell genome fragment pairs comprises two second cell genome segments tethered via a nucleic acid segment comprising a second cell genome library-indicative tag. In some cases, the two first cell genome segments tethered via a nucleic acid segment comprising a first cell genome library-indicative tag indicate a first cell genome configuration in a first cell. In some cases, the two second cell genome segments tethered via a nucleic acid segment comprising a second cell genome library-indicative tag indicate a second cell genome configuration in a second cell, wherein the second cell genome configuration is different from the first cell genome configuration. In some cases, the first cell genome library component is obtained from an isolated eukaryotic nucleus. In some cases, the plurality of first cell genome fragment pairs are bookended by recombinase sites. In some cases, the recombinase site is an integrase integration site. In some cases, the recombinase site is a transposase mosaic end. In some cases, at least one recombinase site of the recombinase sites comprises an exonuclease resistant moiety. In some cases, the exonuclease resistant moiety comprises a phosphorothioate. In some cases, the nucleic acid segment comprising a first cell genome library-indicative tag further comprises a recombinase left border and a recombinase right border. In some cases, the recombinase is an integrase. In some cases, the recombinase is a transposase. In some cases, the nucleic acid segment comprising a first cell genome library-indicative tag comprises an affinity tag. In some cases, the affinity tag comprises biotin. In some cases, at least some library members are clonal copies. In some cases, co-occurrence of read pairs that map to comparable regions of a nucleic acid reference indicate distance of the regions to one another in the cell. In some cases, the distance is a relative distance.

In additional aspects, there are provided systems comprising: a plurality of cell genome aliquots, wherein at least some of the cell genome aliquots comprise genome binding moieties that preserve genome component positional information; and a plurality of recombinase nucleic acid aliquots, wherein at least some of the recombinase nucleic acid aliquots comprise distinguishable sequence relative to at least one other aliquot. In some cases, the recombinase is an integrase. In some cases, the recombinase is a transposase. In some cases, at least some of the cell genome aliquots comprise fragmented genome molecules. In some cases, at least some of the fragmented genome molecules comprise integration site ends. In some cases, the cell genome aliquots comprise eukaryotic cell genome aliquots. In some cases, the genome binding moieties that preserve genome component positional information comprise chromatin constituents. In some cases, the genome binding moieties that preserve genome component positional information comprise nucleosomes. In some cases, the plurality of cell genome aliquots comprise integrase enzymes. In some cases, the plurality of recombinase nucleic acid aliquots comprise integrase nucleic acid molecules having integrase integration sites. In some cases, the plurality of cell genome aliquots comprise transposase enzymes. In some cases, the plurality of recombinase nucleic acid aliquots comprise transposase nucleic acid molecules having transposase mosaic ends. In some cases, at least one integration site of the integration sites comprises an exonuclease resistant moiety. In some cases, at least one mosaic end of the mosaic ends comprises an exonuclease resistant moiety. In some cases, the exonuclease resistant moiety comprises a phosphorothioate. In some cases, at least some of the recombinase nucleic acid molecules comprise a recombinase left border and an recombinase right border. In some cases, the nucleic acid segment comprising a first cell genome library-indicative tag comprises an affinity tag. In some cases, the affinity tag comprises biotin. In some cases, the distinguishable sequence relative to at least one other aliquot comprises a plurality of identical nucleic acid sequences in an aliquot. In some cases, a first aliquot comprises a plurality of nucleic acid molecules having a common distinguishable sequence relative to at least one other aliquot. In some cases, the plurality of cell genome aliquots comprise single-cell genome aliquots.

In additional aspects, there are provided methods of assaying for a chromosomal conformation variation between at least two cells. In some cases, methods comprise obtaining genomic nucleic acids from the two cells wherein chromosomal conformation variation is preserved; introducing internal breaks into genomic nucleic acids from the two cells; and linking two exposed ends adjacent to the internal breaks to one another via one of a plurality of tagged segments, wherein tags of a first cell are distinguishable from tags of a second cell. In some cases, the genomic nucleic acids from the two cells are isolated prior to said linking. In some cases, amplifying nucleic acid molecules resultant from said linking to produce amplicons comprising chromosomal break junction ends linked by an internal segment comprising distinguishable sequence such that chromosomal break junctions from the two cells are distinguishable. In some cases, isolating fragments from a first cell of the two cells comprising two formerly exposed ends linked by a first tagged segment and fragments from a second cell of the at least two cells comprising two formerly exposed ends linked by a second tagged segment. In some cases, obtained paired end sequence information comprising at least some first tag information and at least some second tag information. In some cases, assigning paired ends to a common proximity in a cell. In some cases, assigning paired ends to a common proximity comprises assessing a number of occurrences of paired ends mapping to two common clusters, and correlating the relative proximity to the number of occurrences. In some cases, said fragments from a first cell and said fragments from a second cell are isolated in a common volume. In some cases, said fragments from a first cell and said fragments from a second cell are sequenced in a common volume. In some cases, linking two exposed ends adjacent to the internal breaks to one another comprises linking two exposed ends that were not immediately adjacent prior to said introducing internal breaks. In some cases, linking two exposed ends adjacent to the internal breaks to one another comprises linking two exposed ends that were remote to one another on a common nucleic acid molecule prior to said introducing internal breaks. In some cases, linking two exposed ends adjacent to the internal breaks to one another comprises linking two exposed ends that were in physical proximity to one another prior to said introducing internal breaks. In some cases, the at least two cells comprise at least two cell populations.

**In** further aspects, there are provided methods comprising obtaining a stabilized sample comprising a nucleic acid molecule complexed to at least one nucleic acid binding protein; cleaving the nucleic acid molecule into a plurality of segments comprising at least a first segment and a second segment; attaching adapters comprising first recombinase sites to the first segment and to the second segment; and contacting the first segment and the second segment with a linker comprising second recombinase sites in the presence of a recombinase, thereby generating a linked nucleic acid comprising a first sequence from the first segment, a linker sequence from the linker, and a second sequence from the second segment. In some cases, the recombinase is an integrase. In some cases, the recombinase is a transposase. In some cases, the method further comprises sequencing at least a portion of the linked nucleic acid. In some cases, the sequencing comprises sequencing at least a portion of the first sequence and at least a portion of the second sequence. In some cases, the method further comprises mapping at least a portion of the first sequence and at least a portion of the second sequence to a genome. In some cases, the method further comprises conducting three-dimensional genomic analysis using information from the sequencing. In some cases, the stabilized sample is a cross-linked sample. In some cases, obtaining the stabilized sample comprises obtaining a sample and stabilizing the sample. In some cases, obtaining the stabilized sample comprises obtaining a sample that was previously stabilized. In some cases, the nucleic acid binding protein comprises chromatin or a constituent thereof. In some cases, cleaving comprises enzymatic digestion. In some cases, the enzymatic digestion comprises digestion with one or more restriction enzymes. In some cases, the enzymatic digestion comprises digestion with one or more non-specific nucleases. In some cases, the one or more non-specific nucleases comprise DNase or MNase. In some cases, the attaching the first recombinase sites comprises ligation. In some cases, the first recombinase sites and the second recombinase sites comprise integrase sites attP and attB. In some cases, the adapters further comprise sequencing adapter regions. In some cases, the sequencing adapter regions comprise Y adapters. In some cases, the sequencing adapter regions comprise P5 and/or P7 adapters. In some cases, the first recombinase sites and the second recombinase sites comprise transposase mosaic ends. In some cases, the linker sequence comprises an affinity tag. In some cases, the affinity tag is biotin. In some cases, the linker sequence comprises a barcode sequence. In some cases, the barcode sequence is indicative of a partition of origin. In some cases, the barcode sequence is indicative of a cell of origin. In some cases, the barcode sequence is indicative of a cell population of origin. In some cases, the barcode sequence is indicative of an organism of origin. In some cases, the barcode sequence is indicative of a species of origin.

In further aspects there are provided methods comprising: obtaining a stabilized sample comprising a nucleic acid molecule complexed to at least one nucleic acid binding protein; cleaving the nucleic acid molecule into a plurality of segments comprising at least a first segment and a second segment; attaching the first segment to the second segment, thereby creating proximity ligated segments; recovering the proximity ligated segments; and sequencing at least a portion of the proximity ligated segments, wherein sequencing adapters are not attached to the proximity ligated segments after the recovering. In some cases, attaching is conducted by ligating the first segment to the second segment. In some cases, attaching is conducted using recombinase. In some cases, attaching is conducted via a linker. In some cases, the linker comprises an affinity tag. In some cases, the affinity tag is biotin. In some cases, the method further comprises prior to the attaching in step (c), attaching recombination adapters comprising recombinase sites to the first segment and the second segment. In some cases, the recombination adapters comprise sequencing adapters. In some cases, the sequencing adapters comprise Y adapters. In some cases, the sequencing adapters comprise P5 and/or P7 adapters.

Provided herein are methods comprising: (a) obtaining a stabilized biological sample comprising a nucleic acid molecule complexed to at least one nucleic acid binding protein; (b) contacting the stabilized biological sample to a DNase to cleave the nucleic acid molecule into a plurality of segments; (c) attaching a first segment and a second segment of the plurality of segments at a junction; and (d) subjecting the plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the method further comprises, prior to step (d), preparing a sequencing library from the plurality of segments. In some cases, the method further comprises subjecting the sequencing library to a size selection to obtain a size-selected library. In some cases, the size-selected library is between about 350 bp and 1000 bp in size. In some cases, the size selection is conducted with gel electrophoresis, capillary electrophoresis, size selection beads, or a gel filtration column. In some cases, the method further comprises analyzing the plurality of selected segments to obtain a QC value. In some cases, the QC value is a chromatin digest efficiency (CDE) based on the proportion of segments between 100 and 2500 bp in size prior to step (d) . In some cases, the method further comprises selecting a sample for further analysis when the CDE value is at least 65%. In some cases, the QC value is a chromatin digest index (CDI) based on the ratio of a number of mononucleosome-sized segments to a number of dinucleosome-sized segments prior to step (d). In some cases, the method further comprises selecting a sample for further analysis when the CDI value is greater than -1.5 and less than 1. In some cases, the stabilized biological sample comprises a stabilized cell lysate. In some cases, the stabilized biological sample comprises a stabilized intact cell. In some cases, the stabilized biological sample comprises a stabilized intact nucleus. In some cases, step (b) is conducted prior to lysis of the intact cell or the intact nucleus. In some cases, the method further comprises, prior to step (c), lysing cells and/or nuclei in the stabilized biological sample. In some cases, the stabilized biological sample comprises fewer than 3,000,000 cells. In some cases, the stabilized biological sample comprises fewer than 1,000,000 cells. In some cases, the stabilized biological sample comprises fewer than 100,000 cells. In some cases, the stabilized biological sample comprises less than 10 µg DNA. In some cases, the stabilized biological sample comprises less than 1 µg DNA. In some cases, the DNase is DNase I. In some cases, the DNase is DNase II. In some cases, the DNase is micrococcal nuclease. In some cases, the DNase is selected from one or more of DNase I, DNase II, and micrococcal nuclease. In some cases, the stabilized biological sample has been treated with a crosslinking agent. In some cases, the crosslinking agent is a chemical fixative. In some cases, the chemical fixative comprises formaldehyde. In some cases, the chemical fixative comprises psoralen. In some cases, the chemical fixative comprises disuccinimidyl glutarate (DSG). In some cases, the chemical fixative comprises ethylene glycol bis(succinimidyl succinate) (EGS). In some cases, the crosslinking agent is ultraviolet light. In some cases, the stabilized biological sample is a crosslinked paraffin-embedded tissue sample. In some cases, the method further comprises contacting the plurality of selected segments to an antibody. In some cases, attaching comprises filling in sticky ends using biotin tagged nucleotides and ligating the blunt ends. In some cases, attaching comprises contacting at least the first segment and the second segment to at least one bridge oligonucleotide. In some cases, the bridge oligonucleotide comprises a barcode sequence. In some cases, attaching comprises contacting at least the first segment and the second segment to multiple bridge oligonucleotides in series. In some cases, the attaching results in cells, nuclei, chromosomes, or nucleic acid molecules of the stabilized biological sample receiving a unique sequence of bridge oligonucleotides. In some cases, attaching comprises contacting at least the first segment and the second segment to a barcode. In some cases, the method does not comprise a shearing step. In some cases, the method further comprises: (e) obtaining at least some sequence on each side of the junction to generate a first read pair. In some cases, the method further comprises: (f) mapping the first read pair to a set of contigs; and (g) determining a path through the set of contigs that represents an order and/or orientation to a genome. In some cases, the method further comprises: (f) mapping the first read pair to a set of contigs; and (g) determining, from the set of contigs, a presence of a structural variant or loss of heterozygosity in the stabilized biological sample. In some cases, the method further comprises: (f) mapping the first read pair to a set of contigs; and (g) assigning a variant in the set of contigs to a phase. In some cases, the variant is a human leukocyte antigen (HLA) variant. In some cases, the variant is a killer-cell immunoglobulin-like receptor (KIR) variant. In some cases, the method further comprises: (f) mapping the first read pair to a set of contigs; (g) determining, from the set of contigs, a presence of a variant in the set of contigs; and (h) conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

In additional aspects, there are provided methods comprising: (a) obtaining a stabilized biological sample comprising a nucleic acid molecule complexed to at least one nucleic acid binding protein; (b) contacting the stabilized biological sample to a micrococcal nuclease (MNase) to cleave the nucleic acid molecule into a plurality of segments; and (c) attaching a first segment and a second segment of the plurality of segments at a junction. In some cases, methods herein further comprise (d) subjecting the plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, methods herein further comprise, prior to step (d), preparing a sequencing library from the plurality of segments. In some cases, methods herein further comprise subjecting the sequencing library to a size selection to obtain a size-selected library. In some cases, the size-selected library is between about 350 bp and 1000 bp in size. In some cases, the size selection is conducted with gel electrophoresis, capillary electrophoresis, size selection beads, or a gel filtration column. In some cases, the method further comprises analyzing the plurality of selected segments to obtain a QC value. In some cases, the QC value is a chromatin digest efficiency (CDE) based on the proportion of segments between 100 and 2500 bp in size prior to step (d). In some cases, the method further comprises selecting a sample for further analysis when the CDE value is at least 65%. In some cases, the QC value is a chromatin digest index (CDI) based on the ratio of a number of mononucleosome-sized segments to a number of dinucleosome-sized segments prior to step (d). In some cases, the method further comprises selecting a sample for further analysis when the CDI value is greater than -1.5 and less than 1. In some cases, the stabilized biological sample comprises a stabilized cell lysate. In some cases, the stabilized biological sample comprises a stabilized intact cell. In some cases, the stabilized biological sample comprises a stabilized intact nucleus. In some cases, step (b) is conducted prior to lysis of the intact cell or the intact nucleus. In some cases, methods herein further comprise, prior to step (c), lysing cells and/or nuclei in the stabilized biological sample. In some cases, the stabilized biological sample comprises fewer than 3,000,000 cells. In some cases, the stabilized biological sample comprises fewer than 1,000,000 cells. In some cases, the stabilized biological sample comprises fewer than 100,000 cells. In some cases, the stabilized biological sample comprises less than 10 µg DNA. In some cases, the stabilized biological sample comprises less than 1 µg DNA. In some cases, the stabilized biological sample is further treated with a DNase. In some cases, the DNase is DNase I. In some cases, the DNase is DNase II. In some cases, the DNase is selected from one or more of DNase I and DNase II. In some cases, the stabilized biological sample has been treated with a crosslinking agent. In some cases, the crosslinking agent is a chemical fixative. In some cases, the chemical fixative comprises formaldehyde. In some cases, the chemical fixative comprises psoralen. In some cases, the chemical fixative comprises disuccinimidyl glutarate (DSG). In some cases, the chemical fixative comprises ethylene glycol bis(succinimidyl succinate) (EGS). In some cases, the crosslinking agent is ultraviolet light. In some cases, the stabilized biological sample is a crosslinked paraffin-embedded tissue sample. In some cases, methods herein comprise contacting the plurality of selected segments to an antibody. In some cases, attaching comprises filling in sticky ends using biotin tagged nucleotides and ligating the blunt ends. In some cases, attaching comprises contacting at least the first segment and the second segment to at least one bridge oligonucleotide. In some cases, the bridge oligonucleotide comprises a barcode sequence. In some cases, attaching comprises contacting at least the first segment and the second segment to multiple bridge oligonucleotides in series. In some cases, the attaching results in cells, nuclei, chromosomes, or nucleic acid molecules of the stabilized biological sample receiving a unique sequence of bridge oligonucleotides. In some cases, attaching comprises contacting at least the first segment and the second segment to a barcode. In some cases, the method does not comprise a shearing step. In some cases, methods herein further comprise: (e) obtaining at least some sequence on each side of the junction to generate a first read pair. In some cases, methods herein further comprise: (f) mapping the first read pair to a set of contigs; and (g) determining a path through the set of contigs that represents an order and/or orientation to a genome. In some cases, methods herein further comprise: (f) mapping the first read pair to a set of contigs; and (g) determining, from the set of contigs, a presence of a structural variant or loss of heterozygosity in the stabilized biological sample. In some cases, methods herein further comprise: (f) mapping the first read pair to a set of contigs; and (g) assigning a variant in the set of contigs to a phase. In some cases, the variant is a human leukocyte antigen (HLA) variant. In some cases, the variant is a killer-cell immunoglobulin-like receptor (KIR) variant. In some cases, methods herein further comprise: (f) mapping the first read pair to a set of contigs; (g) determining, from the set of contigs, a presence of a variant in the set of contigs; and (h) conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample

### BRIEF DESCRIPTION OF THE DRAWINGS

The patent application file contains at least one drawing executed in color. Copies of this patent application with color drawing(s) will be provided by the Office upon request and payment of the necessary fee.

An understanding of the features and advantages of the present invention will be obtained by reference to the following detailed description that sets forth illustrative embodiments, in which the principles of the invention are utilized, and the accompanying drawings of which:
**FIG. 1A** and **FIG. 1B** illustrate insufficiently processed **(****FIG. 1A****)** and sufficiently processed **(****FIG. 1B****)** stabilized tissue sample.
**FIG. 2** illustrates quantification and fragment size analysis.
**FIG. 3** illustrates various components of an exemplary computer system according to various embodiments of the present disclosure.
**FIG. 4** is a block diagram illustrating the architecture of an exemplary computer system that can be used in connection with various embodiments of the present disclosure.
**FIG. 5** is a diagram illustrating an exemplary computer network that can be used in connection with various embodiments of the present disclosure.
**FIG. 6** is a block diagram illustrating the architecture of another exemplary computer system that can be used in connection with various embodiments of the present disclosure.
**FIG. 7** shows a graph of read pair separation distributions for DNase-C compared with MNase-C.
**FIG. 8** shows a graph of the cumulative distribution of linkage distance as computed for chromosome 1 for DNase-C compared with MNase-C.
**FIG. 9** shows a graph of relative read coverage around high occupancy CTCF binding sites for various times and conditions of MNase digestion.
**FIG. 10** shows a digest pattern of MNase treated samples and a calculated ratio of mono:di nucleosomes in each sample for various times and conditions of MNase digestion.
**FIG. 11** shows ChIP-seq and HiChIP results compared to peaks reported in the Encyclopedia of DNA Elements (ENCODE) from the University of California, Santa Cruz (UCSC) Genome Browser.
**FIG. 12** shows the relative read coverage around CTCF binding sites for the HiChIP samples.
**FIG. 13** shows contact maps for read pairs presented over graphs of read coverage showing pile-ups of reads associated with the targeted proteins (as shown in **FIG. 11**) and over a graph of gene annotations.
**FIG. 14** shows the same comparison of MNase HiChIP results to ENCODE peaks as in **FIG. 11****,** but for sample replicates on the same and subsequent days.
**FIG. 15** shows an exemplary workflow for improved proximity ligation using a bridge oligonucleotide to link each DNA segment according to various embodiments of the present disclosure.
**FIG. 16** shows an exemplary workflow using a splitting and pooling approach according to various embodiments of the present disclosure.
**FIG. 17** shows an exemplary workflow using a splitting and pooling approach according to various embodiments of the present disclosure.
**FIG. 18** shows an example of combinations of barcodes and a bridge resulting from the splitting and pooling approach according to various embodiments of the present disclosure.
**FIGS. 19A-19D** show steps of exemplary integrase activity. At **FIG. 19A****,** one sees phiC31 pro-phage DNA integrate into Streptomyces sp. At **FIG. 19B****,** one sees that the integrase binds to the phage attB sites and an attP sequence in the bacterial genome and triggers strand exchange. At **FIG. 19C****,** one sees integration resolve into two new sequences attL and attR that are three bases shorter. At **FIG. 19D** one sees that a circular linker is not needed for integration.
**FIG. 20** depicts attP delivery by adapter ligation to DNase digested chromatin as an example of delivery to internal exposed ends of an integration site.
**FIG. 21** indicates that sequence flanking the 33 base attB segment can be replaced, for example, using a partition-distinguishing segment.
**FIG. 22** indicates that integration of attB linear DNA causes intra aggregate ligation.
**FIGS. 23A-23D** depict library preparation approaches. At **FIG. 23A****,** one sees biotin integrated in the attB containing molecule, while the attP adapter carries phospho-thiolated nucleotides. At **FIG. 23B****,** one sees that streptavidin pull down is used to pull down only the biotin-containing molecules. At **FIG. 23C****,** one sees that attP-specific amplification is used to amplify only the integrated molecules. At **FIG. 23D****,** one sees that alternately or in combination, exonuclease activity is used to remove non-integrated molecules and non-adaptor-ligated nucleic acids.
**FIG. 24** depicts using integration for single nuclei tagging of proximity ligation events.
**FIG. 25** shows an exemplary schematic for recombinase (e.g., integrase) based proximity ligation, including (from top to bottom of figure) fragmentation (e.g., by enzymatic digestion, such as with DNase), end polishing and A-tailing, ligation of recombination sites (e.g., A-tail compatible attB sites), recombination with linkers (e.g., attP linkers), and cross-link reversal, resulting in proximity ligated nucleic acids from different regions of the genome.
**FIG. 26** shows exemplary nucleic acid sequences for recombinase based proximity ligation, including (from top to bottom of figure) exemplary EP overhang attB sites ligated to unrecombined gDNA, exemplary attB sites ligated to unrecombined gDNA, exemplary unrecombined biotin linker with attP sites, one end of linker recombined to attB site on gDNA, and both ends of linker recombined with attB site on gDNA.
**FIG. 27** shows exemplary design and nucleic acid sequence for an attB site with sequencing adapter sites (e.g., P7 and P5 sequencing Y-adapter), on its own (top) and ligated to unrecombined gDNA (bottom).

### DETAILED DESCRIPTION

In one aspect, provided herein are compositions, systems, and methods related to the determination of genomic sequence including long-range and structural genomic information, the determination of nucleic acid physical conformation in a cell, and for generating extremely long-range read pairs for nucleic acids with improved results over methods previously disclosed in the art. Methods herein can utilize techniques including, but not limited to, DNase digestion, micrococcal nuclease (MNase) digestion, recombinase treatment, size selection, QC controls, whole cell or whole nuclei nuclease digestion, single cell analysis, and low input requirements to achieve optimal results. Methods herein can also include utilizing an immunoglobulin binding protein, or fragment thereof, to target an oligonucleotide or an oligonucleotide and a nuclease to an antibody binding site in the nucleic acid sample. Also provided herein are improved methods of HiChIP, HiChIRP, and Methyl-HiC.

In another aspect, provided herein are embodiments related to nucleic acid conformation assessment, nucleic acid sequence analysis, or nucleic acid phase information determination, for single cells or pluralities or populations of cells.

In some cases, conformation-preserved or conformation-reconstructed nucleic acid samples can be fragmented and distributed in aliquots or partitions to which aliquot-distinguishing sequence segments can be added so that, upon analysis of a paired end library generated from the samples, paired ends can be assigned to a partition, or cell, of origin. Thus cell-specific variation in sequence and/or three-dimensional nucleic acid configuration can be determined.

### Nucleic Acid Conformation Assessment

Disclosed herein are compositions, systems and methods related to the determination of nucleic acid physical conformation in a cell, such as a single cell or a population of cells, distinguishable from a physical conformation of a second cell or population of cells. Through practice of the disclosure herein, nucleic acid molecules indicative of three-dimensional nucleic acid relative position can be generated and optionally provided with a tag (e.g., nucleic acid barcode) to discern a common cell or population of origin for a plurality of molecules.

Through practice of the disclosed methods herein, nucleic acids can be obtained so as to preserve all or at least some of their three-dimensional configuration in a cell. Exposed nucleic acid loops of such nucleic acids can be cleaved to expose internal segment ends that are randomly reattached to one another such that exposed ends in physical proximity are more likely to become attached to one another (proximity attachment). Accordingly, by determining which exposed ends become attached to one another, one may obtain data informative of the physical proximity of the end-adjacent nucleic acids in a native cell configuration.

Related approaches are disclosed in, for example US9434985B2 to Dekker et al. published September 6, 2016.

Through practice of the disclosed methods herein, paired-end library constituents can be further tagged or otherwise provided with sequence information indicative of cell of origin, such that conformational differences among individual cells of a population are readily discerned for a population of cells, or such that conformational differences between a first population of cells and a second population of cells are readily discerned, even when they are concurrently analyzed. Tags can comprise, for example, nucleic acid barcodes. In some cases, tags can comprise a junction between two nucleic acid segments that are not contiguous in the genome. Nucleic acid molecules can be generated such that when sequenced in full or in part, one often obtains at least some genomic sequence sufficient to map each genomic end to its genomic locus and further obtains a tagging or linking sequence sufficient to identify a precise or likely cell or cell population of origin. Accordingly, one obtains sequence information informative of two regions of a genome being in physical proximity to one another, while also obtaining information informative of the cell or cell population in which this physical conformation occurs, such that it can be assessed in the context of other physical conformation information co-occurring in that cell or cell population.

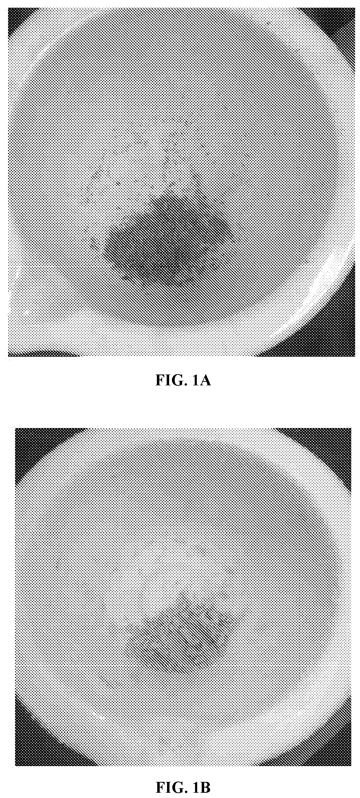

Genomic or other nucleic acids in cells can be stabilized and, for eukaryotic cells, nuclei are optionally isolated according to methods known in the art such as those referenced herein or otherwise known. For example, at **FIG. 1A** and **FIG. 1B** processed stabilized tissue samples are illustrated. **FIG. 1A** illustrates insufficiently processed tissue sample. **FIG. 1B** illustrates sufficiently processed stabilized tissue sample.

Nucleic acids consistent with the disclosure herein include any number of cellular nucleic acids, such as prokaryotic primary genome or plasmid nucleic acids, eukaryotic nuclear, mitochondrial or plastid nucleic acids, or in some cases cytoplasmic nucleic acids such as rRNA, mRNA, or exogenous nucleic acids in a sample such as viral or other pathogen or other exogenous nucleic acids of a sample.

Stabilized nucleic acids can be distributed in some cases such that at least some nucleic acids are distributed into individual partitions. Exemplary partitions include wells, droplets in an emulsion, or surface positions (e.g., array spots, beads, etc.) comprising distinct patches of differentially sequenced linker molecules as described elsewhere herein. Additional partitions known in the art or available to one of skill in the art are also contemplated and consistent with the methods, compositions, and systems disclosed herein.

Stabilized nucleic acids can be fragmented so as to expose internal breaks for later reconnection so as to obtain nucleic acid configuration information for a particular cell. A number of fragmentation approaches are known in the art and consistent with the disclosure herein. Nucleic acids can be fragmented using one or more populations of restriction endonucleases, programmable endonucleases such as CRISPR/Cas molecules coupled to guide RNA, non-specific endonucleases (e.g., DNase), tagmentation, shearing, sonication, heating, or other mechanism. In some cases, the DNase is non-sequence specific. In some cases, the DNase is active for both single-stranded DNA and double-stranded DNA. In some cases, the DNase is specific for double-stranded DNA. In some cases, the DNase is preferential to double-stranded DNA. In some cases, the DNase is specific for single-stranded DNA. In some cases, the DNase is preferential to single-stranded DNA. In some cases, the DNase is DNase I. In some cases, the DNase is DNase II. In some cases, the DNase is selected from one or more of DNase I and DNase II. In some cases, the DNase is micrococcal nuclease. In some cases, the DNase is selected from one or more of DNase I, DNase II, and micrococcal nuclease. Other suitable nucleases are also within the scope of this disclosure.

In particular, the disclosure of WO2014121091A1 to Green et al. published August 7, 2014 (later published as US20150363550A1 on December 17, 2015 and issued as US10089437B2 on October 2, 2018) is referenced. Similarly, the disclosure of WO2016019360A1 to Fields et al. published on February 4, 2016 (later published as US20170335369A1 on November 23, 2017) is referenced. Similarly, the disclosure of WO2017147279A1 to Green et al. published August 31, 2017 is referenced.

Nucleic acids can be bound to a surface prior to or after attachment. Exemplary surfaces include, but are not limited to, beads, arrays, and wells. In some cases, the surface is a solid phase reversible immobilization (SPRI) surface, such as a SPRI bead. Binding nucleic acids to a surface prior to attachment can improve performance of downstream steps, such as reducing inter-chromosomal ligations or attachments and increasing intra-chromosomal ligations or attachments.

Nucleic acids may be immunoprecipitated prior to or after attachment. Such methods can involve fragmenting chromatin and then contacting the fragments with an antibody that specifically recognizes and binds to acetylated histones, particularly H3. Examples of such antibodies include, but are not limited to, Anti Acetylated Histone H3, available from Upstate Biotechnology, Lake Placid, N.Y. The polynucleotides from the immunoprecipitate can subsequently be collected from the immunoprecipitate. Similar targeted enrichment methods also can be employed with target-specific compounds including but not limited to aptamers, oligonucleotides or other nucleic acid probes, and nucleic-acid guided nucleases (e.g., Cas-family enzymes such as Cas9, including catalytically-inactive or "dead" nucleases).

Linking nucleic acids, such as linking nucleic acids having barcodes, partition-specific sequences, or partition-identifying sequences, can be attached to exposed internal ends so as to generate nucleic acid segments having a left genomic segment, a linking region often having partition-specific or partition-identifying sequence (e.g., nucleic acid barcode), and a right genomic segment, wherein the left genomic segment and the right genomic segment map to genomic segments in physical proximity in the source cell.

Prior to attachment of exposed nucleic acid ends, the ends can be processed. Such processing can include end polishing or blunt ending. Blunt ended exposed nucleic acid ends can be ligated, for example directly to other blunt ended exposed nucleic acid ends, or to adapters or linkers. Such processing can include generating overhangs, for example, by tailing (e.g., A-tailing or adenylation). In one example, the overhang is one nucleotide in size. In one example, the overhang is a single A nucleotide. Tailed exposed nucleic acid ends can be ligated, for example, directly to other tailed exposed nucleic acid ends, or to adapters or linkers. In some cases, blunt ending or tailing can incorporate affinity tagged nucleic acids, such as biotinylated nucleic acids. Affinity tags can be used, for example, in downstream capture or enrichment steps. In other cases, blunt ending or tailing can be performed without incorporating affinity tagged nucleic acids (e.g., without biotinylated nucleic acids). Affinity tags, if desired, can be added subsequently, for example, in an adapter or a linker (e.g., a bridge). In one example, exposed nucleic acids are end polished, overhangs are generated, and exposed ends are attached via a bridge oligo.

Attachment can be direct, such as via ligation.

Attachment can be via a linker or bridge, such as by ligation of one or more linker or bridge nucleic acids connecting one exposed nucleic acid end to another.

Attachment can be through the use of capping nucleic acid adapter segments such as those consistent with recombinase incorporation, such as integrase or transposase incorporation. Adapters with recombinase sites can be added to exposed nucleic acid ends, and those ends can then be connected, for example, by recombination.

Taking phiC31 integrase barcode delivery as an example, linkers such as cell-identifying or cell-specific linkers (e.g., nucleic acid barcodes) can be enzymatically added as follows.

Subsequent to exposure of internal nucleic acid ends, integrase sites can be ligated to exposed nucleic acid ends such as internal ends or exposed linear chromosome ends, such as those from which telomeres have been removed. Exemplary integration sites are attP phiC31 integrase integration sites or nucleic acids comprising attP integration sites, although other integration sites are consistent with the disclosure herein. Ligation results in a population of nucleic acid fragments, at least some of which individually comprise a cellular nucleic acid segment bordered at each end by an integration site, such as a segment comprising an attP segment. In various embodiments, either one or both of fragmentation and integration site attachment occur prior to partitioning, or either one or both of fragmentation and integration site attachment occur subsequent to partitioning.

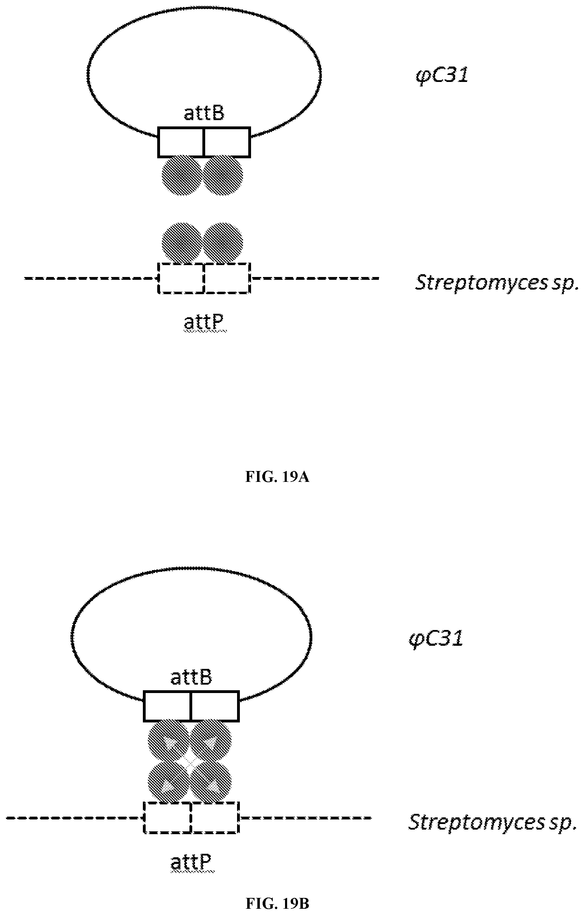

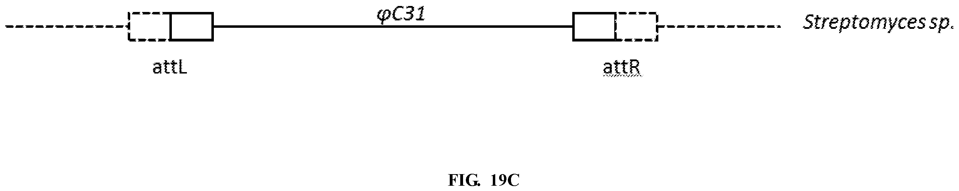

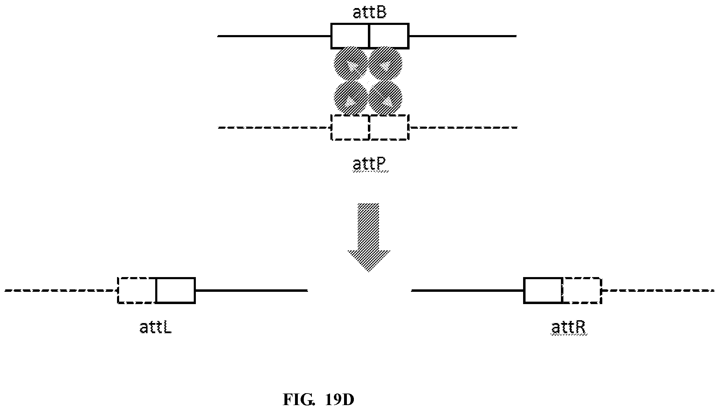

**FIG. 19A-D** show an exemplary schematic of a phiC31 integrase-based attachment approach. At **FIG. 19A****,** one sees a schematic of a phiC31 integration into Streptomyces via integrase. Nucleic acids comprising attP (denoted by a dashed line) and attB (denoted by a solid line) sites are indicated, although in various embodiments sites other than attB and attP, and enzymatic activities other than integrase, are also contemplated and consistent with the disclosure herein. At **FIG. 19B****,** one sees that integrase and associated proteins (denoted by circles) binds to the phage attB sites and an attP sequence in the bacterial genome and triggers strand exchange. At **FIG. 19C****,** one sees the result of the integration event. Integration resolves into a linear nucleic acid lacking attB and attP, but having an attL and attR that are chimeric fragments of portions of attB and attP. The attL and attR sites are 3 bp shorter and different in sequence compared to attB and attP. At **FIG. 19D****,** one sees that a circular integration or linker genome is not required. Integration of a linear DNA containing the attB site will cause cleavage of the attP containing DNA.

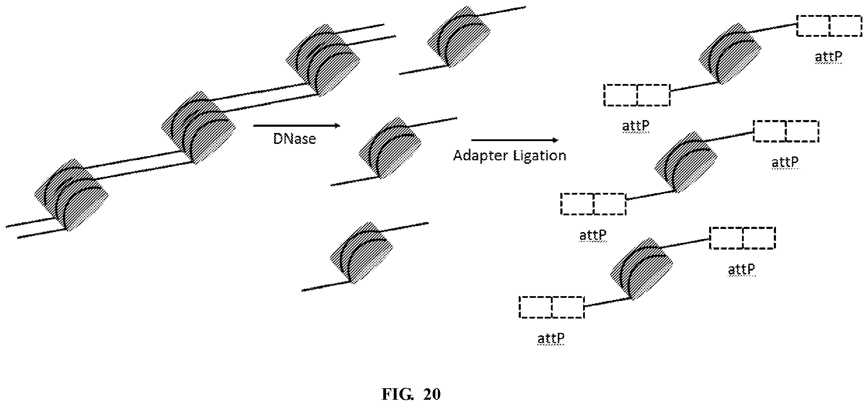

**FIG. 20** depicts delivery of integrase sites to exposed internal ends of stabilized nucleic acids as contemplated herein. For example, attP can be delivered by adapter ligation onto exposed internal ends of DNase digested chromatin (denoted by cylinders). Nucleic acids can be stabilized so as to preserve contact with binding moieties such as nucleosomes, so as in some cases to preserve phase information or three-dimensional physical location.

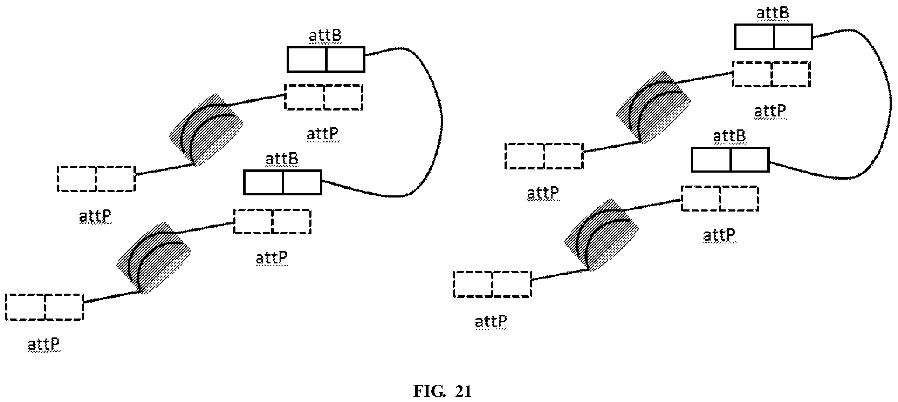

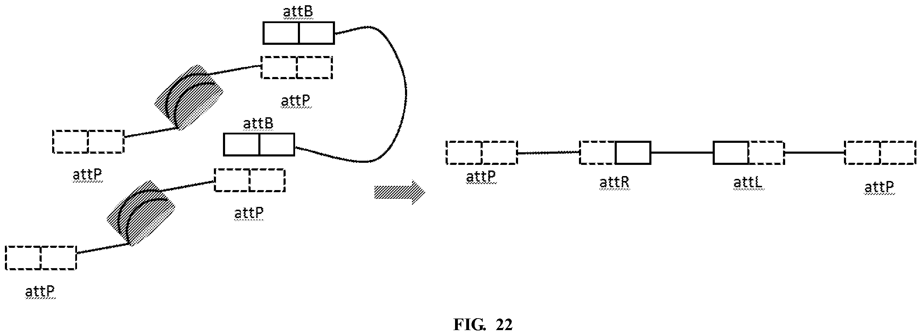

**FIG. 21** shows generation of linker constructs using integrase sites such as attB sites. For example, a minimal 33-nucleotide attB site is sufficient for integration. Flanking sequences can be replaced using sequences of choice, such as barcodes or other sequences that designate nucleic acids from a particular source (e.g., cell, droplet or other partition, organism). **FIG. 22** demonstrates intra-aggregate ligation via integration of attB linear DNA. The result is a linear molecule having exposed internal ends of nucleic acid segments that were in phase or in physical proximity being joined on a single library constituent. The library constituent is bounded by intact integration sites (attP, in this case), but the internal integration sites have been destroyed and replaced by attR and attL borders, such that attP related primers may amplify the library fragment. By obtaining internal end adjacent sequence and mapping it to a genome or contig set, one is able to assign the contigs or genome segments to a common phase or to a common three-dimensional location within a cell.

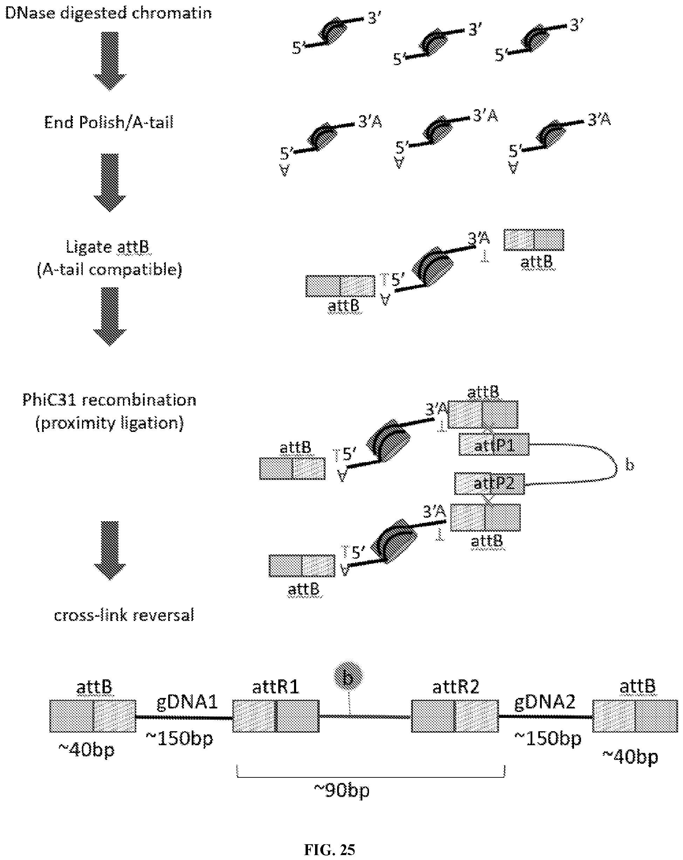

**FIG. 25** shows another example of a recombination-based proximity ligation protocol. Genomic DNA comprising cross-linked chromatin is digested, for example, with DNase. Exposed ends are polished and A-tailed, e.g., with a single A base overhang. A-tail compatible adapters comprising recombinase sites, such as attB sites, are ligated to the exposed ends. Linkers with corresponding recombinase sites, such as attP sites, are contacted to the sample and recombination is performed with a recombinase enzyme (e.g., phiC31) to achieve proximity ligation. Linkers optionally comprise affinity agents, such as biotin (b), to enable downstream pull-down or other purification or processing. Cross-linking is reversed and proximity ligated nucleic acids are recovered, for example comprising ~40 bp of attB site, followed by ~150 bp of genomic DNA region 1, followed by ~90 bp of linker sequence including attR sites and affinity agent, followed by ~150 bp of genomic DNA region 2, followed by ~40 bp of attB site.

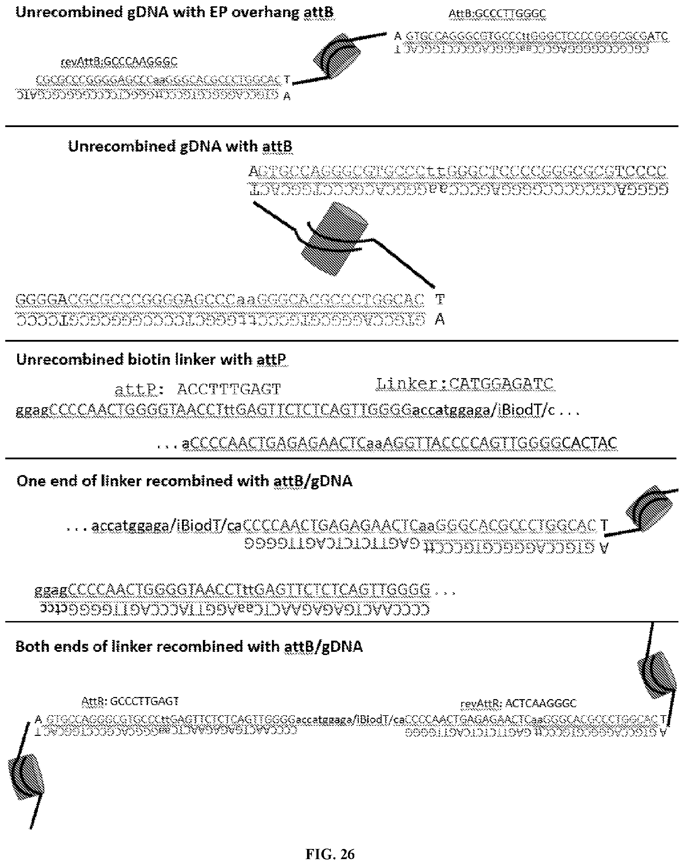

**FIG. 26** shows a similar protocol as that shown in **FIG. 25****,** with exemplary adapter and linker sequences. At top is shown unrecombined gDNA with EP overhang attB adapters, with sequence GTGCCAGGGCGTGCCCttGGGCTCCCCGGGCGCGATC comprising the attB site GCCCTTGGGC, with complement sequence CGCGCCCGGGGAGCCCaaGGGCACGCCCTGGCAC comprising the reverse attB site GCCCAAGGGC. Second from top is shown unrecombined gDNA with attB adapters, with sequence GTGCCAGGGCGTGCCCttGGGCTCCCCGGGCGCGTCCCC and complement sequence GGGGACGCGCCCGGGGAGCCCaaGGGCACGCCCTGGCAC. Third from top is shown an unrecombined linker comprising attP sites and biotin, with sequence ggagCCCCAACTGGGGTAACCTttGAGTTCTCTCAGTTGGGGaccatggaga/iBiodT/c aCCCCAACTGAGAGAACTCaaAGGTTACCCCAGTTGGGGCACTAC comprising attP sites with sequence ACCTTTGAGT and linker sequence CATGGAGATC. Fourth from top is shown one end of linker recombined with attB/gDNA, with sequence ggagCCCCAACTGGGGTAACCTttGAGTTCTCTCAGTTGGGG accatggaga/iBiodT/caCCCCAACTGAGAGAACTCaaGGGCACGCCCTGGCAC. At bottom is shown both ends of linker recombined with attB/gDNA, with sequence GTGCCAGGGCGTGCCCttGAGTTCTCTCAGTTGGGGaccatggaga/iBiodT/caCCCCAACTGAGAGA ACTCaaGGGCACGCCCTGGCAC, comprising attR site with sequence GCCCTTGAGT and reverse attR site with sequence ACTCAAGGGC.

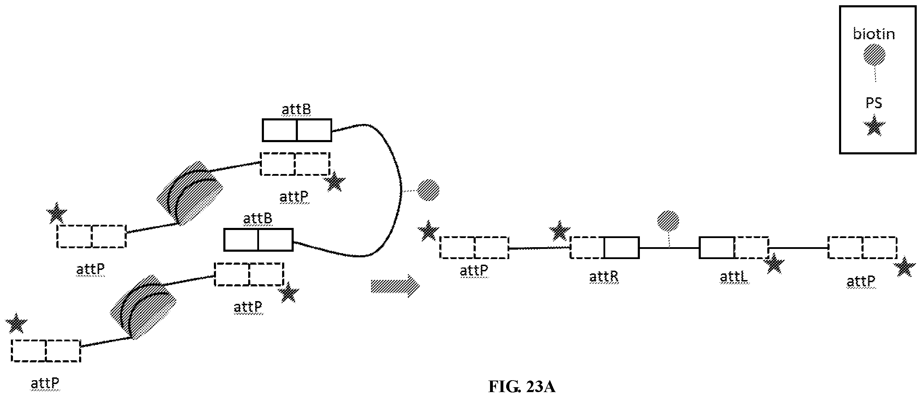

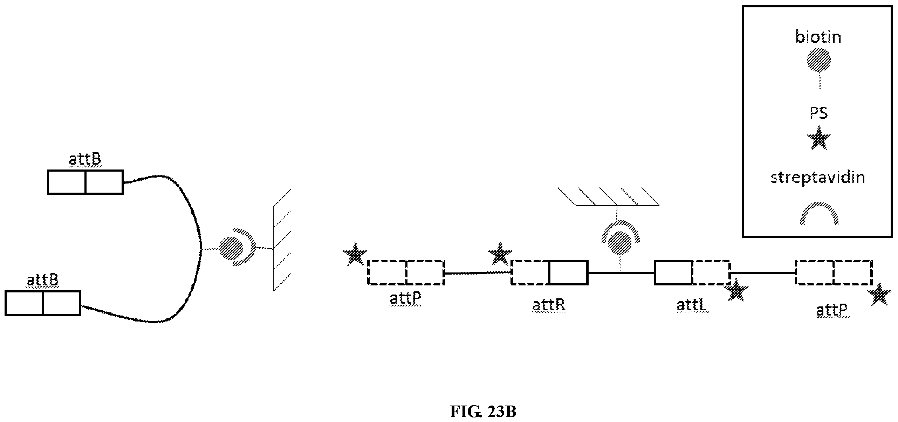

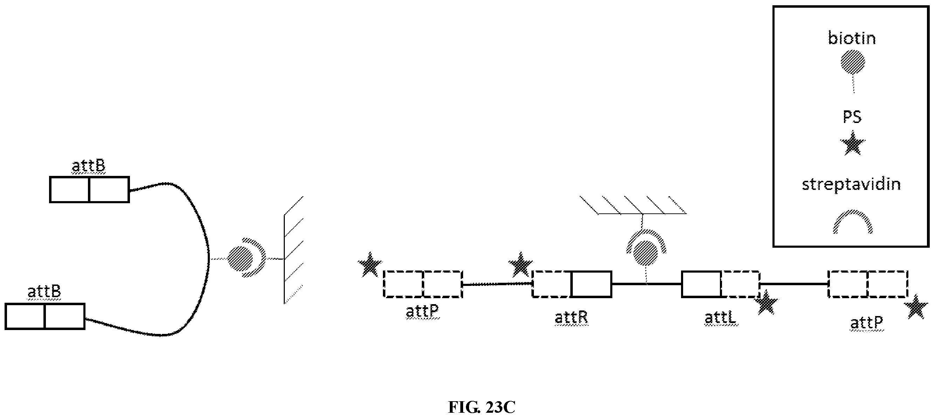

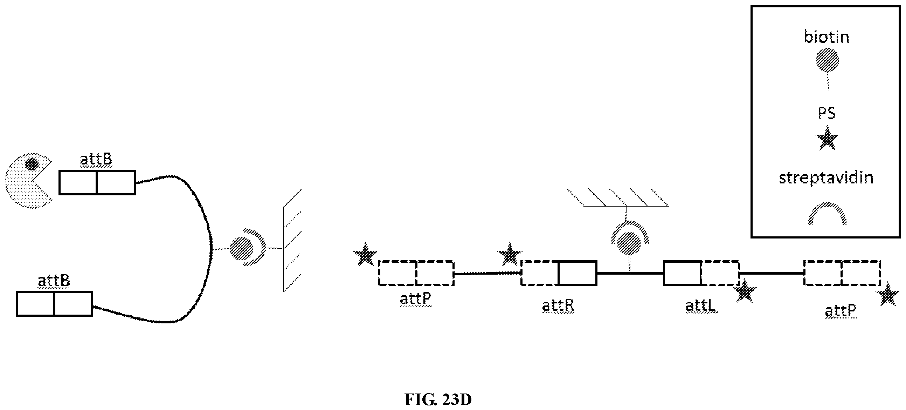

**FIG. 23A** shows exemplary linker molecule and adapter molecule modifications to facilitate library generation. A linker molecule is given an affinity tag (in this case biotin, denoted by a circle) while the adapters are furnished with an exonuclease resistant modification (in this case phosphothioation (PS), denoted by a star). The affinity tag facilitates isolation of linker molecules independent of whether they integrated into end-adjacent molecules. The exonuclease resistant modification on the linker facilitates the selective degradation of nucleic acid molecules to which a linker was not added and to linker molecules that did not integrate with end-adjacent nucleic acid sample molecules. **FIG. 23B** shows affinity purification (in this case streptavidin, denoted by a half-circle arc) of tagged molecules independent of whether they integrated into ligation sites added to internal ends. **FIG. 23C** indicates that attP-directed amplification is used to selectively amplify affinity-isolated molecules that preserve integrase sites such as attP sites (in this case by targeting such sites with primers). The presence of an affinity tag and attP sites indicates a molecule for which successful integration event occurred. **FIG. 23D** indicates an alternative whereby exonuclease (denoted by a circular sector or "Pac-Man") is used to clear affinity tagged molecules lacking exonuclease resistant modification (in this case, phosphothiolation). The presence of an affinity tag and exonuclease resistant sites indicates a molecule for which successful integration event occurred.

Alternatively, a transposase, such as Tn3, Tn5, Tn7, or sleeping beauty transposase can be used for barcode delivery. Subsequent to exposure of internal nucleic acid ends, mosaic ends can be ligated to exposed nucleic acid ends such as internal ends or exposed linear chromosome ends, such as those from which telomeres have been removed. Exemplary mosaic ends are Tn5 mosaic ends or nucleic acids comprising Tn5 mosaic ends, although other mosaic ends are consistent with the disclosure herein. Ligation results in a population of nucleic acid fragments, at least some of which individually comprise a cellular nucleic acid segment bordered at each end by a mosaic end, such as a Tn5 mosaic end.

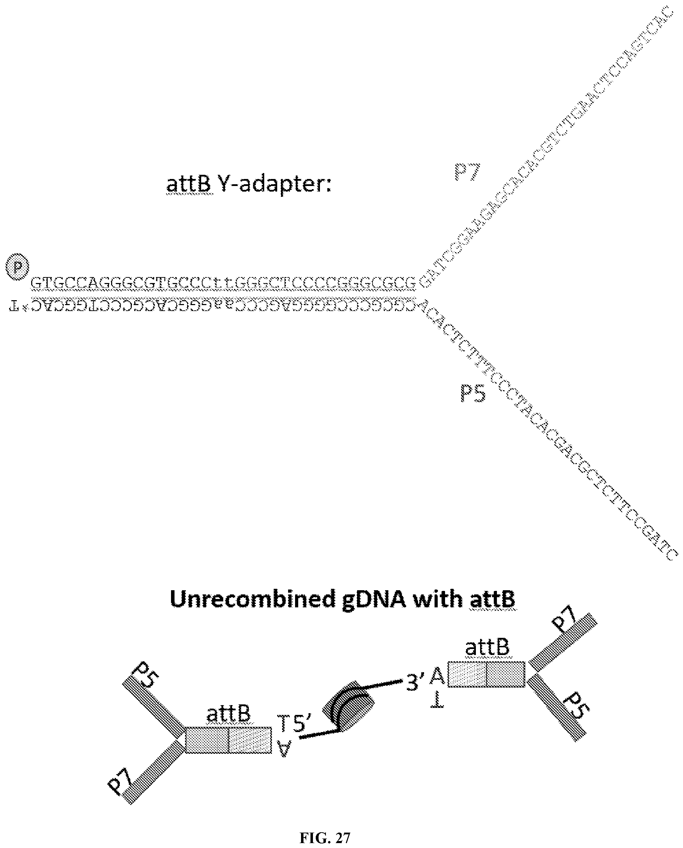

Recombinase adapter molecules can also comprise sequencing adapter sites, for example P5 and P7 sites. **FIG. 27** (top) shows an exemplary attB adapter with a sequencing Y-adapter, with attB adapter sequence GTGCCAGGGCGTGCCCttGGGCTCCCCGGGCGCG, P7 sequence GATCGGAAGAGCACACGTCTGAACTCCAGTCAC, and P5 sequence ACACTCTTTCCCTACACGACGCTCTTCCGATC. **FIG. 27** (bottom) shows a schematic of unrecombined gDNA with a recombinase adapter with sequencing adapter. The sequencing adapter is attached to the part of the attB site which will remain with the genomic DNA post-recombination, allowing sequencing post-recombination, including without further amplification or adapter ligation. Use of recombinase adapters that comprise sequencing adapters, including but not limited to that shown in **FIG. 27****,** can enable direct sequencing of proximity ligation products, without need for amplification or separate adapter incorporation steps. This can reduce biases such as amplification biases in the resulting sequence information.

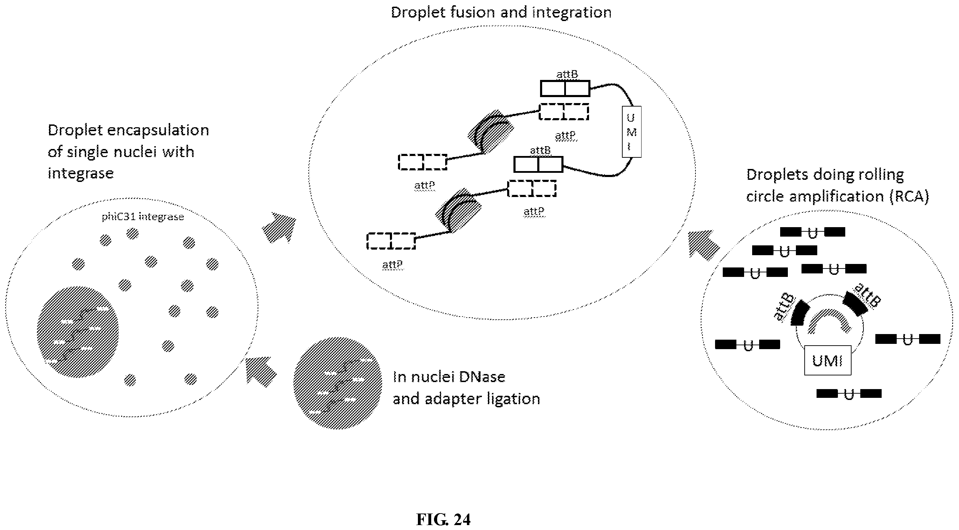

In various embodiments, either one or both of fragmentation and mosaic end attachment occur prior to partitioning, or either one or both of fragmentation and mosaic end attachment occur subsequent to partitioning. **FIG. 24** depicts an exemplary system for single-cell HiC (or other proximity ligation techniques) using integrase mediated intra-aggregate ligation. Single cell nuclei are encapsulated in a first set of partitions in combination with an integrase. The partitions are in this case, droplets in an emulsion. Nuclei are subjected to strand breakage so as to generate internal exposed ends and to preserve local three-dimensional information. Adapters are ligated onto exposed internal ends. The adapters optionally comprise exonuclease-resistant ends. In this embodiment, the adapters do not convey partition-distinguishing information. In a second set of partitions, linkers having partition distinguishing sequence such as unique molecular identifiers (UMIs) are encapsulated and optionally subjected to amplification and cleavage-directed linearization. The first and second sets of partitions are merged in an approximately 1:1 ratio, or under conditions such that nucleic acids from two cells are unlikely to be combined into a single resultant partition.

Recombinase sites, such as integrase sites or mosaic ends can be in some cases carried on unmodified single or double stranded fragments to be ligated onto internal nucleic acid ends. Alternately, so as to facilitate subsequent sequencing library clean-up, some single or double stranded fragments harboring integration sites such as attP sequences or mosaic ends such as Tn3, Tn5, Tn7, or sleeping beauty transposase mosaic ends can comprise at least one modification, such as a modification that interferes with exonuclease or other nucleic acid degrading activity. Examples include thiosulphate modification so as to preclude exonuclease degradation of fragments to which a double stranded fragment harboring an integration site has been added to each end.

Often, recombinase sites, such as integration sites or mosaic ends are nonspecific, in that the sequence in such integration sites or mosaic ends, such as attP sequence or Tn3, Tn5, Tn7, or sleeping beauty transposase mosaic end, is not used to designate a cell source of the adjacent nucleic acid. Alternately, often subsequent to nucleic acid partitioning, partitions can be provided with adapters having distinct, specific or cell-distinguishing sequence (e.g., nucleic acid barcode) adjacent to integration sites or mosaic ends, or can be provided with distinct integration sites or mosaic ends, such that nucleic acids of a first partition receive integration segments or mosaic ends having a first identifying segment while nucleic acid segments of a second partition receive integration segments having a second identifying segment.

Fragments having recombinase borders, such as borders comprising integrase attP segments, can be then contacted to integration sites, such as attB phiC31 integration sites, in a common solution. In an example, the integration enzyme can comprise a phi31 integrase, integration borders can comprise attP segments, and integration sites can comprise attB integration sites. Alternatively, fragments have mosaic end borders, such as Tn3, Tn5, Tn7, or sleeping beauty transposase mosaic end borders.

When recombinase sites such as attB integration sites or Tn3, Tn5, Tn7, or sleeping beauty transposase mosaic ends flank a linking segment having a sequence that identifies a partition or cell, such as one that is specific to a segment or cell source (e.g., nucleic acid barcode), that sequence identifies the adjacent cellular nucleic acid as arising from a particular or a common cell source or partition, such that multiple exposed ends from a common cell joined by a common cell-distinguishing or partition distinguishing segment can be readily identified as arising from a common cell even if they are bulked with fragments of a second partition prior to or concurrent with sequence determination.

When cell-distinguishing sequence is delivered via a recombinase site-bordered fragment, the integration or transposition is preferably performed subsequent to partitioning. Nucleic acid contents of at least some partitions can be thereby distinguished by the cell-distinguishing sequence of its linkers, such that even after nucleic acids form multiple cell sources are bulked for sequencing, one is able to assign internal end pairs, and the proximity information assigned to the vicinity to which they map in a contig set up to and including a largely or completely sequenced genome, to a common cell distinguished from at least one other cell of a sample, such that differences in predicted nucleic acid three dimensional conformation can be established.

A recombination site-bordered fragment variously comprises a left border fragment and a right border fragment (attB sites or Tn3, Tn5, Tn7, or sleeping beauty transposase mosaic ends, for example) linked by a linker region optionally comprising cell or partition designating sequence (e.g., nucleic acid barcode). The linker region optionally further comprises a moiety to facilitate subsequent isolation. A number of affinity tags or modified bases are consistent with the disclosure herein. Exemplary moieties facilitate physical or chemical isolation of linkers subsequent to integrase or transposase treatment. Any number of affinity tags known to one of skill in the art are consistent with the disclosure herein, such as one or a plurality of biotin tags that may facilitate avidin- or streptavidin-based isolation. Alternately, any antigen, receptor or ligand that facilitates isolation without interfering with integrase or transposase activity is suitable for some embodiments herein.

As mentioned above, some library generation approaches comprise a clean-up step, such as a step to selectively remove unincorporated reagents. Exonuclease treatment, for example, is often used to selectively remove unattached linker molecules, genomic fragments to which no integration site has been attached, or both unattached linker molecules and genomic fragments to which no integration site has been attached. A genomic fragment ligated to an integration site fragment having an exonuclease resistant modification such as a thiosulphate backbone is resistant to exonuclease degradation from that end, and a nucleic acid molecule bounded on both ends by an integration site fragment having an exonuclease resistant modification such as a thiosulphate backbone is resistant to degradation at both ends and can survive exonuclease treatment.

Alternately or in combination, some linker molecules comprise a counter-affinity tag on an opposite side of a recombination site such as an attP integration site or a Tn3, Tn5, Tn7, or sleeping beauty transposase mosaic end, such that the counter-affinity tag is removed pursuant to a successful recombination reaction. In such cases, unwanted reagents can be removed by contacting to a binding partner of the counter-affinity tag.

Integrase activity partially destroys both integration sites, such as attB and attP sites, as part of the integration event. Accordingly, by designing primers to anneal to ligated adapter sites such as attP integration sites, alone or in combination with linker-based isolation, one may generate clonal amplicons spanning at least one linker such that cell or aliquot-distinguishing information and internal end adjacent information is amplified, in some cases facilitating sequencing or other downstream analysis.

Following library generation and optionally library clean-up, nucleic acids can be sequenced completely or partially, so as to obtain information sufficient for the cell-distinguished or cell-specific three-dimensional nucleic acid position assessment. As mentioned above, sequencing is preferably performed such that one obtains at least some genomic sequence sufficient to map each genomic end of a library constituent to its genomic locus and further obtains a linking sequence sufficient to identify a precise or likely cell of origin. Accordingly, one obtains sequence information informative of two regions of a genome being in physical proximity to one another, while also obtaining information informative of the cell in which this physical conformation occurs, such that it can be assessed in the context of other physical conformation information co-occurring in that cell. Often this information is obtained through paired-end sequencing rather than through full length sequencing, although both approaches and others are consistent with the disclosure herein.

The compositions and methods related to the determination of nucleic acid physical conformation in a cell such as a single cell distinguishable from a physical conformation on a second cell can be implemented on a number of systems consistent with the disclosure herein. Some systems comprise distribution of fixed cellular nucleic acid material into first droplets of an emulsion or in wells, e.g. on a well plate. These droplets further comprise recombinase sites, such as integrase sites or mosaic ends, optionally modified to be exonuclease resistant as described herein, as well as integrase or transposase enzymes and ligase enzymes. Separately, linker nucleic acid molecules can be configured for delivery to the first droplets of the emulsion. The linker nucleic acids can be optionally distributed into droplets of a second emulsion or second wells and optionally amplified, for example using rolling circle amplification, and processed to generate multiple copies of a given linker molecule per second emulsion droplet.

Second emulsion droplets and first emulsion droplets can be then merged pairwise so as to assemble integrase or transposase-ligates nucleic acid fragments with integrase or transposase compatible linkers, often exhibiting a uniform label per droplet. However, droplets having two or more identifiers per nucleic acid sample can be still capable of yielding meaningful data, particularly when data analysis indicates the presence of more than one type of tag in a droplet.

As an alternative to pairwise merger, in some cases integrase or transposase-compatible linkers can be delivered as colonies of solid particles in a reagent stream that is contacted to first emulsion droplet via droplet to stream merger, such as that described in US20170335369A1, published November 23, 2017. Linker nucleic acids can be optionally amplified on solid particles or in gels. First emulsion droplets can be merged to the stream and second emulsion droplets can be recovered by segmenting or partitioning the stream so that a desired proportion of nucleic acid clusters to linker particles, such as 1: 1 greater than 1:1 or less than 1:1 is obtained.

Alternately, some systems and methods comprise distribution of fixed cellular nucleic acid material into wells of a chip or plate, followed by delivery of linker nucleic acids into the partitions, either unamplified or amplified as discussed above.

Alternately, in some cases delivery of linker nucleic acids is not temporally separated from partitioning. Rather, linker nucleic acids or an enzymatic activity or factor necessary for enzymatic activity is sequestered until a particular treatment, such as heat, electromagnetic activation or other administration so as to temporally activate the enzymatic activity leading to covalent binding of the linker to the nucleic acid sample exposed ends, such as via the linker.

A number of integration enzymes are consistent with the disclosure herein. PhiC31 integrase, such as that commercially available by ThermoFisher, exhibits a number of benefits for the practice of the methods, operation of the systems and for use in the compositions herein. Some benefits of this integrase are as follows. It uses the small integration sites (attB / attP). The enzyme itself is a small single polypeptide. Integration is irreversible without use of a separate enzyme to excise integration events. Activity is high, and the enzyme is readily engineered to alter activity. Nonetheless, its use is not required to the exclusion of other enzymes, as a number of integration systems are consistent with the disclosure herein. Aspects of the present disclosure may be described with respect to PhiC31 integrase, though use of any compatible enzymes is contemplated.

A number of transposase enzymes are consistent with the disclosure herein. Tn5 transposase, such as that commercially available by Lucigen, exhibits a number of benefits for the practice of the methods, operation of the systems, and for use in the compositions herein. Some benefits of this transposase are as follows: Tn5 uses a 19 bp mosaic end recognition sequence, insertions have little bias and are stable, and Tn5 can be delivered to cells for in vivo transposition or isolated nucleic acids for in vitro reactions. Nonetheless, its use is not required to the exclusion of other enzymes, as a number of transposase systems, such as Tn3, Tn7, or sleeping beauty transposase are consistent with the disclosure herein. Aspects of the present disclosure may be described with respect to Tn3, Tn5, Tn7, or sleeping beauty transposase, though use of any compatible enzyme is contemplated.

Sequence information obtained from library constituents is assessed through a number of approaches, such as those known in the art in the context of Hi-C, Chicago^{®} *in vitro* proximity ligation, or other three-dimensional conformational analysis. Importantly, cell-specific read pair frequencies can be obtained, such that the frequency of end adjacent sequence mapping to particular regions of a genome or particular contig can be assessed on a cell-specific basis. That is, one is able to assess the cell-specific occurrence of a likely three-dimensional conformation. In some cases, one is also able to assess the cell-specific strength of signal, correlating to cell-specific distance in the three dimensional conformation, such that one is able to conclude that certain regions of a nucleic acid are in relatively close proximity in one cell relative to a second cell where they are in comparable but 'weaker' or more distant proximity, while in a third cell there is no signal indicative of proximity. That is, both qualitative and quantitative assessments of three-dimensional configuration are consistent with the disclosure herein. In some cases, the proximity of one region to a second region is assessed at least in part by counting the number of cluster constituents of a first cluster that co-occur in paired end reads with cluster constituents of a second cluster, particularly in library constituents sharing a common partition-distinguishing sequence such as a unique partition tag.

Configuration information need not be made through multiple occurrence of identical end-adjacent sequence in multiple library constituents. Rather, in some cases end adjacent sequence that maps to near a second end adjacent sequence mapping site (to a common 'cluster') can re-enforce three-dimensional conformation assessments when both members of the cluster map to non-identical regions of a second cluster on a second region of an nucleic acid reference such as a genome.

**In** some cases, the methods disclosed herein are used to label and/or associate polynucleotides or sequence segments thereof, and to utilize that data for various applications. In some cases, the disclosure provides methods that produce a highly contiguous and accurate human genomic assembly with less than about 10,000, about 20,000, about 50,000, about 100,000, about 200,000, about 500,000, about 1 million, about 2 million, about 5 million, about 10 million, about 20 million, about 30 million, about 40 million, about 50 million, about 60 million, about 70 million, about 80 million, about 90 million, about 100 million, about 200 million, about 300 million, about 400 million, about 500 million, about 600 million, about 700 million, about 800 million, about 900 million, or about 1 billion read pairs. In some cases, the disclosure provides methods that phase, or assign physical linkage information to, about 50%, 60%, 70%, 75%, 80%, 85%, 90%, 91%, 92%, 93%, 94%, 95%, 96%, 97%, 98%, 99% or more of heterozygous variants in a human genome with about 50%, 60%, 70%, 75%, 80%, 85%, 90%, 91%, 92%, 93%, 94%, 95%, 96%, 97%, 98%, 99%, or greater accuracy.

In some embodiments, the compositions and methods described herein allow for the investigation of meta-genomes, for example those found in the human gut. Accordingly, the partial or whole genomic sequences of some or all organisms that inhabit a given ecological environment can be investigated. Examples include random sequencing of all gut microbes, the microbes found on certain areas of skin, and the microbes that live in toxic waste sites. The composition of the microbe population in these environments can be determined using the compositions and methods described herein and as well as the aspects of interrelated biochemistries encoded by their respective genomes. The methods described herein can enable metagenomic studies from complex biological environments, for example, those that comprise more than 2, 3, 4, 5, 6, 7, 8, 9, 10, 12, 15, 20, 25, 30, 40, 50, 60, 70, 80, 90, 100, 125, 150, 175, 200, 250, 300, 400, 500, 600, 700, 800, 900, 1000, 5000, 10000 or more organisms and/or variants of organisms.

Accordingly, methods disclosed herein may be applied to intact human genomic DNA samples but may also be applied to a broad diversity of nucleic acid samples, such as reverse-transcribed RNA samples, circulating free DNA samples, cancer tissue samples, crime scene samples, archaeological samples, nonhuman genomic samples, or environmental samples such as environmental samples comprising genetic information from more than one organism, such as an organism that is not easily cultured under laboratory conditions.

High degrees of accuracy required by cancer genome sequencing can be achieved using the methods and systems described herein. Inaccurate reference genomes can make base-calling challenges when sequencing cancer genomes. Heterogeneous samples and small starting materials, for example a sample obtained by biopsy introduce additional challenges. Further, detection of large-scale structural variants and/or losses of heterozygosity is often crucial for cancer genome sequencing, as well as the ability to differentiate between somatic variants and errors in base-calling.

Systems and methods described herein may generate accurate long sequences from complex samples containing 2, 3, 4, 5, 6, 7, 8, 9, 10, 12, 15, 20 or more varying genomes. Mixed samples of normal, benign, and/or tumor origin may be analyzed, optionally without the need for a normal control. In some embodiments, starting samples as little as 100ng or even as little as hundreds of genome equivalents are utilized to generate accurate long sequences. Systems and methods described herein may allow for detection of large scale structural variants and rearrangements, Phased variant calls may be obtained over long sequences spanning about 1 kbp, about 2 kbp, about 5 kbp, about 10 kbp, about 20 kbp, about 50 kbp, about 100 kbp, about 200 kbp, about 500 kbp, about 1 Mbp, about 2 Mbp, about 5 Mbp, about 10 Mbp, about 20 Mbp, about 50 Mbp, or about 100 Mbp or more nucleotides. For example, phase variant call may be obtained over long sequences spanning about 1 Mbp or about 2 Mbp.

In certain aspects, the methods disclosed herein are used to assemble a plurality of contigs originating from a single DNA molecule. In some cases, the method comprises generating a plurality of read-pairs from the single DNA molecule that is cross-linked to a plurality of nanoparticles and assembling the contigs using the read-pairs. In certain cases, single DNA molecule is cross-linked outside of a cell. In some cases, at least 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, or 50% of the read-pairs span a distance greater than 1kB, 2kB, 3kB, 4kB, 5kB, 6kB, 7kB, 8kB, 9kB, 10kB, 15kB, 20kB, 30kB, 40kB, 50kB, 60kB, 70kB, 80kB, 90kB, 100kB, 150kB, 200kB, 250kB, 300kB, 400kB, 500kB, 600kB, 700kB, 800kB, 900kB, or 1MB on the single DNA molecule. In certain cases, at least 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, or 20% of the read-pairs span a distance greater than 5kB, 6kB, 7kB, 8kB, 9kB, 10kB, 15kB, 20kB, 30kB, 40kB, 50kB, 60kB, 70kB, 80kB, 90kB, 100kB, 150kB, or 200kB on the single DNA molecule. In further cases, at least 0.5%, 0.6%, 0.7%, 0.8%, 0.9%, 1%, 2%, 3%, 4%, or 5% of the read-pairs span a distance greater than 20kB, 30kB, 40kB, 50kB, 60kB, 70kB, 80kB, 90kB, or 100kB on the single DNA molecule. In particular cases, at least 1% or 5% of the read pairs span a distance greater than 50kB or 100kB on the single DNA molecule. In some cases, the read-pairs are generated within 1, 2, 3, 4, 5, 6, 7, 8, 9, 10, 11, 12, 13, 14, 15, 16, 17, 18, 19, 20, 30, 40, 50 or 60 days. In certain cases, the read-pairs are generated within 3, 4, 5, 6, 7, 8, 9, 10, 11, 12, 13, 14, 15, 16, 17 or 18 days. In further cases, the read-pairs are generated within 7, 8, 9, 10, 11, 12, 13, or 14 days. In particular cases, the read-pairs are generated within 7 or 14 days.

Haplotypes determined using the methods and systems described herein may be assigned to computational resources, for example computational resources over a network, such as a cloud system. Short variant calls can be corrected, if necessary, using relevant information that is stored in the computational resources. Structural variants can be detected based on the combined information from short variant calls and the information stored in the computational resources. Problematic parts of the genome, such as segmental duplications, regions prone to structural variation, the highly variable and medically relevant MHC region, centromeric and telomeric regions, and other heterochromatic regions including those with repeat regions, low sequence accuracy, high variant rates, ALU repeats, segmental duplications, or any other relevant problematic parts known in the art, can be reassembled for increased accuracy.

A sample type can be assigned to the sequence information either locally or in a networked computational resource, such as a cloud. In cases where the source of the information is known, for example when the source of the information is from a cancer or normal tissue, the source can be assigned to the sample as part of a sample type. Other sample type examples generally include, but are not limited to, tissue type, sample collection method, presence of infection, type of infection, processing method, size of the sample, etc. In cases where a complete or partial comparison genome sequence is available, such as a normal genome in comparison to a cancer genome, the differences between the sample data and the comparison genome sequence can be determined and optionally output.

The methods of the present disclosure can be used in the analysis of genetic information of selective genomic regions of interest as well as genomic regions which may interact with the selective region of interest. Amplification methods as disclosed herein can be used in the devices, kits, and methods known to the art for genetic analysis, such as, but not limited to those found in U.S. Pat. Nos. 6,449,562, 6,287,766, 7,361,468, 7,414,117, 6,225,109, and 6,110,709. In some cases, amplification methods of the present disclosure can be used to amplify target nucleic acid for DNA hybridization studies to determine the presence or absence of polymorphisms. The polymorphisms, or alleles, can be associated with diseases or conditions such as genetic disease. In some other cases, the polymorphisms can be associated with susceptibility to diseases or conditions, for example, polymorphisms associated with addiction, degenerative and age related conditions, cancer, and the like. In other cases, the polymorphisms can be associated with beneficial traits such as increased coronary health, or resistance to diseases such as HIV or malaria, or resistance to degenerative diseases such as osteoporosis, Alzheimer's or dementia.

The compositions and methods of the disclosure can be used for diagnostic, prognostic, therapeutic, patient stratification, drug development, treatment selection, and screening purposes. The present disclosure provides the advantage that many different target molecules can be analyzed at one time from a single biomolecular sample using the methods of the disclosure. This allows, for example, for several diagnostic tests to be performed on one sample.

The methods provided herein can greatly advance the field of genomics by overcoming the substantial barriers posed by these repetitive regions and can thereby enable important advances in many domains of genomic analysis. To perform a *de novo* assembly with previous technologies, one must either settle for an assembly fragmented into many small scaffolds or commit substantial time and resources to producing a large-insert library or using other approaches to generate a more contiguous assembly. Such approaches may include acquiring very deep sequencing coverage, constructing BAC or fosmid libraries, optical mapping, or, most likely, some combination of these and other techniques. The intense resource and time requirements put such approaches out of reach for most small labs and prevents studying non-model organisms. Since the methods described herein can produce very long-range read-sets, *de novo* assembly may be achieved with a single sequencing run. This cuts assembly costs by orders of magnitude and shorten the time required from months or years to weeks. In some cases, the methods disclosed herein allow for generating a plurality of read-sets in less than 14 days, less than 13 days, less than 12 days, less than 11 days, less than 10 days, less than 9 days, less than 8 days, less than 7 days, less than 6 days, less than 5 days, less than 4 days, less than 3 days, less than 2 days, less than 1 day or in a range between any two of foregoing specified time periods. In some cases, the methods allow for generating a plurality of read-sets in about 10 days to 14 days. Building genomes for even the most niche of organisms would become routine, phylogenetic analyses would suffer no lack of comparisons, and projects such as Genome 10k could be realized.

The methods described herein allow for assignment of previously provided, previously generated, or de novo synthesized contig information into physical linkage groups such as chromosomes or shorter contiguous nucleic acid molecules. Similarly, the methods disclosed herein allow said contigs to be positioned relative to one another in linear order along a physical nucleic acid molecule. Similarly, the methods disclosed herein allow said contigs to be oriented relative to one another in linear order along a physical nucleic acid molecule.

Similarly, the methods disclosed herein can provide advances in structural and phasing analyses for medical purposes. There is astounding heterogeneity among cancers, individuals with the same type of cancer, or even within the same tumor. Teasing out the causative from consequential effects requires very high precision and throughput at a low per-sample cost. In the domain of personalized medicine, one of the gold standards of genomic care is a sequenced genome with all variants thoroughly characterized and phased, including large and small structural rearrangements and novel mutations. To achieve this with previous technologies demands effort akin to that required for a *de novo* assembly, which is currently too expensive and laborious to be a routine medical procedure. In some cases, the methods disclosed herein rapidly produce complete, accurate genomes at low cost and thereby yield many highly sought capabilities in the study and treatment of human disease.

Further, applying the methods disclosed herein to phasing can combine the convenience of statistical approaches with the accuracy of familial analysis, providing savings - money, labor, and samples - greater than those using either method alone. De novo variant phasing, a highly desirable phasing analysis that is prohibitive with previous technologies, can be performed readily using the methods disclosed herein. This is particularly important as the vast majority of human variation is rare (less than 5% minor allele frequency). Phasing information is valuable for population genetic studies that gain significant advantages from networks of highly connected haplotypes (collections of variants assigned to a single chromosome), relative to unlinked genotypes. Haplotype information may enable higher resolution studies of historical changes in population size, migrations, and exchange between subpopulations, and allows us to trace specific variants back to particular parents and grandparents. This in turn clarifies the genetic transmission of variants associated with disease, and the interplay between variants when brought together in a single individual. In further cases, the methods of the disclosure enable the preparation, sequencing, and analysis of extremely long range read-set (XLRS) or extremely long range read-pair (XLRP) libraries.

In some embodiments of the disclosure, a tissue or a DNA sample from a subject is provided and the method returns an assembled genome, alignments with called variants (including large structural variants), phased variant calls, or any additional analyses. In other embodiments, the methods disclosed herein provide XLRP libraries directly for the individual.

In various embodiments, the methods disclosed herein generate extremely long-range read pairs separated by large distances. The upper limit of this distance may be improved by the ability to collect DNA samples of large size. In some cases, the read pairs span up to 50, 60, 70, 80, 90, 100, 125, 150, 175, 200, 225, 250, 300, 400, 500, 600, 700, 800, 900, 1000, 1500, 2000, 2500, 3000, 4000, 5000 kbp or more in genomic distance. In some cases, the read pairs span up to 500 kbp in genomic distance. In other cases, the read pairs span up to 2000 kbp in genomic distance. The methods disclosed herein can integrate and build upon standard techniques in molecular biology, and are further well-suited for increases in efficiency, specificity, and genomic coverage. In some cases, the read pairs are generated in less than about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 60, or 90 days. In some cases, the read pairs are generated in less than about 14 days. In further cases, the read pairs are generated in less about 10 days. In some cases, the methods of the present disclosure provide greater than about 5%, about 10%, about 15 %, about 20%, about 30%, about 40%, about 50%, about 60%, about 70%, about 80%, about 90%, about 95%, about 99%, or about 100% of the read pairs with at least about 50%, about 60%, about 70%, about 80%, about 90%, about 95%, about 99%, or about 100% accuracy in correctly ordering and/or orientating the plurality of contigs. In some cases, the methods provide about 90 to 100% accuracy in correctly ordering and/or orientating the plurality of contigs.

In other embodiments, the methods disclosed herein are used with currently employed sequencing technology. In some cases, the methods are used in combination with well-tested and/or widely deployed sequencing instruments. In further embodiments, the methods disclosed herein are used with technologies and approaches derived from currently employed sequencing technology.

The methods disclosed herein can dramatically simplify de novo genomic assembly for a wide range of organisms. Using previous technologies, such assemblies are currently limited by the short inserts of economical mate-pair libraries. While it may be possible to generate read pairs at genomic distances up to the 40-50 kbp accessible with fosmids, these are expensive, cumbersome, and too short to span the longest repetitive stretches, including those within centromeres, which in humans range in size from 300 kbp to 5 Mbp. In some cases, the methods disclosed herein provide read pairs capable of spanning large distances (e.g., megabases or longer) and thereby overcome these scaffold integrity challenges. Accordingly, producing chromosome-level assemblies may be routine by utilizing the methods disclosed herein. Similarly, the acquisition of long-range phasing information can provide tremendous additional power to population genomic, phylogenetic, and disease studies. In certain cases, the methods disclosed herein enable accurate phasing for large numbers of individuals, thus extending the breadth and depth of our ability to probe genomes at the population and deep-time levels.

In the realm of personalized medicine, the XLRS read-sets generated from the methods disclosed herein represents a meaningful advance toward accurate, low-cost, phased, and rapidly produced personal genomes. Previous methods are insufficient in their ability to phase variants at long distances, thereby preventing the characterization of the phenotypic impact of compound heterozygous genotypes. Additionally, structural variants of substantial interest for genomic diseases are difficult to accurately identify and characterize with previous techniques due to their large size in comparison to the reads and read inserts used to study them. Read-sets spanning tens of kilobases to megabases or longer can help alleviate this difficulty, thereby allowing for highly parallel and personalized analyses of structural variation.

Basic evolutionary and biomedical research can be driven by technological advances in high-throughput sequencing. It is now relatively inexpensive to generate massive quantities of DNA sequence data. However, it is difficult in theory and in practice to produce high-quality, highly contiguous genome sequences with previous technologies. Further, many organisms, including humans, are diploid, wherein each individual has two haploid copies of the genome. At sites of heterozygosity (e.g., where the allele given by the mother differs from the allele given by the father), it is difficult to know which sets of alleles came from which parent (known as haplotype phasing). This information can be critically important for performing a number of evolutionary and biomedical studies such as disease and trait association studies.

The present disclosure provides methods for genome assembly that combine technologies for DNA preparation with tagged sequence reads for high-throughput discovery of short, intermediate and long-term connections corresponding to sequence reads from a single physical nucleic acid molecule bound to a complex such as a chromatin complex within a given genome. The disclosure further provides methods using these connections to assist in genome assembly, for haplotype phasing, and/or for metagenomic studies. While the methods presented herein can be used to determine the assembly of a subject's genome, it should also be understood that in certain cases the methods presented herein are used to determine the assembly of portions of the subject's genome such as chromosomes, or the assembly of the subject's chromatin of varying lengths. It should also be understood that, in certain cases, the methods presented herein are used to determine or direct the assembly of non-chromosomal nucleic acid molecules. Indeed, any nucleic acid the sequencing of which is complicated by the presence of repetitive regions separating non-repetitive contigs may be facilitated using the methods disclosed herein.

In further cases, the methods disclosed herein allow for accurate and predictive results for genotype assembly, haplotype phasing, and metagenomics with small amounts of materials. In some cases, less than about 100 picograms (pg), about 200 pg, about 300 pg, about 400 pg, about 500 pg, about 600 pg, about 700 pg, about 800 pg, about 900 pg, about 1.0 nanograms (ng), about 2.0 ng, about 3.0 ng, about 4.0 ng, about 5.0 ng, about 6.0 ng, about 7.0 ng, about 8.0 ng, about 9.0 ng, about 10 ng, about 15 ng, about 20 ng, about 30 ng, about 40 ng, about 50 ng, about 60 ng, about 70 ng, about 80 ng, about 90 ng, about 100 ng, about 200 ng, about 300 ng, about 400 ng, about 500 ng, about 600 ng, about 700 ng, about 800 ng, about 900 ng, about 1.0 micrograms (µg), about 1.2 µg, about 1.4 µg, about 1.6 µg, about 1.8 µg, about 2.0 µg, about 2.5 µg, about 3.0 µg, about 3.5 µg, about 4.0 µg, about 4.5 µg, about 5.0 µg, about 6.0 µg, about 7.0 µg, about 8.0 µg, about 9.0 µg, about 10 µg, about 15 µg, about 20 µg, about 30 µg, about 40 µg, about 50 µg, about 60 µg, about 70 µg, about 80 µg, about 90 µg, about 100 µg, about 150 µg, about 200 µg, about 300 µg, about 400 µg, about 500 µg, about 600 µg, about 700 µg, about 800 µg, about 900 µg, or about 1000 µg of DNA is used with the methods disclosed herein. In some cases, the DNA used in the methods disclosed herein is extracted from less than about 10,000,000, about 5,000,000, about 4,000,000, about 3,000,000, about 2,000,000, about 1,000,000, about 500,000, about 200,000, about 100,000, about 50,000, about 20,000, about 10,000, about 5,000, about 2,000, about 1,000, about 500, about 200, about 100, about 50, about 20, or about 10 cells.

In diploid genomes, it often important to know which allelic variants are physically linked on the same chromosome rather than mapping to the homologous position on a chromosome pair. Mapping an allele or other sequence to a specific physical chromosome of a diploid chromosome pair is known as the haplotype phasing. Short reads from high-throughput sequence data rarely allow one to directly observe which allelic variants are linked, particularly, as is most often the case, if the allelic variants are separated by a greater distance than the longest single read. Computational inference of haplotype phasing can be unreliable at long distances. Methods disclosed herein allow for determining which allelic variants are physically linked using allelic variants on read pairs.

In various cases, the methods and compositions of the disclosure enable the haplotype phasing of diploid or polyploid genomes with regard to a plurality of allelic variants. Methods described herein thus provide for the determination of linked allelic variants based on variant information from labeled sequence segments and/or assembled contigs using the same. Cases of allelic variants include, but are not limited to, those that are known from the 1000genomes, UK10K, HapMap and other projects for discovering genetic variation among humans. In some cases, disease association to a specific gene are revealed more easily by having haplotype phasing data as demonstrated, for example, by the finding of unlinked, inactivating mutations in both copies of SH3TC2 leading to Charcot-Marie-Tooth neuropathy (Lupski JR, Reid JG, Gonzaga-Jauregui C, et al. N. Engl. J. Med. 362:1181-91, 2010) and unlinked, inactivating mutations in both copies of ABCG5 leading to hypercholesterolemia 9 (Rios J, Stein E, Shendure J, et al. Hum. Mol. Genet. 19:4313-18, 2010).

Humans are heterozygous at an average of 1 site in 1,000. In some cases, a single lane of data using high throughput sequencing methods generates at least about 150,000,000 reads. In further cases, individual reads are about 100 base pairs long. If we assume input DNA fragments average 150 kbp in size and we get 100 paired-end reads per fragment, then we expect to observe 30 heterozygous sites per set, i.e., per 100 read-pairs. Every read-pair containing a heterozygous site within a set is in phase (i.e., molecularly linked) with respect to all other read-pairs within the same set. This property enables greater power for phasing with sets as opposed to singular pairs of reads in some cases. With approximately 3 billion bases in the human genome, and one in one-thousand being heterozygous, there are approximately 3 million heterozygous sites in an average human genome. With about 45,000,000 read pairs that contain heterozygous sites, the average coverage of each heterozygous site to be phased using a single lane of a high throughput sequence method is about (15X), using a typical high throughput sequencing machine. A diploid human genome can therefore be reliably and completely phased with one lane of a high-throughput sequence data relating sequence variants from a sample that is prepared using the methods disclosed herein. In some cases, a lane of data is a set of DNA sequence read data. In further cases, a lane of data is a set of DNA sequence read data from a single run of a high throughput sequencing instrument.

As the human genome consists of two homologous sets of chromosomes, understanding the true genetic makeup of an individual requires delineation of the maternal and paternal copies or haplotypes of the genetic material. Obtaining a haplotype in an individual is useful in several ways. For example, haplotypes are useful clinically in predicting outcomes for donor-host matching in organ transplantation. Haplotypes are increasingly used to detect disease associations. In genes that show compound heterozygosity, haplotypes provide information as to whether two deleterious variants are located on the same allele (that is, 'in cis', to use genetics terminology) or on two different alleles ('in trans'), greatly affecting the prediction of whether inheritance of these variants is harmful, and impacting conclusions as to whether an individual carries a functional allele and a single nonfunctional allele having two deleterious variant positions, or whether that individual carries two nonfunctional alleles, each with a different defect. Haplotypes from groups of individuals have provided information on population structure of interest to both epidemiologists and anthropologists and informative of the evolutionary history of the human race. In addition, widespread allelic imbalances in gene expression have been reported, and suggest that genetic or epigenetic differences between allele phase may contribute to quantitative differences in expression. An understanding of haplotype structure will delineate the mechanisms of variants that contribute to allelic imbalances.

In certain embodiments, the methods disclosed herein comprise an in vitro technique to fix and capture associations among distant regions of a genome as needed for long-range linkage and phasing. In some cases, the method comprises constructing and sequencing one or more read-sets to deliver very genomically distant read pairs. In further cases, each read-set comprises two or more reads that are labeled by a common barcode, which may represent two or more sequence segments from a common polynucleotide. In some cases, the interactions primarily arise from the random associations within a single polynucleotide. In some cases, the genomic distance between sequence segments are inferred because sequence segments near to each other in a polynucleotide interact more often and with higher probability, while interactions between distant portions of the molecule are less frequent. Consequently, there is a systematic relationship between the number of pairs connecting two loci and their proximity on the input DNA.

In some aspects, the disclosure provides methods and compositions that produce data to achieve extremely high phasing accuracy. In comparison to previous methods, the methods described herein can phase a higher proportion of the variants. In some cases, phasing is achieved while maintaining high levels of accuracy. In further cases, this phase information is extended to longer ranges, for example greater than about 200 kbp, about 300 kbp, about 400 kbp, about 500 kbp, about 600 kbp, about 700 kbp, about 800 kbp, about 900 kbp, about 1 Mbp, about 2 Mbp, about 3 Mbp, about 4 Mbp, about 5 Mbp, or about 10 Mbp, or longer than about 10 Mbp, up to and including the entire length of a chromosome. In some embodiments, more than 90% of the heterozygous SNPs for a human sample is phased at an accuracy greater than 99% using less than about 250 million reads, e.g., by using only 1 lane of Illumina HiSeq data. In other cases, more than about 40%, 50%, 60%, 70%, 80%, 90%, 95% or 99% of the heterozygous SNPs for a human sample is phased at an accuracy greater than about 70%, 80%, 90%, 95%, or 99% using less than about 250 million or about 500 million reads, e.g., by using only 1 or 2 lanes of Illumina HiSeq data. In some cases, more than 95% or 99% of the heterozygous SNPs for a human sample are phased at an accuracy greater than about 95% or 99% using less about 250 million or about 500 million reads. In further cases, additional variants are captured by increasing the read length to about 200 bp, 250 bp, 300 bp, 350 bp, 400 bp, 450 bp, 500 bp, 600 bp, 800 bp, 1000 bp, 1500 bp, 2 kbp, 3 kbp, 4 kbp, 5 kbp, 10 kbp, 20 kbp, 50 kbp, or 100 kbp.

The composition and methods of the disclosure can be used in gene expression analysis. The methods described herein discriminate between nucleotide sequences. The difference between the target nucleotide sequences can be, for example, a single nucleic acid base difference, a nucleic acid deletion, a nucleic acid insertion, or rearrangement. Such sequence differences involving more than one base can also be detected. The process of the present disclosure is able to detect infectious diseases, genetic diseases, and cancer. It is also useful in environmental monitoring, forensics, and food science. Examples of genetic analyses that can be performed on nucleic acids include, e.g., SNP detection, STR detection, RNA expression analysis, promoter methylation, gene expression, virus detection, viral subtyping and drug resistance.

The present methods can be applied to the analysis of biomolecular samples obtained or derived from a patient so as to determine whether a diseased cell type is present in the sample, the stage of the disease, the prognosis for the patient, the ability to the patient to respond to a particular treatment, or the best treatment for the patient. The present methods can also be applied to identify biomarkers for a particular disease.

In some embodiments, the methods described herein are used in the diagnosis of a condition. As used herein the term "diagnose" or "diagnosis" of a condition may include predicting or diagnosing the condition, determining predisposition to the condition, monitoring treatment of the condition, diagnosing a therapeutic response of the disease, or prognosis of the condition, condition progression, or response to particular treatment of the condition. For example, a blood sample can be assayed according to any of the methods described herein to determine the presence and/or quantity of markers of a disease or malignant cell type in the sample, thereby diagnosing or staging a disease or a cancer.

In some embodiments, the methods and composition described herein are used for the diagnosis and prognosis of a condition.

Numerous immunologic, proliferative and malignant diseases and disorders are especially amenable to the methods described herein. Immunologic diseases and disorders include allergic diseases and disorders, disorders of immune function, and autoimmune diseases and conditions. Allergic diseases and disorders include but are not limited to allergic rhinitis, allergic conjunctivitis, allergic asthma, atopic eczema, atopic dermatitis, and food allergy. Immunodeficiencies include but are not limited to severe combined immunodeficiency (SCID), hypereosinophilic syndrome, chronic granulomatous disease, leukocyte adhesion deficiency I and II, hyper IgE syndrome, Chediak Higashi, neutrophilias, neutropenias, aplasias, Agammaglobulinemia, hyper-IgM syndromes, DiGeorge/Velocardial-facial syndromes and Interferon gamma-TH1 pathway defects. Autoimmune and immune dysregulation disorders include but are not limited to rheumatoid arthritis, diabetes, systemic lupus erythematosus, Graves' disease, Graves ophthalmopathy, Crohn's disease, multiple sclerosis, psoriasis, systemic sclerosis, goiter and struma lymphomatosa (Hashimoto's thyroiditis, lymphadenoid goiter), alopecia aerata, autoimmune myocarditis, lichen sclerosis, autoimmune uveitis, Addison's disease, atrophic gastritis, myasthenia gravis, idiopathic thrombocytopenic purpura, hemolytic anemia, primary biliary cirrhosis, Wegener's granulomatosis, polyarteritis nodosa, and inflammatory bowel disease, allograft rejection and tissue destructive from allergic reactions to infectious microorganisms or to environmental antigens.

Proliferative diseases and disorders that may be evaluated by the methods of the disclosure include, but are not limited to, hemangiomatosis in newborns; secondary progressive multiple sclerosis; chronic progressive myelodegenerative disease; neurofibromatosis; ganglioneuromatosis; keloid formation; Paget's Disease of the bone; fibrocystic disease (e.g., of the breast or uterus); sarcoidosis; Peronies and Duputren's fibrosis, cirrhosis, atherosclerosis and vascular restenosis.

Malignant diseases and disorders that may be evaluated by the methods of the disclosure include both hematologic malignancies and solid tumors.

Hematologic malignancies are especially amenable to the methods of the disclosure when the sample is a blood sample, because such malignancies involve changes in blood-borne cells. Such malignancies include non-Hodgkin's lymphoma, Hodgkin's lymphoma, non-B cell lymphomas, and other lymphomas, acute or chronic leukemias, polycythemias, thrombocythemias, multiple myeloma, myelodysplastic disorders, myeloproliferative disorders, myelofibroses, atypical immune lymphoproliferations and plasma cell disorders.

Plasma cell disorders that may be evaluated by the methods of the disclosure include multiple myeloma, amyloidosis and Waldenstrom's macroglobulinemia.

Examples of solid tumors include, but are not limited to, colon cancer, breast cancer, lung cancer, prostate cancer, brain tumors, central nervous system tumors, bladder tumors, melanomas, liver cancer, osteosarcoma and other bone cancers, testicular and ovarian carcinomas, head and neck tumors, and cervical neoplasms.

Genetic diseases can also be detected by the process of the present disclosure. This can be carried out by prenatal or post-natal screening for chromosomal and genetic aberrations or for genetic diseases. Examples of detectable genetic diseases include: 21 hydroxylase deficiency, cystic fibrosis, Fragile X Syndrome, Turner Syndrome, Duchenne Muscular Dystrophy, Down Syndrome or other trisomies, heart disease, single gene diseases, HLA typing, phenylketonuria, sickle cell anemia, Tay-Sachs Disease, thalassemia, Klinefelter Syndrome, Huntington Disease, autoimmune diseases, lipidosis, obesity defects, hemophilia, inborn errors of metabolism, and diabetes.

The methods described herein can be used to diagnose pathogen infections, for example infections by intracellular bacteria and viruses, by determining the presence and/or quantity of markers of bacterium or virus, respectively, in the sample.

A wide variety of infectious diseases can be detected by the process of the present disclosure. The infectious diseases can be caused by bacterial, viral, parasite, and fungal infectious agents. The resistance of various infectious agents to drugs can also be determined using the present disclosure.

Bacterial infectious agents which can be detected by the present disclosure include Escherichia coli, Salmonella, Shigella, Klebsiella, Pseudomonas, Listeria monocytogenes, Mycobacterium tuberculosis, Mycobacterium aviumintracellulare, Yersinia, Francisella, Pasteurella, Brucella, Clostridia, Bordetella pertussis, Bacteroides, Staphylococcus aureus, Streptococcus pneumonia, B-Hemolytic strep., Corynebacteria, Legionella, Mycoplasma, Ureaplasma, Chlamydia, Neisseria gonorrhea, Neisseria meningitides, Hemophilus influenza, Enterococcus faecalis, Proteus vulgaris, Proteus mirabilis, Helicobacter pylori, Treponema palladium, Borrelia burgdorferi, Borrelia recurrentis, Rickettsial pathogens, Nocardia, and Acitnomycetes.

Fungal infectious agents which can be detected by the present disclosure include Cryptococcus neoformans, Blastomyces dermatitidis, Histoplasma capsulatum, Coccidioides immitis, Paracoccidioides brasiliensis, Candida albicans, Aspergillus fumigautus, Phycomycetes (Rhizopus), Sporothrix schenckii, Chromomycosis, and Maduromycosis.

Viral infectious agents which can be detected by the present disclosure include human immunodeficiency virus, human T-cell lymphocytotrophic virus, hepatitis viruses (e.g., Hepatitis B Virus and Hepatitis C Virus), Epstein-Barr virus, cytomegalovirus, human papillomaviruses, orthomyxo viruses, paramyxo viruses, adenoviruses, corona viruses, rhabdo viruses, polio viruses, toga viruses, bunya viruses, arena viruses, rubella viruses, and reo viruses.

Parasitic agents which can be detected by the present disclosure include Plasmodium falciparum, Plasmodium malaria, Plasmodium vivax, Plasmodium ovale, Onchoverva volvulus, Leishmania, Trypanosoma spp., Schistosoma spp., Entamoeba histolytica, Cryptosporidum, Giardia spp., Trichimonas spp., Balatidium coli, Wuchereria bancrofti, Toxoplasma spp., Enterobius vermicularis, Ascaris lumbricoides, Trichuris trichiura, Dracunculus medinesis, trematodes, Diphyllobothrium latum, Taenia spp., Pneumocystis carinii, and Necator americanis.

The present disclosure is also useful for detection of drug resistance by infectious agents. For example, vancomycin-resistant Enterococcus faecium, methicillin-resistant Staphylococcus aureus, penicillin-resistant Streptococcus pneumoniae, multi-drug resistant Mycobacterium tuberculosis, and AZT-resistant human immunodeficiency virus can all be identified with the present disclosure.

Thus, the target molecules detected using the compositions and methods of the disclosure can be either patient markers (such as a cancer marker) or markers of infection with a foreign agent, such as bacterial or viral markers.

The compositions and methods of the disclosure can be used to identify and/or quantify a target molecule whose abundance is indicative of a biological state or disease condition, for example, blood markers that are upregulated or downregulated as a result of a disease state.

In some embodiments, the methods and compositions of the present disclosure can be used for cytokine expression. The low sensitivity of the methods described herein would be helpful for early detection of cytokines, e.g., as biomarkers of a condition, diagnosis or prognosis of a disease such as cancer, and the identification of subclinical conditions.

The different samples from which the target polynucleotides are derived can comprise multiple samples from the same individual, samples from different individuals, or combinations thereof. In some embodiments, a sample comprises a plurality of polynucleotides from a single individual. In some embodiments, a sample comprises a plurality of polynucleotides from two or more individuals. An individual is any organism or portion thereof from which target polynucleotides can be derived, non-limiting examples of which include plants, animals, fungi, protists, monerans, viruses, mitochondria, and chloroplasts. Sample polynucleotides can be isolated from a subject, such as a cell sample, tissue sample, or organ sample derived therefrom, including, for example, cultured cell lines, biopsy, blood sample, or fluid sample containing a cell. The subject may be an animal, including but not limited to, an animal such as a cow, a pig, a mouse, a rat, a chicken, a cat, a dog, etc., and is usually a mammal, such as a human. Samples can also be artificially derived, such as by chemical synthesis. In some embodiments, the samples comprise DNA. In some embodiments, the samples comprise genomic DNA. In some embodiments, the samples comprise mitochondrial DNA, chloroplast DNA, plasmid DNA, bacterial artificial chromosomes, yeast artificial chromosomes, oligonucleotide tags, or combinations thereof. In some embodiments, the samples comprise DNA generated by primer extension reactions using any suitable combination of primers and a DNA polymerase, including but not limited to polymerase chain reaction (PCR), reverse transcription, and combinations thereof. Where the template for the primer extension reaction is RNA, the product of reverse transcription is referred to as complementary DNA (cDNA). Primers useful in primer extension reactions can comprise sequences specific to one or more targets, random sequences, partially random sequences, and combinations thereof. Reaction conditions suitable for primer extension reactions are known in the art. In general, sample polynucleotides comprise any polynucleotide present in a sample, which may or may not include target polynucleotides.

In some embodiments, nucleic acid template molecules (e.g., DNA or RNA) are isolated from a biological sample containing a variety of other components, such as proteins, lipids and non-template nucleic acids. Nucleic acid template molecules can be obtained from any cellular material, obtained from an animal, plant, bacterium, fungus, or any other cellular organism. Biological samples for use in the present disclosure include viral particles or preparations. Nucleic acid template molecules can be obtained directly from an organism or from a biological sample obtained from an organism, e.g., from blood, urine, cerebrospinal fluid, seminal fluid, saliva, sputum, stool and tissue. Any tissue or body fluid specimen may be used as a source for nucleic acid for use in the disclosure. Nucleic acid template molecules can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA. A sample may also be isolated DNA from a non-cellular origin, e.g., amplified/isolated DNA from the freezer.

Methods for the extraction and purification of nucleic acids are well known in the art. For example, nucleic acids can be purified by organic extraction with phenol, phenol/chloroform/isoamyl alcohol, or similar formulations, including TRIzol and TriReagent. Other non-limiting examples of extraction techniques include: (1) organic extraction followed by ethanol precipitation, e.g., using a phenol/chloroform organic reagent (Ausubel *et al.,* 1993), with or without the use of an automated nucleic acid extractor, e.g., the Model 341 DNA Extractor available from Applied Biosystems (Foster City, Calif.); (2) stationary phase adsorption methods (U.S. Pat. No. 5,234,809; Walsh *et al.,* 1991); and (3) salt-induced nucleic acid precipitation methods (Miller *et al.,* (1988), such precipitation methods being typically referred to as "salting-out" methods. Another example of nucleic acid isolation and/or purification includes the use of magnetic particles to which nucleic acids can specifically or non-specifically bind, followed by isolation of the beads using a magnet, and washing and eluting the nucleic acids from the beads (see, e.g., U.S. Pat. No. 5,705,628). In some embodiments, the above isolation methods may be preceded by an enzyme digestion step to help eliminate unwanted protein from the sample, e.g., digestion with proteinase K, or other like proteases. See, e.g., U.S. Pat. No. 7,001,724. If desired, RNase inhibitors may be added to the lysis buffer. For certain cell or sample types, it may be desirable to add a protein denaturation/digestion step to the protocol. Purification methods may be directed to isolate DNA, RNA, or both. When both DNA and RNA are isolated together during or subsequent to an extraction procedure, further steps may be employed to purify one or both separately from the other. Sub-fractions of extracted nucleic acids can also be generated, for example, purification by size, sequence, or other physical or chemical characteristic. In addition to an initial nucleic isolation step, purification of nucleic acids can be performed after any step in the methods of the disclosure, such as to remove excess or unwanted reagents, reactants, or products.

Nucleic acid template molecules can be obtained as described in U.S. Patent Application Publication Number US2002/0190663 A1, published Oct. 9, 2003. Generally, nucleic acid can be extracted from a biological sample by a variety of techniques such as those described by Maniatis, et al., Molecular Cloning: A Laboratory Manual, Cold Spring Harbor, N.Y., pp. 280-281 (1982). In some cases, the nucleic acids can be first extract from the biological samples and then cross-linked *in vitro.* In some cases, native association proteins (e.g., histones) can be further removed from the nucleic acids.

In other embodiments, the disclosure can be easily applied to any high molecular weight double stranded DNA including, for example, DNA isolated from tissues, cell culture, bodily fluids, animal tissue, plant, bacteria, fungi, viruses, etc.

### Hi-C Methods Comprising Size Selection

Provided herein are methods comprising obtaining a stabilized biological sample comprising a nucleic acid molecule complexed to at least one nucleic acid binding protein; contacting the stabilized biological sample to a DNase to cleave the nucleic acid molecule into a plurality of segments; attaching a first segment and a second segment of the plurality of segments at a junction; and subjecting the plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the plurality of selected segments is between about 100 bp and about 600 bp, between about 100 bp and about 700 bp, between about 100 bp and about 800 bp, between about 100 bp and about 900 bp, between about 100 bp and about 1000 bp, between about 100 bp and about 1100 bp, between about 100 bp and about 1200 bp, between about 100 bp and about 1300 bp, between about 100 bp and about 1400 bp, between about 100 bp and about 1500 bp, between about 100 bp and about 1600 bp, between about 100 bp and about 1700 bp, between about 100 bp and about 1800 bp, between about 100 bp and about 1900 bp, between about 100 bp and about 2000 bp, between about 100 bp and about 2100 bp, between about 100 bp and about 2200 bp, between about 100 bp and about 2300 bp, between about 100 bp and about 2400 bp, or between about 100 bp and about 2500 bp.

In another aspect of methods involving a size selection step provided herein, methods further comprise, prior a size selection step, preparing a sequencing library from the plurality of segments. In some embodiments, the method further comprises subjecting the sequencing library to a size selection to obtain a size-selected library. In some cases, the size-selected library is between about 350 bp and about 1000 bp in size. In some cases, the size-selected library is between about 100 bp and about 2500 bp in size, for example, between about 100 bp and about 350 bp, between about 350 bp and about 500 bp, between about 500 bp and about 1000 bp, between about 1000 and about 1500 bp and about 2000 bp, between about 2000 bp and about 2500 bp, between about 350 bp and about 1000 bp, between about 350 bp and about 1500 bp, between about 350 bp and about 2000 bp, between about 350 bp and about 2500 bp, between about 500 bp and about 1500 bp, between about 500 bp and about 2000 bp, between about 500 bp and about 3500 bp, between about 1000 bp and about 1500 bp, between about 1000 bp and about 2000 bp, between about 1000 bp and about 2500 bp, between about 1500 bp and about 2000 bp, between about 1500 bp and about 2500 bp, or between about 2000 bp and about 2500 bp.

Size selection utilized in methods involving a size selection step provided herein can be conducted with gel electrophoresis, capillary electrophoresis, size selection beads, a gel filtration column, other suitable methods, or combinations thereof.

In another aspect, methods involving a size selection step provided herein can further comprise analyzing the plurality of selected segments to obtain a QC value. In some cases, a QC value is selected from a chromatin digest efficiency (CDE) and a chromatin digest index (CDI). A CDE is calculated as the proportion of segments having a desired length. For example, in some cases, the CDE is calculated as the proportion of segments between 100 and 2500 bp in size prior to size selection. In some cases, a sample is selected for further analysis when the CDE value is at least 65%. In some cases, a sample is selected for further analysis when the CDE value is at least about 50%, at least about 55%, at least about 60%, at least about 65%, at least about 70%, at least about 75%, at least about 80%, at least about 85%, at least about 90%, or at least about 95%. A CDI is calculated as a ratio of a number of mononucleosome-sized segments to a number of dinucleosome-sized segments prior to size selection. For example, a CDI may be calculated as a logarithm of the ratio of fragments having a size of 600-2500 bp versus fragments having a size of 100-600 bp. In some cases, a sample is selected for further analysis when the CDI value is greater than -1.5 and less than 1. In some cases, a sample is selected for further analysis when the CDI value is greater than about -2 and less than about 1.5, greater than about -1.9 and less than about 1.5, greater than about -1.8 and less than about 1.5, greater than about -1.7 and less than about 1.5, greater than about -1.6 and less than about 1.5, greater than about -1.5 and less than about 1.5, greater than about -1.4 and less than about 1.5, greater than about -1.3 and less than about 1.5, greater than about -1.2 and less than about 1.5, greater than about -1.1 and less than about 1.5, greater than about -2 and less than about 1.5, greater than about -1 and less than about 1.5, greater than about -0.9 and less than about 1.5, greater than about - 0.8 and less than about 1.5, greater than about -0.7 and less than about 1.5, greater than about -0.6 and less than about 1.5, greater than about -0.5 and less than about 1.5, greater than about -2 and less than about 1.4, greater than about -2 and less than about 1.3, greater than about -2 and less than about 1.2, greater than about -2 and less than about 1.1, greater than about -2 and less than about 1, greater than about -2 and less than about 0.9, greater than about -2 and less than about 0.8, greater than about -2 and less than about 0.7, greater than about -2 and less than about 0.6, or greater than about -2 and less than about 0.5.

In another aspect, stabilized biological samples used in methods involving a size selection step herein comprise biological material that has been treated with a stabilizing agent. In some cases, the stabilized biological sample comprises a stabilized cell lysate. Alternatively, the stabilized biological sample comprises a stabilized intact cell. Alternatively, the stabilized biological sample comprises a stabilized intact nucleus. In some cases, contacting the stabilized intact cell or intact nucleus sample to a DNase is conducted prior to lysis of the intact cell or the intact nucleus. In some cases, cells and/or nuclei are lysed prior to attaching a first segment and a second segment of a plurality of segments at a junction.

In another aspect, methods involving a size selection step herein are conducted on small samples containing few cells or small amounts of nucleic acid. For example, in some cases, the stabilized biological sample comprises fewer than 3,000,000 cells. In some cases, the stabilized biological sample comprises fewer than 2,000,000 cells. In some cases, the stabilized biological sample comprises fewer than 1,000,000 cells. In some cases, the stabilized biological sample comprises fewer than 500,000 cells. In some cases, the stabilized biological sample comprises fewer than 400,000 cells. In some cases, the stabilized biological sample comprises fewer than 300,000 cells. In some cases, the stabilized biological sample comprises fewer than 200,000 cells. In some cases, the stabilized biological sample comprises fewer than 100,000 cells. In some cases, the stabilized biological sample comprises less than 10 µg DNA. In some cases, the stabilized biological sample comprises less than 9 µg DNA. In some cases, the stabilized biological sample comprises less than 8 µg DNA. In some cases, the stabilized biological sample comprises less than 7 µg DNA. In some cases, the stabilized biological sample comprises less than 6 µg DNA. In some cases, the stabilized biological sample comprises less than 5 µg DNA. In some cases, the stabilized biological sample comprises less than 4 µg DNA. In some cases, the stabilized biological sample comprises less than 3 µg DNA. In some cases, the stabilized biological sample comprises less than 2 µg DNA. In some cases, the stabilized biological sample comprises less than 1 µg DNA. In some cases, the stabilized biological sample comprises less than 0.5 µg DNA.

In another aspect, methods involving a size selection step herein can be conducted on individual or single cells. For example, methods herein may be conducted on cells distributed into individual partitions. Exemplary partitions include, but are not limited to, wells, droplets in an emulsion, or surface positions (e.g., array spots, beads, etc.) comprising distinct patches of differentially sequenced linker molecules as described elsewhere herein. Additional partitions are also contemplated and consistent with the methods, compositions, and systems disclosed herein.

In additional aspects, stabilized biological samples used in methods involving a size selection step herein are treated with a nuclease, such as a DNase to create fragments of DNA. In some cases, the DNase is non-sequence specific. In some cases, the DNase is active for both single-stranded DNA and double-stranded DNA. In some cases, the DNase is specific for double-stranded DNA. In some cases, the DNase preferentially cleaves double-stranded DNA. In some cases, the DNase is specific for single-stranded DNA. In some cases, the DNase preferentially cleaves single-stranded DNA. In some cases, the DNase is DNase I. In some cases, the DNase is DNase II. In some cases, the DNase is selected from one or more of DNase I and DNase II. In some cases, the DNase is micrococcal nuclease. In some cases, the DNase is selected from one or more of DNase I, DNase II, and micrococcal nuclease. In some cases, the DNase may be coupled or fused to an immunoglobulin binding protein or a fragment thereof. The immunoglobulin binding protein may be, for example, a Protein A, a Protein G, a Protein A/G, or a Protein L. In some embodiments, the DNase may be coupled to a fusion protein including two or more immunoglobulin binding proteins and/or fragments thereof. Other suitable nucleases are also within the scope of this disclosure.

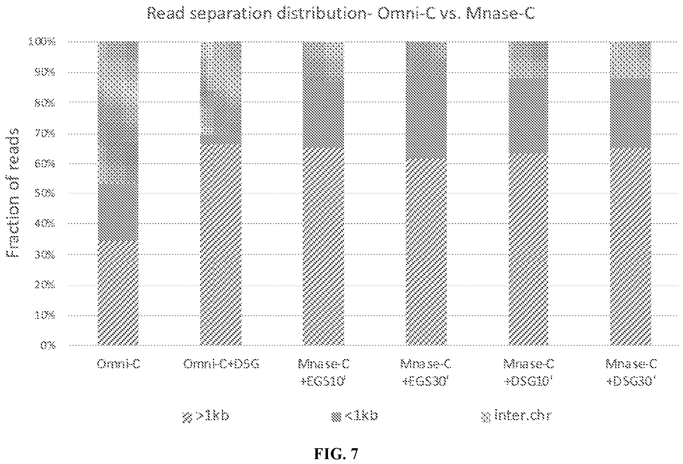

In additional aspects, stabilized biological samples as provided herein for use in methods involving a size selection step are treated with one or more crosslinking agents. In some cases, the crosslinking agent is a chemical fixative. In some cases, the chemical fixative comprises formaldehyde, which has a spacer arm length of about 2.3-2.7 angstrom (A). In some cases, the chemical fixative comprises a crosslinking agent with a long spacer arm length, For example, the crosslinking agent can have a spacer length of at least about 3 A, 4 A, 5 A, 6 A, 7 A, 8 A, 9 A, 10 A, 11 A, 12 A, 13 A, 14 A, 15 A, 16 A, 17 A, 18 A, 19 A, or 20 A. The chemical fixative can comprise ethylene glycol bis(succinimidyl succinate) (EGS), which has a spacer arm with length about 16.1 A. The chemical fixative can comprise disuccinimidyl glutarate (DSG), which has a spacer arm with length about 7.7 A. In some cases, the chemical fixative comprises formaldehyde and EGS, formaldehyde and DSG, or formaldehyde, EGS, and DSG. In some cases where multiple chemical fixatives are employed, each chemical fixative is used sequentially; in other cases, some or all of the multiple chemical fixatives are applied to the sample at the same time. The use of crosslinkers with long spacer arms can increase the fraction of read pairs with large (e.g., > 1 kb) read pair separation distances. For example, **FIG. 7** shows a comparison of resulting libraries (both DNase and MNase digested) crosslinked with formaldehyde alone versus crosslinked with formaldehyde plus DSG or EGS. DSG has NHS ester reactive groups at both ends and can be reactive towards amino groups (e.g., primary amines). DSG is membrane-permeable, allowing for intracellular crosslinking. DSG can increase crosslinking efficiency compared to disuccinimidyl suberate (DSS) in some applications. EGS has NHS ester reactive groups at both ends and can be reactive towards amino groups (e.g., primary amines). EGS is membrane-permeable, allowing for intracellular crosslinking. EGS crosslinks can be reversed, for example, by treatment with hydroxylamine for 3 to 6 hours at pH 8.5; in an example, lactose dehydrogenase retained 60% of its activity after reversible crosslinking with EGS. In some cases, the chemical fixative comprises psoralen. In some cases, the crosslinking agent is ultraviolet light. In some cases, the stabilized biological sample is a crosslinked paraffin-embedded tissue sample.

In further aspects, methods involving a size selection step provided herein comprise contacting the plurality of selected segments to an antibody.

In additional aspects, methods involving a size selection step provided herein comprise attaching a first segment and a second segment of a plurality of segments at a junction. In some cases, attaching comprises filling in sticky ends using biotin tagged nucleotides and ligating the blunt ends. In some cases, attaching comprises contacting at least the first segment and the second segment to a bridge oligonucleotide. In some cases, attaching comprises contacting at least the first segment and the second segment to a barcode. In some embodiments, bridge oligonucleotides herein can be from at least about 5 nucleotides in length to about 50 nucleotides in length. In some embodiments, bridge oligonucleotides herein can be from about 15 to about 18 nucleotides in length. In some embodiments, bridge oligonucleotides can be about 5, about 6, about 7, about 8, about 9, about 10, about 11, about 12, about 13, about 14, about 15, about 16, about 17, about 18, about 19, about 20, about 25, about 30, about 35, about 40, about 45, or about 50 nucleotides in length. In some embodiments, bridge oligonucleotides herein may comprise a barcode. In some embodiments, bridge oligonucleotides can comprise multiple barcodes. In some embodiments, bridge oligonucleotides comprise multiple bridge oligonucleotides connected together. In some embodiments, bridge oligonucleotides may be coupled or linked to an immunoglobulin binding protein or fragment thereof, such as a Protein A, a Protein G, a Protein A/G, or a Protein L. In some cases, coupled bridge oligonucleotides may be delivered to a location in the sample nucleic acid where an antibody is bound.

A splitting and pooling approach can be employed to produce bridge oligonucleotides with unique barcodes. A population of samples can be split into multiple groups, bridge oligonucleotides can be attached to the samples such that the bridge oligonucleotide barcodes are different between groups but the same within a group, the groups of samples can be pooled together again, and this process can be repeated multiple times. Iterating this process can ultimately result in each sample in the population having a unique series of bridge oligonucleotide barcodes, allowing single-sample (e.g., single cell, single nucleus, single chromosome) analysis. In one illustrative example, a sample of crosslinked digested nuclei attached to a solid support of beads is split across 8 tubes, each containing 1 of 8 unique members of a first adaptor group (first iteration) comprising double-stranded DNA (dsDNA) adaptors to be ligated. Each of the 8 adaptors can have the same 5' overhang sequence for ligation to the nucleic acid ends of the cross-linked chromatin aggregates in the nuclei, but otherwise has a unique dsDNA sequence. After the first adaptor group is ligated, the nuclei can be pooled back together and washed to remove the ligation reaction components. The scheme of distributing, ligating, and pooling can be repeated 2 additional times (2 iterations). Following ligation of members from each adaptor group, a cross-linked chromatin aggregate can be attached to multiple barcodes in series. In some cases, the sequential ligation of a plurality of members of a plurality of adaptor groups (iterations) results in barcode combinations. The number of barcode combinations available depends on the number of groups per iteration and the total number of barcode oligonucleotides used. For example, 3 iterations comprising 8 members each can have 83 possible combinations. In some cases, barcode combinations are unique. In some cases, barcode combinations are redundant. The total number of barcode combinations can be adjusted by increasing or decreasing the number of groups receiving unique barcodes and/or increasing or decreasing the number of iterations. When more than one adaptor group is used, a distributing, attaching, and pooling scheme can be used for iterative adaptor attachment. In some cases, the scheme of distributing, attaching, and pooling can be repeated at least 3, 4, 5, 6, 7, 8, 9, or 10 additional times. In some cases, the members of the last adaptor group include a sequence for subsequent enrichment of adaptor-attached DNA, for example, during sequencing library preparation through PCR amplification.

In additional aspects, methods involving a size selection step herein do not comprise a shearing step (e.g., the nucleic acid is not sheared).

In further aspects of methods involving a size selection step herein, methods comprise obtaining at least some sequence on each side of the junction to generate a first read pair. For example, the methods may comprise obtaining at least about 50 bp, at least about 100 bp, at least about 150 bp, at least about 200 bp, at least about 250 bp, or at least about 300 bp of sequence on each side of the junction to generate a first read pair.

In additional aspects of methods involving a size selection step herein, methods comprise mapping the first read pair to a set of contigs, and determining a path through the set of contigs that represents an order and/or orientation to a genome.

In further aspects of methods involving a size selection step herein, methods comprise mapping the first read pair to a set of contigs; and determining, from the set of contigs, a presence of a structural variant or loss of heterozygosity in the stabilized biological sample.

In additional aspects of methods involving a size selection step herein, methods comprise mapping the first read pair to a set of contigs, and assigning a variant in the set of contigs to a phase.

In further aspects of methods involving a size selection step herein, methods comprise mapping the first read pair to a set of contigs; determining, from the set of contigs, a presence of a variant in the set of contigs, and conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

### Hi-C Methods Comprising a QC Calculation

Additionally, provided herein are methods comprising obtaining a stabilized biological sample comprising a nucleic acid molecule complexed to at least one nucleic acid binding protein, contacting the stabilized biological sample to a DNase to cleave the nucleic acid molecule into a plurality of segments, attaching a first segment and a second segment of the plurality of segments at a junction, and analyzing the plurality of segments to determine a QC value. In some cases, a QC value is selected from a chromatin digest efficiency (CDE) and a chromatin digest index (CDI). A CDE is calculated as the proportion of segments having a desired length. For example, in some cases, the CDE is calculated as the proportion of segments between 100 and 2500 bp in size prior to size selection. In some cases, a sample is selected for further analysis when the CDE value is at least 65%. In some cases, a sample is selected for further analysis when the CDE value is at least about 50%, at least about 55%, at least about 60%, at least about 65%, at least about 70%, at least about 75%, at least about 80%, at least about 85%, at least about 90%, or at least about 95%. A CDI is calculated as a ratio of a number of mononucleosome-sized segments to a number of dinucleosome-sized segments prior to size selection. For example, a CDI may be calculated as a logarithm of the ratio of fragments having a size 600-2500 bp versus fragments having a size 100-600 bp. In some cases, a sample is selected for further analysis when the CDI value is greater than -1.5 and less than 1. In some cases, a sample is selected for further analysis when the CDI value is greater than about -2 and less than about 1.5, greater than about -1.9 and less than about 1.5, greater than about -1.8 and less than about 1.5, greater than about -1.7 and less than about 1.5, greater than about -1.6 and less than about 1.5, greater than about -1.5 and less than about 1.5, greater than about -1.4 and less than about 1.5, greater than about -1.3 and less than about 1.5, greater than about -1.2 and less than about 1.5, greater than about -1.1 and less than about 1.5, greater than about -2 and less than about 1.5, greater than about -1 and less than about 1.5, greater than about -0.9 and less than about 1.5, greater than about -0.8 and less than about 1.5, greater than about -0.7 and less than about 1.5, greater than about -0.6 and less than about 1.5, greater than about -0.5 and less than about 1.5, greater than about -2 and less than about 1.4, greater than about -2 and less than about 1.3, greater than about -2 and less than about 1.2, greater than about -2 and less than about 1.1, greater than about -2 and less than about 1, greater than about -2 and less than about 0.9, greater than about -2 and less than about 0.8, greater than about -2 and less than about 0.7, greater than about -2 and less than about 0.6, or greater than about -2 and less than about 0.5.

In another aspect, methods involving a QC determination step herein may comprise subjecting a plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the plurality of selected segments is between about 100 bp and about 600 bp, between about 100 bp and about 700 bp, between about 100 bp and about 800 bp, between about 100 bp and about 900 bp, between about 100 bp and about 1000 bp, between about 100 bp and about 1100 bp, between about 100 bp and about 1200 bp, between about 100 bp and about 1300 bp, between about 100 bp and about 1400 bp, between about 100 bp and about 1500 bp, between about 100 bp and about 1600 bp, between about 100 bp and about 1700 bp, between about 100 bp and about 1800 bp, between about 100 bp and about 1900 bp, between about 100 bp and about 2000 bp, between about 100 bp and about 2100 bp, between about 100 bp and about 2200 bp, between about 100 bp and about 2300 bp, between about 100 bp and about 2400 bp, or between about 100 bp and about 2500 bp.

In another aspect of methods involving a QC determination step provided herein, methods can further comprise, prior to a size selection step, preparing a sequencing library from the plurality of segments. In some embodiments, the method further comprises subjecting the sequencing library to a size selection to obtain a size-selected library. In some cases, the size-selected library is between about 350 bp and about 1000 bp in size. In some cases, the size-selected library is between about 100 bp and about 2500 bp in size, for example, between about 100 bp and about 350 bp, between about 350 bp and about 500 bp, between about 500 bp and about 1000 bp, between about 1000 and about 1500 bp and about 2000 bp, between about 2000 bp and about 2500 bp, between about 350 bp and about 1000 bp, between about 350 bp and about 1500 bp, between about 350 bp and about 2000 bp, between about 350 bp and about 2500 bp, between about 500 bp and about 1500 bp, between about 500 bp and about 2000 bp, between about 500 bp and about 3500 bp, between about 1000 bp and about 1500 bp, between about 1000 bp and about 2000 bp, between about 1000 bp and about 2500 bp, between about 1500 bp and about 2000 bp, between about 1500 bp and about 2500 bp, or between about 2000 bp and about 2500 bp.

Size selection utilized in methods involving a QC determination step herein may be conducted with gel electrophoresis, capillary electrophoresis, size selection beads, a gel filtration column, or combinations thereof. Other suitable methods of size selection are also within the scope of this disclosure.

In another aspect, stabilized biological samples used in involving a QC determination step herein comprise biological material that has been treated with a stabilizing agent. In some cases, the stabilized biological sample comprises a stabilized cell lysate. Alternatively, the stabilized biological sample comprises a stabilized intact cell. Alternatively, the stabilized biological sample comprises a stabilized intact nucleus. In some cases, contacting the stabilized intact cell or intact nucleus sample to a DNase is conducted prior to lysis of the intact cell or the intact nucleus. In some cases, cells and/or nuclei are lysed prior to attaching a first segment and a second segment of a plurality of segments at a junction.

In another aspect, methods involving a QC determination step herein are conducted on small samples containing few cells or small amounts of nucleic acid. In some cases, the stabilized biological sample comprises fewer than 3,000,000 cells. In some cases, the stabilized biological sample comprises fewer than 2,000,000 cells. In some cases, the stabilized biological sample comprises fewer than 1,000,000 cells. In some cases, the stabilized biological sample comprises fewer than 500,000 cells. In some cases, the stabilized biological sample comprises fewer than 400,000 cells. In some cases, the stabilized biological sample comprises fewer than 300,000 cells. In some cases, the stabilized biological sample comprises fewer than 200,000 cells. In some cases, the stabilized biological sample comprises fewer than 100,000 cells. In some cases, the stabilized biological sample comprises less than 10 µg DNA. In some cases, the stabilized biological sample comprises less than 9 µg DNA. In some cases, the stabilized biological sample comprises less than 8 µg DNA. In some cases, the stabilized biological sample comprises less than 7 µg DNA. In some cases, the stabilized biological sample comprises less than 6 µg DNA. In some cases, the stabilized biological sample comprises less than 5 µg DNA. In some cases, the stabilized biological sample comprises less than 4 µg DNA. In some cases, the stabilized biological sample comprises less than 3 µg DNA. In some cases, the stabilized biological sample comprises less than 2 µg DNA. In some cases, the stabilized biological sample comprises less than 1 µg DNA. In some cases, the stabilized biological sample comprises less than 0.5 µg DNA.

In another aspect, methods involving a QC determination step herein can be conducted on individual or single cells. For example, methods herein may be conducted on cells distributed into individual partitions. Exemplary partitions include, but are not limited to, wells, droplets in an emulsion, or surface positions (e.g., array spots, beads, etc.) comprising distinct patches of differentially sequenced linker molecules as described elsewhere herein. Additional partitions are also contemplated and consistent with the methods, compositions, and systems disclosed herein.

In additional aspects, stabilized biological samples used in methods involving a QC determination step herein are treated with a nuclease, such as a DNase to create fragments of DNA. In some cases, the DNase is non-sequence specific. In some cases, the DNase is active for both single-stranded DNA and double-stranded DNA. In some cases, the DNase is specific for double-stranded DNA. In some cases, the DNase preferentially cleaves double-stranded DNA. In some cases, the DNase is specific for single-stranded DNA. In some cases, the DNase preferentially cleaves single-stranded DNA. In some cases, the DNase is DNase I. In some cases, the DNase is DNase II. In some cases, the DNase is selected from one or more of DNase I and DNase II. In some cases, the DNase is micrococcal nuclease. In some cases, the DNase is selected from one or more of DNase I, DNase II, and micrococcal nuclease. In some cases, the DNase may be coupled or fused to an immunoglobulin binding protein or fragment thereof, such as a Protein A, a Protein G, a Protein A/G, or a Protein L. Other suitable nucleases are also within the scope of this disclosure.

In additional aspects, stabilized biological samples used in methods involving a QC determination step herein are treated with a crosslinking agent. In some cases, the crosslinking agent is a chemical fixative. In some cases, the chemical fixative comprises formaldehyde, which has a spacer arm length of about 2.3-2.7 angstrom (A). In some cases, the chemical fixative comprises a crosslinking agent with a long spacer arm length, For example, the crosslinking agent can have a spacer length of at least about 3 A, 4 A, 5 A, 6 A, 7 A, 8 A, 9 A, 10 A, 11 A, 12 A, 13 A, 14 A, 15 A, 16 A, 17 A, 18 A, 19 A, or 20 A. The chemical fixative can comprise ethylene glycol bis(succinimidyl succinate) (EGS), which has a spacer arm with length about 16.1 A. The chemical fixative can comprise disuccinimidyl glutarate (DSG), which has a spacer arm with length about 7.7 A. In some cases, the chemical fixative comprises formaldehyde and EGS, formaldehyde and DSG, or formaldehyde, EGS, and DSG. In some cases where multiple chemical fixatives are employed, each chemical fixative is used sequentially; in other cases, some or all of the multiple chemical fixatives are applied to the sample at the same time. The use of crosslinkers with long spacer arms can increase the fraction of read pairs with large (e.g., > 1 kb) read pair separation distances. For example, **FIG. 7** shows a comparison of resulting libraries (both DNase and MNase digested) crosslinked with formaldehyde alone versus crosslinked with formaldehyde plus DSG or EGS. DSG has NHS ester reactive groups at both ends and can be reactive towards amino groups (e.g., primary amines). DSG is membrane-permeable, allowing for intracellular crosslinking. DSG can increase crosslinking efficiency compared to disuccinimidyl suberate (DSS) in some applications. EGS has NHS ester reactive groups at both ends and can be reactive towards amino groups (e.g., primary amines). EGS is membrane-permeable, allowing for intracellular crosslinking. EGS crosslinks can be reversed, for example, by treatment with hydroxylamine for 3 to 6 hours at pH 8.5; in an example, lactose dehydrogenase retained 60% of its activity after reversible crosslinking with EGS. In some cases, the chemical fixative comprises psoralen. In some cases, the crosslinking agent is ultraviolet light. In some cases, the stabilized biological sample is a crosslinked paraffin-embedded tissue sample.

In further aspects, methods involving a QC determination step provided herein comprise contacting the plurality of selected segments to an antibody.

In additional aspects, methods involving a QC determination step provided herein comprise attaching a first segment and a second segment of a plurality of segments at a junction. In some cases, attaching comprises filling in sticky ends using biotin tagged nucleotides and ligating the blunt ends. In some cases, attaching comprises contacting at least the first segment and the second segment to a bridge oligonucleotide. In some cases, attaching comprises contacting at least the first segment and the second segment to a barcode. In some embodiments, bridge oligonucleotides herein can be from at least about 5 nucleotides in length to about 50 nucleotides in length. In some embodiments, bridge oligonucleotides herein can be from about 15 to about 18 nucleotides in length. In some embodiments, bridge oligonucleotides can be about 5, about 6, about 7, about 8, about 9, about 10, about 11, about 12, about 13, about 14, about 15, about 16, about 17, about 18, about 19, about 20, about 25, about 30, about 35, about 40, about 45, or about 50 nucleotides in length. In some embodiments, bridge oligonucleotides herein may comprise a barcode. In some embodiments, bridge oligonucleotides can comprise multiple barcodes. In some embodiments, bridge oligonucleotides comprise multiple bridge oligonucleotides connected together. In some embodiments, bridge oligonucleotides may be coupled or linked to an immunoglobulin binding protein or fragment thereof, such as a Protein A, a Protein G, a Protein A/G, or a Protein L. In some cases, coupled bridge oligonucleotides may be delivered to a location in the sample nucleic acid where an antibody is bound.

In additional aspects, methods involving a QC determination step herein do not comprise a shearing step.

In further aspects of methods involving a QC determination step herein, methods comprise obtaining at least some sequence on each side of the junction to generate a first read pair. For example, the methods may comprise obtaining at least about 50 bp, at least about 100 bp, at least about 150 bp, at least about 200 bp, at least about 250 bp, or at least about 300 bp of sequence on each side of the junction to generate a first read pair.

In additional aspects of methods involving a QC determination step herein, methods comprise mapping the first read pair to a set of contigs and determining a path through the set of contigs that represents an order and/or orientation to a genome.

In further aspects of methods involving a QC determination step herein, methods may comprise mapping the first read pair to a set of contigs and determining, from the set of contigs, a presence of a structural variant or loss of heterozygosity in the stabilized biological sample.

In additional aspects of methods involving a QC determination step herein, methods comprise mapping the first read pair to a set of contigs and assigning a variant in the set of contigs to a phase.

In further aspects of methods involving a QC determination step herein, methods comprise mapping the first read pair to a set of contigs; determining, from the set of contigs, a presence of a variant in the set of contigs; and conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

### Hi-C Methods Comprising Whole Cell or Whole Nuclei Digestion

Further provided herein are methods comprising obtaining a stabilized biological sample comprising a nucleic acid molecule complexed to at least one nucleic acid binding protein; contacting the stabilized biological sample to a DNase to cleave the nucleic acid molecule into a plurality of segments; and attaching a first segment and a second segment of the plurality of segments at a junction, wherein the stabilized biological sample comprises intact cells and/or intact nuclei. In some cases, the stabilized biological sample comprises a stabilized intact cell. Alternatively, or in combination, the stabilized biological sample comprises a stabilized intact nucleus. In some cases, contacting the stabilized intact cell or intact nucleus sample to a DNase is conducted prior to lysis of the intact cell or the intact nucleus. In some cases, cells and/or nuclei are lysed prior to attaching a first segment and a second segment of a plurality of segments at a junction.

In another aspect, methods involving digestion of whole cells or whole nuclei herein can comprise subjecting a plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the plurality of selected segments is between about 100 bp and about 600 bp, between about 100 bp and about 700 bp, between about 100 bp and about 800 bp, between about 100 bp and about 900 bp, between about 100 bp and about 1000 bp, between about 100 bp and about 1100 bp, between about 100 bp and about 1200 bp, between about 100 bp and about 1300 bp, between about 100 bp and about 1400 bp, between about 100 bp and about 1500 bp, between about 100 bp and about 1600 bp, between about 100 bp and about 1700 bp, between about 100 bp and about 1800 bp, between about 100 bp and about 1900 bp, between about 100 bp and about 2000 bp, between about 100 bp and about 2100 bp, between about 100 bp and about 2200 bp, between about 100 bp and about 2300 bp, between about 100 bp and about 2400 bp, or between about 100 bp and about 2500 bp.

In another aspect of methods involving digestion of whole cells or whole nuclei provided herein, methods further comprise, prior a size selection step, preparing a sequencing library from the plurality of segments. In some embodiments, the method further comprises subjecting the sequencing library to a size selection to obtain a size-selected library. In some cases, the size-selected library is between about 350 bp and about 1000 bp in size. In some cases, the size-selected library is between about 100 bp and about 2500 bp in size, for example, between about 100 bp and about 350 bp, between about 350 bp and about 500 bp, between about 500 bp and about 1000 bp, between about 1000 and about 1500 bp and about 2000 bp, between about 2000 bp and about 2500 bp, between about 350 bp and about 1000 bp, between about 350 bp and about 1500 bp, between about 350 bp and about 2000 bp, between about 350 bp and about 2500 bp, between about 500 bp and about 1500 bp, between about 500 bp and about 2000 bp, between about 500 bp and about 3500 bp, between about 1000 bp and about 1500 bp, between about 1000 bp and about 2000 bp, between about 1000 bp and about 2500 bp, between about 1500 bp and about 2000 bp, between about 1500 bp and about 2500 bp, or between about 2000 bp and about 2500 bp.

Size selection utilized in methods involving digestion of whole cells or whole nuclei herein can be conducted with gel electrophoresis, capillary electrophoresis, size selection beads, a gel filtration column, or combinations thereof.

In another aspect, methods involving digestion of whole cells or whole nuclei herein may comprise further analyzing the plurality of selected segments to obtain a QC value. In some cases, a QC value is selected from a chromatin digest efficiency (CDE) and a chromatin digest index (CDI). A CDE is calculated as the proportion of segments having a desired length. For example, in some cases, the CDE is calculated as the proportion of segments between 100 and 2500 bp in size prior to size selection. In some cases, a sample is selected for further analysis when the CDE value is at least 65%. In some cases, a sample is selected for further analysis when the CDE value is at least about 50%, at least about 55%, at least about 60%, at least about 65%, at least about 70%, at least about 75%, at least about 80%, at least about 85%, at least about 90%, or at least about 95%. A CDI is calculated as a ratio of a number of mononucleosome-sized segments to a number of dinucleosome-sized segments prior to size selection. For example, a CDI may be calculated as a logarithm of the ratio of fragments having a size 600-2500 bp versus fragments having a size 100-600 bp. In some cases, a sample is selected for further analysis when the CDI value is greater than -1.5 and less than 1. In some cases, a sample is selected for further analysis when the CDI value is greater than about -2 and less than about 1.5, greater than about -1.9 and less than about 1.5, greater than about -1.8 and less than about 1.5, greater than about -1.7 and less than about 1.5, greater than about -1.6 and less than about 1.5, greater than about -1.5 and less than about 1.5, greater than about -1.4 and less than about 1.5, greater than about -1.3 and less than about 1.5, greater than about -1.2 and less than about 1.5, greater than about -1.1 and less than about 1.5, greater than about -2 and less than about 1.5, greater than about -1 and less than about 1.5, greater than about -0.9 and less than about 1.5, greater than about -0.8 and less than about 1.5, greater than about -0.7 and less than about 1.5, greater than about -0.6 and less than about 1.5, greater than about -0.5 and less than about 1.5, greater than about -2 and less than about 1.4, greater than about -2 and less than about 1.3, greater than about -2 and less than about 1.2, greater than about -2 and less than about 1.1, greater than about -2 and less than about 1, greater than about -2 and less than about 0.9, greater than about -2 and less than about 0.8, greater than about -2 and less than about 0.7, greater than about -2 and less than about 0.6, or greater than about -2 and less than about 0.5.

In another aspect, methods involving digestion of whole cells or whole nuclei herein are conducted on small samples containing few cells or small amounts of nucleic acid. In some cases, the stabilized biological sample comprises fewer than 3,000,000 cells. In some cases, the stabilized biological sample comprises fewer than 2,000,000 cells. In some cases, the stabilized biological sample comprises fewer than 1,000,000 cells. In some cases, the stabilized biological sample comprises fewer than 500,000 cells. In some cases, the stabilized biological sample comprises fewer than 400,000 cells. In some cases, the stabilized biological sample comprises fewer than 300,000 cells. In some cases, the stabilized biological sample comprises fewer than 200,000 cells. In some cases, the stabilized biological sample comprises fewer than 100,000 cells. In some cases, the stabilized biological sample comprises less than 10 µg DNA. In some cases, the stabilized biological sample comprises less than 9 µg DNA. In some cases, the stabilized biological sample comprises less than 8 µg DNA. In some cases, the stabilized biological sample comprises less than 7 µg DNA. In some cases, the stabilized biological sample comprises less than 6 µg DNA. In some cases, the stabilized biological sample comprises less than 5 µg DNA. In some cases, the stabilized biological sample comprises less than 4 µg DNA. In some cases, the stabilized biological sample comprises less than 3 µg DNA. In some cases, the stabilized biological sample comprises less than 2 µg DNA. In some cases, the stabilized biological sample comprises less than 1 µg DNA. In some cases, the stabilized biological sample comprises less than 0.5 µg DNA.

In another aspect, methods involving a digestion of whole cells or whole nuclei herein may be conducted on individual or single cells. For example, methods herein may be conducted on cells distributed into individual partitions. Exemplary partitions include, but are not limited to, wells, droplets in an emulsion, or surface positions (e.g., array spots, beads, etc.) comprising distinct patches of differentially sequenced linker molecules as described elsewhere herein. Additional partitions are also contemplated and consistent with the methods, compositions, and systems disclosed herein.

In additional aspects, stabilized biological samples used in methods involving digestion of whole cells or whole nuclei herein are treated with a nuclease, such as a DNase to create fragments of DNA. In some cases, the DNase is non-sequence specific. In some cases, the DNase is active for both single-stranded DNA and double-stranded DNA. In some cases, the DNase is specific for double-stranded DNA. In some cases, the DNase preferentially cleaves double-stranded DNA. In some cases, the DNase is specific for single-stranded DNA. In some cases, the DNase preferentially cleaves single-stranded DNA. In some cases, the DNase is DNase I. In some cases, the DNase is DNase II. In some cases, the DNase is selected from one or more of DNase I and DNase II. In some cases, the DNase is micrococcal nuclease. In some cases, the DNase is selected from one or more of DNase I, DNase II, and micrococcal nuclease. In some cases, the DNase may be coupled or fused to an immunoglobulin binding protein or fragment thereof, such as a Protein A, a Protein G, a Protein A/G, or a Protein L. Other suitable nucleases are also within the scope of this disclosure.

In additional aspects, stabilized biological samples used in methods involving digestion of whole cells or whole nuclei herein are treated with a crosslinking agent. In some cases, the crosslinking agent is a chemical fixative. In some cases, the chemical fixative comprises formaldehyde, which has a spacer arm length of about 2.3-2.7 angstrom (A). In some cases, the chemical fixative comprises a crosslinking agent with a long spacer arm length, For example, the crosslinking agent can have a spacer length of at least about 3 A, 4 A, 5 A, 6 A, 7 A, 8 A, 9 A, 10 A, 11 A, 12 A, 13 A, 14 A, 15 A, 16 A, 17 A, 18 A, 19 A, or 20 A. The chemical fixative can comprise ethylene glycol bis(succinimidyl succinate) (EGS), which has a spacer arm with length about 16.1 A. The chemical fixative can comprise disuccinimidyl glutarate (DSG), which has a spacer arm with length about 7.7 A. In some cases, the chemical fixative comprises formaldehyde and EGS, formaldehyde and DSG, or formaldehyde, EGS, and DSG. In some cases where multiple chemical fixatives are employed, each chemical fixative is used sequentially; in other cases, some or all of the multiple chemical fixatives are applied to the sample at the same time. The use of crosslinkers with long spacer arms can increase the fraction of read pairs with large (e.g., > 1 kb) read pair separation distances. For example, FIG. 7 shows a comparison of resulting libraries (both DNase and MNase digested) crosslinked with formaldehyde alone versus crosslinked with formaldehyde plus DSG or EGS. DSG has NHS ester reactive groups at both ends and can be reactive towards amino groups (e.g., primary amines). DSG is membrane-permeable, allowing for intracellular crosslinking. DSG can increase crosslinking efficiency compared to disuccinimidyl suberate (DSS) in some applications. EGS has NHS ester reactive groups at both ends and can be reactive towards amino groups (e.g., primary amines). EGS is membrane-permeable, allowing for intracellular crosslinking. EGS crosslinks can be reversed, for example, by treatment with hydroxylamine for 3 to 6 hours at pH 8.5; in an example, lactose dehydrogenase retained 60% of its activity after reversible crosslinking with EGS. In some cases, the chemical fixative comprises psoralen. In some cases, the crosslinking agent is ultraviolet light. In some cases, the stabilized biological sample is a crosslinked paraffin-embedded tissue sample.

In further aspects, methods involving digestion of whole cells or whole nuclei provided herein comprise contacting the plurality of selected segments to an antibody.

In additional aspects, methods involving digestion of whole cells or whole nuclei provided herein comprise attaching a first segment and a second segment of a plurality of segments at a junction. In some cases, attaching comprises filling in sticky ends using biotin tagged nucleotides and ligating the blunt ends. In some cases, attaching comprises contacting at least the first segment and the second segment to a bridge oligonucleotide. In some cases, attaching comprises contacting at least the first segment and the second segment to a barcode. In some embodiments, bridge oligonucleotides herein may be from at least about 5 nucleotides in length to about 50 nucleotides in length. In some embodiments, bridge oligonucleotides herein may be from about 15 to about 18 nucleotides in length. In some embodiments, bridge oligonucleotides may be about 5, about 6, about 7, about 8, about 9, about 10, about 11, about 12, about 13, about 14, about 15, about 16, about 17, about 18, about 19, about 20, about 25, about 30, about 35, about 40, about 45, or about 50 nucleotides in length. In some embodiments, bridge oligonucleotides herein can comprise a barcode. In some embodiments, bridge oligonucleotides can comprise multiple barcodes. In some embodiments, bridge oligonucleotides comprise multiple bridge oligonucleotides connected together. In some embodiments, bridge oligonucleotides may be coupled or linked to an immunoglobulin binding protein or fragment thereof, such as a Protein A, a Protein G, a Protein A/G, or a Protein L. In some cases, coupled bridge oligonucleotides may be delivered to a location in the sample nucleic acid where an antibody is bound.

A splitting and pooling approach can be employed to produce bridge oligonucleotides with unique barcodes. A population of samples can be split into multiple groups, bridge oligonucleotides can be attached to the samples such that the bridge oligonucleotide barcodes are different between groups but the same within a group, the groups of samples can be pooled together again, and this process can be repeated multiple times. Iterating this process can ultimately result in each sample in the population having a unique series of bridge oligonucleotide barcodes, allowing single-sample (e.g., single cell, single nucleus, single chromosome) analysis. In one illustrative example, a sample of crosslinked digested nuclei attached to a solid support of beads is split across 8 tubes, each containing 1 of 8 unique members of a first adaptor group (first iteration) comprising double-stranded DNA (dsDNA) adaptors to be ligated. Each of the 8 adaptors can have the same 5' overhang sequence for ligation to the nucleic acid ends of the cross-linked chromatin aggregates in the nuclei, but otherwise has a unique dsDNA sequence. After the first adaptor group is ligated, the nuclei can be pooled back together and washed to remove the ligation reaction components. The scheme of distributing, ligating, and pooling can be repeated 2 additional times (2 iterations). Following ligation of members from each adaptor group, a cross-linked chromatin aggregate can be attached to multiple barcodes in series. In some cases, the sequential ligation of a plurality of members of a plurality of adaptor groups (iterations) results in barcode combinations. The number of barcode combinations available depends on the number of groups per iteration and the total number of barcode oligonucleotides used. For example, 3 iterations comprising 8 members each can have 83 possible combinations. In some cases, barcode combinations are unique. In some cases, barcode combinations are redundant. The total number of barcode combinations can be adjusted by increasing or decreasing the number of groups receiving unique barcodes and/or increasing or decreasing the number of iterations. When more than one adaptor group is used, a distributing, attaching, and pooling scheme can be used for iterative adaptor attachment. In some cases, the scheme of distributing, attaching, and pooling can be repeated at least 3, 4, 5, 6, 7, 8, 9, or 10 additional times. In some cases, the members of the last adaptor group include a sequence for subsequent enrichment of adaptor-attached DNA, for example, during sequencing library preparation through PCR amplification.

In additional aspects, methods involving digestion of whole cells or whole nuclei herein do not comprise a shearing step.

In further aspects of methods involving digestion of whole cells or whole nuclei herein, methods comprise obtaining at least some sequence on each side of the junction to generate a first read pair. For example, the methods may comprise obtaining at least about 50 bp, at least about 100 bp, at least about 150 bp, at least about 200 bp, at least about 250 bp, or at least about 300 bp of sequence on each side of the junction to generate a first read pair.

In additional aspects of methods involving digestion of whole cells or whole nuclei herein, methods comprise mapping the first read pair to a set of contigs and determining a path through the set of contigs that represents an order and/or orientation to a genome.

In further aspects of methods involving digestion of whole cells or whole nuclei herein, methods comprise mapping the first read pair to a set of contigs; and determining, from the set of contigs, a presence of a structural variant or loss of heterozygosity in the stabilized biological sample.

In additional aspects of methods involving digestion of whole cells or whole nuclei herein, methods comprise mapping the first read pair to a set of contigs and assigning a variant in the set of contigs to a phase.

In further aspects of methods involving digestion of whole cells or whole nuclei herein, methods comprise mapping the first read pair to a set of contigs; determining, from the set of contigs, a presence of a variant in the set of contigs; and conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

### Hi-C Methods Having Low Nucleic Acid Input Requirements

Additionally provided herein are methods comprising obtaining a stabilized biological sample comprising a nucleic acid molecule complexed to at least one nucleic acid binding protein; contacting the stabilized biological sample to a DNase to cleave the nucleic acid molecule into a plurality of segments; and attaching a first segment and a second segment of the plurality of segments at a junction, wherein the stabilized biological sample comprises fewer than 3,000,000 cells or less than 10 µg DNA. In some cases, the stabilized biological sample comprises fewer than 3,000,000 cells. In some cases, the stabilized biological sample comprises fewer than 2,000,000 cells. In some cases, the stabilized biological sample comprises fewer than 1,000,000 cells. In some cases, the stabilized biological sample comprises fewer than 500,000 cells. In some cases, the stabilized biological sample comprises fewer than 400,000 cells. In some cases, the stabilized biological sample comprises fewer than 300,000 cells. In some cases, the stabilized biological sample comprises fewer than 200,000 cells. In some cases, the stabilized biological sample comprises fewer than 100,000 cells. In some cases, the stabilized biological sample comprises less than 10 µg DNA. In some cases, the stabilized biological sample comprises less than 9 µg DNA. In some cases, the stabilized biological sample comprises less than 8 µg DNA. In some cases, the stabilized biological sample comprises less than 7 µg DNA. In some cases, the stabilized biological sample comprises less than 6 µg DNA. In some cases, the stabilized biological sample comprises less than 5 µg DNA. In some cases, the stabilized biological sample comprises less than 4 µg DNA. In some cases, the stabilized biological sample comprises less than 3 µg DNA. In some cases, the stabilized biological sample comprises less than 2 µg DNA. In some cases, the stabilized biological sample comprises less than 1 µg DNA. In some cases, the stabilized biological sample comprises less than 0.5 µg DNA.

In another aspect, methods having low nucleic acid input requirements herein may be conducted on individual or single cells. For example, methods herein may be conducted on cells distributed into individual partitions. Exemplary partitions include, but are not limited to, wells, droplets in an emulsion, or surface positions (e.g., array spots, beads, etc.) comprising distinct patches of differentially sequenced linker molecules as described elsewhere herein. Additional partitions are also contemplated and consistent with the methods, compositions, and systems disclosed herein.

In another aspect, methods having low nucleic acid input requirements herein comprise subjecting a plurality of segments to size selection to obtain a plurality of selected segments. In some cases, the plurality of selected segments is about 145 to about 600 bp. In some cases, the plurality of selected segments is about 100 to about 2500 bp. In some cases, the plurality of selected segments is about 100 to about 600 bp. In some cases, the plurality of selected segments is about 600 to about 2500 bp. In some cases, the plurality of selected segments is between about 100 bp and about 600 bp, between about 100 bp and about 700 bp, between about 100 bp and about 800 bp, between about 100 bp and about 900 bp, between about 100 bp and about 1000 bp, between about 100 bp and about 1100 bp, between about 100 bp and about 1200 bp, between about 100 bp and about 1300 bp, between about 100 bp and about 1400 bp, between about 100 bp and about 1500 bp, between about 100 bp and about 1600 bp, between about 100 bp and about 1700 bp, between about 100 bp and about 1800 bp, between about 100 bp and about 1900 bp, between about 100 bp and about 2000 bp, between about 100 bp and about 2100 bp, between about 100 bp and about 2200 bp, between about 100 bp and about 2300 bp, between about 100 bp and about 2400 bp, or between about 100 bp and about 2500 bp.

In another aspect of methods having low nucleic acid input requirements provided herein, methods further comprise, prior a size selection step, preparing a sequencing library from the plurality of segments. In some embodiments, the method further comprises subjecting the sequencing library to a size selection to obtain a size-selected library. In some cases, the size-selected library is between about 350 bp and about 1000 bp in size. In some cases, the size-selected library is between about 100 bp and about 2500 bp in size, for example, between about 100 bp and about 350 bp, between about 350 bp and about 500 bp, between about 500 bp and about 1000 bp, between about 1000 and about 1500 bp and about 2000 bp, between about 2000 bp and about 2500 bp, between about 350 bp and about 1000 bp, between about 350 bp and about 1500 bp, between about 350 bp and about 2000 bp, between about 350 bp and about 2500 bp, between about 500 bp and about 1500 bp, between about 500 bp and about 2000 bp, between about 500 bp and about 3500 bp, between about 1000 bp and about 1500 bp, between about 1000 bp and about 2000 bp, between about 1000 bp and about 2500 bp, between about 1500 bp and about 2000 bp, between about 1500 bp and about 2500 bp, or between about 2000 bp and about 2500 bp.

Size selection utilized in methods having low nucleic acid input requirements herein is often conducted with gel electrophoresis, capillary electrophoresis, size selection beads, a gel filtration column, or combinations thereof.

In another aspect, methods having low nucleic acid input requirements herein may further comprise analyzing the plurality of selected segments to obtain a QC value. In some cases, a QC value is selected from a chromatin digest efficiency (CDE) and a chromatin digest index (CDI). A CDE is calculated as the proportion of segments having a desired length. For example, in some cases, the CDE is calculated as the proportion of segments between 100 and 2500 bp in size prior to size selection. In some cases, a sample is selected for further analysis when the CDE value is at least 65%. In some cases, a sample is selected for further analysis when the CDE value is at least about 50%, at least about 55%, at least about 60%, at least about 65%, at least about 70%, at least about 75%, at least about 80%, at least about 85%, at least about 90%, or at least about 95%. A CDI is calculated as a ratio of a number of mononucleosome-sized segments to a number of dinucleosome-sized segments prior to size selection. For example, a CDI may be calculated as a logarithm of the ratio of fragments having a size 600-2500 bp versus fragments having a size 100-600 bp. In some cases, a sample is selected for further analysis when the CDI value is greater than -1.5 and less than 1. In some cases, a sample is selected for further analysis when the CDI value is greater than about -2 and less than about 1.5, greater than about -1.9 and less than about 1.5, greater than about -1.8 and less than about 1.5, greater than about -1.7 and less than about 1.5, greater than about -1.6 and less than about 1.5, greater than about -1.5 and less than about 1.5, greater than about -1.4 and less than about 1.5, greater than about -1.3 and less than about 1.5, greater than about -1.2 and less than about 1.5, greater than about -1.1 and less than about 1.5, greater than about -2 and less than about 1.5, greater than about -1 and less than about 1.5, greater than about -0.9 and less than about 1.5, greater than about -0.8 and less than about 1.5, greater than about -0.7 and less than about 1.5, greater than about -0.6 and less than about 1.5, greater than about -0.5 and less than about 1.5, greater than about -2 and less than about 1.4, greater than about -2 and less than about 1.3, greater than about -2 and less than about 1.2, greater than about -2 and less than about 1.1, greater than about -2 and less than about 1, greater than about -2 and less than about 0.9, greater than about -2 and less than about 0.8, greater than about -2 and less than about 0.7, greater than about -2 and less than about 0.6, or greater than about -2 and less than about 0.5.

In another aspect, stabilized biological samples used in methods having low nucleic acid input requirements herein comprise biological material that has been treated with a stabilizing agent. In some cases, the stabilized biological sample comprises a stabilized cell lysate. Alternatively, the stabilized biological sample comprises a stabilized intact cell. Alternatively, the stabilized biological sample comprises a stabilized intact nucleus. In some cases, contacting the stabilized intact cell or intact nucleus sample to a DNase is conducted prior to lysis of the intact cell or the intact nucleus. In some cases, cells and/or nuclei are lysed prior to attaching a first segment and a second segment of a plurality of segments at a junction.

In additional aspects, stabilized biological samples used in methods having low nucleic acid input requirements herein are treated with a nuclease, such as a DNase to create fragments of DNA. In some cases, the DNase is non-sequence specific. In some cases, the DNase is active for both single-stranded DNA and double-stranded DNA. In some cases, the DNase is specific for double-stranded DNA. In some cases, the DNase preferentially cleaves double-stranded DNA. In some cases, the DNase is specific for single-stranded DNA. In some cases, the DNase preferentially cleaves single-stranded DNA. In some cases, the DNase is DNase I. In some cases, the DNase is DNase II. In some cases, the DNase is selected from one or more of DNase I and DNase II. In some cases, the DNase is micrococcal nuclease. In some cases, the DNase is selected from one or more of DNase I, DNase II, and micrococcal nuclease. In some cases, the DNase may be coupled or fused to an immunoglobulin binding protein or fragment thereof, such as a Protein A, a Protein G, a Protein A/G, or a Protein L. Other suitable nucleases are also within the scope of this disclosure.

In additional aspects, stabilized biological samples used in methods having low nucleic acid input requirements herein are treated with a crosslinking agent. In some cases, the crosslinking agent is a chemical fixative. In some cases, the chemical fixative comprises formaldehyde, which has a spacer arm length of about 2.3-2.7 angstrom (A). In some cases, the chemical fixative comprises a crosslinking agent with a long spacer arm length, For example, the crosslinking agent can have a spacer length of at least about 3 A, 4 A, 5 A, 6 A, 7 A, 8 A, 9 A, 10 A, 11 A, 12 A, 13 A, 14 A, 15 A, 16 A, 17 A, 18 A, 19 A, or 20 A. The chemical fixative can comprise ethylene glycol bis(succinimidyl succinate) (EGS), which has a spacer arm with length about 16.1 A. The chemical fixative can comprise disuccinimidyl glutarate (DSG), which has a spacer arm with length about 7.7 A. In some cases, the chemical fixative comprises formaldehyde and EGS, formaldehyde and DSG, or formaldehyde, EGS, and DSG. In some cases where multiple chemical fixatives are employed, each chemical fixative is used sequentially; in other cases, some or all of the multiple chemical fixatives are applied to the sample at the same time. The use of crosslinkers with long spacer arms can increase the fraction of read pairs with large (e.g., > 1 kb) read pair separation distances. For example, FIG. 7 shows a comparison of resulting libraries (both DNase and MNase digested) crosslinked with formaldehyde alone versus crosslinked with formaldehyde plus DSG or EGS. DSG has NHS ester reactive groups at both ends and can be reactive towards amino groups (e.g., primary amines). DSG is membrane-permeable, allowing for intracellular crosslinking. DSG can increase crosslinking efficiency compared to disuccinimidyl suberate (DSS) in some applications. EGS has NHS ester reactive groups at both ends and can be reactive towards amino groups (e.g., primary amines). EGS is membrane-permeable, allowing for intracellular crosslinking. EGS crosslinks can be reversed, for example, by treatment with hydroxylamine for 3 to 6 hours at pH 8.5; in an example, lactose dehydrogenase retained 60% of its activity after reversible crosslinking with EGS. In some cases, the chemical fixative comprises psoralen. In some cases, the crosslinking agent is ultraviolet light. In some cases, the stabilized biological sample is a crosslinked paraffin-embedded tissue sample.

In further aspects, methods provided herein comprise contacting the plurality of selected segments to an antibody.

In additional aspects, methods having low nucleic acid input requirements provided herein comprise attaching a first segment and a second segment of a plurality of segments at a junction. In some cases, attaching comprises filling in sticky ends using biotin tagged nucleotides and ligating the blunt ends. In some cases, attaching comprises contacting at least the first segment and the second segment to a bridge oligonucleotide. In some cases, attaching comprises contacting at least the first segment and the second segment to a barcode. In some embodiments, bridge oligonucleotides herein may be from at least about 5 nucleotides in length to about 50 nucleotides in length. In some embodiments, bridge oligonucleotides herein may be from about 15 to about 18 nucleotides in length. In some embodiments, bridge oligonucleotides may be about 5, about 6, about 7, about 8, about 9, about 10, about 11, about 12, about 13, about 14, about 15, about 16, about 17, about 18, about 19, about 20, about 25, about 30, about 35, about 40, about 45, or about 50 nucleotides in length. In some embodiments, bridge oligonucleotides herein may comprise a barcode. In some embodiments, bridge oligonucleotides can comprise multiple barcodes. In some embodiments, bridge oligonucleotides comprise multiple bridge oligonucleotides connected together. In some embodiments, bridge oligonucleotides may be coupled or linked to an immunoglobulin binding protein or fragment thereof, such as a Protein A, a Protein G, a Protein A/G, or a Protein L. In some cases, coupled bridge oligonucleotides may be delivered to a location in the sample nucleic acid where an antibody is bound.

A splitting and pooling approach can be employed to produce bridge oligonucleotides with unique barcodes. A population of samples can be split into multiple groups, bridge oligonucleotides can be attached to the samples such that the bridge oligonucleotide barcodes are different between groups but the same within a group, the groups of samples can be pooled together again, and this process can be repeated multiple times. Iterating this process can ultimately result in each sample in the population having a unique series of bridge oligonucleotide barcodes, allowing single-sample (e.g., single cell, single nucleus, single chromosome) analysis. In one illustrative example, a sample of crosslinked digested nuclei attached to a solid support of beads is split across 8 tubes, each containing 1 of 8 unique members of a first adaptor group (first iteration) comprising double-stranded DNA (dsDNA) adaptors to be ligated. Each of the 8 adaptors can have the same 5' overhang sequence for ligation to the nucleic acid ends of the cross-linked chromatin aggregates in the nuclei, but otherwise has a unique dsDNA sequence. After the first adaptor group is ligated, the nuclei can be pooled back together and washed to remove the ligation reaction components. The scheme of distributing, ligating, and pooling can be repeated 2 additional times (2 iterations). Following ligation of members from each adaptor group, a cross-linked chromatin aggregate can be attached to multiple barcodes in series. In some cases, the sequential ligation of a plurality of members of a plurality of adaptor groups (iterations) results in barcode combinations. The number of barcode combinations available depends on the number of groups per iteration and the total number of barcode oligonucleotides used. For example, 3 iterations comprising 8 members each can have 83 possible combinations. In some cases, barcode combinations are unique. In some cases, barcode combinations are redundant. The total number of barcode combinations can be adjusted by increasing or decreasing the number of groups receiving unique barcodes and/or increasing or decreasing the number of iterations. When more than one adaptor group is used, a distributing, attaching, and pooling scheme can be used for iterative adaptor attachment. In some cases, the scheme of distributing, attaching, and pooling can be repeated at least 3, 4, 5, 6, 7, 8, 9, or 10 additional times. In some cases, the members of the last adaptor group include a sequence for subsequent enrichment of adaptor-attached DNA, for example, during sequencing library preparation through PCR amplification.

In additional aspects, methods having low nucleic acid input requirements herein do not comprise a shearing step.

In further aspects of methods having low nucleic acid input requirements herein, methods comprise obtaining at least some sequence on each side of the junction to generate a first read pair. For example, the methods may comprise obtaining at least about 50 bp, at least about 100 bp, at least about 150 bp, at least about 200 bp, at least about 250 bp, or at least about 300 bp of sequence on each side of the junction to generate a first read pair.

In additional aspects of methods having low nucleic acid input requirements herein, methods comprise mapping the first read pair to a set of contigs and determining a path through the set of contigs that represents an order and/or orientation to a genome.

In further aspects of methods having low nucleic acid input requirements herein, methods comprise mapping the first read pair to a set of contigs and determining, from the set of contigs, a presence of a structural variant or loss of heterozygosity in the stabilized biological sample.

In additional aspects of methods having low nucleic acid input requirements herein, methods comprise mapping the first read pair to a set of contigs and assigning a variant in the set of contigs to a phase.

In further aspects of methods having low nucleic acid input requirements herein, methods comprise mapping the first read pair to a set of contigs; determining, from the set of contigs, a presence of a variant in the set of contigs; and conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

### Hi-C Methods Using Micrococcal Nuclease (MNase)

Additionally, provided herein are methods that may comprise obtaining a stabilized biological sample comprising a nucleic acid molecule complexed to at least one nucleic acid binding protein; contacting the stabilized biological sample to a micrococcal nuclease (MNase) to cleave the nucleic acid molecule into a plurality of segments; and attaching a first segment and a second segment of the plurality of segments at a junction. Use of MNase in methods herein may provide specific information about where DNA binding proteins are bound to the chromatin with up to single base pair resolution because, for example, MNase can cleave all base pairs not bound to a DNA binding protein. In addition, use of MNase digestion may allow for creation of contact maps and topologically associated domains to decipher three-dimensional chromatin structural information. In some cases, the MNase may be coupled or fused to an immunoglobulin binding protein or fragment thereof, such as a Protein A, a Protein G, a Protein A/G, or a Protein L.

For example, MNase Hi-C methods can provide locations of protein binding or genome contact interactions at a resolution of less than or equal to about 1 bp, 2 bp, 3 bp, 4 bp, 5 bp, 6 bp, 7 bp, 8 bp, 9 bp, 10 bp, 20 bp, 30 bp, 40 bp, 50 bp, 60 bp, 70 bp, 80 bp, 90 bp, 100 bp, 200 bp, 300 bp, 400 bp, 500 bp, 600 bp, 700 bp, 800 bp, 900 bp, 1000 bp, 2000 bp, 3000 bp, 4000 bp, 5000 bp, 6000 bp, 7000 bp, 8000 bp, 9000 bp, 10 kb, 20 kb, 30 kb, 40 kb, 50 kb, 60 kb, 70 kb, 80 kb, 90 kb, or 100 kb. In some cases, protein binding sites, protein footprints, contact interactions, or other features can be mapped to within 1000 bp, within 900 bp, within 800 bp, within 700 bp, within 600 bp, within 500 bp, within 400 bp, within 300 bp, within 200 bp, within 190 bp, within 180 bp, within 170 bp, within 160 bp, within 150 bp, within 140 bp, within 130 bp, within 120 bp, within 110 bp, within 100 bp, within 90 bp, within 80 bp, within 70 bp, within 60 bp, within 50 bp, within 40 bp, within 30 bp, within 20 bp, within 10 bp, within 9 bp, within 8 bp, within 7 bp, within 6 bp, within 5 bp, within 4 bp, within 3 bp, within 2 bp, or within 1 bp.

In certain aspects, methods involving a MNase digestion step may further comprise subjecting a plurality of segments to size selection to obtain a plurality of selected segments. In some cases, the plurality of selected segments can be from about 145 to about 600 bp. In some cases, the plurality of selected segments can be from about 100 to about 2500 bp. In some cases, the plurality of selected segments can be from about 100 to about 600 bp. In some cases, the plurality of selected segments can be from about 600 to about 2500 bp. In some cases, the plurality of selected segments can be from about 100 bp to about 600 bp, from about 100 bp to about 700 bp, from about 100 bp to about 800 bp, from about 100 bp to about 900 bp, from about 100 bp to about 1000 bp, from about 100 bp to about 1100 bp, from about 100 bp to about 1200 bp, from about 100 bp to about 1300 bp, from about 100 bp to about 1400 bp, from about 100 bp to about 1500 bp, from about 100 bp to about 1600 bp, from about 100 bp to about 1700 bp, from about 100 bp to about 1800 bp, from about 100 bp to about 1900 bp, from about 100 bp to about 2000 bp, from about 100 bp to about 2100 bp, from about 100 bp to about 2200 bp, from about 100 bp to about 2300 bp, from about 100 bp to about 2400 bp, or from about 100 bp to about 2500 bp.

In another aspect of methods involving a MNase digestion step as provided herein, the methods may further comprise preparing a sequencing library from the plurality of segments. In some embodiments, the method may further comprise subjecting the sequencing library to a size selection to obtain a size-selected library. In some cases, the size-selected library may be from about 350 bp to about 1000 bp in size. In some cases, the size-selected library may be from about 100 bp to about 2500 bp in size, for example, from about 100 bp to about 350 bp, from about 350 bp to about 500 bp, from about 500 bp to about 1000 bp, from about 1000 to about 1500 bp, from about 2000 bp to about 2500 bp, from about 350 bp to about 1000 bp, from about 350 bp to about 1500 bp, from about 350 bp to about 2000 bp, from about 350 bp to about 2500 bp, from about 500 bp to about 1500 bp, from about 500 bp to about 2000 bp, from about 500 bp to about 3500 bp, from about 1000 bp to about 1500 bp, from about 1000 bp to about 2000 bp, from about 1000 bp to about 2500 bp, from about 1500 bp to about 2000 bp, from about 1500 bp to about 2500 bp, or from about 2000 bp to about 2500 bp.

In another aspect, methods involving a MNase digestion step as provided herein can further comprise analyzing the plurality of segments to obtain a QC value. In some cases, a QC value may be selected from a chromatin digest efficiency (CDE) and a chromatin digest index (CDI). A CDE can be calculated as the proportion of segments having a desired length. For example, in some cases, the CDE can be calculated as the proportion of segments from 100 bp to 2500 bp in size prior to size selection. In some cases, a sample may be selected for further analysis when the CDE value is at least 65%. In some cases, a sample may be selected for further analysis when the CDE value is at least about 50%, at least about 55%, at least about 60%, at least about 65%, at least about 70%, at least about 75%, at least about 80%, at least about 85%, at least about 90%, or at least about 95%.

A CDI can be calculated as a ratio of a number of mononucleosome-sized segments to a number of dinucleosome-sized segments prior to size selection. For example, a CDI may be calculated as a logarithm of the ratio of fragments having a size of 600-2500 bp versus fragments having a size of 100-600 bp. In some cases, a sample may be selected for further analysis when the CDI value is greater than - 1.5 and less than 1. In some cases, a sample may be selected for further analysis when the CDI value is greater than about -2 and less than about 1.5, greater than about -1.9 and less than about 1.5, greater than about -1.8 and less than about 1.5, greater than about -1.7 and less than about 1.5, greater than about -1.6 and less than about 1.5, greater than about -1.5 and less than about 1.5, greater than about -1.4 and less than about 1.5, greater than about -1.3 and less than about 1.5, greater than about -1.2 and less than about 1.5, greater than about -1.1 and less than about 1.5, greater than about -2 and less than about 1.5, greater than about -1 and less than about 1.5, greater than about -0.9 and less than about 1.5, greater than about - 0.8 and less than about 1.5, greater than about -0.7 and less than about 1.5, greater than about -0.6 and less than about 1.5, greater than about -0.5 and less than about 1.5, greater than about -2 and less than about 1.4, greater than about -2 and less than about 1.3, greater than about -2 and less than about 1.2, greater than about -2 and less than about 1.1, greater than about -2 and less than about 1, greater than about -2 and less than about 0.9, greater than about -2 and less than about 0.8, greater than about -2 and less than about 0.7, greater than about -2 and less than about 0.6, or greater than about -2 and less than about 0.5.

In another aspect, stabilized biological samples used in methods involving a MNase digestion step as provided herein may comprise biological material that has been treated with a stabilizing agent. In some cases, the stabilized biological sample may comprise a stabilized cell lysate. Alternatively, the stabilized biological sample may comprise a stabilized intact cell. Alternatively, the stabilized biological sample may comprise a stabilized intact nucleus. In some cases, contacting the stabilized intact cell or intact nucleus sample to a MNase may be conducted prior to lysis of the intact cell or the intact nucleus. In some cases, cells and/or nuclei may be lysed prior to attaching a first segment and a second segment of a plurality of segments at a junction.

In another aspect, methods involving a MNase digestion step as provided herein may be conducted on small samples containing few cells or small amounts of nucleic acid. For example, in some cases, the stabilized biological sample may comprise fewer than 3,000,000 cells. In some cases, the stabilized biological sample may comprise fewer than 2,000,000 cells. In some cases, the stabilized biological sample may comprise fewer than 1,000,000 cells. In some cases, the stabilized biological sample may comprise fewer than 500,000 cells. In some cases, the stabilized biological sample may comprise fewer than 400,000 cells. In some cases, the stabilized biological sample may comprise fewer than 300,000 cells. In some cases, the stabilized biological sample may comprise fewer than 200,000 cells. In some cases, the stabilized biological sample may comprise fewer than 100,000 cells. In some cases, the stabilized biological sample may comprise less than 10 µg DNA. In some cases, the stabilized biological sample may comprise less than 9 µg DNA. In some cases, the stabilized biological sample may comprise less than 8 µg DNA. In some cases, the stabilized biological sample may comprise less than 7 µg DNA. In some cases, the stabilized biological sample may comprise less than 6 µg DNA. In some cases, the stabilized biological sample may comprise less than 5 µg DNA. In some cases, the stabilized biological sample may comprise less than 4 µg DNA. In some cases, the stabilized biological sample may comprise less than 3 µg DNA. In some cases, the stabilized biological sample may comprise less than 2 µg DNA. In some cases, the stabilized biological sample comprises less than 1 µg DNA. In some cases, the stabilized biological sample comprises less than 0.5 µg DNA.

In another aspect, methods involving a MNase digestion step herein may be conducted on individual or single cells. For example, methods herein may be conducted on cells distributed into individual partitions. Exemplary partitions include, but are not limited to, wells, droplets in an emulsion, or surface positions (e.g., array spots, beads, etc.) comprising distinct patches of differentially sequenced linker molecules as described elsewhere herein. Additional partitions are also contemplated and consistent with the methods, compositions, and systems disclosed herein.

In additional aspects, stabilized biological samples used in methods involving a MNase digestion step herein may be further treated with an additional nuclease, such as a DNase to create fragments of DNA. In some cases, the DNase may be non-sequence specific. In some cases, the DNase may be active for both single-stranded DNA and double-stranded DNA. In some cases, the DNase may be specific for double-stranded DNA. In some cases, the DNase may preferentially cleave double-stranded DNA. In some cases, the DNase may be specific for single-stranded DNA. In some cases, the DNase may preferentially cleave single-stranded DNA. In some cases, the DNase can be DNase I. In some cases, the DNase can be DNase II. In some cases, the DNase may be selected from one or more of DNase I and DNase II. In some cases, the DNase may be coupled or fused to an immunoglobulin binding protein or fragment thereof, such as a Protein A, a Protein G, a Protein A/G, or a Protein L. Other suitable nucleases are also within the scope of this disclosure.

In additional aspects, stabilized biological samples as provided herein for use in methods involving a MNase digestion step can be treated with a crosslinking agent. In some cases, the crosslinking agent may be a chemical fixative. In some cases, the chemical fixative comprises formaldehyde, which has a spacer arm length of about 2.3-2.7 angstrom (A). In some cases, the chemical fixative comprises a crosslinking agent with a long spacer arm length, For example, the crosslinking agent can have a spacer length of at least about 3 A, 4 A, 5 A, 6 A, 7 A, 8 A, 9 A, 10 A, 11 A, 12 A, 13 A, 14 A, 15 A, 16 A, 17 A, 18 A, 19 A, or 20 A. The chemical fixative can comprise ethylene glycol bis(succinimidyl succinate) (EGS), which has a spacer arm with length about 16.1 A. The chemical fixative can comprise disuccinimidyl glutarate (DSG), which has a spacer arm with length about 7.7 A. In some cases, the chemical fixative comprises formaldehyde and EGS, formaldehyde and DSG, or formaldehyde, EGS, and DSG. In some cases where multiple chemical fixatives are employed, each chemical fixative is used sequentially; in other cases, some or all of the multiple chemical fixatives are applied to the sample at the same time. The use of crosslinkers with long spacer arms can increase the fraction of read pairs with large (e.g., > 1 kb) read pair separation distances. For example, **FIG. 7** shows a comparison of resulting libraries (both DNase and MNase digested) crosslinked with formaldehyde alone versus crosslinked with formaldehyde plus DSG or EGS. DSG has NHS ester reactive groups at both ends and can be reactive towards amino groups (e.g., primary amines). DSG is membrane-permeable, allowing for intracellular crosslinking. DSG can increase crosslinking efficiency compared to disuccinimidyl suberate (DSS) in some applications. EGS has NHS ester reactive groups at both ends and can be reactive towards amino groups (e.g., primary amines). EGS is membrane-permeable, allowing for intracellular crosslinking. EGS crosslinks can be reversed, for example, by treatment with hydroxylamine for 3 to 6 hours at pH 8.5; in an example, lactose dehydrogenase retained 60% of its activity after reversible crosslinking with EGS. In some cases, the chemical fixative may comprise psoralen. In some cases, the crosslinking agent may be ultraviolet light. In some cases, the stabilized biological sample may be a crosslinked paraffin-embedded tissue sample.

In further aspects, methods involving a MNase digestion step provided herein may comprise contacting the plurality of selected segments to an antibody. In some cases, an immunoglobulin binding protein or fragment thereof tethered to an oligonucleotide adaptor may be targeted to the antibody bound to a plurality of selected segments.

In additional aspects, methods involving a MNase digestion step provided herein may comprise attaching a first segment and a second segment of a plurality of segments at a junction. In some cases, attaching may comprise filling in sticky ends using biotin tagged nucleotides and ligating the blunt ends. In some cases, attaching may comprise contacting at least the first segment and the second segment to a bridge oligonucleotide. In some cases, attaching may comprise contacting at least the first segment and the second segment to a barcode. In some embodiments, bridge oligonucleotides herein may be from at least about 5 nucleotides in length to about 50 nucleotides in length. In some embodiments, bridge oligonucleotides herein may be from about 15 to about 18 nucleotides in length. In some embodiments, bridge oligonucleotides may be about 5, about 6, about 7, about 8, about 9, about 10, about 11, about 12, about 13, about 14, about 15, about 16, about 17, about 18, about 19, about 20, about 25, about 30, about 35, about 40, about 45, or about 50 nucleotides in length. In some embodiments, bridge oligonucleotides herein may comprise a barcode.

In further aspects of methods involving a MNase digestion step herein, methods can comprise obtaining at least some sequence on each side of the junction to generate a first read pair. For example, the methods may comprise obtaining at least about 50 bp, at least about 100 bp, at least about 150 bp, at least about 200 bp, at least about 250 bp, or at least about 300 bp of sequence on each side of the junction to generate a first read pair.

In additional aspects of methods involving a MNase digestion step herein, methods can comprise mapping the first read pair to a set of contigs, and determining a path through the set of contigs that represents an order and/or orientation to a genome.

In further aspects of methods involving a MNase digestion step herein, methods can comprise mapping the first read pair to a set of contigs; and determining, from the set of contigs, a presence of a structural variant or loss of heterozygosity in the stabilized biological sample.

In additional aspects of methods involving a MNase digestion step herein, methods can comprise mapping the first read pair to a set of contigs, and assigning a variant in the set of contigs to a phase.

In further aspects of methods involving a MNase digestion step herein, methods can comprise mapping the first read pair to a set of contigs; determining, from the set of contigs, a presence of a variant in the set of contigs, and conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

### Improved Methods for HiChIP, HiChIRP, and Methyl HiC

HiChIP is an approach combining methods of HiC with methods of chromatin immunoprecipitation, allowing targeted analysis of interactions involving one or more proteins of interest. A proximity ligated nucleic acid can be prepared, and targeted regions can be immunoprecipitated for further analysis. HiChIRP, a related approach, uses chromatin isolation by RNA purification (ChIRP) enrichment in combination with HiC methods, enabling the interrogation of RNAs, such as of the scaffolding function of long non-coding RNAs (lncRNAs). Methyl-HiC combines methylation analysis with HiC methods, allowing simultaneous capture of chromosome conformation and DNA methylome information. Methyl-HiC can reveal coordinated DNA methylation status between distal genomic segments that are in spatial proximity in the nucleus, delineate heterogeneity of both the chromatin architecture and DNA methylome in a mixed population, and enable simultaneous characterization of cell-type-specific chromatin organization and epigenome in complex tissues. These methods and other methods can be improved by use of the techniques of the present disclosure, including but not limited to size selection steps, surface binding steps (e.g., binding to a bead such as a SPRI bead), use of bridge oligonucleotides to conduct proximity ligation, use of recombination to conduct proximity ligation, and others.

In additional aspects, provided herein are improved methods for HiChIP, HiChIRP, and Methyl HiC that can comprise obtaining a stabilized biological sample comprising a nucleic acid molecule complexed to at least one nucleic acid binding protein, for example, by immunoprecipitation of nucleic acids bound to the nucleic acid binding protein or by immunoprecipitation of methylated nucleic acids; contacting the stabilized biological sample to a DNase to cleave the nucleic acid molecule into a plurality of segments; attaching a first segment and a second segment of the plurality of segments at a junction; and subjecting the plurality of segments to size selection to obtain a plurality of selected segments. Alternatively, or in combination, methods herein can comprise obtaining a stabilized biological sample comprising a nucleic acid molecule complexed to at least one nucleic acid binding protein, for example, by immunoprecipitation of nucleic acids bound to the nucleic acid binding protein or by immunoprecipitation of methylated nucleic acids; contacting the stabilized biological sample to a micrococcal nuclease (MNase) to cleave the nucleic acid molecule into a plurality of segments; and attaching a first segment and a second segment of the plurality of segments at a junction.

In some aspects of improved methods for HiChIP, HiChIRP, and Methyl HiC herein, the stabilized biological sample can comprise intact cells and/or intact nuclei. In some cases, the stabilized biological sample can comprise a stabilized intact cell. Alternatively, or in combination, the stabilized biological sample can comprise a stabilized intact nucleus. In some cases, contacting the stabilized intact cell or intact nucleus sample to a DNase may be conducted prior to lysis of the intact cell or the intact nucleus. In some cases, cells and/or nuclei may be lysed prior to attaching a first segment and a second segment of a plurality of segments at a junction.

In another aspect, methods involving improved methods for HiChIP, HiChIRP, and Methyl HiC herein can comprise subjecting a plurality of segments to size selection to obtain a plurality of selected segments. In some cases, the plurality of selected segments may be from about 145 to about 600 bp. In some cases, the plurality of selected segments may be from about 100 to about 2500 bp. In some cases, the plurality of selected segments may be from about 100 to about 600 bp. In some cases, the plurality of selected segments may be from about 600 to about 2500 bp. In some cases, the plurality of selected segments may be from about 100 bp to about 600 bp, from about 100 bp to about 700 bp, from about 100 bp to about 800 bp, from about 100 bp to about 900 bp, from about 100 bp to about 1000 bp, from about 100 bp to about 1100 bp, from about 100 bp to about 1200 bp, from about 100 bp to about 1300 bp, from about 100 bp to about 1400 bp, from about 100 bp to about 1500 bp, from about 100 bp to about 1600 bp, from about 100 bp to about 1700 bp, from about 100 bp to about 1800 bp, from about 100 bp to about 1900 bp, from about 100 bp to about 2000 bp, from about 100 bp to about 2100 bp, from about 100 bp to about 2200 bp, from about 100 bp to about 2300 bp, from about 100 bp to about 2400 bp, or from about 100 bp to about 2500 bp.

In another aspect of methods involving improved methods for HiChIP, HiChIRP, and Methyl HiC herein, the methods may further comprise, prior to a size selection step, preparing a sequencing library from the plurality of segments. In some embodiments, the method may further comprise subjecting the sequencing library to a size selection to obtain a size-selected library. In some cases, the size-selected library may be from about 350 bp to about 1000 bp in size. In some cases, the size-selected library may be from about 100 bp to about 2500 bp in size, for example, from about 100 bp to about 350 bp, from about 350 bp to about 500 bp, from about 500 bp to about 1000 bp, from about 1000 to about 1500 bp, from about 2000 bp to about 2500 bp, from about 350 bp to about 1000 bp, from about 350 bp to about 1500 bp, from about 350 bp to about 2000 bp, from about 350 bp to about 2500 bp, from about 500 bp to about 1500 bp, from about 500 bp to about 2000 bp, from about 500 bp to about 3500 bp, from about 1000 bp to about 1500 bp, from about 1000 bp to about 2000 bp, from about 1000 bp to about 2500 bp, from about 1500 bp to about 2000 bp, from about 1500 bp to about 2500 bp, or from about 2000 bp to about 2500 bp.

Size selection utilized in methods involving improved methods for HiChIP, HiChIRP and Methyl HiC herein can be conducted with gel electrophoresis, capillary electrophoresis, size selection beads, a gel filtration column, combinations thereof, or any other suitable method.

In another aspect, methods involving improved methods for HiChIP, HiChIRP, and Methyl HiC herein may comprise further analyzing the plurality of selected segments to obtain a QC value. In some cases, a QC value may be selected from a chromatin digest efficiency (CDE) and a chromatin digest index (CDI). A CDE can be calculated as the proportion of segments having a desired length. For example, in some cases, the CDE can be calculated as the proportion of segments from 100 to 2500 bp in size prior to size selection. In some cases, a sample may be selected for further analysis when the CDE value is at least 65%. In some cases, a sample may be selected for further analysis when the CDE value is at least about 50%, at least about 55%, at least about 60%, at least about 65%, at least about 70%, at least about 75%, at least about 80%, at least about 85%, at least about 90%, or at least about 95%.

A CDI can be calculated as a ratio of a number of mononucleosome-sized segments to a number of dinucleosome-sized segments prior to size selection. For example, a CDI may be calculated as a logarithm of the ratio of fragments having a size 600-2500 bp versus fragments having a size 100-600 bp. In some cases, a sample may be selected for further analysis when the CDI value is greater than -1.5 and less than 1. In some cases, a sample may be selected for further analysis when the CDI value is greater than about -2 and less than about 1.5, greater than about -1.9 and less than about 1.5, greater than about - 1.8 and less than about 1.5, greater than about -1.7 and less than about 1.5, greater than about -1.6 and less than about 1.5, greater than about -1.5 and less than about 1.5, greater than about -1.4 and less than about 1.5, greater than about -1.3 and less than about 1.5, greater than about -1.2 and less than about 1.5, greater than about -1.1 and less than about 1.5, greater than about -2 and less than about 1.5, greater than about -1 and less than about 1.5, greater than about -0.9 and less than about 1.5, greater than about -0.8 and less than about 1.5, greater than about -0.7 and less than about 1.5, greater than about -0.6 and less than about 1.5, greater than about -0.5 and less than about 1.5, greater than about -2 and less than about 1.4, greater than about -2 and less than about 1.3, greater than about -2 and less than about 1.2, greater than about -2 and less than about 1.1, greater than about -2 and less than about 1, greater than about -2 and less than about 0.9, greater than about -2 and less than about 0.8, greater than about -2 and less than about 0.7, greater than about -2 and less than about 0.6, or greater than about -2 and less than about 0.5.

In another aspect, methods involving improved methods for HiChIP, HiChIRP, and Methyl HiC herein can be conducted on small samples containing few cells or small amounts of nucleic acid. In some cases, the stabilized biological sample may comprise fewer than 3,000,000 cells. In some cases, the stabilized biological sample may comprise fewer than 2,000,000 cells. In some cases, the stabilized biological sample may comprise fewer than 1,000,000 cells. In some cases, the stabilized biological sample may comprise fewer than 500,000 cells. In some cases, the stabilized biological sample may comprise fewer than 400,000 cells. In some cases, the stabilized biological sample may comprise fewer than 300,000 cells. In some cases, the stabilized biological sample may comprise fewer than 200,000 cells. In some cases, the stabilized biological sample may comprise fewer than 100,000 cells. In some cases, the stabilized biological sample may comprise less than 10 µg DNA. In some cases, the stabilized biological sample may comprise less than 9 µg DNA. In some cases, the stabilized biological sample may comprise less than 8 µg DNA. In some cases, the stabilized biological sample may comprise less than 7 µg DNA. In some cases, the stabilized biological sample may comprise less than 6 µg DNA. In some cases, the stabilized biological sample may comprise less than 5 µg DNA. In some cases, the stabilized biological sample may comprise less than 4 µg DNA. In some cases, the stabilized biological sample may comprise less than 3 µg DNA. In some cases, the stabilized biological sample may comprise less than 2 µg DNA. In some cases, the stabilized biological sample may comprise less than 1 µg DNA. In some cases, the stabilized biological sample may comprise less than 0.5 µg DNA.

In another aspect, methods involving improved methods for HiChIP, HiChIRP, and Methyl HiC herein may be conducted on individual or single cells. For example, methods herein may be conducted on cells distributed into individual partitions. Exemplary partitions include, but are not limited to, wells, droplets in an emulsion, or surface positions (e.g., array spots, beads, etc.) comprising distinct patches of differentially sequenced linker molecules as described elsewhere herein. Additional partitions are also contemplated and consistent with the methods, compositions, and systems disclosed herein.

In additional aspects, stabilized biological samples used in methods involving improved methods for HiChIP, HiChIRP, and Methyl HiC herein can be treated with a nuclease, such as a DNase, to create fragments of DNA. In some cases, the DNase may be a non-sequence specific. In some cases, the DNase may be active for both single-stranded DNA and double-stranded DNA. In some cases, the DNase may be specific for double-stranded DNA. In some cases, the DNase may preferentially cleave double-stranded DNA. In some cases, the DNase may be specific for single-stranded DNA. In some cases, the DNase may preferentially cleave single-stranded DNA. In some cases, the DNase may be DNase I. In some cases, the DNase may be DNase II. In some cases, the DNase may be selected from one or more of DNase I and DNase II. In some cases, the DNase may be micrococcal nuclease. In some cases, the DNase may be selected from one or more of DNase I, DNase II, and micrococcal nuclease. In some cases, the DNase may be coupled or fused to an immunoglobulin binding protein or fragment thereof, such as a Protein A, a Protein G, a Protein A/G, or a Protein L. Other suitable nucleases are also within the scope of this disclosure.

In additional aspects, stabilized biological samples used in methods involving improved methods for HiChIP, HiChIRP, and Methyl HiC herein may be treated with a crosslinking agent. In some cases, the crosslinking agent may be a chemical fixative. In some cases, the chemical fixative comprises formaldehyde, which has a spacer arm length of about 2.3-2.7 angstrom (A). In some cases, the chemical fixative comprises a crosslinking agent with a long spacer arm length, For example, the crosslinking agent can have a spacer length of at least about 3 A, 4 A, 5 A, 6 A, 7 A, 8 A, 9 A, 10 A, 11 A, 12 A, 13 A, 14 A, 15 A, 16 A, 17 A, 18 A, 19 A, or 20 A. The chemical fixative can comprise ethylene glycol bis(succinimidyl succinate) (EGS), which has a spacer arm with length about 16.1 A. The chemical fixative can comprise disuccinimidyl glutarate (DSG), which has a spacer arm with length about 7.7 A. In some cases, the chemical fixative comprises formaldehyde and EGS, formaldehyde and DSG, or formaldehyde, EGS, and DSG. In some cases where multiple chemical fixatives are employed, each chemical fixative is used sequentially; in other cases, some or all of the multiple chemical fixatives are applied to the sample at the same time. The use of crosslinkers with long spacer arms can increase the fraction of read pairs with large (e.g., > 1 kb) read pair separation distances. For example, FIG. 7 shows a comparison of resulting libraries (both DNase and MNase digested) crosslinked with formaldehyde alone versus crosslinked with formaldehyde plus DSG or EGS. DSG has NHS ester reactive groups at both ends and can be reactive towards amino groups (e.g., primary amines). DSG is membrane-permeable, allowing for intracellular crosslinking. DSG can increase crosslinking efficiency compared to disuccinimidyl suberate (DSS) in some applications. EGS has NHS ester reactive groups at both ends and can be reactive towards amino groups (e.g., primary amines). EGS is membrane-permeable, allowing for intracellular crosslinking. EGS crosslinks can be reversed, for example, by treatment with hydroxylamine for 3 to 6 hours at pH 8.5; in an example, lactose dehydrogenase retained 60% of its activity after reversible crosslinking with EGS. In some cases, the chemical fixative may comprise psoralen. In some cases, the crosslinking agent may be ultraviolet light. In some cases, the stabilized biological sample may be a crosslinked paraffin-embedded tissue sample.

In additional aspects, methods involving improved methods for HiChIP, HiChIRP, and Methyl HiC herein may comprise attaching a first segment and a second segment of a plurality of segments at a junction. In some cases, attaching can comprise filling in sticky ends using biotin tagged nucleotides and ligating the blunt ends. In some cases, attaching can comprise contacting at least the first segment and the second segment to a bridge oligonucleotide. In some cases, attaching can comprise contacting at least the first segment and the second segment to a barcode. In some embodiments, bridge oligonucleotides herein may be from at least about 5 nucleotides in length to about 50 nucleotides in length. In some embodiments, bridge oligonucleotides herein may be from about 15 to about 18 nucleotides in length. In some embodiments, bridge oligonucleotides may be about 5, about 6, about 7, about 8, about 9, about 10, about 11, about 12, about 13, about 14, about 15, about 16, about 17, about 18, about 19, about 20, about 25, about 30, about 35, about 40, about 45, or about 50 nucleotides in length. In some embodiments, bridge oligonucleotides herein may comprise a barcode.

In additional aspects, methods involving improved methods for HiChIP, HiChIRP, and Methyl HiC herein may not comprise a shearing step.

In further aspects of methods involving improved methods for HiChIP, HiChIRP, and Methyl HiC herein, methods may comprise obtaining at least some sequence on each side of the junction to generate a first read pair. For example, the methods may comprise obtaining at least about 50 bp, at least about 100 bp, at least about 150 bp, at least about 200 bp, at least about 250 bp, or at least about 300 bp of sequence on each side of the junction to generate a first read pair.

In additional aspects of methods involving improved methods for HiChIP, HiChIRP, and Methyl HiC herein, methods may comprise mapping the first read pair to a set of contigs and determining a path through the set of contigs that represents an order and/or orientation to a genome.

In further aspects of methods involving improved methods for HiChIP, HiChIRP, and Methyl HiC herein, methods may comprise mapping the first read pair to a set of contigs; and determining, from the set of contigs, a presence of a structural variant or loss of heterozygosity in the stabilized biological sample.

In additional aspects of methods involving digestion of whole cells or whole nuclei herein, methods may comprise mapping the first read pair to a set of contigs and assigning a variant in the set of contigs to a phase.

In further aspects of methods involving improved methods for HiChIP, HiChIRP, and Methyl HiC herein, methods may comprise mapping the first read pair to a set of contigs; determining, from the set of contigs, a presence of a variant in the set of contigs; and conducting a step selected from one or more of: (1) identifying a disease stage, a prognosis, or a course of treatment for the stabilized biological sample; (2) selecting a drug based on the presence of the variant; or (3) identifying a drug efficacy for the stabilized biological sample.

### Generating Long-Range Read Pairs

The disclosure provides methods for generating extremely long-range read pairs and to utilize that data for the advancement of all of the aforementioned pursuits. In some embodiments, the disclosure provides methods that produce a highly contiguous and accurate human genomic assembly with only ~300 million read pairs. In other embodiments, the disclosure provides methods that phase 90% or more of heterozygous variants in a human genome with 99% or greater accuracy. Further, the range of the read pairs generated by the disclosure can be extended to span much larger genomic distances. The assembly is produced from a standard shotgun library in addition to an extremely long-range read pair library. In yet other embodiments, the disclosure provides software that is capable of utilizing both of these sets of sequencing data. Phased variants are produced with a single long-range read pair library, the reads from which are mapped to a reference genome and then used to assign variants to one of the individual's two parental chromosomes. Finally, the disclosure provides for the extraction of even larger DNA fragments using known techniques, so as to generate exceptionally long reads.

The mechanism by which these repeats obstruct assembly and alignment processes is fairly straightforward and is ultimately a consequence of ambiguity. In the case of large repetitive regions, the difficulty can be one of span. If a read or read pair is not long enough to span a repetitive region, one may not be able to confidently connect regions bordering the repetitive element. In the case of smaller repetitive elements, the problem can be primarily placement. When a region is flanked by two repetitive elements that are common in the genome, determining its exact placement becomes difficult, if not impossible, due to the similarity of the flanking elements to all others of their class. In both cases, it is the lack of distinguishing information in the repeat that makes the identification, and thus placement of a particular repeat, challenging. What is needed is the ability to experimentally establish connection between unique segments hemmed or separated by repetitive regions.

The methods of the disclosure advance the field of genomics by overcoming the substantial barriers posed by these repetitive regions, and can thereby enable important advances in many domains of genomic analysis. To perform a de novo assembly with previous technologies, one must either settle for an assembly fragmented into many small scaffolds or commit substantial time and resources to producing a large-insert library or using other approaches to generate a more contiguous assembly. Such approaches may include acquiring very deep sequencing coverage, constructing BAC or fosmid libraries, optical mapping, or, some combination of these and/or other techniques. The intense resource and time requirements put such approaches out of reach for most small labs and prevents studying non-model organisms. Since the methods described herein can produce very long-range read pairs, de novo assembly can be achieved with a single sequencing run. This would cut assembly costs by orders of magnitude and shorten the time required from months or years to weeks. In some cases, the methods disclosed herein allow for generating a plurality of read-pairs in less than 14 days, less than 13 days, less than 12 days, less than 11 days, less than 10 days, less than 9 days, less than 8 days, less than 7 days, less than 6 days, less than 5 days, less than 4 days, or in a range between any two of foregoing specified time periods. For example, the methods can allow for generating a plurality of read-pairs in about 10 days to 14 days. Building genomes for even the most niche of organisms would become routine, phylogenetic analyses would suffer no lack of comparisons, and projects such as Genome 10k could be realized.

Similarly, structural and phasing analyses for medical purposes also remain challenging. There is astounding heterogeneity among cancers, individuals with the same type of cancer, or even within the same tumor. Teasing out the causative from consequential effects requires very high precision and throughput at a low per-sample cost. In the domain of personalized medicine, one of the gold standards of genomic care is a sequenced genome with all variants thoroughly characterized and phased, including large and small structural rearrangements and novel mutations. To achieve this with previous technologies demands effort akin to that required for a de novo assembly, which is currently too expensive and laborious to be a routine medical procedure. The disclosed methods can rapidly produce complete, accurate genomes at low cost and can thereby yield many highly sought capabilities in the study and treatment of human disease.

Applying the methods disclosed herein to phasing can combine the convenience of statistical approaches with the accuracy of familial analysis, providing savings - money, labor, and samples - than using either method alone. De novo variant phasing, a highly desirable phasing analysis that is prohibitive with previous technologies, can be performed readily using the methods disclosed herein. This is particularly important as the vast majority of human variation is rare (less than 5% minor allele frequency). Phasing information is valuable for population genetic studies that gain significant advantages from networks of highly connected haplotypes (collections of variants assigned to a single chromosome), relative to unlinked genotypes. Haplotype information can enable higher resolution studies of historical changes in population size, migrations, and exchange between subpopulations, and allows us to trace specific variants back to particular parents and grandparents. This in turn clarifies the genetic transmission of variants associated with disease, and the interplay between variants when brought together in a single individual. The methods of the disclosure can eventually enable the preparation, sequencing, and analysis of extremely long range read pair (XLRP) libraries.

In some embodiments of the disclosure, a tissue or a DNA sample from a subject can be provided and the method can return an assembled genome, alignments with called variants (including large structural variants), phased variant calls, or any additional analyses. In other embodiments, the methods disclosed herein can provide XLRP libraries directly for the individual.

### Extremely Long-Range Read Pairs

In various embodiments of the disclosure, the methods disclosed herein can generate extremely long-range read pairs separated by large distances. The upper limit of this distance may be improved by the ability to collect DNA samples of large size. In some cases, the read pairs can span up to50, 60, 70, 80, 90, 100, 125, 150, 175, 200, 225, 250, 300, 400, 500, 600, 700, 800, 900, 1000, 1500, 2000, 2500, 3000, 4000, 5000 kbp or more in genomic distance. In some examples, the read pairs can span up to 500 kbp in genomic distance. In other examples, the read pairs can span up to 2000 kbp in genomic distance. The methods disclosed herein can integrate and build upon standard techniques in molecular biology, and are further well-suited for increases in efficiency, specificity, and genomic coverage. In some cases, the read pairs can be generated in less than about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 60, or 90 days. In some examples, the read pairs can be generated in less than about 14 days. In further examples, the read pairs can be generated in less about 10 days. In some cases, the methods of the present disclosure can provide greater than about 5%, about 10%, about 15 %, about 20%, about 30%, about 40%, about 50%, about 60%, about 70%, about 80%, about 90%, about 95%, about 99%, or about 100% of the read pairs with at least about 50%, about 60%, about 70%, about 80%, about 90%, about 95%, about 99%, or about 100% accuracy in correctly ordering and/or orientating the plurality of contigs. For example, the methods can provide about 90 to 100% accuracy in correctly ordering and/or orientating the plurality of contigs.

In other embodiments, the methods disclosed herein can be used with currently employed sequencing technology. For example, the methods can be used in combination with well-tested and/or widely deployed sequencing instruments. In further embodiments, the methods disclosed herein can be used with technologies and approaches derived from currently employed sequencing technology.

The methods of the disclosure dramatically simplify de novo genomic assembly for a wide range of organisms. Using previous technologies, such assemblies are currently limited by the short inserts of economical mate-pair libraries. While it may be possible to generate read pairs at genomic distances up to the 40-50 kbp accessible with fosmids, these are expensive, cumbersome, and too short to span the longest repetitive stretches, including those within centromeres, which - in humans - can range in size from 300 kbp to 5 Mbp. The methods disclosed herein can provide read pairs capable of spanning large distances (e.g., megabases or longer) and thereby overcome these scaffold integrity challenges. Accordingly, producing chromosome-level assemblies can be routine by utilizing the methods of the disclosure. More laborious avenues for assembly - currently costing research labs incredible amounts of time and money, and prohibiting expansive genomic catalogs - may become unnecessary, freeing up resources for more meaningful analyses. Similarly, the acquisition of long-range phasing information can provide tremendous additional power to population genomic, phylogenetic, and disease studies. The methods disclosed herein enable accurate phasing for large numbers of individuals, thus extending the breadth and depth of our ability to probe genomes at the population and deep-time levels.

In the realm of personalized medicine, the XLRP read pairs generated from the methods disclosed herein represent a meaningful advance toward accurate, low-cost, phased, and rapidly produced personal genomes. Current methods are insufficient in their ability to phase variants at long distances, thereby preventing the characterization of the phenotypic impact of compound heterozygous genotypes. Additionally, structural variants of substantial interest for genomic diseases are difficult to accurately identify and characterize with current techniques due to their large size in comparison to reads and read pair inserts used to study them. Read pairs spanning tens of kilobases to megabases or longer can help alleviate this difficulty, thereby allowing for highly parallel and personalized analyses of structural variation.

Basic evolutionary and biomedical research is being driven by technological advances in high-throughput sequencing. Whereas whole genome sequencing and assembly used to be the provenance of large genome sequencing centers, commercially available sequencers are now inexpensive enough that most research universities have one or several of these machines. It is now relatively inexpensive to generate massive quantities of DNA sequence data. However, it remains difficult in theory and in practice to produce high-quality, highly contiguous genome sequences with current technology. Furthermore, because most organisms that one would care to analyze, including humans, are diploid, each individual has two haploid copies of the genome. At sites of heterozygosity (e.g., where the allele given by the mother differs from the allele given by the father), it is difficult to know which sets of alleles came from which parent (known as haplotype phasing). This information can be used for performing a number of evolutionary and biomedical studies such as disease and trait association studies.

In various embodiments, the disclosure provides methods for genome assembly that combine technologies for DNA preparation with paired-end sequencing for high-throughput discovery of short, intermediate and long-term connections within a given genome. The disclosure further provides methods using these connections to assist in genome assembly, for haplotype phasing, and/or for metagenomic studies. While the methods presented herein can be used to determine the assembly of a subject's genome, it should also be understood that the methods presented herein can also be used to determine the assembly of portions of the subject's genome such as chromosomes, or the assembly of the subject's chromatin of varying lengths.

In some embodiments, the disclosure provides for one or more methods disclosed herein that comprise the step of generating a plurality of contigs from sequencing fragments of target DNA obtained from a subject. Long stretches of target DNA can be fragmented by cutting the DNA with one or more nucleases (e.g., DNase I, DNase II, micrococcal nuclease, etc.). The resulting fragments can be sequenced using high-throughput sequencing methods to obtain a plurality of sequencing reads. Examples of high-throughput sequencing methods which can be used with the methods of the disclosure include, but are not limited to, 454 pyrosequencing methods developed Roche Diagnostics, "clusters" sequencing methods developed by Illumina, SOLiD and Ion semiconductor sequencing methods developed by Life Technologies, and DNA nanoball sequencing methods developed by Complete Genomics. Overlapping ends of different sequencing reads can then be assembled to form a contig. Alternatively, fragmented target DNA can be cloned into vectors. Cells or organisms are then transfected with the DNA vectors to form a library. After replicating the transfected cells or organisms, the vectors are isolated and sequenced to generate a plurality of sequencing reads. The overlapping ends of different sequencing reads can then be assembled to form a contig.

Genome assembly, especially with high-throughput sequencing technology, can be problematic. Often, the assembly consists of thousands or tens of thousands of short contigs. The order and orientation of these contigs is generally unknown, limiting the usefulness of the genome assembly. Technologies exist to order and orient these scaffolds, but they are generally expensive, labor intensive, and often fail in discovering very long-range interactions.

Samples comprising target DNA used to generate contigs can be obtained from a subject by any number of means, including by taking bodily fluids (e.g., blood, urine, serum, lymph, saliva, buccal swab, anal and vaginal secretions, perspiration and semen, etc.), taking tissue, or by collecting cells/organisms. The sample obtained may be comprised of a single type of cell/organism, or may be comprised multiple types of cells/organisms. The DNA can be extracted and prepared from the subject's sample. For example, the sample may be treated to lyse a cell comprising the polynucleotide, using known lysis buffers, sonication techniques, electroporation, and the like. The target DNA may be further purified to remove contaminants, such as proteins, by using alcohol extractions, cesium gradients, and/or column chromatography.

In other embodiments of the disclosure, a method to extract very high molecular weight DNA is provided. In some cases, the data from an XLRP library can be improved by increasing the fragment size of the input DNA. In some examples, extracting megabase-sized fragments of DNA from a cell can produce read pairs separated by megabases in the genome. In some cases, the produced read-pairs can provide sequence information over a span of greater than about 10 kB, about 50 kB, about 100 kB, about 200 kB, about 500 kB, about 1 Mb, about 2 Mb, about 5 Mb, about 10 Mb, or about 100 Mb. In some examples, the read-pairs can provide sequence information over a span of greater than about 500 kB. In further examples, the read-pairs can provide sequence information over a span of greater than about 2 Mb. In some cases, the very high molecular weight DNA can be extracted by very gentle cell lysis (Teague, B. et al. (2010) Proc. Nat. Acad. Sci. USA 107(24), 10848-53) and agarose plugs (Schwartz, D. C., & Cantor, C. R. (1984) Cell, 37(1), 67-75). In other cases, commercially available machines that can purify DNA molecules up to megabases in length can be used to extract very high molecular weight DNA.

### Probing Physical Layout of Chromosomes

In various embodiments, the disclosure provides for one or more methods disclosed herein that comprise the step of probing the physical layout of chromosomes within living cells. Examples of techniques to probe the physical layout of chromosomes through sequencing include the "C" family of techniques, such as chromosome conformation capture ("3C"), circularized chromosome conformation capture ("4C"), carbon-copy chromosome capture ("5C"), and Hi-C based methods; and ChIP based methods, such as ChIP-loop, ChIA-PET, and HiChIP. These techniques utilize the fixation of chromatin in live cells to cement spatial relationships in the nucleus. Subsequent processing and sequencing of the products allows a researcher to recover a matrix of proximate associations among genomic regions. With further analysis these associations can be used to produce a three-dimensional geometric map of the chromosomes as they are physically arranged in live nuclei. Such techniques describe the discrete spatial organization of chromosomes in live cells, and provide an accurate view of the functional interactions among chromosomal loci. One issue that plagued these functional studies was the presence of nonspecific interactions, associations present in the data that are attributable to nothing more than chromosomal proximity. In the disclosure, these nonspecific intrachromosomal interactions are captured by the methods presented herein so as to provide valuable information for assembly.

In some embodiments, the intrachromosomal interactions correlate with chromosomal connectivity. In some cases, the intrachromosomal data can aid genomic assembly. In some cases, the chromatin is reconstructed in vitro. This can be advantageous because chromatin - particularly histones, the major protein component of chromatin - is important for fixation under the most common "C" family of techniques for detecting chromatin conformation and structure through sequencing: 3C, 4C, 5C, and Hi-C. Chromatin is highly non-specific in terms of sequence and will generally assemble uniformly across the genome. In some cases, the genomes of species that do not use chromatin can be assembled on a reconstructed chromatin and thereby extend the horizon for the disclosure to all domains of life.

A chromatin conformation capture technique is summarized. In brief, cross-links are created between genome regions that are in close physical proximity. Crosslinking of proteins (such as histones) to the DNA molecule, e.g., genomic DNA, within chromatin can be accomplished according to a suitable method described in further detail elsewhere herein or otherwise known in the art. In some cases, two or more nucleotide sequences can be cross-linked via proteins bound to one or more nucleotide sequences. One approach is to expose the chromatin to ultraviolet irradiation (Gilmour et al., Proc. Nat'l. Acad. Sci. USA 81:4275-4279, 1984). Crosslinking of polynucleotide segments may also be performed utilizing other approaches, such as chemical or physical (e.g., optical) crosslinking. Suitable chemical crosslinking agents include, but are not limited to, formaldehyde and psoralen (Solomon et al., Proc. Nat'l. Acad. Sci. USA 82:6470-6474, 1985; Solomon et al., Cell 53:937-947, 1988). For example, cross-linking can be performed by adding 2% formaldehyde to a mixture comprising the DNA molecule and chromatin proteins. Other examples of agents that can be used to cross-link DNA include, but are not limited to, UV light, mitomycin C, nitrogen mustard, melphalan, 1,3-butadiene diepoxide, cis diamminedichloroplatinum(II) and cyclophosphamide. Suitably, the cross-linking agent will form cross-links that bridge relatively short distances-such as about 2 A-thereby selecting intimate interactions that can be reversed.

In some embodiments, the DNA molecule may be immunoprecipitated prior to or after crosslinking. In some cases, the DNA molecule can be fragmented. Fragments may be contacted with a binding partner, such as an antibody that specifically recognizes and binds to acetylated histones, e.g., H3. Examples of such antibodies include, but are not limited to, Anti Acetylated Histone H3, available from Upstate Biotechnology, Lake Placid, N.Y. The polynucleotides from the immunoprecipitate can subsequently be collected from the immunoprecipitate. Prior to fragmenting the chromatin, the acetylated histones can be crosslinked to adjacent polynucleotide sequences. The mixture is then treated to fractionate polynucleotides in the mixture. Fractionation techniques herein comprise use of deoxyribonuclease (DNase) enzymes. DNases suitable for methods herein include, but are not limited to, DNase I, DNase II, and micrococcal nuclease. The resulting fragments can vary in size. The resulting fragments may also comprise a single-stranded overhand at the 5' or 3' end.

In some embodiments, fragments of about 145 bp to about 600 bp can be obtained. Alternatively, fragments of about 100 bp to about 2500 bp, about 100 bp to about 600 bp, or about 600 to about 2500 can be obtained. The sample can be prepared for sequencing of coupled sequence segments that are cross-linked. In some cases, a single, short stretch of polynucleotide can be created, for example, by ligating two sequence segments that were intramolecularly crosslinked. Sequence information may be obtained from the sample using any suitable sequencing technique described in further detail elsewhere herein or other suitable methods, such as a high-throughput sequencing method. For example, ligation products can be subjected to paired-end sequencing obtaining sequence information from each end of a fragment. Pairs of sequence segments can be represented in the obtained sequence information, associating haplotyping information over a linear distance separating the two sequence segments along the polynucleotide.

One feature of the data generated by Hi-C is that most reads pairs, when mapped back to the genome, are found to be in close linear proximity. That is, most read pairs are found to be close to one another in the genome. In the resulting data sets, the probability of intrachromosomal contacts is on average much higher than that of interchromosomal contacts, as expected if chromosomes occupy distinct territories. Moreover, although the probability of interaction decays rapidly with linear distance, even loci separated by > 200 Mb on the same chromosome are more likely to interact than loci on different chromosomes. In detecting long-range intra-chromosomal and especially inter-chromosomal contacts, this "background" of short and intermediate range intra-chromosomal contacts is background noise to be factored out using Hi-C analysis.

Notably, Hi-C experiments in eukaryotes have shown, in addition to species-specific and cell type-specific chromatin interactions, two canonical interaction patterns. One pattern, distance-dependent decay (DDD), is a general trend of decay in interaction frequency as a function of genomic distance. The second pattern, cis-trans ratio (CTR), is a significantly higher interaction frequency between loci located on the same chromosome, even when separated by tens of megabases of sequence, versus loci on different chromosomes. These patterns may reflect general polymer dynamics, where proximal loci have a higher probability of randomly interacting, as well as specific nuclear organization features such as the formation of chromosome territories, the phenomenon of interphase chromosomes tending to occupy distinct volumes in the nucleus with little mixing. Although the exact details of these two patterns may vary between species, cell types and cellular conditions, they are ubiquitous and prominent. These patterns are so strong and consistent that they are used to assess experiment quality and are usually normalized out of the data in order to reveal detailed interactions. However, in the methods disclosed herein, genome assembly can take advantage of the three-dimensional structure of genomes. Features which make the canonical Hi-C interaction patterns a hindrance for the analysis of specific looping interactions, namely their ubiquity, strength and consistency, can be used as powerful tool for estimating the genomic position of contigs.

In a particular implementation, examination of the physical distance between intra-chromosomal read pairs indicates several useful features of the data with respect to genome assembly. First, shorter range interactions are more common than longer-range interactions. That is, each read of a read-pair is more likely to be mated with a region close by in the actual genome than it is to be with a region that is far away. Second, there is a long tail of intermediate and long-range interactions. That is, read-pairs carry information about intra-chromosomal arrangement at kilobase (kB) or even megabase (Mb) distances. For example, read-pairs can provide sequence information over a span of greater than about 10 kB, about 50 kB, about 100 kB, about 200 kB, about 500 kB, about 1 Mb, about 2 Mb, about 5 Mb, about 10 Mb, or about 100 Mb. These features of the data simply indicate that regions of the genome that are nearby on the same chromosome are more likely to be in close physical proximity - an expected result because they are chemically linked to one another through the DNA backbone. It was speculated that genome-wide chromatin interaction data sets, such as those generated by Hi-C, would provide long-range information about the grouping and linear organization of sequences along entire chromosomes.

Although the experimental methods for Hi-C are straightforward and relatively low cost, current protocols for genome assembly and haplotyping require 3-5 million cells, a fairly large amount of material that may not be feasible to obtain, particularly from certain human patient samples. By contrast, the methods disclosed herein include methods that allow for accurate and predictive results for genotype assembly, haplotype phasing, and metagenomics with significantly less material from cells. For example, less than about 0.1 µg, about 0.2 µg, about 0.3 µg, about 0.4 µg, about 0.5 µg, about 0.6 µg, about 0.7 µg, about 0.8 µg, about 0.9 µg, about 1.0 µg, about 1.2 µg, about 1.4 µg, about 1.6 µg, about 1.8 µg, about 2.0 µg, about 2.5 µg, about 3.0 µg, about 3.5 µg, about 4.0 µg, about 4.5 µg, about 5.0 µg, about 6.0 µg, about 7.0 µg, about 8.0 µg, about 9.0 µg, about 10 µg, about 15 µg, about 20 µg, about 30 µg, about 40 µg, about 50 µg, about 60 µg, about 70 µg, about 80 µg, about 90 µg, about 100 µg, about 150 µg, about 200 µg, about 300 µg, about 400 µg, about 500 µg, about 600 µg, about 700 µg, about 800 µg, about 900 µg, about 1000 µg, about 1200 µg, about 1400 µg, about 1600 µg, about 1800 µg, about 2000 µg, about 2200 µg, about 2400 µg, about 2600 µg, about 2800 µg, about 3000 µg, about 3200 µg, about 3400 µg, about 3600 µg, about 3800 µg, about 4000 µg, about 4200 µg, about 4400 µg, about 4600 µg, about 4800 µg, about 5000 µg, about 5200 µg, about 5400 µg, about 5600 µg, about 5800 µg, about 6000 µg, about 6200 µg, about 6400 µg, about 6600 µg, about 6800 µg, about 7000 µg, about 7200 µg, about 7400 µg, about 7600 µg, about 7800 µg, about 8000 µg, about 8200 µg, about 8400 µg, about 8600 µg, about 8800 µg, about 9000 µg, about 9200 µg, about 9400 µg, about 9600 µg, about 9800 µg, or about 10,000 µg of DNA can be used with the methods disclosed herein. In some examples, the DNA used in the methods disclosed herein can be extracted from less than about 3,000,000, about 2,500,000, about 2,000,000, about 1,500,000, about 1,000,000, about 500,000, about 100,000, about 50,000, about 10,000, about 5,000, about 1,000, about 500, or about 100 cells.

Universally, procedures for probing the physical layout of chromosomes, such as Hi-C based techniques, utilize chromatin that is formed within a cell/organism, such as chromatin isolated from cultured cells or primary tissue. The disclosure provides not only for the use of such techniques with chromatin isolated from a cell/organism but also with reconstituted chromatin. Reconstituted chromatin is differentiated from chromatin formed within a cell/organism over various features. First, for many samples, the collection of naked DNA samples can be achieved by using a variety of noninvasive to invasive methods, such as by collecting bodily fluids, swabbing buccal or rectal areas, taking epithelial samples, etc. Second, reconstituting chromatin substantially prevents the formation of inter-chromosomal and other long-range interactions that generate artifacts for genome assembly and haplotype phasing. In some cases, a sample may have less than about 20, 15, 12, 11, 10, 9, 8, 7, 6, 5, 4, 3, 2, 1, 0.5, 0.4, 0.3, 0.2, 0.1% or less inter-chromosomal or intermolecular crosslinking according to the methods and compositions of the disclosure. In some examples, the sample may have less than about 5% inter-chromosomal or intermolecular crosslinking. In some examples, the sample may have less than about 3% inter-chromosomal or intermolecular crosslinking. In further examples, may have less than about 1% inter-chromosomal or intermolecular crosslinking. Third, the frequency of sites that are capable of crosslinking and thus the frequency of intramolecular crosslinks within the polynucleotide can be adjusted. For example, the ratio of DNA to histones can be varied, such that the nucleosome density can be adjusted to a desired value. In some cases, the nucleosome density is reduced below the physiological level. Accordingly, the distribution of crosslinks can be altered to favor longer-range interactions. In some embodiments, sub-samples with varying cross-linking density may be prepared to cover both short- and long-range associations. For example, the crosslinking conditions can be adjusted such that at least about 1%, about 2%, about 3%, about 4%, about 5%, about 6%, about 7%, about 8%, about 9%, about 10%, about 11%, about 12%, about 13%, about 14%, about 15%, about 16%, about 17%, about 18%, about 19%, about 20%, about 25%, about 30%, about 40%, about 45%, about 50%, about 60%, about 70%, about 80%, about 90%, about 95%, or about 100% of the crosslinks occur between DNA segments that are at least about 50 kb, about 60 kb, about 70 kb, about 80 kb, about 90 kb, about 100 kb, about 110 kb, about 120 kb, about 130 kb, about 140 kb, about 150 kb, about 160 kb, about 180 kb, about 200 kb, about 250 kb, about 300 kb, about 350 kb, about 400 kb, about 450 kb, or about 500 kb apart on the sample DNA molecule.

### Contact Mapping and Topology

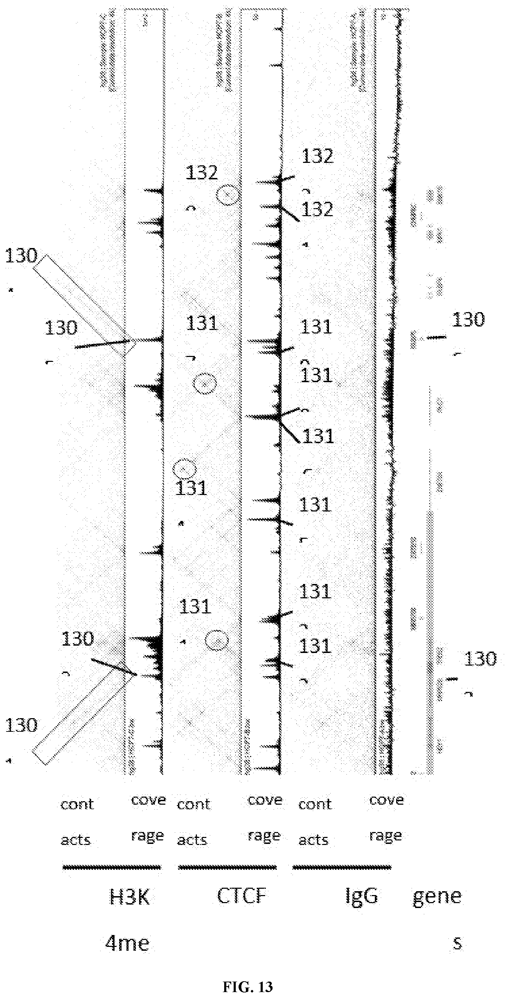

Read pairs generated by methods of the present disclosure can be used to analyze the three-dimensional structure of a genome and of chromosomes and nucleic acid molecules therein. As discussed herein, each read in a read pair can be mapped to different regions in the genome. It can be inferred that, for a given read pair, the two different regions in the genome that they map to would have been in spatial proximity to each other, in order to be able to be ligated together. By plotting read pairs from a sample according to the coordinates of both reads in the read pair, a contact map can be created for the sample. Example contact maps can be seen in **FIG. 13****,** where each point on the contact map represents a read pair plotted according to the mapped locations of its read pairs.

Analysis of contacts throughout a sample can allow analysis of the structure of chromosomes and genomes. The organization of a genome into A and B compartments, active and inactive compartments, chromosomal compartments, euchromatin and heterochromatin, topologically-associating domains (TADs) including TAD subtypes, and other structures, can be analyzed, on scales as large as kilobase- or megabase-scale. Analysis of contact maps can also allow detection of genomic features such as structural variants such as rearrangements, translocations, copy number variations, inversions, deletions, and insertions.

Methods of the present disclosure can provide locations of protein binding, structural variation, or genome contact interactions at a resolution of less than or equal to about 1 bp, 2 bp, 3 bp, 4 bp, 5 bp, 6 bp, 7 bp, 8 bp, 9 bp, 10 bp, 20 bp, 30 bp, 40 bp, 50 bp, 60 bp, 70 bp, 80 bp, 90 bp, 100 bp, 200 bp, 300 bp, 400 bp, 500 bp, 600 bp, 700 bp, 800 bp, 900 bp, 1000 bp, 2000 bp, 3000 bp, 4000 bp, 5000 bp, 6000 bp, 7000 bp, 8000 bp, 9000 bp, 10 kb, 20 kb, 30 kb, 40 kb, 50 kb, 60 kb, 70 kb, 80 kb, 90 kb, or 100 kb. In some cases, protein binding sites, protein footprints, contact interactions, or other features can be mapped to within 1000 bp, within 900 bp, within 800 bp, within 700 bp, within 600 bp, within 500 bp, within 400 bp, within 300 bp, within 200 bp, within 190 bp, within 180 bp, within 170 bp, within 160 bp, within 150 bp, within 140 bp, within 130 bp, within 120 bp, within 110 bp, within 100 bp, within 90 bp, within 80 bp, within 70 bp, within 60 bp, within 50 bp, within 40 bp, within 30 bp, within 20 bp, within 10 bp, within 9 bp, within 8 bp, within 7 bp, within 6 bp, within 5 bp, within 4 bp, within 3 bp, within 2 bp, or within 1 bp. In an example, methods of the present disclosure can enable resolution of sites (e.g., protein binding sites such as CTCF sites) that are within 10,000 bp, 5,000 bp, 2,000 bp, or 1,000 bp of each other on a genome. In some cases, improved resolution or mapping can be achieved by the use of MNase or other endonucleases that degrade unprotected nucleic acids (e.g., nucleic acids not within the footprint of a binding protein), thereby resulting in proximity ligation events that occur at the edge of a protected region (e.g., a protein footprint).

### Contig Mapping

In various embodiments, the disclosure provides a variety of methods that enable the mapping of the plurality of read pairs to the plurality of contigs. There are several publicly available computer programs for mapping reads to contig sequences. These read-mapping programs data also provide data describing how unique a particular read-mapping is within the genome. From the population of reads that map uniquely, with high confidence within a contig, we can infer the distribution of distances between reads in each read pair. For read pairs whose reads map confidently to different contigs, this mapping data implies a connection between the two contigs in question. It also implies a distance between the two contigs that is proportional to the distribution of distances learned from the analysis described above. Thus, each read pair whose reads map to different contigs implies a connection between those two contigs in a correct assembly. The connections inferred from all such mapped read pairs can be summarized in an adjacency matrix wherein each contig is represented by both a row and column. Read pairs that connect contigs are marked as a non-zero value in the corresponding row and column denoting the contigs to which the reads in the read pair were mapped. Most of the read pairs will map within in a contig, and from which the distribution of distances between read pairs can be learned, and from which an adjacency matrix of contigs can be constructed using read pairs that map to different contigs.

In various embodiments, the disclosure provides methods comprising constructing an adjacency matrix of contigs using the read-mapping data from the read-pair data. In some embodiments, the adjacency matrix uses a weighting scheme for read pairs that incorporate the tendency for short-range interactions over long-range interactions. Read pairs spanning shorter distances are generally more common than read pairs that span longer distances. A function describing the probability of a particular distance can be fit using the read pair data that map to a single contig to learn this distribution. Therefore, one important feature of read pairs that map to different contigs is the position on the contig where they map. For read pairs that both map near one end of a contig, the inferred distance between these contigs can be short and therefore the distance between the joined reads small. Since shorter distances between read pairs are more common than longer distances, this configuration provides stronger evidence that these two contigs are adjacent than would reads mapping far from the edges of the contig. Therefore, the connections in the adjacency matrix are further weighted by the distance of the reads to the edge of the contigs. In further embodiments, the adjacency matrix can further be re-scaled to down-weight the high number of contacts on some contigs that represent promiscuous regions of the genome. These regions of the genome, identifiable by having a high proportion of reads mapping to them, are a priori more likely to contain spurious read mappings that might misinform assembly. In yet further embodiments, this scaling can be directed by searching for one or more conserved binding sites for one or more agents that regulate the scaffolding interactions of chromatin, such as transcriptional repressor CTCF, endocrine receptors, cohesins, or covalently modified histones.

In some embodiments, the disclosure provides for one or more methods disclosed herein that comprise a step of analyzing the adjacency matrix to determine a path through the contigs that represent their order and/or orientation to the genome. In other embodiments, the path through the contigs can be chosen so that each contig is visited exactly once. In further embodiments, the path through the contigs is chosen so that the path through the adjacency matrix maximizes the sum of edge-weights visited. In this way, the most probably contig connections are proposed for the correct assembly. In yet further embodiments, the path through the contigs can be chosen so that each contig is visited exactly once and that edge-weighting of adjacency matrix is maximized.

### Haplotype Phasing

In diploid genomes, it often important to know which allelic variants are linked on the same chromosome. This is known as the haplotype phasing. Short reads from high-throughput sequence data rarely allow one to directly observe which allelic variants are linked. Computational inference of haplotype phasing can be unreliable at long distances. The disclosure provides one or more methods that allow for determining which allelic variants are linked using allelic variants on read pairs. In some cases, phasing with methods of the present disclosure is conducted without imputation.

In various embodiments, the methods and compositions of the disclosure enable the haplotype phasing of diploid or polyploid genomes with regard to a plurality of allelic variants. The methods described herein can thus provide for the determination of linked allelic variants that are linked based on variant information from read pairs and/or assembled contigs using the same. Examples of allelic variants include, but are not limited to, those that are known from the 1000genomes, UK10K, HapMap and other projects for discovering genetic variation among humans. Disease association to a specific gene can be revealed more easily by having haplotype phasing data as demonstrated, for example, by the finding of unlinked, inactivating mutations in both copies of SH3TC2 leading to Charcot-Marie-Tooth neuropathy (Lupski JR, Reid JG, Gonzaga-Jauregui C, et al. N. Engl. J. Med. 362:1181-91, 2010) and unlinked, inactivating mutations in both copies of ABCG5 leading to hypercholesterolemia 9 (Rios J, Stein E, Shendure J, et al. Hum. Mol. Genet. 19:4313-18, 2010).

Humans are heterozygous at an average of 1 site in 1,000. In some cases, a single lane of data using high-throughput sequencing methods can generate at least about 150,000,000 read pairs. Read pairs can be about 100 base pairs long. From these parameters, one-tenth of all reads from a human sample is estimated to cover a heterozygous site. Thus, on average one-hundredth of all read pairs from a human sample is estimated to cover a pair of heterozygous sites. Accordingly, about 1,500,000 read pairs (one-hundredth of 150,000,000) provide phasing data using a single lane. With approximately 3 billion bases in the human genome, and one in one-thousand being heterozygous, there are approximately 3 million heterozygous sites in an average human genome. With about 1,500,000 read pairs that represent a pair of heterozygous sites, the average coverage of each heterozygous site to be phased using a single lane of a high-throughput sequence method is about (1X), using a typical high-throughput sequencing machine. A diploid human genome can therefore be reliably and completely phased with one lane of a high-throughput sequence data relating sequence variants from a sample that is prepared using the methods disclosed herein. In some examples, a lane of data can be a set of DNA sequence read data. In further examples, a lane of data can be a set of DNA sequence read data from a single run of a high-throughput sequencing instrument.

As the human genome consists of two homologous sets of chromosomes, understanding the true genetic makeup of an individual requires delineation of the maternal and paternal copies or haplotypes of the genetic material. Obtaining a haplotype in an individual is useful in several ways. First, haplotypes are useful clinically in predicting outcomes for donor-host matching in organ transplantation and are increasingly used as a means to detect disease associations. Second, in genes that show compound heterozygosity, haplotypes provide information as to whether two deleterious variants are located on the same allele, greatly affecting the prediction of whether inheritance of these variants is harmful. Third, haplotypes from groups of individuals have provided information on population structure and the evolutionary history of the human race. Lastly, recently described widespread allelic imbalances in gene expression suggest that genetic or epigenetic differences between alleles may contribute to quantitative differences in expression. An understanding of haplotype structure will delineate the mechanisms of variants that contribute to allelic imbalances.

In certain embodiments, the methods disclosed herein comprise an in vitro technique to fix and capture associations among distant regions of a genome as needed for long-range linkage and phasing. In some cases, the method comprises constructing and sequencing an XLRP library to deliver very genomically distant read pairs. In some cases, the interactions primarily arise from the random associations within a single DNA fragment. In some examples, the genomic distance between segments can be inferred because segments that are near to each other in a DNA molecule interact more often and with higher probability, while interactions between distant portions of the molecule will be less frequent. Consequently, there is a systematic relationship between the number of pairs connecting two loci and their proximity on the input DNA. The disclosure can produce read pairs capable of spanning the largest DNA fragments in an extraction. The input DNA for this library had a maximum length of 150 kbp, which is the longest meaningful read pair observed from the sequencing data. This suggests that the present method can link still more genomically distant loci if provided larger input DNA fragments. By applying improved assembly software tools that are specifically adapted to handle the type of data produced by the present method, a complete genomic assembly may be possible.

Extremely high phasing accuracy can be achieved by the data produced using the methods and compositions of the disclosure. In comparison to previous methods, the methods described herein can phase a higher proportion of the variants. Phasing can be achieved while maintaining high levels of accuracy. The techniques herein can allow for phasing at an accuracy of greater than about 70%, 80%, 90%, 95%, 96%, 97%, 98%, 99%, 99.9%, 99.99%, or 99.999%. The techniques herein can allow for accurate phasing with less than about 500x sequencing depth, 450x sequencing depth, 400x sequencing depth, 350x sequencing depth, 300x sequencing depth, 250x sequencing depth, 200x sequencing depth, 150x sequencing depth, 100x sequencing depth, or 50x sequencing depth. This phase information can be extended to longer ranges, for example, greater than about 200 kbp, about 300 kbp, about 400 kbp, about 500 kbp, about 600 kbp, about 700 kbp, about 800 kbp, about 900 kbp, about 1Mbp, about 2Mbp, about 3 Mbp, about 4 Mbp, about 5Mbp, or about 10 Mbp. In some embodiments, more than 90% of the heterozygous SNPs for a human sample can be phased at an accuracy greater than 99% using less than about 250 million reads or read pairs, e.g., by using only 1 lane of Illumina HiSeq data. In other cases, more than about 40%, 50%, 60%, 70%, 80%, 90 %, 95%, or 99% of the heterozygous SNPs for a human sample can be phased at an accuracy greater than about 70%, 80%, 90%, 95%, 96%, 97%, 98%, 99%, 99.9%, 99.99%, or 99.999% using less than about 250 million or about 500 million reads or read pairs, e.g., by using only 1 or 2 lanes of Illumina HiSeq data. For example, more than 95% or 99% of the heterozygous SNPs for a human sample can be phase at an accuracy greater than about 95% or 99% using less about 250 million or about 500 million reads. In further cases, additional variants can be captured by increasing the read length to about 200 bp, 250 bp, 300 bp, 350 bp, 400 bp, 450 bp, 500 bp, 600 bp, 800 bp, 1000 bp, 1500 bp, 2 kbp, 3 kbp, 4 kbp, 5 kbp, 10 kbp, 20 kbp, 50 kbp, or 100 kbp.

In other embodiments of the disclosure, the data from an XLRP library can be used to confirm the phasing capabilities of the long-range read pairs. The accuracy of those results is on par with the best technologies previously available, but further extending to significantly longer distances. The current sample preparation protocol for a particular sequencing method recognizes variants located within a read-length, e.g., 150 bp, of a targeted site for phasing. In one example, from an XLRP library built for NA12878, a benchmark sample for assembly, 44% of the 1,703,909 heterozygous SNPs present were phased with an accuracy greater than 99%. In some cases, this proportion can be expanded to nearly all variable sites with the judicious choice of enzymes or with digestion conditions.

Haplotype phasing can include phasing the human leukocyte antigen (HLA) region (e.g., Class I HLA-A, B, and C; Class II HLA-DRB1/3/4/5, HLA-DQA1, HLA-DQB1, HLA-DPA1, HLA-DPB1). The HLA region of the genome is densely polymorphic and can be difficult to sequence or phase with standard sequencing approaches. Techniques of the present disclosure can provide for improved sequencing and phasing accuracy of the HLA region of the genome. Using techniques of the present disclosure, the HLA region of the genome can be phased accurately as part of phasing larger regions (e.g., chromosome arms, chromosomes, whole genomes) or on its own (e.g., by targeted enrichment such as hybrid capture). In an example, the HLA region on its own was phased accurately at a sequencing depth of approximately 300x. These techniques can provide advantages over traditional approaches for HLA analysis, such as long-range PCR; for example, long-range PCR can involve complex protocols and many separate reactions. As discussed further herein, samples can be multiplexed for sequencing analysis, for example by including sample-identifying barcodes in bridge oligonucleotides or elsewhere, and de-multiplexing the sequence information based on the barcodes. In an example, multiple samples are subjected to proximity ligation, barcoded with sample-identifying barcodes (e.g., in the bridge oligonucleotide), the HLA region is targeted (e.g., by hybrid capture), and multiplexed sequencing is conducted, allowing phasing of the HLA region for multiple samples. In some cases, phasing the HLA region is conducted without imputation.

Haplotype phasing can include phasing the killer cell immunoglobulin-like receptor (KIR) region. The KIR region of the genome is highly homologous and structurally dynamic due to transposon-mediated recombination, and can be difficult to sequence or phase with standard sequencing approaches. Techniques of the present disclosure can provide for improved sequencing and phasing accuracy of the KIR region of the genome. Using techniques of the present disclosure, the KIR region of the genome can be phased accurately as part of phasing larger regions (e.g., chromosome arms, chromosomes, whole genomes) or on its own (e.g., by targeted enrichment such as hybrid capture). These techniques can provide advantages over traditional approaches for HLA analysis, such as long-range PCR; for example, long-range PCR can involve complex protocols and many separate reactions. As discussed further herein, samples can be multiplexed for sequencing analysis, for example by including sample-identifying barcodes in bridge oligonucleotides or elsewhere, and de-multiplexing the sequence information based on the barcodes. In an example, multiple samples are subjected to proximity ligation, barcoded with sample-identifying barcodes (e.g., in the bridge oligonucleotide), the KIR region is targeted (e.g., by hybrid capture), and multiplexed sequencing is conducted, allowing phasing of the KIR region for multiple samples. At least about 8, 9, 10, 11, 12, 13, 14, 15, 16, 17, or more genes and/or pseudogenes can be phased. In some cases, phasing the KIR region is conducted without imputation.

### Metagenomics Analysis

In some embodiments, the compositions and methods described herein allow for the investigation of meta-genomes, for example, those found in the human gut. Accordingly, the partial or whole genomic sequences of some or all organisms that inhabit a given ecological environment can be investigated. Examples include random sequencing of all gut microbes, the microbes found on certain areas of skin, and the microbes that live in toxic waste sites. The composition of the microbe population in these environments can be determined using the compositions and methods described herein and as well as the aspects of interrelated biochemistries encoded by their respective genomes. The methods described herein can enable metagenomic studies from complex biological environments, for example, those that comprise more than 2, 3, 4, 5, 6, 7, 8, 9, 10, 12, 15, 20, 25, 30, 40, 50, 60, 70, 80, 90, 100, 125, 150, 175, 200, 250, 300, 400, 500, 600, 700, 800, 900, 1000, 5000, 10000 or more organisms and/or variants of organisms.

High degrees of accuracy required by cancer genome sequencing can be achieved using the methods and systems described herein. Inaccurate reference genomes can make base-calling challenges when sequencing cancer genomes. Heterogeneous samples and small starting materials, for example, a sample obtained by biopsy introduce additional challenges. Further, detection of large-scale structural variants and/or losses of heterozygosity is often crucial for cancer genome sequencing, as well as the ability to differentiate between somatic variants and errors in base-calling.

### Improved Sequencing Accuracy

Systems and methods described herein may generate accurate long sequences from complex samples containing 2, 3, 4, 5, 6, 7, 8, 9, 10, 12, 15, 20 or more varying genomes. Mixed samples of normal, benign, and/or tumor origin may be analyzed, optionally without the need for a normal control. In some embodiments, starting samples as little as 100 ng or even as little as hundreds of genome equivalents are utilized to generate accurate long sequences. Systems and methods described herein may allow for detection of large scale structural variants and rearrangements, Phased variant calls may be obtained over long sequences spanning about 1 kbp, about 2 kbp, about 5 kbp, about 10 kbp, about 20 kbp, about 50 kbp, about 100 kbp, about 200 kbp, about 500 kbp, about 1 Mbp, about 2 Mbp, about 5 Mbp, about 10 Mbp, about 20 Mbp, about 50 Mbp, or about 100 Mbp or more nucleotides. For example, phase variant call may be obtained over long sequences spanning about 1 Mbp or about 2 Mbp.

Haplotypes determined using the methods and systems described herein may be assigned to computational resources, for example, computational resources over a network, such as a cloud system. Short variant calls can be corrected, if necessary, using relevant information that is stored in the computational resources. Structural variants can be detected based on the combined information from short variant calls and the information stored in the computational resources. Problematic parts of the genome, such as segmental duplications, regions prone to structural variation, the highly variable and medically relevant MHC region, centromeric and telomeric regions, and other heterochromatic regions including, but not limited to, those with repeat regions, low sequence accuracy, high variant rates, ALU repeats, segmental duplications, or any other relevant problematic parts known in the art, can be reassembled for increased accuracy.

A sample type can be assigned to the sequence information either locally or in a networked computational resource, such as a cloud. In cases where the source of the information is known, for example, when the source of the information is from a cancer or normal tissue, the source can be assigned to the sample as part of a sample type. Other sample type examples generally include, but are not limited to, tissue type, sample collection method, presence of infection, type of infection, processing method, size of the sample, etc. In cases where a complete or partial comparison genome sequence is available, such as a normal genome in comparison to a cancer genome, the differences between the sample data and the comparison genome sequence can be determined and optionally output.

### Clinical Applications

The methods of the present disclosure can be used in the analysis of genetic information of selective genomic regions of interest as well as genomic regions which may interact with the selective region of interest. Amplification methods as disclosed herein can be used in the devices, kits, and methods known to the art for genetic analysis, such as, but not limited to, those found in U.S. Pat. Nos. 6,449,562, 6,287,766, 7,361,468, 7,414,117, 6,225,109, and 6,110,709. In some cases, amplification methods of the present disclosure can be used to amplify target nucleic acid for DNA hybridization studies to determine the presence or absence of polymorphisms. The polymorphisms, or alleles, can be associated with diseases or conditions such as genetic disease. In other cases, the polymorphisms can be associated with susceptibility to diseases or conditions, for example, polymorphisms associated with addiction, degenerative and age-related conditions, cancer, and the like. In other cases, the polymorphisms can be associated with beneficial traits such as increased coronary health, or resistance to diseases such as HIV or malaria, or resistance to degenerative diseases such as osteoporosis, Alzheimer's or dementia.

The compositions and methods of the disclosure can be used for diagnostic, prognostic, therapeutic, patient stratification, drug development, treatment selection, and screening purposes. The present disclosure provides the advantage that many different target molecules can be analyzed at one time from a single biomolecular sample using the methods of the disclosure. This allows, for example, for several diagnostic tests to be performed on one sample.

The composition and methods of the disclosure can be used in genomics. The methods described herein can provide an answer rapidly which is very desirable for this application. The methods and composition described herein can be used in the process of finding biomarkers that may be used for diagnostics or prognostics and as indicators of health and disease. The methods and composition described herein can be used to screen for drugs, e.g., drug development, selection of treatment, determination of treatment efficacy and/or identify targets for pharmaceutical development. The ability to test gene expression on screening assays involving drugs is very important because proteins are the final gene product in the body. In some embodiments, the methods and compositions described herein will measure both protein and gene expression simultaneously which will provide the most information regarding the particular screening being performed.

The composition and methods of the disclosure can be used in gene expression analysis. The methods described herein discriminate between nucleotide sequences. The difference between the target nucleotide sequences can be, for example, a single nucleic acid base difference, a nucleic acid deletion, a nucleic acid insertion, or rearrangement. Such sequence differences involving more than one base can also be detected. The process of the present disclosure is able to detect infectious diseases, genetic diseases, and cancer. It is also useful in environmental monitoring, forensics, and food science. Examples of genetic analyses that can be performed on nucleic acids include, e.g., SNP detection, STR detection, RNA expression analysis, promoter methylation, gene expression, virus detection, viral subtyping and drug resistance.

The present methods can be applied to the analysis of biomolecular samples obtained or derived from a patient so as to determine whether a diseased cell type is present in the sample, the stage of the disease, the prognosis for the patient, the ability to the patient to respond to a particular treatment, or the best treatment for the patient. The present methods can also be applied to identify biomarkers for a particular disease.

In some embodiments, the methods described herein are used in the diagnosis of a condition. As used herein the term "diagnose" or "diagnosis" of a condition may include predicting or diagnosing the condition, determining predisposition to the condition, monitoring treatment of the condition, diagnosing a therapeutic response of the disease, or prognosis of the condition, condition progression, or response to particular treatment of the condition. For example, a blood sample can be assayed according to any of the methods described herein to determine the presence and/or quantity of markers of a disease or malignant cell type in the sample, thereby diagnosing or staging a disease or a cancer.

In some embodiments, the methods and composition described herein are used for the diagnosis and prognosis of a condition.

Numerous immunologic, proliferative and malignant diseases and disorders are especially amenable to the methods described herein. Immunologic diseases and disorders include allergic diseases and disorders, disorders of immune function, and autoimmune diseases and conditions. Allergic diseases and disorders include, but are not limited to, allergic rhinitis, allergic conjunctivitis, allergic asthma, atopic eczema, atopic dermatitis, and food allergy. Immunodeficiencies include, but are not limited to, severe combined immunodeficiency (SCID), hypereosinophilic syndrome, chronic granulomatous disease, leukocyte adhesion deficiency I and II, hyper IgE syndrome, Chediak Higashi, neutrophilias, neutropenias, aplasias, Agammaglobulinemia, hyper-IgM syndromes, DiGeorge/Velocardial-facial syndromes and Interferon gamma-TH1 pathway defects. Autoimmune and immune dysregulation disorders include, but are not limited to, rheumatoid arthritis, diabetes, systemic lupus erythematosus, Graves' disease, Graves ophthalmopathy, Crohn's disease, multiple sclerosis, psoriasis, systemic sclerosis, goiter and struma lymphomatosa (Hashimoto's thyroiditis, lymphadenoid goiter), alopecia aerata, autoimmune myocarditis, lichen sclerosis, autoimmune uveitis, Addison's disease, atrophic gastritis, myasthenia gravis, idiopathic thrombocytopenic purpura, hemolytic anemia, primary biliary cirrhosis, Wegener's granulomatosis, polyarteritis nodosa, and inflammatory bowel disease, allograft rejection and tissue destructive from allergic reactions to infectious microorganisms or to environmental antigens.

Proliferative diseases and disorders that may be evaluated by the methods of the disclosure include, but are not limited to, hemangiomatosis in newborns; secondary progressive multiple sclerosis; chronic progressive myelodegenerative disease; neurofibromatosis; ganglioneuromatosis; keloid formation; Paget's Disease of the bone; fibrocystic disease (e.g., of the breast or uterus); sarcoidosis; Peronies and Duputren's fibrosis, cirrhosis, atherosclerosis and vascular restenosis.

Malignant diseases and disorders that may be evaluated by the methods of the disclosure include both hematologic malignancies and solid tumors.

Hematologic malignancies are especially amenable to the methods of the disclosure when the sample is a blood sample, because such malignancies involve changes in blood-borne cells. Such malignancies include non-Hodgkin's lymphoma, Hodgkin's lymphoma, non-B cell lymphomas, and other lymphomas, acute or chronic leukemias, polycythemias, thrombocythemias, multiple myeloma, myelodysplastic disorders, myeloproliferative disorders, myelofibroses, atypical immune lymphoproliferations and plasma cell disorders.

Plasma cell disorders that may be evaluated by the methods of the disclosure include multiple myeloma, amyloidosis and Waldenstrom's macroglobulinemia.

Example of solid tumors include, but are not limited to, colon cancer, breast cancer, lung cancer, prostate cancer, brain tumors, central nervous system tumors, bladder tumors, melanomas, liver cancer, osteosarcoma and other bone cancers, testicular and ovarian carcinomas, head and neck tumors, and cervical neoplasms.

Genetic diseases can also be detected by the process of the present disclosure. This can be carried out by prenatal or post-natal screening for chromosomal and genetic aberrations or for genetic diseases. Examples of detectable genetic diseases include: 21 hydroxylase deficiency, cystic fibrosis, Fragile X Syndrome, Turner Syndrome, Duchenne Muscular Dystrophy, Down Syndrome or other trisomies, heart disease, single gene diseases, HLA typing, phenylketonuria, sickle cell anemia, Tay-Sachs Disease, thalassemia, Klinefelter Syndrome, Huntington Disease, autoimmune diseases, lipidosis, obesity defects, hemophilia, inborn errors of metabolism, and diabetes.

Methods of the present disclosure can be used to detect genetic or genomic features associated with genetic diseases including, but not limited to, gene fusions, structural variants, rearrangements, and changes in topology such as missing or altered TAD boundaries, changes in TAD subtype, changes in compartment, changes in chromatin type, and changes in modification status such as methylation status (e.g., CpG methylation, H3K4me3, H3K27me3, or other histone methylation).

The methods described herein can be used to diagnose pathogen infections, for example, infections by intracellular bacteria and viruses, by determining the presence and/or quantity of markers of bacterium or virus, respectively, in the sample.

A wide variety of infectious diseases can be detected by the process of the present disclosure. The infectious diseases can be caused by bacterial, viral, parasite, and fungal infectious agents. The resistance of various infectious agents to drugs can also be determined using the present disclosure.

Bacterial infectious agents which can be detected by the present disclosure include *Escherichia coli, Salmonella, Shigella, Klebsiella, Pseudomonas, Listeria monocytogenes, Mycobacterium tuberculosis, Mycobacterium aviumintracellulare, Yersinia, Francisella, Pasteurella, Brucella, Clostridia, Bordetella pertussis, Bacteroides, Staphylococcus aureus, Streptococcus pneumonia, B-Hemolytic strep., Corynebacteria, Legionella, Mycoplasma, Ureaplasma, Chlamydia, Neisseria gonorrhea, Neisseria meningitides, Hemophilus influenza, Enterococcus faecalis, Proteus vulgaris, Proteus mirabilis, Helicobacter pylori, Treponema palladium, Borrelia burgdorferi, Borrelia recurrentis, Rickettsial* pathogens, *Nocardia,* and *Acitnomycetes.*

Fungal infectious agents which can be detected by the present disclosure include *Cryptococcus neoformans, Blastomyces dermatitidis, Histoplasma capsulatum, Coccidioides immitis, Paracoccidioides brasiliensis, Candida albicans, Aspergillus fumigautus, Phycomycetes (Rhizopus), Sporothrix schenckii, Chromomycosis,* and*Maduromycosis.*

Viral infectious agents which can be detected by the present disclosure include human immunodeficiency virus, human T-cell lymphocytotrophic virus, hepatitis viruses (e.g., Hepatitis B Virus and Hepatitis C Virus), Epstein-Barr virus, cytomegalovirus, human papillomaviruses, orthomyxo viruses, paramyxo viruses, adenoviruses, corona viruses, rhabdo viruses, polio viruses, toga viruses, bunya viruses, arena viruses, rubella viruses, and reo viruses.

Parasitic agents which can be detected by the present disclosure include *Plasmodium falciparum, Plasmodium malaria, Plasmodium vivax, Plasmodium ovale, Onchoverva volvulus, Leishmania, Trypanosoma* spp., *Schistosoma* spp., *Entamoeba histolytica, Cryptosporidum, Giardia* spp., *Trichimonas* spp., *Balatidium coli, Wuchereria bancrofti, Toxoplasma* spp., *Enterobius vermicularis, Ascaris lumbricoides, Trichuris trichiura, Dracunculus medinesis, Trematodes, Diphyllobothrium latum, Taenia* spp., *Pneumocystis carinii,* and *Necator americanis.*

The present disclosure is also useful for detection of drug resistance by infectious agents. For example, vancomycin-resistant *Enterococcus faecium,* methicillin-resistant *Staphylococcus aureus,* penicillin-resistant *Streptococcus pneumoniae,* multi-drug resistant *Mycobacterium tuberculosis,* and AZT-resistant human immunodeficiency virus can all be identified with the present disclosure.

Thus, the target molecules detected using the compositions and methods of the disclosure can be either patient markers (such as a cancer marker) or markers of infection with a foreign agent, such as bacterial or viral markers.

The compositions and methods of the disclosure can be used to identify and/or quantify a target molecule whose abundance is indicative of a biological state or disease condition, for example, blood markers that are upregulated or downregulated as a result of a disease state.

**In** some embodiments, the methods and compositions of the present disclosure can be used for cytokine expression. The low sensitivity of the methods described herein would be helpful for early detection of cytokines, e.g., as biomarkers of a condition, diagnosis or prognosis of a disease such as cancer, and the identification of subclinical conditions.

Methods of the present disclosure can be used to detect genetic or genomic features associated with cancer including, but not limited to, gene fusions, structural variants, rearrangements, and changes in topology such as missing or altered TAD boundaries, changes in TAD subtype, changes in compartment, changes in chromatin type, and changes in modification status such as methylation status (e.g., CpG methylation, H3K4me3, H3K27me3, or other histone methylation).

### Samples

The different samples from which the target polynucleotides are derived can comprise multiple samples from the same individual, samples from different individuals, or combinations thereof. In some embodiments, a sample comprises a plurality of polynucleotides from a single individual. In some embodiments, a sample comprises a plurality of polynucleotides from two or more individuals. An individual is any organism or portion thereof from which target polynucleotides can be derived, non-limiting examples of which include plants, animals, fungi, protists, monerans, viruses, mitochondria, and chloroplasts. Sample polynucleotides can be isolated from a subject, such as a cell sample, tissue sample, or organ sample derived therefrom, including, for example, cultured cell lines, biopsy, blood sample, or fluid sample containing a cell. The subject may be an animal including, but not limited to, an animal such as a cow, a pig, a mouse, a rat, a chicken, a cat, a dog, etc., and is usually a mammal, such as a human. Samples can also be artificially derived, such as by chemical synthesis. In some embodiments, the samples comprise DNA. In some embodiments, the samples comprise genomic DNA. In some embodiments, the samples comprise mitochondrial DNA, chloroplast DNA, plasmid DNA, bacterial artificial chromosomes, yeast artificial chromosomes, oligonucleotide tags, or combinations thereof. In some embodiments, the samples comprise DNA generated by primer extension reactions using any suitable combination of primers and a DNA polymerase including, but not limited to, polymerase chain reaction (PCR), reverse transcription, and combinations thereof. Where the template for the primer extension reaction is RNA, the product of reverse transcription is referred to as complementary DNA (cDNA). Primers useful in primer extension reactions can comprise sequences specific to one or more targets, random sequences, partially random sequences, and combinations thereof. Reaction conditions suitable for primer extension reactions are known in the art. In general, sample polynucleotides comprise any polynucleotide present in a sample, which may or may not include target polynucleotides.

In some embodiments, nucleic acid template molecules (e.g., DNA or RNA) are isolated from a biological sample containing a variety of other components, such as proteins, lipids and non-template nucleic acids. Nucleic acid template molecules can be obtained from any cellular material, obtained from an animal, plant, bacterium, fungus, or any other cellular organism. Biological samples for use in the present disclosure include viral particles or preparations. Nucleic acid template molecules can be obtained directly from an organism or from a biological sample obtained from an organism, e.g., from blood, urine, cerebrospinal fluid, seminal fluid, saliva, sputum, stool and tissue. Any tissue or body fluid specimen may be used as a source for nucleic acid for use in the disclosure. Nucleic acid template molecules can also be isolated from cultured cells, such as a primary cell culture or a cell line. The cells or tissues from which template nucleic acids are obtained can be infected with a virus or other intracellular pathogen. A sample can also be total RNA extracted from a biological specimen, a cDNA library, viral, or genomic DNA. A sample may also be isolated DNA from a non-cellular origin, e.g., amplified/isolated DNA from the freezer.

Methods for the extraction and purification of nucleic acids are well known in the art. For example, nucleic acids can be purified by organic extraction with phenol, phenol/chloroform/isoamyl alcohol, or similar formulations, including TRIzol and TriReagent. Other non-limiting examples of extraction techniques include: (1) organic extraction followed by ethanol precipitation, e.g., using a phenol/chloroform organic reagent (Ausubel et al., 1993), with or without the use of an automated nucleic acid extractor, e.g., the Model 341 DNA Extractor available from Applied Biosystems (Foster City, Calif.); (2) stationary phase adsorption methods (U.S. Pat. No. 5,234,809; Walsh et al., 1991); and (3) salt-induced nucleic acid precipitation methods (Miller et al., (1988), such precipitation methods being typically referred to as "salting-out" methods. Another example of nucleic acid isolation and/or purification includes the use of magnetic particles to which nucleic acids can specifically or non-specifically bind, followed by isolation of the beads using a magnet, and washing and eluting the nucleic acids from the beads (see, e.g., U.S. Pat. No. 5,705,628). In some embodiments, the above isolation methods may be preceded by an enzyme digestion step to help eliminate unwanted protein from the sample, e.g., digestion with proteinase K, or other like proteases (see, e.g., U.S. Pat. No. 7,001,724). If desired, RNase inhibitors may be added to the lysis buffer. For certain cell or sample types, it may be desirable to add a protein denaturation/digestion step to the protocol. Purification methods may be directed to isolate DNA, RNA, or both. When both DNA and RNA are isolated together during or subsequent to an extraction procedure, further steps may be employed to purify one or both separately from the other. Sub-fractions of extracted nucleic acids can also be generated, for example, purification by size, sequence, or other physical or chemical characteristic. In addition to an initial nucleic isolation step, purification of nucleic acids can be performed after any step in the methods of the disclosure, such as to remove excess or unwanted reagents, reactants, or products.

Nucleic acid template molecules can be obtained as described in U.S. Patent Application Publication Number US2002/0190663 A1, published Oct. 9, 2003. Generally, nucleic acid can be extracted from a biological sample by a variety of techniques such as those described by Maniatis, et al., Molecular Cloning: A Laboratory Manual, Cold Spring Harbor, N.Y., pp. 280-281 (1982). In some cases, the nucleic acids can be first extracted from the biological samples and then cross-linked in vitro. In some cases, native association proteins (e.g., histones) can be further removed from the nucleic acids.

In other embodiments, the disclosure can be easily applied to any high molecular weight double stranded DNA including, for example, DNA isolated from tissues, cell culture, bodily fluids, animal tissue, plant, bacteria, fungi, viruses, etc.

In some embodiments, each of the plurality of independent samples can independently comprise at least about 1 ng, 2 ng ,5 ng, 10 ng, 20 ng, 30 ng, 40 ng, 50 ng, 75 ng, 100 ng, 150 ng, 200 ng, 250 ng, 300 ng, 400 ng, 500 ng, 1 µg, 1.5 µg, 2 µg, 5 µg, 10 µg, 20 µg, 50 µg, 100 µg, 200 µg, 500 µg, or 1000 µg, or more of nucleic acid material. In some embodiments, each of the plurality of independent samples can independently comprise less than about 1 ng, 2 ng, 5ng, 10 ng, 20 ng, 30 ng, 40 ng, 50 ng, 75 ng, 100 ng, 150 ng, 200 ng, 250 ng, 300 ng, 400 ng, 500 ng, 1 µg, 1.5 µg, 2 µg, 5 µg, 10 µg, 20 µg, 50 µg, 100 µg, 200 µg, 500 µg, or 1000 µg, or more of nucleic acid.

In some embodiments, end repair is performed to generate blunt end 5' phosphorylated nucleic acid ends using commercial kits, such as those available from Epicentre Biotechnologies (Madison, WI).

### Adaptors

An adaptor oligonucleotide includes any oligonucleotide having a sequence, at least a portion of which is known, that can be joined to a target polynucleotide. Adaptor oligonucleotides can comprise DNA, RNA, nucleotide analogues, non-canonical nucleotides, labeled nucleotides, modified nucleotides, or combinations thereof. Adaptor oligonucleotides can be single-stranded, double-stranded, or partial duplex. In general, a partial-duplex adaptor comprises one or more single-stranded regions and one or more double-stranded regions. Double-stranded adaptors can comprise two separate oligonucleotides hybridized to one another (also referred to as an "oligonucleotide duplex"), and hybridization may leave one or more blunt ends, one or more 3' overhangs, one or more 5' overhangs, one or more bulges resulting from mismatched and/or unpaired nucleotides, or any combination of these. In some embodiments, a single-stranded adaptor comprises two or more sequences that are able to hybridize with one another. When two such hybridizable sequences are contained in a single-stranded adaptor, hybridization yields a hairpin structure (hairpin adaptor). When two hybridized regions of an adaptor are separated from one another by a non-hybridized region, a "bubble" structure results. Adaptors comprising a bubble structure can consist of a single adaptor oligonucleotide comprising internal hybridizations, or may comprise two or more adaptor oligonucleotides hybridized to one another. Internal sequence hybridization, such as between two hybridizable sequences in an adaptor, can produce a double-stranded structure in a single-stranded adaptor oligonucleotide. Adaptors of different kinds can be used in combination, such as a hairpin adaptor and a double-stranded adaptor, or adaptors of different sequences. Hybridizable sequences in a hairpin adaptor may or may not include one or both ends of the oligonucleotide. When neither of the ends are included in the hybridizable sequences, both ends are "free" or "overhanging." When only one end is hybridizable to another sequence in the adaptor, the other end forms an overhang, such as a 3' overhang or a 5' overhang. When both the 5'-terminal nucleotide and the 3'-terminal nucleotide are included in the hybridizable sequences, such that the 5'-terminal nucleotide and the 3'-terminal nucleotide are complementary and hybridize with one another, the end is referred to as "blunt." Different adaptors can be joined to target polynucleotides in sequential reactions or simultaneously. For example, the first and second adaptors can be added to the same reaction. Adaptors can be manipulated prior to combining with target polynucleotides. For example, terminal phosphates can be added or removed.

Adaptors can contain one or more of a variety of sequence elements including, but not limited to, one or more amplification primer annealing sequences or complements thereof, one or more sequencing primer annealing sequences or complements thereof, one or more barcode sequences, one or more common sequences shared among multiple different adaptors or subsets of different adaptors, one or more restriction enzyme recognition sites, one or more overhangs complementary to one or more target polynucleotide overhangs, one or more probe binding sites (e.g., for attachment to a sequencing platform, such as a flow cell for massive parallel sequencing, such as developed by Illumina, Inc.), one or more random or near-random sequences (e.g., one or more nucleotides selected at random from a set of two or more different nucleotides at one or more positions, with each of the different nucleotides selected at one or more positions represented in a pool of adaptors comprising the random sequence), and combinations thereof. Two or more sequence elements can be non-adjacent to one another (e.g., separated by one or more nucleotides), adjacent to one another, partially overlapping, or completely overlapping. For example, an amplification primer annealing sequence can also serve as a sequencing primer annealing sequence. Sequence elements can be located at or near the 3' end, at or near the 5' end, or in the interior of the adaptor oligonucleotide. When an adaptor oligonucleotide is capable of forming secondary structure, such as a hairpin, sequence elements can be located partially or completely outside the secondary structure, partially or completely inside the secondary structure, or in between sequences participating in the secondary structure. For example, when an adaptor oligonucleotide comprises a hairpin structure, sequence elements can be located partially or completely inside or outside the hybridizable sequences (the "stem"), including in the sequence between the hybridizable sequences (the "loop"). In some embodiments, the first adaptor oligonucleotides in a plurality of first adaptor oligonucleotides having different barcode sequences comprise a sequence element common among all first adaptor oligonucleotides in the plurality. In some embodiments, all second adaptor oligonucleotides comprise a sequence element common among all second adaptor oligonucleotides that is different from the common sequence element shared by the first adaptor oligonucleotides. A difference in sequence elements can be any such that at least a portion of different adaptors do not completely align, for example, due to changes in sequence length, deletion or insertion of one or more nucleotides, or a change in the nucleotide composition at one or more nucleotide positions (such as a base change or base modification). In some embodiments, an adaptor oligonucleotide comprises a 5' overhang, a 3' overhang, or both that is complementary to one or more target polynucleotides. Complementary overhangs can be one or more nucleotides in length including, but not limited to, 1, 2, 3, 4, 5, 6, 7, 8, 9, 10, 11, 12, 13, 14, 15, or more nucleotides in length. For example, the complementary overhangs can be about 1, 2, 3, 4, 5 or 6 nucleotides in length. Complementary overhangs may comprise a fixed sequence. Complementary overhangs may comprise a random sequence of one or more nucleotides, such that one or more nucleotides are selected at random from a set of two or more different nucleotides at one or more positions, with each of the different nucleotides selected at one or more positions represented in a pool of adaptors with complementary overhangs comprising the random sequence. In some embodiments, an adaptor overhang consists of an adenine or a thymine.

Adaptor oligonucleotides can have any suitable length, at least sufficient to accommodate the one or more sequence elements of which they are comprised. In some embodiments, adaptors are about, less than about, or more than about, 10, 15, 20, 25, 30, 35, 40, 45, 50, 55, 60, 65, 70, 75, 80, 90, 100, 200, or more nucleotides in length. In some examples, the adaptors can be about 10 to about 50 nucleotides in length. In further examples, the adaptors can be about 20 to about 40 nucleotides in length.

As used herein, the term "barcode" refers to a known nucleic acid sequence that allows some feature of a polynucleotide with which the barcode is associated to be identified. In some embodiments, the feature of the polynucleotide to be identified is the sample from which the polynucleotide is derived. In some embodiments, barcodes can be at least 3, 4, 5, 6, 7, 8, 9, 10, 11, 12, 13, 14, 15, or more nucleotides in length. For example, barcodes can be at least 10, 11, 12, 13, 14, or 15 nucleotides in length. In some embodiments, barcodes can be shorter than 10, 9, 8, 7, 6, 5, or 4 nucleotides in length. For example, barcodes can be shorter than 10 nucleotides in length. In some embodiments, barcodes associated with some polynucleotides are of different length than barcodes associated with other polynucleotides. In general, barcodes are of sufficient length and comprise sequences that are sufficiently different to allow the identification of samples based on barcodes with which they are associated. In some embodiments, a barcode, and the sample source with which it is associated, can be identified accurately after the mutation, insertion, or deletion of one or more nucleotides in the barcode sequence, such as the mutation, insertion, or deletion of 1, 2, 3, 4, 5, 6, 7, 8, 9, 10, or more nucleotides. In some examples, 1, 2 or 3 nucleotides can be mutated, inserted and/or deleted. In some embodiments, each barcode in a plurality of barcodes differ from every other barcode in the plurality at least two nucleotide positions, such as at least 2, 3, 4, 5, 6, 7, 8, 9, 10, or more positions. In some examples, each barcode can differ from every other barcode by in at least 2, 3, 4 or 5 positions. In some embodiments, both a first site and a second site comprise at least one of a plurality of barcode sequences. In some embodiments, barcodes for second sites are selected independently from barcodes for first adaptor oligonucleotides. In some embodiments, first sites and second sites having barcodes are paired, such that sequences of the pair comprise the same or different one or more barcodes. In some embodiments, the methods of the disclosure further comprise identifying the sample from which a target polynucleotide is derived based on a barcode sequence to which the target polynucleotide is joined. In general, a barcode may comprise a nucleic acid sequence that when joined to a target polynucleotide serves as an identifier of the sample from which the target polynucleotide was derived.

Adaptor oligonucleotides may be coupled, linked, or tethered to an immunoglobulin or an immunoglobulin binding protein or fragment thereof. For example, after in situ genomic digestion of a crosslinked sample with a DNase, such as MNase, one or more antibodies may be added to the sample to bind the digested chromatin, such as at methylated sites or transcription factor binding sites. Next, a biotinylated adaptor oligonucleotide coupled, linked, or tethered to an immunoglobulin binding protein or fragment thereof, such as a Protein A, a Protein G, a Protein A/G, or a Protein L, may be added to the sample to target the adaptors to one or more specific sites in the chromatin. The sample may then be treated with a ligase to effect proximity ligation. Moreover, streptavidin may be used to isolate DNA that has been ligated to the adaptors. Crosslinks may then be reversed before amplifying the sample using PCR and sequencing. Alternatively, adaptor linked oligonucleotides may comprise modified nucleotides capable of linking to a purification reagent using click chemistry.

### Bridge Oligonucleotides

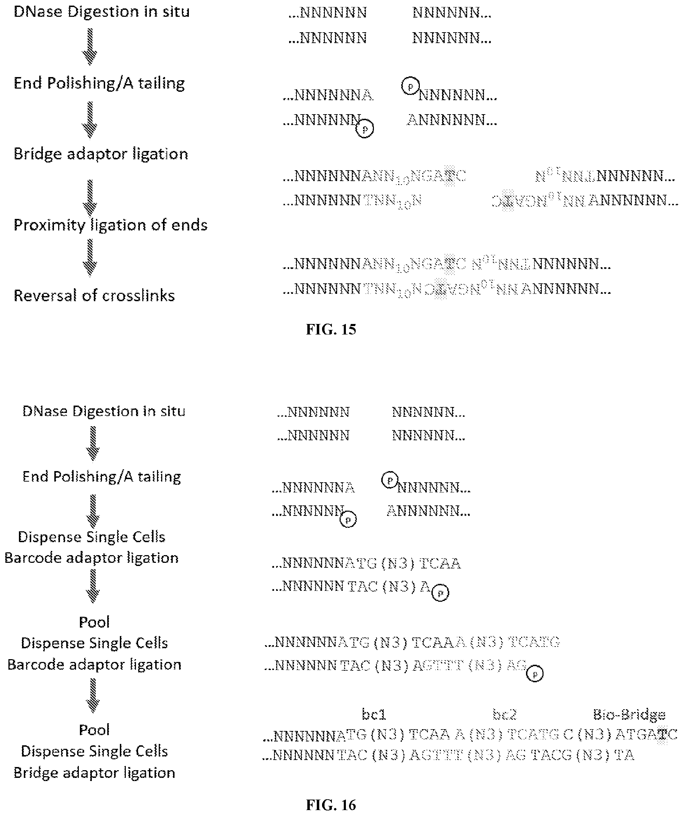

Methods provided herein can comprise attaching a first segment and a second segment of a plurality of segments at a junction. In some cases, attaching can comprise filling in sticky ends using biotin tagged nucleotides and ligating the blunt ends. In certain cases, attaching can comprise contacting at least the first segment and the second segment to a bridge oligonucleotide. **FIG. 15** illustrates an exemplary workflow using a bridge oligonucleotide to connect a first segment and a second segment where a nucleic acid is digested in situ to form the first segment and the second segment. The ends are polished and polyadenylated before ligating a bridge oligonucleotide to each of the first segment and the second segment. The first segment and the second segment are then ligated to create a junction comprising a bridge oligonucleotide. In various cases, attaching can comprise contacting at least the first segment and the second segment to a barcode.

In some embodiments, bridge oligonucleotides as provided herein can be from at least about 5 nucleotides in length to about 50 nucleotides in length. In certain embodiments, the bridge oligonucleotides can be from about 15 nucleotides in length to about 18 nucleotides in length. In various embodiments, the bridge oligonucleotides can be at least 5, at least 6, at least 7, at least 8, at least 9, at least 10, at least 11, at least 12, at least 13, at least 14, at least 15, at least 16, at least 17, at least 18, at least 19, at least 20, at least 25, at least 30, at least 35, at least 40, at least 45, at least 50, or more nucleotides in length. In an example, the bridge oligonucleotides are at least 10 nucleotides in length. In another example, the bridge oligonucleotides are 12 nucleotides in length or about 12 nucleotides in length. In some cases, bridge oligonucleotides of at least 10 bp can increase stability and reduce adverse proximity ligation events, such as short inserts, interchromosomal ligations, non-specific ligations, and bridge self-ligations.

In some embodiments, the bridge oligonucleotides may comprise a barcode. In certain embodiments, the bridge oligonucleotides can comprise multiple barcodes (e.g., two or more barcodes). In various embodiments, the bridge oligonucleotides can comprise multiple bridge oligonucleotides coupled or connected together. In some embodiments, the bridge oligonucleotides may be coupled or linked to an immunoglobulin binding protein or fragment thereof, such as a Protein A, a Protein G, a Protein A/G, or a Protein L. In some cases, coupled bridge oligonucleotides may be delivered to a location in the sample nucleic acid where an antibody is bound.

A splitting and pooling approach can be employed to produce bridge oligonucleotides with unique barcodes. A population of samples can be split into multiple groups, bridge oligonucleotides can be attached to the samples such that the bridge oligonucleotide barcodes are different between groups but the same within a group, the groups of samples can be pooled together again, and this process can be repeated multiple times. For example, a population of polynucleotides can be split into Group A and Group B. First bridge oligonucleotides can be attached to the polynucleotides in Group A and second bridge oligonucleotides can be attached to the polynucleotides in Group B. Accordingly, the bridge oligonucleotide barcodes are the same within Group A, but the bridge oligonucleotides are different between Group A and Group B. Iterating this process can ultimately result in each sample in the population having a unique series of bridge oligonucleotide barcodes, allowing single-sample (e.g., single cell, single nucleus, single chromosome) analysis. In one illustrative example, a sample of crosslinked digested nuclei attached to a solid support of beads is split across 8 tubes, each containing 1 of 8 unique members of a first adaptor group (first iteration) comprising double-stranded DNA (dsDNA) adaptors to be ligated. Each of the 8 adaptors can have the same 5' overhang sequence for ligation to the nucleic acid ends of the cross-linked chromatin aggregates in the nuclei, but otherwise has a unique dsDNA sequence. After the first adaptor group is ligated, the nuclei can be pooled back together and washed to remove the ligation reaction components. The scheme of distributing, ligating, and pooling can be repeated 2 additional times (2 iterations). Following ligation of members from each adaptor group, a cross-linked chromatin aggregate can be attached to multiple barcodes in series. In some cases, the sequential ligation of a plurality of members of a plurality of adaptor groups (iterations) results in barcode combinations. The number of barcode combinations available depends on the number of groups per iteration and the total number of barcode oligonucleotides used. For example, 3 iterations comprising 8 members each can have 83 possible combinations. In some cases, barcode combinations are unique. In some cases, barcode combinations are redundant. The total number of barcode combinations can be adjusted by increasing or decreasing the number of groups receiving unique barcodes and/or increasing or decreasing the number of iterations. When more than one adaptor group is used, a distributing, attaching, and pooling scheme can be used for iterative adaptor attachment. In some cases, the scheme of distributing, attaching, and pooling can be repeated at least 3, 4, 5, 6, 7, 8, 9, or 10 additional times. In some cases, the members of the last adaptor group include a sequence for subsequent enrichment of adaptor-attached DNA, for example, during sequencing library preparation through PCR amplification.

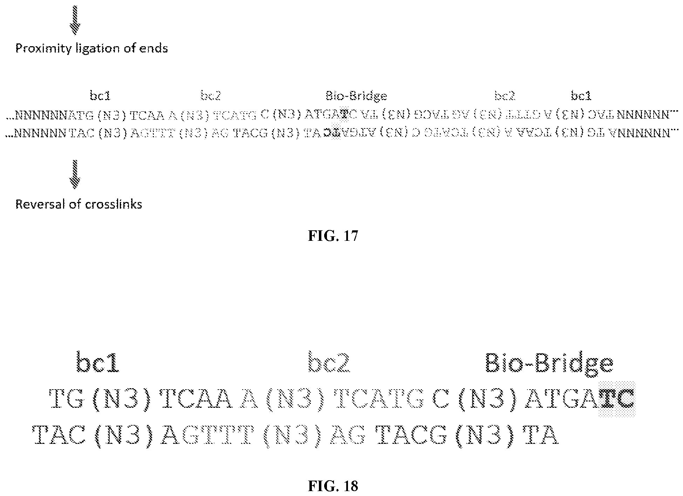

Iterating this process (of splitting and pooling) can ultimately result in each sample in the population having a unique series of bridge oligonucleotide barcodes, allowing single-sample (e.g., single cell, single nucleus, and single chromosome) analysis. **FIG. 16** and **FIG. 17** show an exemplary workflow using a splitting and pooling approach, where the nucleic acid is digested in situ and then end polished and polyadenylated. Single cells are dispensed, and a barcode is ligated to the ends present in each cell (e.g., barcode bc1). Cells are pooled and then single cells are isolated, and a second barcode is ligated to the ends present in each cell (e.g., barcode bc2). Cells are pooled again and separated into single cells before ligating a bridge adaptor (e.g., Bio-Bridge), which can be ligated to another DNA segment forming a junction between two segments having a unique combination of barcodes and adaptors identifying the cell from which the junction was derived (e.g., barcodes bc1 and bc2). The bridge adaptor can comprise one or more affinity reagents, such as biotin, for subsequent pull-down or other purification. **FIG. 18** shows an example of combinations of barcodes and a bridge resulting from the splitting and pooling approach.

In another illustrative example, a sample of crosslinked digested nuclei attached to a solid support of beads can be split across eight tubes, each containing one of eight unique members of a first adaptor group (first iteration) comprising double-stranded DNA (dsDNA) adaptors to be ligated. Each of the eight adaptors can have the same 5' overhang sequence for ligation to the nucleic acid ends of the cross-linked chromatin aggregates in the nuclei, but otherwise have a unique dsDNA sequence. After the first adaptor group is ligated, the nuclei can be pooled back together and washed to remove the ligation reaction components. The scheme of distributing, ligating, and pooling can be repeated two additional times (two iterations). Following ligation of members from each adaptor group, a cross-linked chromatin aggregate can be attached to multiple barcodes in series.

In some cases, the sequential ligation of a plurality of members of a plurality of adaptor groups (iterations) can result in barcode combinations. The number of barcode combinations available can depend on the number of groups per iteration and the total number of barcode oligonucleotides used. For example, three iterations comprising eight members each can have 83 possible combinations. In some cases, barcode combinations are unique. In certain cases, barcode combinations are redundant. The total number of barcode combinations can be adjusted by increasing or decreasing the number of groups receiving unique barcodes and/or increasing or decreasing the number of iterations. When more than one adaptor group is used, a distributing, attaching, and pooling scheme can be used for iterative adaptor attachment. In various cases, the scheme of distributing, attaching, and pooling can be repeated at least 3, 4, 5, 6, 7, 8, 9, 10, or more additional times. In some cases, the members of the last adaptor group may include a sequence for subsequent enrichment of adaptor-attached DNA, for example, during sequencing library preparation through PCR amplification.

In some cases, a three oligo design may be used, allowing for a split-pool strategy whereby two 96-well plates combined with eight different biotinylated oligos may be used, allowing for distinct barcoding of 73,728 different molecules. In certain cases, the first two sets of eight oligos are not biotinylated and the third set of eight oligos is biotinylated. In various cases, each barcoded oligonucleotide is directional allowing only one oligo to be added in each round. The bridge oligonucleotide can have a sequence that allows it to match up with a corresponding end.

In certain cases, the barcodes and adaptors may have a shorter sequence to reduce the amount of sequence space taken by the fully ligated bridges. In various cases, the bridge may take up 30 bp of sequence space. In some cases, the bridge may take up 54 bp of sequence space but offer additional positions for unique molecular identifiers (UMIs). In certain cases, UMIs may enable single-cell identification with 73,728 different combinations. In various cases, the first two oligo sets are unmodified and the third oligo set is biotinylated.

Barcode sequences in bridge adapters can be used to allow multiplexed sequencing of samples. For example, proximity ligation can be conducted on several different samples, with each sample using bridge oligonucleotides with different barcode sequences. The samples can then be pooled for multiplexed sequencing analysis, and sequence information can be de-multiplexed back to the individual samples based on the barcode sequences.

### Nucleic Acids

In eukaryotes, genomic DNA is packed into chromatin to consist as chromosomes within the nucleus. The basic structural unit of chromatin is the nucleosome, which consists of 146 base pairs (bp) of DNA wrapped around a histone octamer. The histone octamer consists of two copies each of the core histone H2A-H2B dimers and H3-H4 dimers. Nucleosomes are regularly spaced along the DNA in what is commonly referred to as "beads on a string."

The assembly of core histones and DNA into nucleosomes is mediated by chaperone proteins and associated assembly factors. Nearly all of these factors are core histone-binding proteins. Some of the histone chaperones, such as nucleosome assembly protein-1 (NAP-1), exhibit a preference for binding to histones H3 and H4. It has also been observed that newly synthesized histones are acetylated and then subsequently deacetylated after assembly into chromatin. The factors that mediate histone acetylation or deacetylation therefore play an important role in the chromatin assembly process.

In general, two in vitro methods have been developed for reconstituting or assembling chromatin. One method is ATP-independent, while the second is ATP-dependent. The ATP-independent method for reconstituting chromatin involves the DNA and core histones plus either a protein like NAP-1 or salt to act as a histone chaperone. This method results in a random arrangement of histones on the DNA that does not accurately mimic the native core nucleosome particle in the cell. These particles are often referred to as mononucleosomes because they are not regularly ordered, extended nucleosome arrays and the DNA sequence used is usually not longer than 250 bp (Kundu, T. K. et al., Mol. Cell 6: 551-561, 2000). To generate an extended array of ordered nucleosomes on a greater length of DNA sequence, the chromatin can be assembled through an ATP-dependent process.

The ATP-dependent assembly of periodic nucleosome arrays, which are similar to those seen in native chromatin, requires the DNA sequence, core histone particles, a chaperone protein and ATP-utilizing chromatin assembly factors. ACF (ATP-utilizing chromatin assembly and remodeling factor) or RSF (remodeling and spacing factor) are two widely researched assembly factors that are used to generate extended ordered arrays of nucleosomes into chromatin in vitro (Fyodorov, D.V., and Kadonaga, J.T. Method Enzymol. 371: 499-515, 2003; Kundu, T. K. et al. Mol. Cell 6: 551-561, 2000).

In particular embodiments, the methods of the disclosure can be easily applied to any type of fragmented double stranded DNA including, but not limited to, for example, free DNA isolated from plasma, serum, and/or urine; apoptotic DNA from cells and/or tissues; and/or DNA fragmented enzymatically in vitro (for example, by DNase I).

Nucleic acid obtained from biological samples can be fragmented to produce suitable fragments for analysis. Template nucleic acids may be fragmented to desired length, using a variety of enzymatic methods. DNA may be randomly sheared brief exposure to a DNase. RNA may be fragmented by brief exposure to an RNase, heat plus magnesium, or by shearing. The RNA may be converted to cDNA. If fragmentation is employed, the RNA may be converted to cDNA before or after fragmentation. Nucleic acid molecules may be single-stranded, double-stranded, or double-stranded with single-stranded regions (for example, stem- and loop-structures).

In some embodiments, cross-linked DNA molecules may be subjected to a size selection step. Size selection of the nucleic acids may be performed to cross-linked DNA molecules below or above a certain size. Size selection may further be affected by the frequency of cross-links and/or by the fragmentation method. In some embodiments, a composition may be prepared comprising cross-linking a DNA molecule in the range of about 145 bp to about 600 bp, about 100 bp to about 2500 bp, about 600 to about 2500 bp, about 350 bp to about 1000 bp, or any range bounded by any of these values (e.g., about 100 bp to about 2500 bp).

In some embodiments, sample polynucleotides are fragmented into a population of fragmented DNA molecules of one or more specific size range(s). In some embodiments, fragments can be generated from at least about 1, about 2, about 5, about 10, about 20, about 50, about 100, about 200, about 500, about 1000, about 2000, about 5000, about 10,000, about 20,000, about 50,000, about 100,000, about 200,000, about 500,000, about 1,000,000, about 2,000,000, about 5,000,000, about 10,000,000, or more genome-equivalents of starting DNA. Fragmentation may be accomplished by DNase treatment. In some embodiments, the fragments have an average length from about 10 to about 10,000, about 20,000, about 30,000, about 40,000, about 50,000, about 60,000, about 70,000, about 80,000, about 90,000, about 100,000, about 150,000, about 200,000, about 300,000, about 400,000, about 500,000, about 600,000, about 700,000, about 800,000, about 900,000, about 1,000,000, about 2,000,000, about 5,000,000, about 10,000,000, or more nucleotides. In some embodiments, the fragments have an average length from about 145 bp to about 600 bp, about 100 bp to about 2500 bp, about 600 to about 2500 bp, about 350 bp to about 1000 bp, or any range bounded by any of these values (e.g., about 100 bp to about 2500 bp). In some embodiments, the fragments have an average length less than about 2500 bp, less than about 1200 bp, less than about 1000 bp, less than about 800 bp, less than about 600 bp, less than about 350 bp, or less than about 200 bp. In other embodiments, the fragments have an average length more than about 100 bp, more than about 350 bp, more than about 600 bp, more than about 800 bp, more than about 1000 bp, more than about 1200 bp, or more than about 2000 bp. Non-limiting examples of DNases include DNase I, DNase II, micrococcal nuclease, variants thereof, and combinations thereof. For example, digestion with DNase I can induce random double-stranded breaks in DNA in the absence of Mg++ and in the presence of Mn++. Fragmentation can produce fragments having 5' overhangs, 3' overhangs, blunt ends, or a combination thereof. In some embodiments, the method includes the step of size selecting the fragments via standard methods such as column purification or isolation from an agarose gel.

### Targeted Nuclease Enzymes

Fragmented DNA as provided herein may be created or generated by digestion, such as by in situ digestion with any number of nucleases (e.g., restriction endonucleases) or DNases (e.g., MNase). In some cases, enzymes may be used in combination to achieve the desired digestion or fragmentation. In various cases, nucleases (or domains or fragments thereof) may be targeted to certain genomic sites using one or more antibodies. For example, the crosslinked sample may be contacted to an antibody that binds to certain regions of the DNA, such as a histone binding site, a transcription factor binding site, or a methylated DNA site. A nuclease linked or fused to an immunoglobulin binding protein or fragment thereof, such as a Protein A, a Protein G, a Protein A/G, or a Protein L, can then be added to the sample and the nuclease may digest the DNA only in the region where the antibody bound. This may be done in combination, for example, where a first antibody is bound to the DNA sample, then the nuclease is targeted to the first antibody, then a second antibody is bound to the DNA sample and the nuclease is targeted to the second antibody, and so on to achieve the desired digestion pattern.

### Ligation

In some embodiments, the 5' and/or 3' end nucleotide sequences of fragmented DNA are not modified prior to ligation. For example, cleavage by an enzyme that leaves a predictable blunt end can be followed by ligation of blunt-ended DNA fragments to nucleic acids, such as adaptors, oligonucleotides, or polynucleotides, comprising a blunt end. In some embodiments, the fragmented DNA molecules are blunt-end polished (or "end repaired") to produce DNA fragments having blunt ends, prior to being joined to adaptors. The blunt-end polishing step may be accomplished by incubation with a suitable enzyme, such as a DNA polymerase that has both 3' to 5' exonuclease activity and 5' to 3' polymerase activity, for example, T4 polymerase. In some embodiments, end repair can be followed by an addition of 1, 2, 3, 4, 5, 6, 7, 8, 9, 10, 11, 12, 13, 14, 15, 16, 17, 18, 19, 20 or more nucleotides, such as one or more adenine, one or more thymine, one or more guanine, or one or more cytosine, to produce an overhang. For example, the end pair can be followed by an addition of 1, 2, 3, 4, 5, or 6 nucleotides. DNA fragments having an overhang can be joined to one or more nucleic acids, such as oligonucleotides, adaptor oligonucleotides, or polynucleotides, having a complementary overhang, such as in a ligation reaction. For example, a single adenine can be added to the 3' ends of end repaired DNA fragments using a template independent polymerase, followed by ligation to one or more adaptors each having a thymine at a 3' end. In some embodiments, nucleic acids, such as oligonucleotides or polynucleotides can be joined to blunt end double-stranded DNA molecules which have been modified by extension of the 3' end with one or more nucleotides followed by 5' phosphorylation. In some cases, extension of the 3' end may be performed with a polymerase such as, Klenow polymerase or any of the suitable polymerases provided herein, or by use of a terminal deoxynucleotide transferase, in the presence of one or more dNTPs in a suitable buffer that can contain magnesium. In some embodiments, target polynucleotides having blunt ends are joined to one or more adaptors comprising a blunt end. Phosphorylation of 5' ends of DNA fragment molecules may be performed, for example, with T4 polynucleotide kinase in a suitable buffer containing ATP and magnesium. The fragmented DNA molecules may optionally be treated to dephosphorylate 5' ends or 3' ends, for example, by using enzymes known in the art, such as phosphatases.

The terms "connecting," "joining" and "ligation" as used herein, with respect to two polynucleotides, such as an adaptor oligonucleotide and a target polynucleotide, refers to the covalent attachment of two separate DNA segments to produce a single larger polynucleotide with a contiguous backbone. Methods for joining two DNA segments are known in the art, and include without limitation, enzymatic and non-enzymatic (e.g., chemical) methods. Examples of ligation reactions that are non-enzymatic include the non-enzymatic ligation techniques described in U.S. Pat. Nos. 5,780,613 and 5,476,930. In some embodiments, an adaptor oligonucleotide is joined to a target polynucleotide by a ligase, for example, a DNA ligase or RNA ligase. Multiple ligases, each having characterized reaction conditions, are known in the art, and include, without limitation NAD+-dependent ligases including tRNA ligase, Taq DNA ligase, Thermus filiformis DNA ligase, Escherichia coli DNA ligase, Tth DNA ligase, Thermus scotoductus DNA ligase (I and II), thermostable ligase, Ampligase thermostable DNA ligase, VanC-type ligase, 9° N DNA Ligase, Tsp DNA ligase, and novel ligases discovered by bioprospecting; ATP-dependent ligases including T4 RNA ligase, T4 DNA ligase, T3 DNA ligase, T7 DNA ligase, Pfu DNA ligase, DNA ligase 1, DNA ligase III, DNA ligase IV, and novel ligases discovered by bioprospecting; and wild-type, mutant isoforms, and genetically engineered variants thereof.

Ligation can be between DNA segments having hybridizable sequences, such as complementary overhangs. Ligation can also be between two blunt ends. Generally, a 5' phosphate is utilized in a ligation reaction. The 5' phosphate can be provided by the target polynucleotide, the adaptor oligonucleotide, or both. 5' phosphates can be added to or removed from DNA segments to be joined, as needed. Methods for the addition or removal of 5' phosphates are known in the art, and include without limitation enzymatic and chemical processes. Enzymes useful in the addition and/or removal of 5' phosphates include kinases, phosphatases, and polymerases. In some embodiments, both of the two ends joined in a ligation reaction (e.g., an adaptor end and a target polynucleotide end) provide a 5' phosphate, such that two covalent linkages are made in joining the two ends. In some embodiments, only one of the two ends joined in a ligation reaction (e.g., only one of an adaptor end and a target polynucleotide end) provides a 5' phosphate, such that only one covalent linkage is made in joining the two ends.

In some embodiments, only one strand at one or both ends of a target polynucleotide is joined to an adaptor oligonucleotide. In some embodiments, both strands at one or both ends of a target polynucleotide are joined to an adaptor oligonucleotide. In some embodiments, 3' phosphates are removed prior to ligation. In some embodiments, an adaptor oligonucleotide is added to both ends of a target polynucleotide, wherein one or both strands at each end are joined to one or more adaptor oligonucleotides. When both strands at both ends are joined to an adaptor oligonucleotide, joining can be followed by a cleavage reaction that leaves a 5' overhang that can serve as a template for the extension of the corresponding 3' end, which 3' end may or may not include one or more nucleotides derived from the adaptor oligonucleotide. In some embodiments, a target polynucleotide is joined to a first adaptor oligonucleotide on one end and a second adaptor oligonucleotide on the other end. In some embodiments, two ends of a target polynucleotide are joined to the opposite ends of a single adaptor oligonucleotide. In some embodiments, the target polynucleotide and the adaptor oligonucleotide to which it is joined comprise blunt ends. In some embodiments, separate ligation reactions can be carried out for each sample, using a different first adaptor oligonucleotide comprising at least one barcode sequence for each sample, such that no barcode sequence is joined to the target polynucleotides of more than one sample. A DNA segment or a target polynucleotide that has an adaptor oligonucleotide joined to it is considered "tagged" by the joined adaptor.

In some cases, the ligation reaction can be performed at a DNA segment or target polynucleotide concentration of about 0.1 ng/µL, about 0.2 ng/µL, about 0.3 ng/µL, about 0.4 ng/µL, about 0.5 ng/µL, about 0.6 ng/µL, about 0.7 ng/µL, about 0.8 ng/µL, about 0.9 ng/µL, about 1.0 ng/µL, about 1.2 ng/µL, about 1.4 ng/µL, about 1.6 ng/µL, about 1.8 ng/µL, about 2.0 ng/µL, about 2.5 ng/µL, about 3.0 ng/µL, about 3.5 ng/µL, about 4.0 ng/µL, about 4.5 ng/µL, about 5.0 ng/µL, about 6.0 ng/µL, about 7.0 ng/µL, about 8.0 ng/µL, about 9.0 ng/µL, about 10 ng/µL, about 15 ng/µL, about 20 ng/µL, about 30 ng/µL, about 40 ng/µL, about 50 ng/µL, about 60 ng/µL, about 70 ng/µL, about 80 ng/µL, about 90 ng/µL, about 100 ng/µL, about 150 ng/µL, about 200 ng/µL, about 300 ng/µL, about 400 ng/µL, about 500 ng/µL, about 600 ng/µL, about 800 ng/µL, or about 1000 ng/µL. For example, the ligation can be performed at a DNA segment or target polynucleotide concentration of about 100 ng/µL, about 150 ng/µL, about 200 ng/µL, about 300 ng/µL, about 400 ng/µL, or about 500 ng/µL.

In some cases, the ligation reaction can be performed at a DNA segment or target polynucleotide concentration of about 0.1 to 1000 ng/µL, about 1 to 1000 ng/µL, about 1 to 800 ng/µL, about 10 to 800 ng/µL, about 10 to 600 ng/µL, about 100 to 600 ng/µL, or about 100 to 500 ng/µL.

In some cases, the ligation reaction can be performed for more than about 5 minutes, about 10 minutes, about 20 minutes, about 30 minutes, about 40 minutes, about 50 minutes, about 60 minutes, about 90 minutes, about 2 hours, about 3 hours, about 4 hours, about 5 hours, about 6 hours, about 8 hours, about 10 hours, about 12 hours, about 18 hours, about 24 hours, about 36 hours, about 48 hours, or about 96 hours. In other cases, the ligation reaction can be performed for less than about 5 minutes, about 10 minutes, about 20 minutes, about 30 minutes, about 40 minutes, about 50 minutes, about 60 minutes, about 90 minutes, about 2 hours, about 3 hours, about 4 hours, about 5 hours, about 6 hours, about 8 hours, about 10 hours, about 12 hours, about 18 hours, about 24 hours, about 36 hours, about 48 hours, or about 96 hours. For example, the ligation reaction can be performed for about 30 minutes to about 90 minutes. In some embodiments, joining of an adaptor to a target polynucleotide produces a joined product polynucleotide having a 3' overhang comprising a nucleotide sequence derived from the adaptor.

In some embodiments, after joining at least one adaptor oligonucleotide to a target polynucleotide, the 3' end of one or more target polynucleotides is extended using the one or more joined adaptor oligonucleotides as template. For example, an adaptor comprising two hybridized oligonucleotides that is joined to only the 5' end of a target polynucleotide allows for the extension of the unjoined 3' end of the target using the joined strand of the adaptor as template, concurrently with or following displacement of the unjoined strand. Both strands of an adaptor comprising two hybridized oligonucleotides may be joined to a target polynucleotide such that the joined product has a 5' overhang, and the complementary 3' end can be extended using the 5' overhang as template. As a further example, a hairpin adaptor oligonucleotide can be joined to the 5' end of a target polynucleotide. In some embodiments, the 3' end of the target polynucleotide that is extended comprises one or more nucleotides from an adaptor oligonucleotide. For target polynucleotides to which adaptors are joined on both ends, extension can be carried out for both 3' ends of a double-stranded target polynucleotide having 5' overhangs. This 3' end extension, or "fill-in" reaction, generates a complementary sequence, or "complement," to the adaptor oligonucleotide template that is hybridized to the template, thus filling in the 5' overhang to produce a double-stranded sequence region. Where both ends of a double-stranded target polynucleotide have 5' overhangs that are filled in by extension of the complementary strands' 3' ends, the product is completely double-stranded. Extension can be carried out by any suitable polymerase known in the art, such as a DNA polymerase, many of which are commercially available. DNA polymerases can comprise DNA-dependent DNA polymerase activity, RNA-dependent DNA polymerase activity, or DNA-dependent and RNA-dependent DNA polymerase activity. DNA polymerases can be thermostable or non-thermostable. Examples of DNA polymerases include, but are not limited to, Taq polymerase, Tth polymerase, Tli polymerase, Pfu polymerase, Pfutubo polymerase, Pyrobest polymerase, Pwo polymerase, KOD polymerase, Bst polymerase, Sac polymerase, Sso polymerase, Poc polymerase, Pab polymerase, Mth polymerase, Pho polymerase, ES4 polymerase, VENT polymerase, DEEPVENT polymerase, EX-Taq polymerase, LA-Taq polymerase, Expand polymerases, Platinum Taq polymerases, Hi-Fi polymerase, Tbr polymerase, Tfl polymerase, Tru polymerase, Tac polymerase, Tne polymerase, Tma polymerase, Tih polymerase, Tfi polymerase, Klenow fragment, and variants, modified products and derivatives thereof 3' end extension can be performed before or after pooling of target polynucleotides from independent samples.

### Target Enrichment

In certain embodiments, the disclosure provides methods for the enrichment of a target nucleic acids and analysis of the target nucleic acids. In some cases, the methods for enrichment is in a solution-based format. In some cases, the target nucleic acid can be labeled with a labeling agent. In other cases, the target nucleic acid can be crosslinked to one or more association molecules that are labeled with a labeling agent. Examples of labeling agents include, but are not limited to, biotin, polyhistidine tags, and chemical tags (e.g., alkyne and azide derivatives used in Click Chemistry methods). Further, the labeled target nucleic acid can be captured and thereby enriched by using a capturing agent. The capturing agent can be streptavidin and/or avidin, an antibody, a chemical moiety (e.g., alkyne, azide), and any biological, chemical, physical, or enzymatic agents used for affinity purification known in the art.

In some cases, immobilized or non-immobilized nucleic acid probes can be used to capture the target nucleic acids. For example, the target nucleic acids can be enriched from a sample by hybridization to the probes on a solid support or in solution. In some examples, the sample can be a genomic sample. In some examples, the probes can be an amplicon. The amplicon can comprise a predetermined sequence. Further, the hybridized target nucleic acids can be washed and/or eluted off of the probes. The target nucleic acid can be a DNA, RNA, cDNA, or mRNA molecule.

In some cases, the enrichment method can comprise contacting the sample comprising the target nucleic acid to the probes and binding the target nucleic acid to a solid support. In some cases, the sample can be fragmented using enzymatic methods to yield the target nucleic acids. In some cases, the probes can be specifically hybridized to the target nucleic acids. In some cases, the target nucleic acids can have an average size of about 145 bp to about 600 bp, about 100 bp to about 2500 bp, about 600 to about 2500 bp, or about 350 bp to about 1000 bp. The target nucleic acids can be further separated from the unbound nucleic acids in the sample. The solid support can be washed and/or eluted to provide the enriched target nucleic acids. In some examples, the enrichment steps can be repeated for about 1, 2, 3, 4, 5, 6, 7, 8, 9, or 10 times. For example, the enrichment steps can be repeated for about 1, 2, or 3 times.

In some cases, the enrichment method can comprise providing probe derived amplicons wherein said probes for amplification are attached to a solid support. The solid support can comprise support-immobilized nucleic acid probes to capture specific target nucleic acid from a sample. The probe derived amplicons can hybridize to the target nucleic acids. Following hybridization to the probe amplicons, the target nucleic acids in the sample can be enriched by capturing (e.g., via capturing agents as biotin, antibodies, etc.) and washing and/or eluting the hybridized target nucleic acids from the captured probes. The target nucleic acid sequence(s) may be further amplified using, for example, PCR methods to produce an amplified pool of enriched PCR products.

In some cases, the solid support can be a microarray, a slide, a chip, a microwell, a column, a tube, a particle or a bead. In some examples, the solid support can be coated with streptavidin and/or avidin. In other examples, the solid support can be coated with an antibody. Further, the solid support can comprise a glass, metal, ceramic or polymeric material. In some embodiments, the solid support can be a nucleic acid microarray (e.g., a DNA microarray). In other embodiments, the solid support can be a paramagnetic bead.

In particular embodiments, the disclosure provides methods for amplifying the enriched DNA. In some cases, the enriched DNA is a read-pair. The read-pair can be obtained by the methods of the present disclosure.

In some embodiments, the one or more amplification and/or replication steps are used for the preparation of a library to be sequenced. Any amplification method known in the art may be used. Examples of amplification techniques that can be used include, but are not limited to, quantitative PCR, quantitative fluorescent PCR (QF-PCR), multiplex fluorescent PCR (MF-PCR), real time PCR (RTPCR), single cell PCR, restriction fragment length polymorphism PCR (PCR-RFLP), PCK-RFLPIRT-PCR-IRFLP, hot start PCR, nested PCR, in situ polonony PCR, in situ rolling circle amplification (RCA), bridge PCR, ligation mediated PCR, Qb replicase amplification, inverse PCR, picotiter PCR and emulsion PCR. Other suitable amplification methods include the ligase chain reaction (LCR), transcription amplification, self-sustained sequence replication, selective amplification of target polynucleotide sequences, consensus sequence primed polymerase chain reaction (CP-PCR), arbitrarily primed polymerase chain reaction (AP-PCR), degenerate oligonucleotide-primed PCR (DOP-PCR) and nucleic acid-based sequence amplification (NABSA). Other amplification methods that can be used herein include those described in U.S. Patent Nos. 5,242,794; 5,494,810; 4,988,617; and 6,582,938.

In particular embodiments, PCR is used to amplify DNA molecules after they are dispensed into individual partitions. In some cases, one or more specific priming sequences within amplification adaptors are utilized for PCR amplification. The amplification adaptors may be ligated to fragmented DNA molecules before or after dispensing into individual partitions. Polynucleotides comprising amplification adaptors with suitable priming sequences on both ends can be PCR amplified exponentially. Polynucleotides with only one suitable priming sequence due to, for example, imperfect ligation efficiency of amplification adaptors comprising priming sequences, may only undergo linear amplification. Further, polynucleotides can be eliminated from amplification, for example, PCR amplification, all together, if no adaptors comprising suitable priming sequences are ligated. In some embodiments, the number of PCR cycles vary between 10-30, but can be as low as 9, 8, 7, 6, 5, 4, 3, 2 or less or as high as 40, 45, 50, 55, 60 or more. As a result, exponentially amplifiable fragments carrying amplification adaptors with a suitable priming sequence can be present in much higher (1000 fold or more) concentration compared to linearly amplifiable or un-amplifiable fragments, after a PCR amplification. Benefits of PCR, as compared to whole genome amplification techniques (such as amplification with randomized primers or Multiple Displacement Amplification using phi29 polymerase) include, but are not limited to, a more uniform relative sequence coverage - as each fragment can be copied at most once per cycle and as the amplification is controlled by thermocycling program, a substantially lower rate of forming chimeric molecules than, for example, MDA (Lasken et al., 2007, BMC Biotechnology) - as chimeric molecules pose significant challenges for accurate sequence assembly by presenting nonbiological sequences in the assembly graph, which may result in higher rate of misassemblies or highly ambiguous and fragmented assembly, reduced sequence specific biases that may result from binding of randomized primers commonly used in MDA versus using specific priming sites with a specific sequence, a higher reproducibility in the amount of final amplified DNA product, which can be controlled by selection of the number of PCR cycles, and a higher fidelity in replication with the polymerases that are commonly used in PCR as compared to common whole genome amplification techniques known in the art.

In some embodiments, the fill-in reaction is followed by or performed as part of amplification of one or more target polynucleotides using a first primer and a second primer, wherein the first primer comprises a sequence that is hybridizable to at least a portion of the complement of one or more of the first adaptor oligonucleotides, and further wherein the second primer comprises a sequence that is hybridizable to at least a portion of the complement of one or more of the second adaptor oligonucleotides. Each of the first and second primers may be of any suitable length, such as about, less than about, or more than about 10, 15, 20, 25, 30, 35, 40, 45, 50, 55, 60, 65, 70, 75, 80, 90, 100, or more nucleotides, any portion or all of which may be complementary to the corresponding target sequence (e.g., about, less than about, or more than about 5, 10, 15, 20, 25, 30, 35, 40, 45, 50, or more nucleotides). For example, about 10 to 50 nucleotides can be complementary to the corresponding target sequence.

"Amplification" refers to any process by which the copy number of a target sequence is increased. In some cases, a replication reaction may produce only a single complementary copy/replica of a polynucleotide. Methods for primer-directed amplification of target polynucleotides are known in the art, and include without limitation, methods based on the polymerase chain reaction (PCR). Conditions favorable to the amplification of target sequences by PCR are known in the art, can be optimized at a variety of steps in the process, and depend on characteristics of elements in the reaction, such as target type, target concentration, sequence length to be amplified, sequence of the target and/or one or more primers, primer length, primer concentration, polymerase used, reaction volume, ratio of one or more elements to one or more other elements, and others, some or all of which can be altered. In general, PCR involves the steps of denaturation of the target to be amplified (if double stranded), hybridization of one or more primers to the target, and extension of the primers by a DNA polymerase, with the steps repeated (or "cycled") in order to amplify the target sequence. Steps in this process can be optimized for various outcomes, such as to enhance yield, decrease the formation of spurious products, and/or increase or decrease specificity of primer annealing. Methods of optimization are well known in the art and include adjustments to the type or number of elements in the amplification reaction and/or to the conditions of a given step in the process, such as temperature at a particular step, duration of a particular step, and/or number of cycles.

In some embodiments, an amplification reaction can comprise at least about 5, 10, 15, 20, 25, 30, 35, 40, 50, 60, 70, 80, 90, 100, 150, 200 or more cycles. In some examples, an amplification reaction can comprise at least about 20, 25, 30, 35 or 40 cycles. In some embodiments, an amplification reaction comprises no more than about 5, 10, 15, 20, 25, 35, 40, 50, 60, 70, 80, 90, 100, 150, 200 or more cycles. Cycles can contain any number of steps, such as 1, 2, 3, 4, 5, 6, 7, 8, 9, 10 or more steps. Steps can comprise any temperature or gradient of temperatures, suitable for achieving the purpose of the given step including, but not limited to, 3' end extension (e.g., adaptor fill-in), primer annealing, primer extension, and strand denaturation. Steps can be of any duration including, but not limited to, about, less than about, or more than about 1, 5, 10, 15, 20, 25, 30, 35, 40, 45, 50, 55, 60, 70, 80, 90, 100, 120, 180, 240, 300, 360, 420, 480, 540, 600, 1200, 1800, or more seconds, including indefinitely until manually interrupted. Cycles of any number comprising different steps can be combined in any order. In some embodiments, different cycles comprising different steps are combined such that the total number of cycles in the combination is about, less that about, or more than about 5, 10, 15, 20, 25, 30, 35, 40, 50, 60, 70, 80, 90, 100, 150, 200 or more cycles. In some embodiments, amplification is performed following the fill-in reaction.

In some embodiments, the amplification reaction can be carried out on at least about 1, 2, 3, 4, 5, 6, 7, 8, 9, 10, 12, 14, 16, 18, 20, 25, 30, 40, 50, 100, 200, 300, 400, 500, 600, 800, 1000 ng of the target DNA molecule. In other embodiments, the amplification reaction can be carried out on less than about 1, 2, 3, 4, 5, 6, 7, 8, 9, 10, 12, 14, 16, 18, 20, 25, 30, 40, 50, 100, 200, 300, 400, 500, 600, 800, 1000 ng of the target DNA molecule.

Amplification can be performed before or after pooling of target polynucleotides from independent samples.

Methods of the disclosure involve determining an amount of amplifiable nucleic acid present in a sample. Any known method may be used to quantify amplifiable nucleic acid, and an exemplary method is the polymerase chain reaction (PCR), specifically quantitative polymerase chain reaction (qPCR). qPCR is a technique based on the polymerase chain reaction, and is used to amplify and simultaneously quantify a targeted nucleic acid molecule. qPCR allows for both detection and quantification (as absolute number of copies or relative amount when normalized to DNA input or additional normalizing genes) of a specific sequence in a DNA sample. The procedure follows the general principle of polymerase chain reaction, with the additional feature that the amplified DNA is quantified as it accumulates in the reaction in real time after each amplification cycle. QPCR is described, for example, in Kurnit et al. (U.S. patent number 6,033,854), Wang et al. (U.S. patent number 5,567,583 and 5,348,853), Ma et al. (The Journal of American Science, 2(3), 2006), Heid et al. (Genome Research 986-994, 1996), Sambrook and Russell (Quantitative PCR, Cold Spring Harbor Protocols, 2006), and Higuchi (U.S. patent numbers 6,171,785 and 5,994,056). Other methods of quantification include use of fluorescent dyes that intercalate with double-stranded DNA, and modified DNA oligonucleotide probes that fluoresce when hybridized with a complementary DNA. These methods can be broadly used but are also specifically adapted to real-time PCR as described in further detail as an example. In the first method, a DNA-binding dye binds to all double-stranded (ds)DNA in PCR, resulting in fluorescence of the dye. An increase in DNA product during PCR therefore leads to an increase in fluorescence intensity and is measured at each cycle, thus allowing DNA concentrations to be quantified. The reaction is prepared similarly to a standard PCR reaction, with the addition of fluorescent (ds)DNA dye. The reaction is run in a thermocycler, and after each cycle, the levels of fluorescence are measured with a detector; the dye only fluoresces when bound to the (ds)DNA (i.e., the PCR product). With reference to a standard dilution, the (ds)DNA concentration in the PCR can be determined. Like other real-time PCR methods, the values obtained do not have absolute units associated with it. A comparison of a measured DNA/RNA sample to a standard dilution gives a fraction or ratio of the sample relative to the standard, allowing relative comparisons between different tissues or experimental conditions. To ensure accuracy in the quantification and/or expression of a target gene can be normalized with respect to a stably expressed gene. Copy numbers of unknown genes can similarly be normalized relative to genes of known copy number.

The second method uses a sequence-specific RNA or DNA-based probe to quantify only the DNA containing a probe sequence; therefore, use of the reporter probe significantly increases specificity, and allows quantification even in the presence of some non-specific DNA amplification. This allows for multiplexing, i.e., assaying for several genes in the same reaction by using specific probes with differently colored labels, provided that all genes are amplified with similar efficiency.

This method is commonly carried out with a DNA-based probe with a fluorescent reporter (e.g., 6-carboxyfluorescein) at one end and a quencher (e.g., 6-carboxy-tetramethylrhodamine) of fluorescence at the opposite end of the probe. The close proximity of the reporter to the quencher prevents detection of its fluorescence. Breakdown of the probe by the 5' to 3' exonuclease activity of a polymerase (e.g., Taq polymerase) breaks the reporter-quencher proximity and thus allows unquenched emission of fluorescence, which can be detected. An increase in the product targeted by the reporter probe at each PCR cycle results in a proportional increase in fluorescence due to breakdown of the probe and release of the reporter. The reaction is prepared similarly to a standard PCR reaction, and the reporter probe is added. As the reaction commences, during the annealing stage of the PCR both probe and primers anneal to the DNA target. Polymerization of a new DNA strand is initiated from the primers, and once the polymerase reaches the probe, its 5'-3'-exonuclease degrades the probe, physically separating the fluorescent reporter from the quencher, resulting in an increase in fluorescence. Fluorescence is detected and measured in a real-time PCR thermocycler, and geometric increase of fluorescence corresponding to exponential increase of the product is used to determine the threshold cycle in each reaction.

Relative concentrations of DNA present during the exponential phase of the reaction are determined by plotting fluorescence against cycle number on a logarithmic scale (so an exponentially increasing quantity will give a straight line). A threshold for detection of fluorescence above background is determined. The cycle at which the fluorescence from a sample crosses the threshold is called the cycle threshold, Ct. Since the quantity of DNA doubles every cycle during the exponential phase, relative amounts of DNA can be calculated, e.g., a sample with a Ct of 3 cycles earlier than another has 23 = 8 times more template. Amounts of nucleic acid (e.g., RNA or DNA) are then determined by comparing the results to a standard curve produced by a real-time PCR of serial dilutions (e.g., undiluted, 1:4, 1:16, 1:64) of a known amount of nucleic acid.

In certain embodiments, the qPCR reaction involves a dual fluorophore approach that takes advantage of fluorescence resonance energy transfer (FRET), e.g., LIGHTCYCLER hybridization probes, where two oligonucleotide probes anneal to the amplicon (see, e.g., U.S. patent number 6,174,670). The oligonucleotides are designed to hybridize in a head-to-tail orientation with the fluorophores separated at a distance that is compatible with efficient energy transfer. Other examples of labeled oligonucleotides that are structured to emit a signal when bound to a nucleic acid or incorporated into an extension product include: SCORPIONS probes (e.g., Whitcombe et al., Nature Biotechnology 17:804-807, 1999, and U.S. patent number 6,326,145), Sunrise (or AMPLIFLOUR) primers (e.g., Nazarenko et al., Nuc. Acids Res. 25:2516-2521, 1997, and U.S. patent number 6,117,635), and LUX primers and MOLECULAR BEACONS probes (e.g., Tyagi et al., Nature Biotechnology 14:303-308, 1996 and U.S. patent number 5,989,823).

In other embodiments, a qPCR reaction uses fluorescent Taqman methodology and an instrument capable of measuring fluorescence in real time (e.g., ABI Prism 7700 Sequence Detector). The Taqman reaction uses a hybridization probe labeled with two different fluorescent dyes. One dye is a reporter dye

(6-carboxyfluorescein), the other is a quenching dye (6-carboxy-tetramethylrhodamine). When the probe is intact, fluorescent energy transfer occurs and the reporter dye fluorescent emission is absorbed by the quenching dye. During the extension phase of the PCR cycle, the fluorescent hybridization probe is cleaved by the 5'-3' nucleolytic activity of the DNA polymerase. On cleavage of the probe, the reporter dye emission is no longer transferred efficiently to the quenching dye, resulting in an increase of the reporter dye fluorescent emission spectra. Any nucleic acid quantification method, including real-time methods or single-point detection methods may be used to quantify the amount of nucleic acid in the sample. The detection can be performed by several different methodologies (e.g., staining, hybridization with a labeled probe; incorporation of biotinylated primers followed by avidin-enzyme conjugate detection; incorporation of 32P-labeled deoxynucleotide triphosphates, such as dCTP or dATP, into the amplified segment), as well as any other suitable detection method known in the art for nucleic acid quantification. The quantification may or may not include an amplification step.

In some embodiments, the disclosure provides labels for identifying or quantifying the linked DNA segments. In some cases, the linked DNA segments can be labeled in order to assist in downstream applications, such as array hybridization. For example, the linked DNA segments can be labeled using random priming or nick translation.

A wide variety of labels (e.g., reporters) may be used to label the nucleotide sequences described herein including, but not limited to, during the amplification step. Suitable labels include radionuclides, enzymes, fluorescent, chemiluminescent, or chromogenic agents as well as ligands, cofactors, inhibitors, magnetic particles and the like. Examples of such labels are included in U.S. Pat. No. 3,817,837; U.S. Pat. No. 3,850,752; U.S. Pat. No. 3,939,350; U.S. Pat. No. 3,996,345; U.S. Pat. No. 4,277,437; U.S. Pat. No. 4,275,149 and U.S. Pat. No. 4,366,241.

Additional labels include, but are not limited to, β-galactosidase, invertase, green fluorescent protein, luciferase, chloramphenicol, acetyltransferase, β-glucuronidase, exo-glucanase and glucoamylase. Fluorescent labels may also be used, as well as fluorescent reagents specifically synthesized with particular chemical properties. A wide variety of ways to measure fluorescence are available. For example, some fluorescent labels exhibit a change in excitation or emission spectra, some exhibit resonance energy transfer where one fluorescent reporter loses fluorescence, while a second gains in fluorescence, some exhibit a loss (quenching) or appearance of fluorescence, while some report rotational movements.

Further, in order to obtain sufficient material for labeling, multiple amplifications may be pooled, instead of increasing the number of amplification cycles per reaction. Alternatively, labeled nucleotides can be incorporated in to the last cycles of the amplification reaction, e.g., 30 cycles of PCR (no label) +10 cycles of PCR (plus label).

In particular embodiments, the disclosure provides probes that can attach to the linked DNA segments. As used herein, the term "probe" refers to a molecule (e.g., an oligonucleotide, whether occurring naturally as in a purified restriction digest or produced synthetically, recombinantly or by PCR amplification), that is capable of hybridizing to another molecule of interest (e.g., another oligonucleotide). When probes are oligonucleotides, they may be single-stranded or double-stranded. Probes are useful in the detection, identification and isolation of particular targets (e.g., gene sequences). In some cases, the probes may be associated with a label so that is detectable in any detection system including, but not limited to, enzyme (e.g., ELISA, as well as enzyme-based histochemical assays), fluorescent, radioactive, and luminescent systems

With respect to arrays and microarrays, the term "probe" is used to refer to any hybridizable material that is affixed to the array for the purpose of detecting a nucleotide sequence that has hybridized to said probe. In some cases, the probes can about 10 bp to 500 bp, about 10 bp to 250 bp, about 20 bp to 250 bp, about 20 bp to 200 bp, about 25 bp to 200 bp, about 25 bp to 100 bp, about 30 bp to 100 bp, or about 30 bp to 80 bp. In some cases, the probes can be greater than about 10 bp, about 20 bp, about 30 bp, about 40 bp ,about 50 bp, about 60 bp, about 70 bp, about 80 bp, about 90 bp, about 100 bp, about 150 bp, about 200 bp, about 250 bp, about 300 bp, about 400 bp, or about 500 bp in length. For example, the probes can be about 20 to about 50 bp in length. Examples and rationale for probe design can be found in WO95/11995, EP 717,113 and WO97/29212

The probes, array of probes or set of probes can be immobilized on a support. Supports (e.g., solid supports) can be made of a variety of materials-such as glass, silica, plastic, nylon or nitrocellulose. Supports can be rigid and have a planar surface. Supports can have from about 1 to 10,000,000 resolved loci. For example, a support can have about 10 to 10,000,000, about 10 to 5,000,000, about 100 to 5,000,000, about 100 to 4,000,000, about 1000 to 4,000,000, about 1000 to 3,000,000, about 10,000 to 3,000,000, about 10,000 to 2,000,000, about 100,000 to 2,000,000, or about 100,000 to 1,000,000 resolved loci. The density of resolved loci can be at least about 10, about 100, about 1000, about 10,000, about 100,000 or about 1,000,000 resolved loci within a square centimeter. In some cases, each resolved locus can be occupied by >95% of a single type of oligonucleotide. In other cases, each resolved locus can be occupied by pooled mixtures of probes or a set of probes. In further cases, some resolved loci are occupied by pooled mixtures of probes or a set of probes, and other resolved loci are occupied by >95% of a single type of oligonucleotide.

**In** some cases, the number of probes for a given nucleotide sequence on the array can be in large excess to the DNA sample to be hybridized to such array. For example, the array can have about 10, about 100, about 1000, about 10,000, about 100,000, about 1,000,000, about 10,000,000, or about 100,000,000 times the number of probes relative to the amount of DNA in the input sample.

**In** some cases, an array can have about 10, about 100, about 1000, about 10,000, about 100,000, about 1,000,000, about 10,000,000, about 100,000,000, or about 1,000,000,000 probes.

Arrays of probes or sets of probes may be synthesized in a step-by-step manner on a support or can be attached in presynthesized form. One method of synthesis is VLSIPS^{™} (as described in U.S. Pat. No. 5,143,854 and EP 476,014), which entails the use of light to direct the synthesis of oligonucleotide probes in high-density, miniaturized arrays. Algorithms for design of masks to reduce the number of synthesis cycles are described in U.S. Pat. No. 5,571,639 and U.S. Pat. No. 5,593,839. Arrays can also be synthesized in a combinatorial fashion by delivering monomers to cells of a support by mechanically constrained flowpaths, as described in EP 624,059. Arrays can also be synthesized by spotting reagents on to a support using an ink jet printer (see, for example, EP 728,520).

In some embodiments, the present disclosure provides methods for hybridizing the linked DNA segments onto an array. A "substrate" or an "array" is an intentionally created collection of nucleic acids which can be prepared either synthetically or biosynthetically and screened for biological activity in a variety of different formats (e.g., libraries of soluble molecules; and libraries of oligonucleotides tethered to resin beads, silica chips, or other solid supports). Additionally, the term "array" includes those libraries of nucleic acids which can be prepared by spotting nucleic acids of essentially any length (e.g., from 1 to about 1000 nucleotide monomers in length) onto a substrate.

Array technology and the various associated techniques and applications are described generally in numerous textbooks and documents. For example, these include Lemieux et al., 1998, Molecular Breeding 4, 277-289; Schena and Davis, Parallel Analysis with Biological Chips. in PCR Methods Manual (eds. M. Innis, D. Gelfand, J. Sninsky); Schena and Davis, 1999, Genes, Genomes and Chips. In DNA Microarrays: A Practical Approach (ed. M. Schena), Oxford University Press, Oxford, UK, 1999); The Chipping Forecast (Nature Genetics special issue; January 1999 Supplement); Mark Schena (Ed.), Microarray Biochip Technology, (Eaton Publishing Company); Cortes, 2000, The Scientist 14[17]:25; Gwynn and Page, Microarray analysis: the next revolution in molecular biology, Science, 1999 Aug. 6; and Eakins and Chu, 1999, Trends in Biotechnology, 17, 217-218.

In general, any library may be arranged in an orderly manner into an array, by spatially separating the members of the library. Examples of suitable libraries for arraying include nucleic acid libraries (including DNA, cDNA, oligonucleotide, etc. libraries), peptide, polypeptide and protein libraries, as well as libraries comprising any molecules, such as ligand libraries, among others.

The library can be fixed or immobilized onto a solid phase (e.g., a solid substrate), to limit diffusion and admixing of the members. In some cases, libraries of DNA binding ligands may be prepared. In particular, the libraries may be immobilized to a substantially planar solid phase, including membranes and non-porous substrates such as plastic and glass. Furthermore, the library can be arranged in such a way that indexing (i.e., reference or access to a particular member) is facilitated. In some examples, the members of the library can be applied as spots in a grid formation. Common assay systems may be adapted for this purpose. For example, an array may be immobilized on the surface of a microplate, either with multiple members in a well, or with a single member in each well. Furthermore, the solid substrate may be a membrane, such as a nitrocellulose or nylon membrane (for example, membranes used in blotting experiments). Alternative substrates include glass, or silica-based substrates. Thus, the library can be immobilized by any suitable method known in the art, for example, by charge interactions, or by chemical coupling to the walls or bottom of the wells, or the surface of the membrane. Other means of arranging and fixing may be used, for example, pipetting, drop-touch, piezoelectric means, ink-jet and bubblejet technology, electrostatic application, etc. In the case of silicon-based chips, photolithography may be utilized to arrange and fix the libraries on the chip.

The library may be arranged by being "spotted" onto the solid substrate; this may be done by hand or by making use of robotics to deposit the members. In general, arrays may be described as macroarrays or microarrays, the difference being the size of the spots. Macroarrays can contain spot sizes of about 300 microns or larger and may be easily imaged by existing gel and blot scanners. The spot sizes in microarrays can be less than 200 microns in diameter and these arrays usually contain thousands of spots. Thus, microarrays may require specialized robotics and imaging equipment, which may need to be custom made. Instrumentation is described generally in a review by Cortese, 2000, The Scientist 14[11]:26.

Techniques for producing immobilized libraries of DNA molecules have been described in the art. Generally, most prior art methods described how to synthesize single-stranded nucleic acid molecule libraries, using, for example, masking techniques to build up various permutations of sequences at the various discrete positions on the solid substrate. U.S. Pat. No. 5,837,832 describes an improved method for producing DNA arrays immobilized to silicon substrates based on very large-scale integration technology. In particular, U.S. Pat. No. 5,837,832 describes a strategy called "tiling" to synthesize specific sets of probes at spatially-defined locations on a substrate which may be used to produce the immobilized DNA libraries of the present disclosure. U.S. Pat. No. 5,837,832 also provides references for earlier techniques that may also be used. In other cases, arrays may also be built using photo deposition chemistry.

Arrays of peptides (or peptidomimetics) may also be synthesized on a surface in a manner that places each distinct library member (e.g., unique peptide sequence) at a discrete, predefined location in the array. The identity of each library member is determined by its spatial location in the array. The locations in the array where binding interactions between a predetermined molecule (e.g., a target or probe) and reactive library members occur is determined, thereby identifying the sequences of the reactive library members on the basis of spatial location. These methods are described in U.S. Pat. No. 5,143,854; WO90/15070 and WO92/10092; Fodor et al. (1991) Science, 251: 767; Dower and Fodor (1991) Ann. Rep. Med. Chem., 26: 271

To aid detection, labels can be used (as discussed above)-such as any readily detectable reporter, for example, a fluorescent, bioluminescent, phosphorescent, radioactive, etc. reporter. Such reporters, their detection, coupling to targets/probes, etc. are discussed elsewhere in this document. Labelling of probes and targets is also disclosed in Shalon et al., 1996, Genome Res 6(7):639-45.

Examples of some commercially available microarray formats are set out in Marshall and Hodgson, 1998, Nature Biotechnology, 16(1), 27-31.

**In** order to generate data from array-based assays a signal can be detected to signify the presence of or absence of hybridization between a probe and a nucleotide sequence. Further, direct and indirect labeling techniques can also be utilized. For example, direct labeling incorporates fluorescent dyes directly into the nucleotide sequences that hybridize to the array associated probes (e.g., dyes are incorporated into nucleotide sequence by enzymatic synthesis in the presence of labeled nucleotides or PCR primers). Direct labeling schemes can yield strong hybridization signals, for example, by using families of fluorescent dyes with similar chemical structures and characteristics, and can be simple to implement. In cases comprising direct labeling of nucleic acids, cyanine or alexa analogs can be utilized in multiple-fluor comparative array analyses. In other embodiments, indirect labeling schemes can be utilized to incorporate epitopes into the nucleic acids either prior to or after hybridization to the microarray probes. One or more staining procedures and reagents can be used to label the hybridized complex (e.g., a fluorescent molecule that binds to the epitopes, thereby providing a fluorescent signal by virtue of the conjugation of dye molecule to the epitope of the hybridized species).

### Sequencing

In various embodiments, suitable sequencing methods described herein or otherwise known in the art will be used to obtain sequence information from nucleic acid molecules within a sample. Sequencing can be accomplished through classic Sanger sequencing methods which are well known in the art. Sequence can also be accomplished using high-throughput systems some of which allow detection of a sequenced nucleotide immediately after or upon its incorporation into a growing strand, i.e., detection of sequence in real time or substantially real time. In some cases, high-throughput sequencing generates at least 1,000, at least 5,000, at least 10,000, at least 20,000, at least 30,000, at least 40,000, at least 50,000, at least 100,000 or at least 500,000 sequence reads per hour; where the sequencing reads can be at least about 50, about 60, about 70, about 80, about 90, about 100, about 120, about 150, about 180, about 210, about 240, about 270, about 300, about 350, about 400, about 450, about 500, about 600, about 700, about 800, about 900, or about 1000 bases per read.

Sequencing can be whole-genome, with or without enrichment of particular regions of interest. Sequencing can be targeted to particular regions of the genome. Regions of the genome that can be enriched for or targeted include but are not limited to single genes (or regions thereof), gene panels, gene fusions, human leukocyte antigen (HLA) loci (e.g., Class I HLA-A, B, and C; Class II HLA-DRB1/3/4/5, HLA-DQA1, HLA-DQB1, HLA-DPA1, HLA-DPB1), exonic regions, exome, and other loci. Genomic regions can be relevant to immune response, immune repertoire, immune cell diversity, transcription (e.g., exome), cancers (e.g., BRCA1, BRCA2, panels of genes or regions thereof such as hotspot regions, somatic variants, SNVs, amplifications, fusions, tumor mutational burden (TMB), microsatellite instability (MSI)), cardiac diseases, inherited diseases, and other diseases or conditions. A variety of methods can be used to enrich for or target regions of interest, including but not limited to sequence capture. In some cases, Capture Hi-C (CHi-C) or CHi-C-like protocols are employed, employing a sequence capture step (e.g., by target enrichment array) before or after library preparation.

In some embodiments, high-throughput sequencing involves the use of technology available by Illumina's Genome Analyzer IIX, MiSeq personal sequencer, or HiSeq systems, such as those using HiSeq 2500, HiSeq 1500, HiSeq 2000, or HiSeq 1000 machines. These machines use reversible terminator-based sequencing by synthesis chemistry. These machines can do 200 billion DNA reads or more in eight days. Smaller systems may be utilized for runs within 3, 2, 1 days or less time.

In some embodiments, high-throughput sequencing involves the use of technology available by ABI Solid System. This genetic analysis platform that enables massively parallel sequencing of clonally-amplified DNA fragments linked to beads. The sequencing methodology is based on sequential ligation with dye-labeled oligonucleotides.

The next generation sequencing can comprise ion semiconductor sequencing (e.g., using technology from Life Technologies (Ion Torrent)). Ion semiconductor sequencing can take advantage of the fact that when a nucleotide is incorporated into a strand of DNA, an ion can be released. To perform ion semiconductor sequencing, a high-density array of micromachined wells can be formed. Each well can hold a single DNA template. Beneath the well can be an ion sensitive layer, and beneath the ion sensitive layer can be an ion sensor. When a nucleotide is added to a DNA, H+ can be released, which can be measured as a change in pH. The H+ ion can be converted to voltage and recorded by the semiconductor sensor. An array chip can be sequentially flooded with one nucleotide after another. No scanning, light, or cameras can be required. In some cases, an IONPROTON^{™} Sequencer is used to sequence nucleic acid. In some cases, an IONPGM^{™} Sequencer is used. The Ion Torrent Personal Genome Machine (PGM). The PGM can do 10 million reads in two hours.

In some embodiments, high-throughput sequencing involves the use of technology available by Helicos BioSciences Corporation (Cambridge, Massachusetts) such as the Single Molecule Sequencing by Synthesis (SMSS) method. SMSS is unique because it allows for sequencing the entire human genome in up to 24 hours. Finally, SMSS is described in part in US Publication Application Nos. 20060024711; 20060024678; 20060012793; 20060012784; and 20050100932.

In some embodiments, high-throughput sequencing involves the use of technology available by 454 Lifesciences, Inc. (Branford, Connecticut) such as the PicoTiterPlate device which includes a fiber optic plate that transmits chemiluminescent signal generated by the sequencing reaction to be recorded by a CCD camera in the instrument. This use of fiber optics allows for the detection of a minimum of 20 million base pairs in 4.5 hours.

Methods for using bead amplification followed by fiber optics detection are described in Marguiles, M., et al. "Genome sequencing in microfabricated high-density pricolitre reactors," Nature, doi:10.1038/nature03959; and well as in US Publication Application Nos. 20020012930; 20030068629; 20030100102; 20030148344; 20040248161; 20050079510, 20050124022; and 20060078909.

In some embodiments, high-throughput sequencing is performed using Clonal Single Molecule Array (Solexa, Inc.) or sequencing-by-synthesis (SBS) utilizing reversible terminator chemistry. These technologies are described in part in US Patent Nos. 6,969,488; 6,897,023; 6,833,246; 6,787,308; and US Publication Application Nos. 20040106110; 20030064398; 20030022207; and Constans, A., The Scientist 2003, 17(13):36.

The next generation sequencing technique can comprise real-time (SMRT^{™}) technology by Pacific Biosciences. In SMRT, each of four DNA bases can be attached to one of four different fluorescent dyes. These dyes can be phospho linked. A single DNA polymerase can be immobilized with a single molecule of template single stranded DNA at the bottom of a zero-mode waveguide (ZMW). A ZMW can be a confinement structure which enables observation of incorporation of a single nucleotide by DNA polymerase against the background of fluorescent nucleotides that can rapidly diffuse in an out of the ZMW (in microseconds). It can take several milliseconds to incorporate a nucleotide into a growing strand. During this time, the fluorescent label can be excited and produce a fluorescent signal, and the fluorescent tag can be cleaved off. The ZMW can be illuminated from below. Attenuated light from an excitation beam can penetrate the lower 20-30 nm of each ZMW. A microscope with a detection limit of 20 zepto liters (20x 10⁻²¹ liters) can be created. The tiny detection volume can provide 1000-fold improvement in the reduction of background noise. Detection of the corresponding fluorescence of the dye can indicate which base was incorporated. The process can be repeated.

In some cases, the next generation sequencing is nanopore sequencing (see, e.g., Soni GV and Meller A. (2007) Clin Chem 53: 1996-2001). A nanopore can be a small hole, of the order of about one nanometer in diameter. Immersion of a nanopore in a conducting fluid and application of a potential across it can result in a slight electrical current due to conduction of ions through the nanopore. The amount of current which flows can be sensitive to the size of the nanopore. As a DNA molecule passes through a nanopore, each nucleotide on the DNA molecule can obstruct the nanopore to a different degree. Thus, the change in the current passing through the nanopore as the DNA molecule passes through the nanopore can represent a reading of the DNA sequence. The nanopore sequencing technology can be from Oxford Nanopore Technologies; e.g., a GridlON system. A single nanopore can be inserted in a polymer membrane across the top of a microwell. Each microwell can have an electrode for individual sensing. The microwells can be fabricated into an array chip, with 100,000 or more microwells (e.g., more than 200,000, 300,000, 400,000, 500,000, 600,000, 700,000, 800,000, 900,000, or 1,000,000) per chip. An instrument (or node) can be used to analyze the chip. Data can be analyzed in real-time. One or more instruments can be operated at a time. The nanopore can be a protein nanopore, e.g., the protein alpha-hemolysin, a heptameric protein pore. The nanopore can be a solid-state nanopore made, e.g., a nanometer sized hole formed in a synthetic membrane (e.g., SiNx, or SiO2). The nanopore can be a hybrid pore (e.g., an integration of a protein pore into a solid-state membrane). The nanopore can be a nanopore with an integrated sensor (e.g., tunneling electrode detectors, capacitive detectors, or graphene based nano-gap or edge state detectors (see e.g., Garaj et al. (2010) Nature vol. 67, doi: 10.1038/nature09379)). A nanopore can be functionalized for analyzing a specific type of molecule (e.g., DNA, RNA, or protein). Nanopore sequencing can comprise "strand sequencing" in which intact DNA polymers can be passed through a protein nanopore with sequencing in real time as the DNA translocates the pore. An enzyme can separate strands of a double stranded DNA and feed a strand through a nanopore. The DNA can have a hairpin at one end, and the system can read both strands. In some cases, nanopore sequencing is "exonuclease sequencing" in which individual nucleotides can be cleaved from a DNA strand by a processive exonuclease, and the nucleotides can be passed through a protein nanopore. The nucleotides can transiently bind to a molecule in the pore (e.g., cyclodextran). A characteristic disruption in current can be used to identify bases.

Nanopore sequencing technology from GENIA can be used. An engineered protein pore can be embedded in a lipid bilayer membrane. "Active Control" technology can be used to enable efficient nanopore-membrane assembly and control of DNA movement through the channel. In some cases, the nanopore sequencing technology is from NABsys. Genomic DNA can be fragmented into strands of average length of about 100 kb. The 100 kb fragments can be made single stranded and subsequently hybridized with a 6-mer probe. The genomic fragments with probes can be driven through a nanopore, which can create a current-versus- time tracing. The current tracing can provide the positions of the probes on each genomic fragment. The genomic fragments can be lined up to create a probe map for the genome. The process can be done in parallel for a library of probes. A genome-length probe map for each probe can be generated. Errors can be fixed with a process termed "moving window Sequencing By Hybridization (mwSBH)." In some cases, the nanopore sequencing technology is from IBM/Roche. An electron beam can be used to make a nanopore sized opening in a microchip. An electrical field can be used to pull or thread DNA through the nanopore. A DNA transistor device in the nanopore can comprise alternating nanometer sized layers of metal and dielectric. Discrete charges in the DNA backbone can get trapped by electrical fields inside the DNA nanopore. Turning off and on gate voltages can allow the DNA sequence to be read.

The next generation sequencing can comprise DNA nanoball sequencing (as performed, e.g., by Complete Genomics; see e.g., Drmanac et al. (2010) Science 327: 78-81). DNA can be isolated, fragmented, and size selected. For example, DNA can be fragmented (e.g., by sonication) to a mean length of about 500 bp. Adaptors (Adl) can be attached to the ends of the fragments. The adaptors can be used to hybridize to anchors for sequencing reactions. DNA with adaptors bound to each end can be PCR amplified. The adaptor sequences can be modified so that complementary single strand ends bind to each other forming circular DNA. The DNA can be methylated to protect it from cleavage by a type IIS restriction enzyme used in a subsequent step. An adaptor (e.g., the right adaptor) can have a restriction recognition site, and the restriction recognition site can remain non-methylated. The non-methylated restriction recognition site in the adaptor can be recognized by a restriction enzyme (e.g., Acul), and the DNA can be cleaved by Acul 13 bp to the right of the right adaptor to form linear double stranded DNA. A second round of right and left adaptors (Ad2) can be ligated onto either end of the linear DNA, and all DNA with both adaptors bound can be PCR amplified (e.g., by PCR). Ad2 sequences can be modified to allow them to bind each other and form circular DNA. The DNA can be methylated, but a restriction enzyme recognition site can remain non-methylated on the left Adl adaptor. A restriction enzyme (e.g., Acul) can be applied, and the DNA can be cleaved 13 bp to the left of the Adl to form a linear DNA fragment. A third round of right and left adaptor (Ad3) can be ligated to the right and left flank of the linear DNA, and the resulting fragment can be PCR amplified. The adaptors can be modified so that they can bind to each other and form circular DNA. A type III restriction enzyme (e.g., EcoP15) can be added; EcoP15 can cleave the DNA 26 bp to the left of Ad3 and 26 bp to the right of Ad2. This cleavage can remove a large segment of DNA and linearize the DNA once again. A fourth round of right and left adaptors (Ad4) can be ligated to the DNA, the DNA can be amplified (e.g., by PCR), and modified so that they bind each other and form the completed circular DNA template.

Rolling circle replication (e.g., using Phi 29 DNA polymerase) can be used to amplify small fragments of DNA. The four adaptor sequences can contain palindromic sequences that can hybridize, and a single strand can fold onto itself to form a DNA nanoball (DNB^{™}) which can be approximately 200-300 nanometers in diameter on average. A DNA nanoball can be attached (e.g., by adsorption) to a microarray (sequencing flowcell). The flow cell can be a silicon wafer coated with silicon dioxide, titanium and hexamehtyldisilazane (HMDS) and a photoresist material. Sequencing can be performed by unchained sequencing by ligating fluorescent probes to the DNA. The color of the fluorescence of an interrogated position can be visualized by a high-resolution camera. The identity of nucleotide sequences between adaptor sequences can be determined.

In some embodiments, high-throughput sequencing can take place using AnyDot.chips (Genovoxx, Germany). In particular, the AnyDot.chips allow for 10x - 50x enhancement of nucleotide fluorescence signal detection. AnyDot.chips and methods for using them are described in part in International Publication Application Nos. WO 02088382, WO 03020968, WO 03031947, WO 2005044836, PCT/EP 05/05657, PCT/EP 05/05655; and German Patent Application Nos. DE 101 49 786, DE 102 14 395, DE 103 56 837, DE 10 2004 009 704, DE 10 2004 025 696, DE 10 2004 025 746, DE 10 2004 025 694, DE 10 2004 025 695, DE 10 2004 025 744, DE 10 2004 025 745, and DE 10 2005 012 301.

Other high-throughput sequencing systems include those disclosed in Venter, J., et al. Science 16 February 2001; Adams, M. et al. Science 24 March 2000; and M. J. Levene, et al. Science 299:682-686, January 2003; as well as US Publication Application No. 20030044781 and 2006/0078937. Overall such systems involve sequencing a target nucleic acid molecule having a plurality of bases by the temporal addition of bases via a polymerization reaction that is measured on a molecule of nucleic acid, i.e., the activity of a nucleic acid polymerizing enzyme on the template nucleic acid molecule to be sequenced is followed in real time. Sequence can then be deduced by identifying which base is being incorporated into the growing complementary strand of the target nucleic acid by the catalytic activity of the nucleic acid polymerizing enzyme at each step in the sequence of base additions. A polymerase on the target nucleic acid molecule complex is provided in a position suitable to move along the target nucleic acid molecule and extend the oligonucleotide primer at an active site. A plurality of labeled types of nucleotide analogs are provided proximate to the active site, with each distinguishable type of nucleotide analog being complementary to a different nucleotide in the target nucleic acid sequence. The growing nucleic acid strand is extended by using the polymerase to add a nucleotide analog to the nucleic acid strand at the active site, where the nucleotide analog being added is complementary to the nucleotide of the target nucleic acid at the active site. The nucleotide analog added to the oligonucleotide primer as a result of the polymerizing step is identified. The steps of providing labeled nucleotide analogs, polymerizing the growing nucleic acid strand, and identifying the added nucleotide analog are repeated so that the nucleic acid strand is further extended, and the sequence of the target nucleic acid is determined.

### Kits

In particular embodiments, the present disclosure further provides kits comprising one or more components of the disclosure. The kits can be used for any application apparent to those of skill in the art, including those described above. The kits can comprise, for example, a plurality of association molecules, a fixative agent, a nuclease, a ligase, and/or a combination thereof. In some cases, the association molecules can be proteins including, for example, histones. In some cases, the fixative agent can be formaldehyde or any other DNA crosslinking agent, including DSG, EGS, or DSS.

In some cases, the kit can further comprise a plurality of beads. The beads can be paramagnetic and/or are coated with a capturing agent. For example, the beads can be coated with streptavidin and/or an antibody.

In some cases, the kit can comprise adaptor oligonucleotides and/or sequencing primers. Further, the kit can comprise a device capable of amplifying the read-pairs using the adaptor oligonucleotides and/or sequencing primers.

In some cases, the kit can also comprise other reagents including, but not limited to, lysis buffers, ligation reagents (e.g., dNTPs, polymerase, polynucleotide kinase, and/ or ligase buffer, etc.), and PCR reagents (e.g., dNTPs, polymerase, and/or PCR buffer, etc.),

The kit can also include instructions for using the components of the kit and/or for generating the read-pairs.

### Computers and Systems

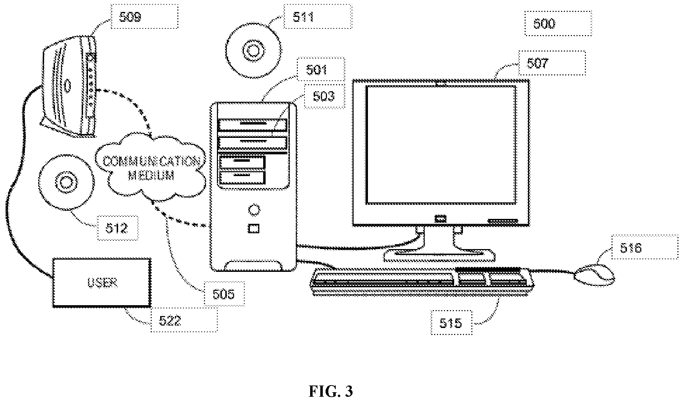

The computer system **500** illustrated in **FIG. 3** may be understood as a logical apparatus that can read instructions from media **511** and/or a network port **505,** which can optionally be connected to server **509** having fixed media **512.** The system, such as shown in **FIG. 3** can include a CPU **501,** disk drives **503,** optional input devices such as keyboard **515** and/or mouse **516** and optional monitor **507.** Data communication can be achieved through the indicated communication medium to a server at a local or a remote location. The communication medium can include any means of transmitting and/or receiving data. For example, the communication medium can be a network connection, a wireless connection or an internet connection. Such a connection can provide for communication over the World Wide Web. It is envisioned that data relating to the present disclosure can be transmitted over such networks or connections for reception and/or review by a party **522** as illustrated in **FIG. 3****.**

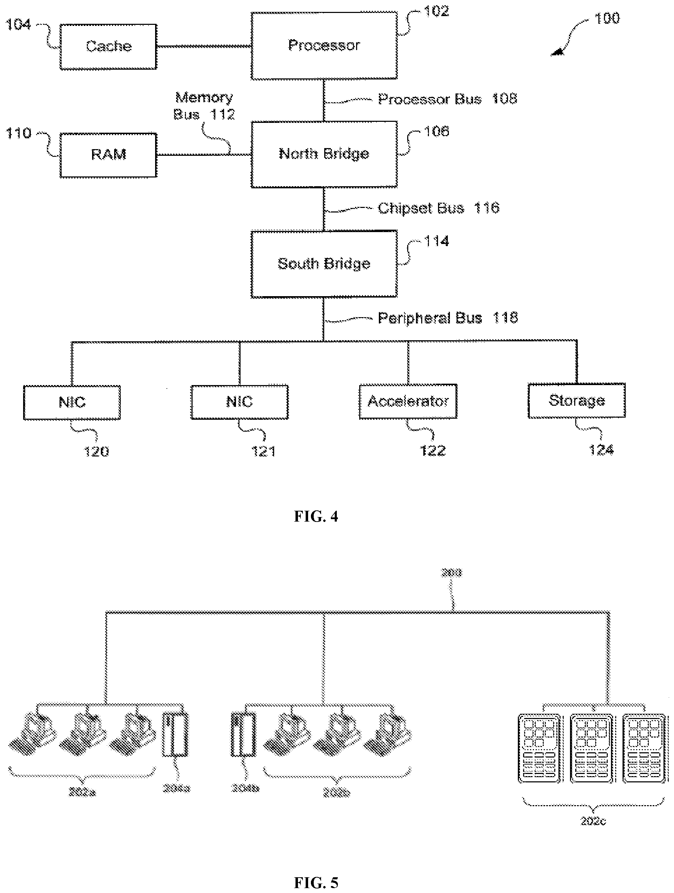

**FIG. 4** is a block diagram illustrating a first example architecture of a computer system **100** that can be used in connection with example embodiments of the present disclosure. As depicted in **FIG. 4****,** the example computer system can include a processor **102** for processing instructions. Non-limiting examples of processors include: Intel Xeon^{™} processor, AMD Opteron^{™} processor, Samsung 32-bit RISC ARM 1176JZ(F)-S v1.0^{™} processor, ARM Cortex-A8 Samsung S5SPC100^{™} processor, ARM Cortex-A8 Apple A4^{™} processor, Marvell PXA 930^{™} processor, or a functionally-equivalent processor. Multiple threads of execution can be used for parallel processing. In some embodiments, multiple processors or processors with multiple cores can also be used, whether in a single computer system, in a cluster, or distributed across systems over a network comprising a plurality of computers, cell phones, and/or personal data assistant devices.

As illustrated in **FIG. 4****,** a high-speed cache **104** can be connected to, or incorporated in, the processor **102** to provide a high-speed memory for instructions or data that have been recently, or are frequently, used by processor **102.** The processor **102** is connected to a north bridge **106** by a processor bus **108.** The north bridge **106** is connected to random access memory (RAM) **110** by a memory bus **112** and manages access to the RAM **110** by the processor **102.** The north bridge **106** is also connected to a south bridge **114** by a chipset bus **116.** The south bridge **114** is, in turn, connected to a peripheral bus **118.** The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus **118.** In some alternative architectures, the functionality of the north bridge can be incorporated into the processor instead of using a separate north bridge chip.

In some embodiments, system **100** can include an accelerator card **122** attached to the peripheral bus **118.** The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage **124** and can be loaded into RAM 110 and/or cache 104 for use by the processor. The system **100** includes an operating system for managing system resources; non-limiting examples of operating systems include: Linux, Windows^{™}, MACOS^{™}, BlackBerry OS^{™}, iOS^{™}, and other functionally-equivalent operating systems, as well as application software running on top of the operating system for managing data storage and optimization in accordance with example embodiments of the present disclosure.

In this example, system **100** also includes network interface cards (NICs) **120** and **121** connected to the peripheral bus for providing network interfaces to external storage, such as Network Attached Storage (NAS) and other computer systems that can be used for distributed parallel processing.

**FIG. 5** is a diagram showing a network **200** with a plurality of computer systems **202a,** and **202b,** a plurality of cell phones and personal data assistants **202c,** and Network Attached Storage (NAS) **204a,** and **204b.** In example embodiments, systems **202a, 202b,** and **202c** can manage data storage and optimize data access for data stored in Network Attached Storage (NAS) **204a** and **204b.** A mathematical model can be used for the data and be evaluated using distributed parallel processing across computer systems **202a,** and **202b,** and cell phone and personal data assistant systems **202c.** Computer systems **202a,** and **202b,** and cell phone and personal data assistant systems **202c** can also provide parallel processing for adaptive data restructuring of the data stored in Network Attached Storage (NAS) **204a** and **204b.** **FIG. 5** illustrates an example only, and a wide variety of other computer architectures and systems can be used in conjunction with the various embodiments of the present disclosure. For example, a blade server can be used to provide parallel processing. Processor blades can be connected through a back plane to provide parallel processing. Storage can also be connected to the back plane or as Network Attached Storage (NAS) through a separate network interface.

In some example embodiments, processors can maintain separate memory spaces and transmit data through network interfaces, back plane or other connectors for parallel processing by other processors. In other embodiments, some or all of the processors can use a shared virtual address memory space.

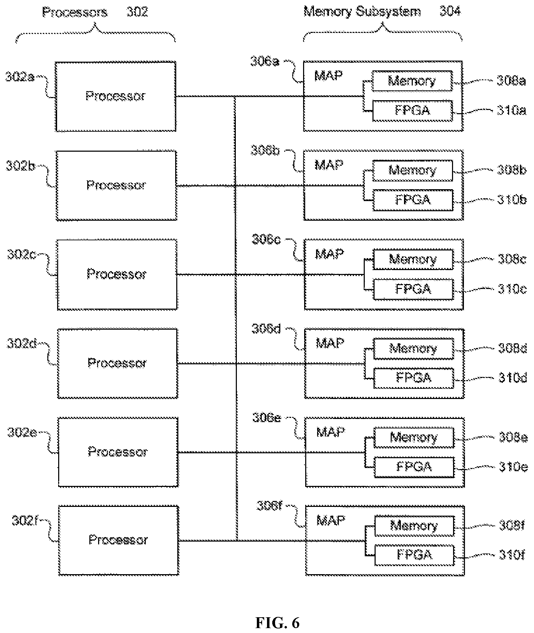

**FIG. 6** is a block diagram of a multiprocessor computer system **300** using a shared virtual address memory space in accordance with an example embodiment. The system includes a plurality of processors **302a-f** that can access a shared memory subsystem **304.** The system incorporates a plurality of programmable hardware memory algorithm processors (MAPs) **306a-f** in the memory subsystem **304.** Each MAP **306a-f** can comprise a memory **308a-f** and one or more field programmable gate arrays (FPGAs) **310a-f.** The MAP provides a configurable functional unit and particular algorithms, or portions of algorithms, can be provided to the FPGAs **310a-f** for processing in close coordination with a respective processor. For example, the MAPs can be used to evaluate algebraic expressions regarding the data model and to perform adaptive data restructuring in example embodiments. In this example, each MAP is globally accessible by all of the processors for these purposes. In one configuration, each MAP can use Direct Memory Access (DMA) to access an associated memory **308a-f,** allowing it to execute tasks independently of, and asynchronously from, the respective microprocessor **302a-f.** In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example embodiments, including systems using any combination of general processors, co-processors, FPGAs and other programmable logic devices, system on chips (SOCs), application specific integrated circuits (ASICs), and other processing and logic elements. In some embodiments, all or part of the computer system can be implemented in software or hardware. Any variety of data storage media can be used in connection with example embodiments, including random access memory, hard drives, flash memory, tape drives, disk arrays, Network Attached Storage (NAS) and other local or distributed data storage devices and systems.

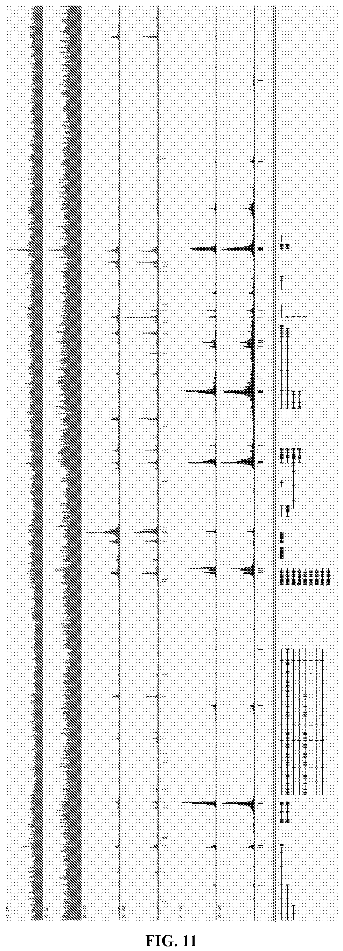

In example embodiments, the computer system can be implemented using software modules executing on any of the above or other computer architectures and systems. In other embodiments, the functions of the system can be implemented partially or completely in firmware, programmable logic devices such as field programmable gate arrays (FPGAs) as referenced in **FIG. 11****,** system on chips

(SOCs), application specific integrated circuits (ASICs), or other processing and logic elements. For example, the Set Processor and Optimizer can be implemented with hardware acceleration through the use of a hardware accelerator card, such as accelerator card 122 illustrated in **FIG. 4****.**

### Definitions

Unless defined otherwise, all technical and scientific terms used herein have the same meaning as commonly understood to one of ordinary skill in the art to which this disclosure belongs. Although any methods and reagents similar or equivalent to those described herein can be used in the practice of the disclosed methods and compositions, the exemplary methods and materials are now described.

As used herein and in the appended claims, the singular forms "a," "and," and "the" include plural referents unless the context clearly dictates otherwise. Thus, for example, reference to "contig" includes a plurality of such contigs and reference to "probing the physical layout of chromosomes" includes reference to one or more methods for probing the physical layout of chromosomes and equivalents thereof known to those skilled in the art, and so forth.

Also, the use of "and" means "and/or" unless stated otherwise. Similarly, "comprise," "comprises," "comprising," "include," "includes," and "including" are interchangeable and not intended to be limiting.

It is to be further understood that where descriptions of various embodiments use the term "comprising," those skilled in the art would understand that in some specific instances, an embodiment can be alternatively described using language "consisting essentially of" or "consisting of."

The term "sequencing read" as used herein, refers to a fragment of DNA in which the sequence has been determined.

The term "contigs" as used herein, refers to contiguous regions of DNA sequence. "Contigs" can be determined by any number methods known in the art, such as, by comparing sequencing reads for overlapping sequences, and/or by comparing sequencing reads against a database of known sequences in order to identify which sequencing reads have a high probability of being contiguous.

The term "subject" as used herein can refer to any eukaryotic or prokaryotic organism.

The term "read pair" or "read-pair" as used herein can refer to two or more elements that are linked to provide sequence information. In some cases, the number of read-pairs can refer to the number of mappable read-pairs. In other cases, the number of read-pairs can refer to the total number of generated read-pairs.

The term "about" as used herein can describe a number, unless otherwise specified, as a range of values including that number plus or minus 10% of that number.

As used herein, "exposed internal ends of a nucleic acid" can refer to exposed ends generated through generation of cleavage sites introduced into stabilized or non-stabilized nucleic acids, such as those introduced so as to access the end-adjacent nucleic acid sequence information to facilitate phase or local three-dimensional structural information.

As used herein, the term "about" a number refers to a range spanning +/- 10% of that number, while "about" a range refers to 10% lower than a stated range limit spanning to 10% greater than a stated range limit.

As used herein, a sequence segment on a linker or otherwise is partition designating, or cell designating when identification of its sequence facilitates assigning adjacent nucleic acid sequence to a particular first partition or cell of origin to the exclusion of a second partition or cell of origin. A distinguishing sequence is in some cases unique to a partition or cell, such that it distinguishes from all other cells, and when this is technically feasible, unique tags facilitate downstream analysis. However, unique sequence is not in all cases required. In some cases, redundant barcoding is resolved computationally downstream, such that a tag that is not unique is nonetheless sufficient to distinguish nucleic acids of a first partition or cell from a second partition or cell.

As used herein, a cluster is a region of a nucleic acid reference to which a plurality of distinct end adjacent sequences or sequence tags map. In some cases, the proximity of one region to a second region is assessed at least in part by counting the number of cluster constituents of a first cluster that co-occur in paired end reads with cluster constituents of a second cluster.

### EXAMPLES

The following examples are given for the purpose of illustrating various embodiments of the invention and are not meant to limit the present invention in any fashion.

### Example 1: Sample Preparation

There are two separate protocols for sample preparation depending on your sample type: cells or tissue. The lysate quantification step is the same for both sample types. Sample preparation should take 2 hours.

Notes: The 10X HiC Wash Buffer, 10X Crosslink Reversal Buffer and 20% SDS might have precipitated in storage. Incubate the solutions at 37 °C for 15 min, until the precipitate is no longer visible. Vortex to mix prior to use. Dilute 10X HiC Wash Buffer to 1X with UltraPure water. Store at room temperature. About 15mL of 1X HiC Wash buffer is needed per sample. 1X HiC Wash Buffer can also be used throughout the rest of the protocol. 1X HiC Wash buffer is stable at room temperature for 2 months. Dilute 10X Crosslink Reversal buffer to 1X with UltraPure water. Store at room temperature. About 1mL of 1X Crosslink Reversal buffer is needed per sample. 1X Crosslink Reversal Buffer can also be used for the Proximity Ligation Protocol. 1X Crosslink Reversal buffer is stable at room temperature for 2 months. Agitating thermal mixer should be set at 1250 rpm for 1.5 mL tubes. Use good laboratory practices, including thawing buffers on ice and vortexing prior to use.

### Protocol for Cells

Notes: It is recommended to use 10 x 10⁶ cells as starting material to account for losses during the washes. If less than 10 x 10⁶ cells are available, refer to low input protocol. Before beginning, prepare fresh 1X Nuclease Digest Buffer and store at room temperature. 1X Nuclease Digest buffer is stable for 1 day at room temperature. To prepare 1X Nuclease Digest Buffer, mix: 140µl UltraPure water; 20 µl 10X Nuclease Digest Buffer; 20 µl 100mM MnCl2; 20 µl 10%Triton.

Cells are harvested and washed in 1X PBS. Cells are counted and 10 x 10⁶ cells are aliquoted and centrifuged at 2000 x g for 5 min. Supernatant is carefully removed. Pellet is resuspended in 5 ml 1x PBS and 135 µl 37% formaldehyde. The sample is transferred to a 5 ml tube and rotated for 10 min at room temperature at a speed such that cells do not settle. The tube is centrifuged at 2000 x g for 5 min. Supernatant is removed carefully with caution as the cell pellet could be loose. The pellet is washed with HiC Wash buffer, first with 200 µl to break up the clump then adding the remaining 4.8 ml, pipetting up and down to fully resuspend the pellet. The tube is spun at 2000 x g for 5 min and supernatant is carefully removed. The wash steps are repeated for a total of 2 washes. After removing the second wash, the pellet is resuspended in 1 ml 1X HiC wash buffer and the pellet is resuspended. Cells are counted and 1 x 10⁶ cells are added to three separate tubes, remaining cells are stored as a pellet frozen at -80 °C. The three tubes are centrifuged at 2000 x g for 5 min and supernatant is removed. Pellets in each tube are resuspended in 50 µl 1X Nuclease Digest Buffer (freshly prepared). Tubes are pre-warmed to 30°C for 2 min in an agitating thermal mixer at 1250 rpm. A fresh 1.5 ml tube with 7.5 µl Nuclease Enzyme Mix is prewarmed at 30 °C for 2 min in an agitating thermal mixer at 1250 rpm. Pre-warmed Nuclease Enzyme mix is transferred to each pre-warmed tube as follows, 0.5 µl to the first tube, 2.0 µl to the second tube, and 4.0 µl to the third tube. Tubes are incubated for exactly 30 min at 30 °C in an agitating thermal mixer at 1250 rpm. The nuclease reaction is stopped by adding 5 µl 0.5 M EDTA and mixing. 3 µl 20% SDS is added to lyse the cells and cells are incubated for 5 min at 30 °C in agitating thermal mixer at 1250 rpm.

### Protocol for Tissue

Notes: It is recommended to use 60 mg tissue. If less than 60 mg of tissue is available refer to low input protocol. Before beginning, fresh 1X Nuclease Digest Buffer is prepared and stored at room temperature. 1X Nuclease Digest buffer is stable for 1 day at room temperature. To prepare 1X Nuclease Digest Buffer, mix: 140µl UltraPure water; 20 µl 10X Nuclease Digest Buffer; 20 µl 100mM MnCl2; 20 µl 10%Triton.

At least 60 mg frozen tissue is weighed out and grinded to a fine powder with mortar and pestle in liquid nitrogen to a consistency illustrated in **FIG. 1A** and **FIG. 1B****,** wherein **FIG. 1A** shows insufficient tissue grinding and **FIG. 1B** shows sufficient tissue grinding. The disrupted tissue is transferred to a 5 ml tube with 5 ml 1X PBS and 135 µl 37% formaldehyde. The tube is rotated for 10 min at room temperature. The tube is centrifuged 2000 x g for 5 min and supernatant is carefully removed. In events where tissue does not pellet, the tube is spun at max speed for 5 min. Pellet is resuspended on 200 µl wash buffer, then 4.8 ml 1X HiC wash buffer is added. The tube is centrifuged for 5 min at 2000 x g and supernatant is removed. The wash step is performed 2 times and the final pellet is resuspended in 1 ml 1X HiC wash buffer. The resuspended cells are passed through a 200 µm filter into a fresh 5 ml tube, changing filters if necessary. An additional 2 ml 1X HiC wash buffer is passed through the 200 µm filter. The sample is separated into three 1 ml aliquots in three separate tubes with each aliquot corresponding to 20 mg tissue. Excess tissue can be pelleted and stored at -80°C. The three tubes are centrifuged at 2000 x g for 5 min and supernatant is removed. Pellets in each tube are resuspended in 50 µl 1X Nuclease Digest Buffer (freshly prepared). Tubes are pre-warmed to 30 °C for 2 min in an agitating thermal mixer at 1250 rpm. A fresh 1.5 ml tube with 7.5 µl Nuclease Enzyme Mix is prewarmed at 30 °C for 2 min in an agitating thermal mixer at 1250 rpm. Pre-warmed Nuclease Enzyme mix is transferred to each pre-warmed tube as follows, 0.5 µl to the first tube, 2.0 µl to the second tube, and 4.0 µl to the third tube. Tubes are incubated for exactly 30 min at 30 °C in an agitating thermal mixer at 1250 rpm. The nuclease reaction is stopped by adding 5 µl 0.5 M EDTA and mixing. 3 µl 20% SDS is added to lyse the cells and cells are incubated for 5 min at 30 °C in agitating thermal mixer at 1250 rpm.

### Example 2: Lysate Quantification

Notes: Lysate Quantification should take 2 hours. 80% ethanol is freshly prepared for DNA purification using SPRIselect beads. The quantification step has two objectives: to determine the volume of sample to use in proximity ligation steps and to determine which of the three tubes obtained in the sample preparation to use in proximity ligation.

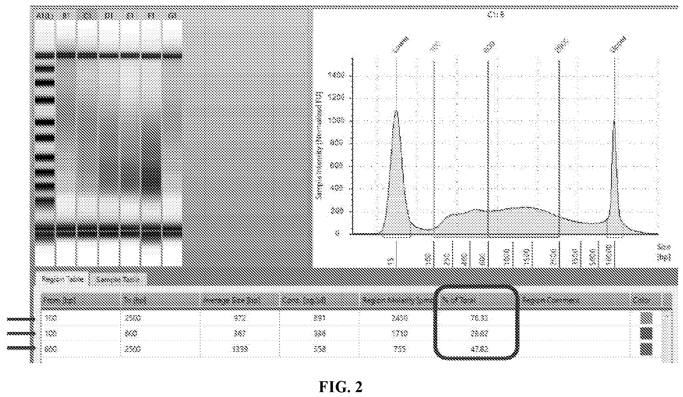

Each lysate is diluted 1:10 by mixing 2 µl lysate from each tube with 18 µl 1 X HiC wash buffer. Undiluted lysate is stored at -80°C. 2.5 µl of each lysate is transferred to a tube with 50 µl crosslink reversal buffer and 1.5 µl proteinase K. The mixtures are mixed by pipetting and incubated for 15 min at 55 °C followed by 45 min at 68 °C in an agitating thermal mixer at 1250 rpm. 100µl SPRIselect beads are added to each tube and vortexed to resuspend, spun down and incubated for 5 min at room temperature away from the magnet. Tubes are placed in the magnet for 5 min or until the solution looks clear and beads have been fully separated. Supernatant is removed and beads are washed twice with 80% ethanol for 1 min without removing tubes from the magnet. After the second wash, tubes were spun down and placed on the magnet for 1 min. The remaining ethanol is removed with a pipette. Beads are air dried for 5 min on the magnet until no ethanol remains but without over-drying. Tubes are taken off the magnet and beads are resuspended in 10 µl TE buffer at pH 8.0. Tubes are vortexed and spun down and put on the magnet for 1 min. 8 µl supernatant is transferred to a fresh tube. Sample is quantified using a Qubit Fluorometer and Qubit dsDNA HS kit. The concentration is recorded in a spreadsheet. Fragment size distribution is determined using a TapeStation D5000 or D5000 HS ScreenTape. If using D5000 HS ScreenTape, samples should be diluted to 1 ng/µl. Regions are analyzed on the TapeStation System: Region 1 100-2500 bp; region 2 100-600 bp, and region 3 600-2500 bp. Percent of total is calculated and recorded. Data appears as shown in **FIG. 2****,** with 76.33% of total from 100 bp to 2500 bp, 28.82% of total from 100 bp to 600 bp, and 47.82% of total from 600 bp to 2500 bp.

Calculate volume of sample corresponding to 1000 ng. Calculate Chromatin Digestion Efficiency (CDE) and Chromatin Digestion Index (CI). Determine which samples pass QC metrics.

### Example 3: Proximity Ligation

Notes: Proximity ligation should take 5.5 hours. The agitating thermal mixer is set at 1250 rpm for 1.5 ml tubes. When placing sample on the magnet, time passes to allow solution to clear fully before removing supernatant. Fresh Bridge Ligation Mix (50 µl) is prepared and stored on ice prior to use. To prepare 50 µl Bridge Ligation Mix, 10 µl 5X ligation buffer, 5 µl bridge, and 35 µl ultrapure water are mixed. 80% ethanol is freshly prepared for DNA purification with SPRIselect beads.

### Bind Chromatin to Chromatin Capture Beads

Chromatin Capture Beads are allowed to come to room temperature and vortexed before use. 100 µl of chromatin capture beads are transferred to a 1.5 ml tube with 1000 ng of sample from Example 1 as calculated. The sample is mixed by pipetting and incubated at room temperature away from the magnet. The tube is placed on the magnet for 5 min or until the solution is clear and beads have separated. Supernatant is removed. Tube is removed from magnetic rack and beads are washed with 150 µl 1X Hi-C wash buffer, mixing by pipetting 10 times the placing on the magnet for 1 min before removing supernatant. The wash step is repeated one time.

### End polishing

Tubes are removed from magnetic rack and 50 µl End Polishing Buffer and 3.5 µl End Polishing enzyme mix are added. Sample is mixed by pipetting and incubated at 22 °C for 30 min followed by 30 min at 65 °C in an agitating thermal mixer at 1250 rpm. Tubes are allowed to reach room temperature then placed on the magnetic rack for 1 min or until the solution looks clear and beads have separated. Supernatant is removed. Tubes are removed from magnetic rack and beads are washed once with 150 µl 1X Hi-C Wash Buffer, mixing by pipetting. Tubes are then placed back on the magnetic rack for 1 min and supernatant is removed.

### Bridge Ligation

Tubes are removed from magnetic rack and 50 µl Bridge Ligation Mix is added (freshly made) with 1 µl T4 DNA ligase. Samples are mixed by pipetting and incubated for 30 min at 22°C. Tubes are placed on the magnetic rack for 1 min or until solution is clear. Supernatant is removed and tube is removed from magnetic rack. Beads are resuspended with 150 µl 1X Hi-C wash buffer and mixed by pipetting. Tubes are placed on magnet for 1 min and supernatant is removed.

### Intra-Aggregate Ligation

Tubes are removed from magnetic rack and added to beads is 50 µl Intra-Aggregate Ligation Buffer and 2 µl Intra-Aggregate Ligation Enzyme mix. Samples are mixed by pipetting and incubated for 1 hour at 22 °C in an agitating thermal mixer. Tubes are placed on the magnetic rack for 1 min or until solution looks clear and the beads have separated. Supernatant is removed.

### Crosslink Reversal

Tubes are removed from magnetic rack and 50 µl plus 1.5 µl proteinase K are added to the beads. Samples are mixed by pipetting and incubated for 15 min at 55 °C, followed by 45 min at 68 °C in an agitating thermal mixer at 1250 rpm.

### DNA Purification on SPRIselect Beads

SPRIselect Beads are vortexed for 30 sec to resuspend. 35 µl resuspended beads are added to the 1.5 ml sample tube. Samples are vortexed and spun down incubating at room temperature for 5 min away from the magnet. Tubes are placed on the magnet for 5 min or until the solution looks clear and the beads have separated. Supernatant is removed. Tubes are left on the magnet for two washes of 150 µl 80% ethanol. Beads are not resuspended in these washes, ethanol is added, incubated for 1 min and ethanol is removed. After the second wash, tubes are spun down and placed on the magnet for 1 min and a pipet is used to remove the last of the ethanol. Beads are air dried for 5 min on the magnet until no ethanol remains, but beads are not over dry. Samples are taken off of the magnet and 52 µl TE Buffer pH 8.0 is added. Samples are vortexed and spun down and incubated for 5 min at room temperature off of the magnet. Tubes are spun down, placed on magnet for one minute. 50 µl supernatant is transferred to a fresh 1.5 ml tube. Beads are discarded. Sample is quantified using Qubit Fluorometer and Qubit dsDNA HS kit. 200 ng is needed to proceed to library preparation step. Purified DNA is stored at 20 °C for up to 6 months.

### Example 4: Library Preparation

Notes: The library preparation protocol does not require fragmentation and should take about 2 hours.

### End Repair

Notes: The End Repair Buffer sometimes precipitates in storage and should be incubated for at least 10 min at 37 °C until there is no visible precipitate. 250 mM DTT is mixed by pipetting to fully mix prior to use.

A 0.2 ml PCR tube is prepared with 48 µl purified sample, 7 µl End Repair Buffer, 3 µl End Repair Enzyme Mix, and 0.5 µl 250 mM DTT. The mixture is mixed by pipetting and spun down. Samples are incubated for 30 min at 20 °C followed by 30 min at 65 °C in a thermal cycler. Samples are held at 12 °C.

### Adaptor Ligation and USER Digest

The 0.2 ml PCR tube containing the sample is mixed with 2.5 µl adaptor for Illumina, 1 µl Ligation Enhancer, and 30 µl Ligation Enzyme Mix. Samples are mixed by pipetting and tubes are spun down. Samples are incubated for 15 min at 20 °C in a thermal cycler and held at 12 °C. 3 µl USER Enzyme mix is added to the PCR tube. Sample is mixed by pipetting and spun down. Sample is incubated for 15 min at 37 °C in a thermal cycler and held at 12 °C.

### DNA Purification

SPRIselect Beads are vortexed for 30 sec to resuspend. 80 µl resuspended beads are added to the PCR tube. Samples are vortexed and spun down incubating at room temperature for 5 min away from the magnet. Tubes are placed on the magnet for 5 min or until the solution looks clear and the beads have separated. Supernatant is removed. Tubes are left on the magnet for two washes of 150 µl 80% ethanol. Beads are not resuspended in these washes, ethanol is added, incubated for 1 min and ethanol is removed. After the second wash, tubes are spun down and placed on the magnet for 1 min and a pipet is used to remove the last of the ethanol. Beads are air dried for 5 min on the magnet until no ethanol remains, but beads are not over dry. Samples are taken off of the magnet and 100 µl TE Buffer pH 8.0 is added. Samples are vortexed and spun down and incubated for 5 min at room temperature off of the magnet. Tubes are spun down, placed on magnet for one minute. 95 µl supernatant is transferred to a fresh 1.5 ml tube. Beads are discarded. Purified DNA is stored at 20 °C overnight.

### Example 5: Ligation Capture and Amplification

Note: The ligation capture and amplification protocol should take two hours.

### Streptavidin Beads Preparation

Note: this step does not involve any DNA sample.

Streptavidin beads are vortexed to resuspend. 25 ml of the resuspended streptavidin beads are transferred to a 1.5 ml tube. Tube containing streptavidin beads is put on the magnet for 5 min and supernatant is removed. Tube is removed from the magnetic rack and streptavidin beads are washed with 200 µl TWB (Red Label) and mixed by pipetting. Sample is placed on the magnetic rack for 1 min and supernatant is removed. Wash step is repeated one time. Streptavidin beads are then resuspended in 100 µl 2X NTP (Yellow Label) and mixed by pipetting.

### Ligation Capture

95 µl of purified DNA is transferred to the 1.5 ml tube with Streptavidin beads resuspended in 100 µl 2X NTB. Tube is vortexed for 10 sec and spun down. Mixture is incubated for 30 min at 25 °C in an agitating thermal mixer.

### Wash Sample on Streptavidin Beads

Note: For each of the washes the tube is removed from the magnetic rack, the indicated buffer is added to the beads, the beads are resuspended, then the tube is placed on the magnet for 1 min and supernatant is removed, taking care to remove all supernatant between each wash.

The tube is spun down then placed on the magnet for 1 min and supernatant is removed. Beads are washed once with 200 µl LWB. Beads are washed twice with 200 µl NWB. Beads are washed twice with 200 µl 1X HiC Wash Buffer.

### Index PCR

Note: Not all PCR enzymes and master mixes are compatible for amplification in the presence of Streptavidin beads so the PCR ready mix supplied is used.

After the last wash is removed, the tube is removed from the magnetic rack and 25 µl HotStart PCR Ready Mix, 5 µl Universal PCR Primer, 5 µl Index Primer (unique to each sample), and 15 µl DNase and RNase-free distilled water is added to the beads. The mixture is mixed by pipetting and transferred to a 0.2 ml PCR tube. The tube is spun down and placed in a thermocycler to run a program as follows: 98 °C 3 min, 12 cycles of (98 °C 20 sec, 65 °C 30 sec, 72 °C 30 sec), 72 °C 1 min, 12 °C hold.

### Size Selection

The PCR tube is spun down and placed on the magnet for 1 min. 47 µl of supernatant is transferred to a 1.5 ml tube and beads are discarded. 53 µl TE buffer pH 8.0 is added to the tube to bring the total volume to 100 µl. SPRIselect beads are vortexed for 30 sec and 45 µl resuspended SPRIselect beads are added to the 1.5 ml tube containing sample. The mixture is vortexed to resuspend, spun down, and incubated for 10 min at room temperature off the magnet. The tube is spin down and placed on the magnet for 5 min. 145 µl supernatant is transferred to a new 1.5 ml tube and beads are discarded. 35 µl SPRIselect beads are added to the 1.5 ml tube, vortexed to resuspend, spun down, and incubated for 10 min at room temperature off the magnet. The tube is spun down and placed on the magnet for 5 min. Supernatant is removed. Tube is left on the magnet and beads are washed twice with 200 µl 80% ethanol. Beads are not resuspended for these washes. The tube is spun down and placed on the magnet for 1 min. A 10 µl pipet tip is used to remove traces of ethanol. Beads are air dried on the magnet for 5 min until no residual ethanol remains, but without over drying. Beads are resuspended in 30 µl TE buffer pH 8.0 mixing by pipetting. The tube is spun down and incubated for 2 min at room temperature off of the magnet. The tube is spun down and placed on the magnet for 1 min. 28 µl of supernatant is transferred to a new 1.5 ml tube. This tube contains the library. The size selected library is quantified using Qubit Fluorometer and Qubit dsDNA HS kit. At least 60 ng DNA is recovered. The library is discarded if less than 60 ng DNA is recovered. A TapeStation or Bioanalyzer is used to verify the size distribution of the size selected library and the size range of the library is between 350 bp and 1000 bp. The library is stored at -20 °C for up to 6 months.

### Example 6: Low Input Sample Preparation

This is used when the recommended input is not available. The lower sample input sometimes results in a lower complexity of the final library.

### Cells

The number of cells available is used and the method in Example 1 for cells is used until the Nuclease step. At the Nuclease step, 0.1 µl pre-warmed Nuclease Enzyme Mix is added to the first tube, 0.5 µl pre-warmed Nuclease Enzyme Mix is added to the second tube, and 2.0 µl Nuclease enzyme mix is added to the third tube.

### Tissues

The amount of tissue available (at least 5 mg) is used and the method in Example 1 for tissues is used until the Nuclease step. At the Nuclease step, 0.1 µl pre-warmed Nuclease Enzyme Mix is added to the first tube, 0.5 µl pre-warmed Nuclease Enzyme Mix is added to the second tube, and 2.0 µl Nuclease enzyme mix is added to the third tube.

### Example 7: Index Primers

The following index primers are used:

| **Table 1: Index Primers** | |
|---|---|
| **Index Primer** | **Sequence** |
| Index Primer 2 | CGATGT |
| Index Primer 4 | TGACCA |
| Index Primer 5 | ACAGTG |
| Index Primer 6 | GCCAAT |
| Index Primer 7 | CAGATC |
| Index Primer 8 | ACTTGA |
| Index Primer 12 | CTTGTA |
| Index Primer 19 | GTGAAA |

Index Primers are selected according to the following scheme:

| **Table 2: Index Primer Selection** | |
|---|---|
| **Number of Libraries** | **Index Primer Combinations** |
| 2 | 6 and 12 |
| 2 | 5 and 19 |
| 3 | 2, 7, and 19 |
| 3 | Either of the 2-plex options plus any other Index Primer |
| 4 | 5, 6, 12, and 19 |
| 4 | Either of the 3-plex options plus any other Index Primer |

### Example 8: Sample Preparation-MNase

There are two separate protocols for sample preparation depending on your sample type: cells or tissue. The lysate quantification step is the same for both sample types. Sample preparation should take 2 hours.

Notes: The 10X HiC Wash Buffer, 10X Crosslink Reversal Buffer, and 20% SDS might have precipitated in storage. Incubate the solutions at 37 °C for 15 min, until the precipitate is no longer visible. Vortex to mix prior to use. Dilute 10X HiC Wash Buffer to 1X with UltraPure water. Store at room temperature. About 15mL of 1X HiC Wash buffer is needed per sample. 1X HiC Wash Buffer can also be used throughout the rest of the protocol. 1X HiC Wash buffer is stable at room temperature for 2 months. Dilute 10X Crosslink Reversal buffer to 1X with UltraPure water. Store at room temperature. About 1mL of 1X Crosslink Reversal buffer is needed per sample. 1X Crosslink Reversal Buffer can also be used for the Proximity Ligation Protocol. 1X Crosslink Reversal buffer is stable at room temperature for 2 months. Agitating thermal mixer should be set at 1250 rpm for 1.5 mL tubes. Use good laboratory practices, including thawing buffers on ice and vortexing prior to use.

### Protocol for Cells

It is recommended to use 10 x 10⁶ cells as starting material to account for losses during the washes. If less than 10 x 10⁶ cells are available, refer to low input protocol. Before beginning, prepare fresh 1X MNase Digest Buffer and store at room temperature. 1X MNase Digest Buffer is stable for 1 day at room temperature. To prepare 1X MNase Digest Buffer, mix: 140µl UltraPure water; 20 µl 10X MNase Digest Buffer; 20 µl 100mM MnCl2; 20 µl 10%Triton.

Cells are harvested and washed in 1X PBS. Cells are counted and 10 x 10⁶ cells are aliquoted and centrifuged at 2000 x g for 5 min. Supernatant is carefully removed. Pellet is resuspended in 5 ml 1x PBS and 135 µl 37% formaldehyde. The sample is transferred to a 5 ml tube and rotated for 10 min at room temperature at a speed such that cells do not settle. The tube is centrifuged at 2000 x g for 5 min. Supernatant is removed carefully with caution as the cell pellet could be loose. The pellet is washed with HiC Wash buffer, first with 200 µl to break up the clump then adding the remaining 4.8 ml, pipetting up and down to fully resuspend the pellet. The tube is spun at 2000 x g for 5 min and supernatant is carefully removed. The wash steps are repeated for a total of 2 washes. After removing the second wash, the pellet is resuspended in 1 ml 1X HiC wash buffer and the pellet is resuspended. Cells are counted and 1 x 10⁶ cells are added to three separate tubes, remaining cells are stored as a pellet frozen at -80 °C. The three tubes are centrifuged at 2000 x g for 5 min and supernatant is removed. Pellets in each tube are resuspended in 50 µl 1X MNase Digest Buffer (freshly prepared). Tubes are pre-warmed to 30 °C for 2 min in an agitating thermal mixer at 1250 rpm. A fresh 1.5 ml tube with 7.5 µl MNase Enzyme Mix is prewarmed at 30 °C for 2 min in an agitating thermal mixer at 1250 rpm. Pre-warmed MNase Enzyme mix is transferred to each pre-warmed tube as follows, 0.5 µl to the first tube, 2.0 µl to the second tube, and 4.0 µl to the third tube. Tubes are incubated for exactly 30 min at 30 °C in an agitating thermal mixer at 1250 rpm. The MNase reaction is stopped by adding 5 µl 0.5 M EDTA and mixing. 3 µl 20% SDS is added to lyse the cells and cells are incubated for 5 min at 30 °C in agitating thermal mixer at 1250 rpm.

### Protocol for Tissue

Notes: It is recommended to use 60 mg tissue. If less than 60 mg of tissue is available refer to low input protocol. Before beginning, fresh 1X MNase Digest Buffer is prepared and stored at room temperature. 1X MNase Digest Buffer is stable for 1 day at room temperature. To prepare 1X MNase Digest Buffer, mix: 140µl UltraPure water; 20 µl 10X MNase Digest Buffer; 20 µl 100mM MnCl2; 20 µl 10%Triton.
At least 60 mg frozen tissue is weighed out and grinded to a fine powder with mortar and pestle in liquid nitrogen to a consistency illustrated in **FIG. 1A** and **FIG. 1B****,** wherein **FIG. 1A** shows insufficient tissue grinding and **FIG. 1B** shows sufficient tissue grinding. The disrupted tissue is transferred to a 5 ml tube with 5 ml 1X PBS and 135 µl 37% formaldehyde. The tube is rotated for 10 min at room temperature. The tube is centrifuged 2000 x g for 5 min and supernatant is carefully removed. In events where tissue does not pellet, the tube is spun at max speed for 5 min. Pellet is resuspended on 200 µl wash buffer, then 4.8 ml 1X HiC wash buffer is added. The tube is centrifuged for 5 min at 2000 x g and supernatant is removed. The wash step is performed 2 times and the final pellet is resuspended in 1 ml 1X HiC wash buffer. The resuspended cells are passed through a 200 µm filter into a fresh 5 ml tube, changing filters if necessary. An additional 2 ml 1X HiC wash buffer is passed through the 200 µm filter. The sample is separated into three 1 ml aliquots in three separate tubes with each aliquot corresponding to 20 mg tissue. Excess tissue can be pelleted and stored at -80 °C. The three tubes are centrifuged at 2000 x g for 5 min and supernatant is removed. Pellets in each tube are resuspended in 50 µl 1X MNase Digest Buffer (freshly prepared). Tubes are pre-warmed to 30 °C for 2 min in an agitating thermal mixer at 1250 rpm. A fresh 1.5 ml tube with 7.5 µl MNase Enzyme Mix is prewarmed at 30 °C for 2 min in an agitating thermal mixer at 1250 rpm. Pre-warmed MNase Enzyme mix is transferred to each pre-warmed tube as follows, 0.5 µl to the first tube, 2.0 µl to the second tube, and 4.0 µl to the third tube. Tubes are incubated for exactly 30 min at 30 °C in an agitating thermal mixer at 1250 rpm. The MNase reaction is stopped by adding 5 µl 0.5 M EDTA and mixing. 3 µl 20% SDS is added to lyse the cells and cells are incubated for 5 min at 30 °C in agitating thermal mixer at 1250 rpm.

### Example 9: Results from MNase-C Libraries

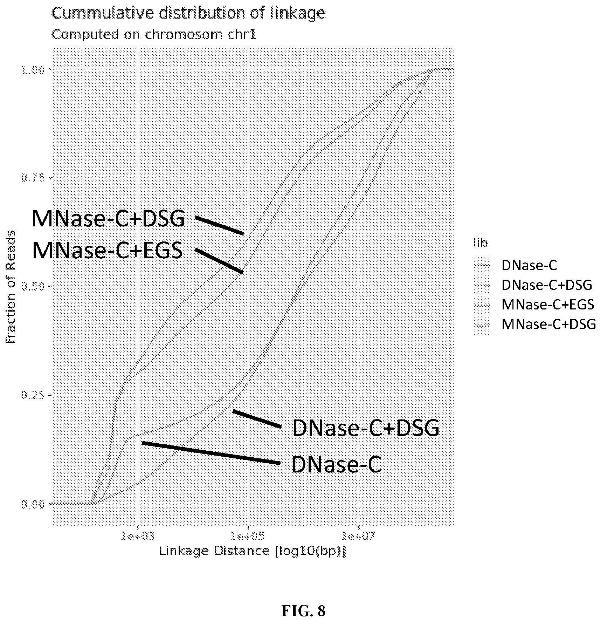

Proximity ligation libraries were prepared using methods herein and sequenced to determine measures of long-range information. **FIG. 7** shows read separation using MNase prepared libraries ("MNase-C") compared with DNase prepared libraries("DNase-C"), as well as with different crosslinking agents. For each bar, the lowest segment shows the percentage of read pairs separated in the genome by more than 1 kb, the middle segment shows the percentage of read pairs separated in the genome by less than 1 kb, and the uppermost segment shows the percentage of read pairs with interchromosomal interactions. From left to right, the bars show results for 1) DNase with formaldehyde crosslinker, 2) DNase with DSG crosslinker, 3) MNase with EGS crosslinker allowed to react for 10 minutes, 4) MNase with EGS crosslinker allowed to react for 30 minutes, 5) MNase with DSG crosslinker allowed to react for 10 minutes, and 6) MNase with DSG crosslinker allowed to react for 30 minutes. **FIG. 8** shows distribution of linkage distance as computed for chromosome 1 of MNase-C prepared libraries (with both DSG and EGS crosslinkers) compared with DNase-C prepared libraries (with both DSG and formaldehyde crosslinkers). DNase-C libraries prepared with the longer spacer arm of DSG show a greater fraction of reads at larger linkage distances compared to DNase-C libraries prepared with formaldehyde, as is also reflected in **FIG. 7****.**

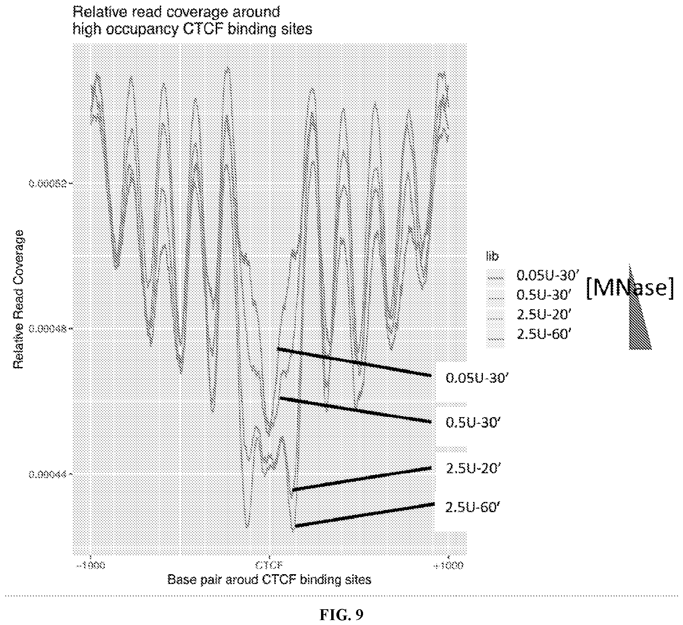

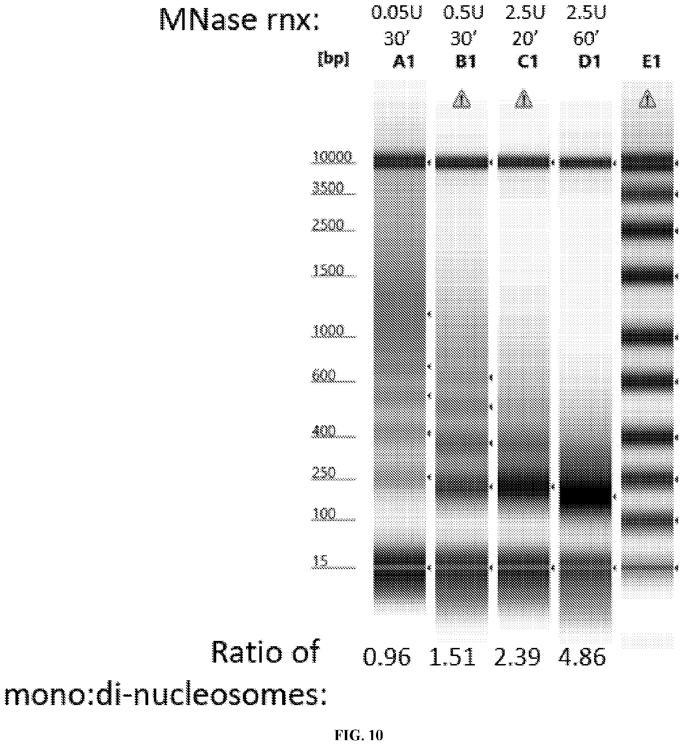

Measures of genome-wide nucleosome mapping were also determined. **FIG. 9** shows relative read coverage around high occupancy CTCF binding sites for libraries prepared with different amounts of MNase and different digestion times: 0.05 units for 30 minutes, 0.5 units for 30 minutes, 2.5 units for 20 minutes, and 2.5 units for 60 minutes, as indicated in the legend. **FIG. 10** shows the ratio of mono:di nucleosomes found in the prepared libraries, at (from left to right) 0.05 U for 30', 0.5 U for 30', 2.5 U for 20', and 2.5 U for 60', with ladder on the far right. Ratio of mono:di-nucleosomes were (from left to right) 0.96, 1.51, 2.39, and 4.86.

### Example 10: HiC Analysis with MNase Digested Samples

A user wishes to determine with high precision the locations of nucleic acid binding proteins in a biological sample. The biological sample is crosslinked using a chemical fixative in order to crosslink nucleic acid binding proteins to the nucleic acids to which they are bound. The fixed sample is then digested with micrococcal nuclease (MNase) which digests all nucleic acids not bound to a protein. MNase treated nucleic acids are then treated with DNA ligase to obtain proximity ligation products. Nucleic acids are purified, and sequencing libraries are made. The sequencing libraries are sequenced to obtain read-pairs for the MNase digested samples. From the read-pairs it is determined with high precision the localization of nucleic acid binding proteins in the biological sample because only sequences from nucleic acids bound to nucleic acid binding proteins are obtained.

### Example 11: HiChIP with MNase Digested Samples

MNase HiChIP analysis was performed as follows: A sample of cells was crosslinked with formaldehyde and DSG crosslinking, digested *in situ* with MNase, and lysed with RIPA lysis buffer. Antibodies for CCCTC-binding factor (CTCF) and H3K4me3-modified histones were contacted to the sample and pulled down via magnetic beads. Proximity ligation was conducted, including end polishing, bridge ligation, and intra-aggregate ligation. Then, crosslinking was reversed, DNA was cleaned up, and sequencing libraries were prepared and sequenced with 20-40 million 2x150 bp reads.

For comparison, ChIP-seq analysis was performed on a parallel set of samples with the same antibodies. The procedure was the same, but without the crosslinking, digestion, and proximity ligation steps.

**FIG. 11** shows ChIP-seq and HiChIP results compared to peaks reported in the Encyclopedia of DNA Elements (ENCODE) from the University of California, Santa Cruz (UCSC) Genome Browser. The negative control libraries show coverage across the whole genome, while the CTCF and H3K4me3 libraries show pile-ups of sequencing reads at loci that match the locations ENCODE peaks for their binding sites. This shows that these libraries do convey ChIP-seq signal, with reads almost entirely coming from the regions where the proteins that correspond to the selected antibodies are expected to bind.

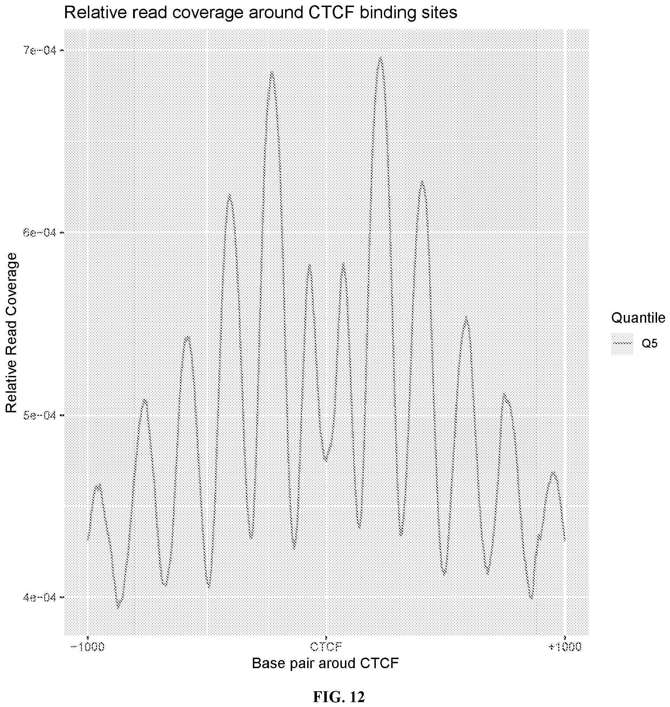

**FIG. 12** shows the relative read coverage around CTCF binding sites for the HiChIP samples. The relative read coverage shows a periodicity of approximately 146 bp, which is consistent with the areas of DNA protected from MNase digestion by the presence of histones in nucleosomes. This shows that these libraries do have the protective profiles expected of an MNase-C library.

In Tables 3-5, inter.chr stands for the percentage of read pairs that are inter-chromosomal (each read in the pair maps to a different chromosome), <1kb stands for the percentage of read pairs that span a separation distance on the genome of less than 1 kb, >1kb stands for the percentage of read pairs that span a separation distance on the genome of greater than 1 kb, fracMapped stands for the percentage of read pairs mapped, and preSeqAt300M stands for the number of unique reads out of a population of 300 million reads.

Table 3 shows typical library quality control (QC) metrics for MNase-C libraries. The percentage of inter-chromosomal read pairs is between 33% and 35%, the percentage of read pairs spanning a separation distance of less than 1 kb is between 7% and 12%, the percentage of read pairs spanning a separation distance of greater than 1 kb is between 55% and 58%, the mapped fraction is between 67% and 79%, and the number of unique reads out of 300 million is between 168 million and 238 million. As shown in Table 4, the QC metrics for MNase HiChIP libraries are relatively similar to those for MNase-C libraries, with inter-chromosomal read pairs between 21.6% and 43.4%, read pairs spanning less than 1 kb between 18.2% and 31.2%, read pairs spanning greater than 1 kb between 25.4% and 56.2%, and between 272 million and 279 million unique reads out of 300 million. By comparison, the QC metrics for ChIP-seq libraries are not like MNase-C libraries (or other proximity ligation libraries), with between 0.46% and 1.65% inter-chromosomal reads, read pairs spanning less than 1 kb between 98.64% and 99.45%, and read pairs spanning greater than 1 kb between 0.09% and 0.16%.

| **Table 3: Typical Library QC Metrics for MNase-C** | | | | | |
|---|---|---|---|---|---|
| **Sample ID** | **inter.chr** | **<1kb** | **>1kb** | **fracMapped** | **preSeqAt300M** |
| **mdev3A** | 35% | 7% | 58% | 67% | 232,517,526 |
| **mdev3B** | 35% | 8% | 57% | 68% | 237,654,318 |
| **mdev3C** | 34% | 11% | 55% | 77% | 218,231,018 |
| **mdev3D** | 33% | 12% | 55% | 77% | 217,283,442 |
| **mdev3E** | 33% | 11% | 56% | 78% | 171,109,061 |
| **mdev3F** | 33% | 11% | 56% | 79% | 168,589,170 |

| **Table 4: QC Metrics for MNase HiChIP Libraries** | | | | |
|---|---|---|---|---|
| | **inter.chr** | **<1kb** | **>1kb** | **preSeqAt300M** |
| **Negative Control** | 21.6% | 22.2% | 56.2% | 270,540,612 |
| **CTCF** | 40.3% | 18.2% | 41.5% | 278,669,408 |
| **H3K36me3** | 43.4% | 31.2% | 25.4% | 272,358,079 |

| **Table 5: QC Metrics for ChIP-seq Libraries** | | | | |
|---|---|---|---|---|
| | **inter.chr** | **<1kb** | **>1kb** | **preSeqAt300M** |
| **Negative Control** | 1.23% | 98.64% | 0.13% | NA |
| **CTCF** | 1.65% | 98.19% | 0.16% | NA |
| **H3K36me3** | 0.46% | 99.45% | 0.09% | NA |

**FIG. 13** shows contact maps for read pairs presented over graphs of read coverage showing pile-ups of reads associated with the targeted proteins (as shown in **FIG. 11****)** and over a graph of gene annotations. H3K4me3 modification is associated with transcription of nearby genes. A dense region of contacts **1301** is seen beginning with a H3K4me3-associated peak **1302** of read coverage and continuing leftward; as shown at the bottom this region **1303** is also annotated as containing genes which are read in the same direction. Similarly, at **1304** a dense region of contacts is seen beginning with another H3K4me3-associated peak **1305** of read coverage and continuing rightward; as shown at the bottom this region **1306** is also annotated as containing the FABP5 gene which is read in the same direction. CTCF is associated with formation of chromatin loops, with two CTCF proteins bound to different loci coming together and the DNA between them forming a loop. Triangles of contact density representing topologically-associated domains (TADs) are seen, for example, those with peaks **1311, 1314, 1317,** and **1320.** Tracing down the left and right edges of these triangles, it can be seen that the boundaries of these regions line up with CTCF-associated peaks (e.g., **1311** with CTCF peaks **1312** and **1313, 1314** with CTCF peaks **1315** and **1316, 1317** with CTCF peaks **1318** and **1319, 1320** with CTCF peaks **1321** and **1322),** allowing discernment of which particular CTCF sites are coming together to form which loops and domains.

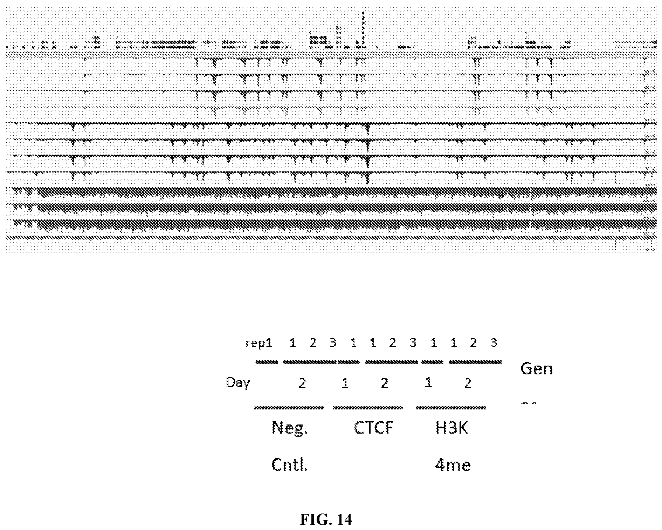

**FIG. 14** shows the same comparison of MNase HiChIP results to ENCODE peaks as in **FIG. 11****,** but for sample replicates on the same and subsequent days. This shows the consistency and reproducibility of the protocol.

Overall, these experiments demonstrate that MNase HiChIP libraries have ChIP-seq characteristics,
MNase properties, Hi-C properties, show Hi-C interactions between protein peaks, and that the protocol is robust and has high reproducibility.

### Example 12: Proximity Ligation Using a Split-Pool Labeling Approach

A stabilized biological sample is obtained comprising cells that have been fixed with a crosslinking agent. The sample is treated with a DNase to digest the DNA in the cells in situ. The sample is then treated with enzymes to polish the ends of the DNA and to polyadenylate the DNA ends. The cells are then dispensed into wells of a 96-well plate with one cell per well. A barcode is added to each well and ligated to the DNA ends in each well. The cells are then pooled and dispensed again with a single cell in each well. A second barcode is added and ligated to the first barcode in each well. The cells are then pooled again and dispensed again with a single cell per well and a bridge adaptor is ligated with an overhang that is compatible with a second bridge adaptor. This approach is illustrated in **FIG. 16****.** The proximal ends are then ligated resulting in a molecule illustrated in **FIG. 17** where each end has two barcodes and a bridge which links to another bridge on the other end. **FIG. 18** shows an example of combinations of barcodes and a bridge resulting from the splitting and pooling approach. Crosslinking is then reversed, nucleic acids are purified and sequenced to obtain sequence information.

### Example 13: Proximity Ligation Using Targeted Adaptors

A stabilized biological sample is obtained comprising cells that have been fixed with a crosslinking agent. The sample is treated with a DNase to digest the DNA in the cells in situ. The sample is then treated with enzymes to polish the ends of the DNA and polyadenylate the DNA ends. The sample is then contacted with an antibody that binds to the histones in the DNA and then the sample is contacted with a plurality of Protein A tethered biotinylated library adaptors before ligating the DNA ends. The adaptors are ligated between proximal ends where the histone binding antibody was bound. Streptavidin is used to pull down the biotinylated adaptors and the crosslinks are reversed on the resulting purified sample. Amplification and PCR are then performed to obtain sequence information.

### Example 14: Aggregate three-dimensional nucleic acid configuration determination may lose cell specific information.

A cell population is collected for three-dimensional nucleic acid configuration analysis. Cells of the population share a nucleic acid configuration that leads regions of chromosome 1 and chromosome 2 to be in proximity, while regions of chromosomes 3 and 4 are in proximity in some but not all of the cells. Stabilized nuclei are partitioned, fragmented to expose internal ends, attB tagged and then contacted to an attP linking nucleic acid population lacking cell-distinguishing information in the presence of phiC31 integrase.

Library constituents are end-sequenced. It is observed that read pairs mapping to chromosome 1 and 2 on a common molecule are differentially observed above background. It is observed that read pairs mapping to chromosome 3 and 4 on a common molecule are differentially observed above background at a lower frequency.

One cannot distinguish whether chromosomes 3 and 4 are in proximity but farther removed from one another than chromosomes 1 and 2, or if there is configuration variation among members of the cell population.

### Example 15: Cell-specific three-dimensional nucleic acid configuration information is preserved through the methods herein

A cell population is collected for three-dimensional nucleic acid configuration analysis. Cells of the population share a nucleic acid configuration that leads regions of chromosome 1 and chromosome 2 to be in proximity, while regions of chromosomes 3 and 4 are in proximity in some but not all of the cells. Stabilized nuclei are partitioned, fragmented to expose internal ends, attB tagged and then contacted to a population of attP linking nucleic acids having cell-distinguishing information in the presence of phiC31 integrase.

Library constituents are end-sequenced so as to obtain both internal-end adjacent sequence and partition distinguishing sequence of the linker. It is observed that read pairs mapping to chromosome 1 and 2 on a common molecule are differentially observed above background independent of partition distinguishing sequence. It is observed that read pairs mapping to chromosome 3 and 4 on a common molecule are observed at a level comparable to that of chromosome 1 and 2 pairs in a first population of cell-distinguished library constituents but are not observed above background in a second population of cell-distinguished library components. It is concluded that the associated segments of chromosomes 3 and 4 exhibit conformational variation within the population, such that some cells exhibit a three-dimensional proximity of segments of chromosome 3 and 4 comparable to that of chromosomes 1 and 2, while other cells do not exhibit a three dimensional proximity of segments of chromosome 3 and 4 comparable to that of chromosomes 1 and 2.

### Example 16: Cell-specific three-dimensional nucleic acid configuration information is quantifiably measured

A cell population is collected for three-dimensional nucleic acid configuration analysis. Cells of the population share a nucleic acid configuration that leads regions of chromosome 1 and chromosome 2 to be in proximity, while regions of chromosomes 3 and 4 vary in their proximity quantitatively among cells. Stabilized nuclei are partitioned, fragmented to expose internal ends, attB tagged and then contacted to a population of attP linking nucleic acids having cell-distinguishing information in the presence of phiC31 integrase.

Library constituents are end-sequenced so as to obtain both internal-end adjacent sequence and partition distinguishing sequence of the linker. It is observed that read pairs mapping to chromosome 1 and 2 on a common molecule are differentially observed above background independent of partition distinguishing sequence. It is observed that read pairs mapping to chromosome 3 and 4 on a common molecule are observed at a level that varies according to cell, as indicated by partition-distinguishing sequence information in combination with read pair frequencies. It is concluded that the indicated segments of chromosomes 1 and 2 are in proximity throughout the population of cells. It is concluded that the associated segments of chromosomes 3 and 4 exhibit conformational variation within the population, such that there is quantitative variation across a continuum as to proximity of the indicated segments of chromosomes 3 and 4.

## Claims

1. A method comprising:
(a) obtaining a cross-linked biological sample comprising a nucleic acid molecule complexed to at least one nucleic acid binding protein;
(b) contacting the cross-linked biological sample to a non-specific endonuclease to cleave the nucleic acid molecule into a plurality of segments;
(c) attaching a first segment and a second segment of the plurality of segments at a junction thereby obtaining a product;
(d) reversing crosslinks in the product of (c) to obtain an additional product; and
(e) subjecting the additional product of (d) to size selection to obtain a plurality of selected segments having a size from 100 bp to 600 bp,
wherein attaching comprises contacting at least the first segment and the second segment to at least one bridge oligonucleotide.

2. The method of claim 1, further comprising, prior to (d), preparing a sequencing library from the plurality of segments and optionally subjecting the sequencing library to a size selection to obtain a size-selected library.

3. The method of any one of claims 1 or claim 2, wherein the cross-linked biological sample comprises a cross-linked cell lysate, a cross-linked intact cell, or a cross-linked intact nucleus.

4. The method of claim 3, wherein the cross-linked biological sample comprise the intact cell or the intact nucleus, and wherein (b) is conducted prior to lysis of the intact cell or the intact nucleus.

5. The method of claim 1, further comprising, prior to (c), lysing cells or nuclei in the cross-linked biological sample.

6. The method of any one of claims 1 to 5, wherein the non-specific endonuclease is selected from one or more of DNase I, DNase II, and micrococcal nuclease.

7. The method of any one of claims 1 to 6, wherein (a) comprises treating a biological sample with a chemical fixative or ultraviolet light to obtain the cross-linked biological sample.

8. The method of any one of claims 1 to 7, further comprising contacting the plurality of selected segments to an antibody and optionally conducting immunoprecipitation on the plurality of segments.

9. The method of any one of claims 1 to 8, wherein attaching in (c) comprises filling in sticky ends using biotin tagged nucleotides, filling in sticky ends using untagged nucleotides, ligating blunt ends, adding overhangs, or contacting at least the first segment and the second segment to a barcode.

10. The method of claim 1, wherein the bridge oligonucleotide comprises a barcode sequence or an affinity tag.

11. The method of claim 1, wherein attaching in (c) comprises contacting at least the first segment and the second segment to multiple bridge oligonucleotides in series.

12. The method of any one of claims 1 to 11, wherein the at least one bridge oligonucleotide is coupled to an immunoglobulin binding protein or a fragment thereof, wherein the immunoglobulin binding protein is optionally selected from a Protein A, a Protein G, a Protein A/G, and a Protein L.

13. The method of claim 1, further comprising:
(f) obtaining at least some sequence on each side of the junction to generate a first read pair.

14. The method of claim 13, further comprising:
(f) mapping the first read pair to a set of contigs; and
(g) determining a path through the set of contigs that represents an order or orientation to a genome.

15. The method of claim 13, further comprising:
(f) mapping the first read pair to a set of contigs; and
(g) determining, from the set of contigs, a presence of a structural variant or loss of heterozygosity in the cross-linked biological sample.

16. The method of claim 13, further comprising:
(f) mapping the first read pair to a set of contigs; and
(g) assigning a variant in the set of contigs to a phase,
wherein the variant is optionally selected from a human leukocyte antigen (HLA) variant or a killer-cell immunoglobulin-like receptor (KIR) variant.

17. The method of claim 13, further comprising:
(f) mapping the first read pair to a set of contigs;
(g) determining, from the set of contigs, a presence of a variant in the set of contigs; and
(h) conducting one or more of: (1) identifying a disease stage, a prognosis, or a course of treatment for a condition associated with the cross-linked biological sample; (2) selecting a drug based on the presence of the variant; or (3) identifying a drug efficacy for a condition associated with the cross-linked biological sample.

18. The method of any one of claims 1 to 17, wherein the non-specific endonuclease is coupled or fused to an immunoglobulin binding protein or a fragment thereof, wherein the immunoglobulin binding protein is optionally selected from a Protein A, a Protein G, a Protein A/G, and a Protein L.

## Patentansprüche

1. Verfahren, umfassend:
(a) Erhalten einer vernetzten biologischen Probe, umfassend ein Nukleinsäuremolekül, das mit wenigstens einem nukleinsäurebindenden Protein komplexiert ist;
(b) Inberührungbringen der vernetzten biologischen Probe mit einer unspezifischen Endonuklease, um das Nukleinsäuremolekül in eine Mehrzahl von Segmenten zu spalten;
(c) Anlagern eines ersten Segments und eines zweiten Segments der Mehrzahl von Segmenten an einer Verbindungsstelle, wobei dadurch ein Produkt erhalten wird;
(d) Umkehren der Vernetzungen in dem Produkt von (c), um ein zusätzliches Produkt zu erhalten; und
(e) Unterziehen des zusätzlichen Produkts von (d) einer Größenauswahl, um eine Mehrzahl von ausgewählten Segmenten mit einer Größe von 100 bp bis 600 bp zu erhalten,
wobei das Anlagern das Inberührungbringen wenigstens des ersten Segments und des zweiten Segments mit wenigstens einem Brücken-Oligonukleotid umfasst.

2. Verfahren nach Anspruch 1, das ferner vor (d) das Herstellen einer Sequenzierungsbibliothek aus der Mehrzahl von Segmenten und optional das Unterziehen der Sequenzierungsbibliothek einer Größenauswahl umfasst, um eine größenausgewählte Bibliothek zu erhalten.

3. Verfahren nach einem der Ansprüche 1 oder 2, wobei die vernetzte biologische Probe ein vernetztes Zell-Lysat, eine vernetzte intakte Zelle oder einen vernetzten intakten Zellkern umfasst.

4. Verfahren nach Anspruch 3, wobei die vernetzte biologische Probe die intakte Zelle oder den intakten Zellkern umfasst, und wobei (b) vor der Lyse der intakten Zelle oder des intakten Kerns durchgeführt wird.

5. Verfahren nach Anspruch 1, das ferner vor (c) das Lysieren von Zellen oder Kernen in der vernetzten biologischen Probe umfasst.

6. Verfahren nach einem der Ansprüche 1 bis 5, wobei die unspezifische Endonuklease aus einer oder mehreren von DNase I, DNase II und Mikrokokken-Nuklease ausgewählt ist.

7. Verfahren nach einem der Ansprüche 1 bis 6, wobei (a) das Behandeln einer biologischen Probe mit einem chemischen Fixiermittel oder ultraviolettem Licht umfasst, um die vernetzte biologische Probe zu erhalten.

8. Verfahren nach einem der Ansprüche 1 bis 7, das ferner das Inberührungbringen der Mehrzahl von ausgewählten Segmenten mit einem Antikörper und optional das Durchführen einer Immunpräzipitation an der Mehrzahl von Segmenten umfasst.

9. Verfahren nach einem der Ansprüche 1 bis 8, wobei das Anlagern in (c) das Auffüllen von klebrigen Enden unter Verwendung von biotinmarkierten Nukleotiden, das Auffüllen von klebrigen Enden unter Verwendung von unmarkierten Nukleotiden, das Ligieren stumpfer Enden, das Hinzufügen von Überhängen oder das Inberührungbringen wenigstens des ersten Segments und des zweiten Segments mit einem Barcode umfasst.

10. Verfahren nach Anspruch 1, wobei das Brücken-Oligonukleotid eine Barcode-Sequenz oder einen Affinitäts-Tag umfasst.

11. Verfahren nach Anspruch 1, wobei das Anlagern in (c) das Inberührungbringen wenigstens des ersten Segments und des zweiten Segments mit mehreren Brücken-Oligonukleotiden in Serie umfasst.

12. Verfahren nach einem der Ansprüche 1 bis 11, wobei das wenigstens eine Brücken-Oligonukleotid an ein immunglobulinbindendes Protein oder ein Fragment davon gekoppelt ist, wobei das immunglobulinbindende Protein optional aus einem Protein A, einem Protein G, einem Protein A/G und einem Protein L ausgewählt ist.

13. Verfahren nach Anspruch 1, das ferner umfasst:
(f) Erhalten wenigstens einiger Sequenz auf jeder Seite der Verbindung, um ein erstes Lesepaar zu erzeugen.

14. Verfahren nach Anspruch 13, das ferner umfasst:
(f) Abbilden des ersten Lesepaares auf einen Satz von Contigs; und
(g) Bestimmen eines Pfades durch den Satz von Contigs, der eine Ordnung oder Orientierung zu einem Genom darstellt.

15. Verfahren nach Anspruch 13, das ferner umfasst:
(f) Abbilden des ersten Lesepaares auf einen Satz von Contigs; und
(g) Bestimmen aus dem Satz von Contigs eines Vorhandenseins einer strukturellen Variante oder eines Verlustes von Heterozygotie in der vernetzten biologischen Probe.

16. Verfahren nach Anspruch 13, das ferner umfasst:
(f) Abbilden des ersten Lesepaares auf einen Satz von Contigs; und
(g) Zuordnen einer Variante in dem Satz von Contigs zu einer Phase,
wobei die Variante optional aus einer humanen Leukozytenantigen- (HLA-) Variante oder einer Killerzellen-Immunglobulin-ähnlichen Rezeptor- (KIR-) Variante ausgewählt ist.

17. Verfahren nach Anspruch 13, das ferner umfasst:
(f) Abbilden des ersten Lesepaares auf einen Satz von Contigs;
(g) Bestimmen aus dem Satz von Contigs eines Vorhandenseins einer Variante in dem Satz von Contigs; und
(h) Durchführen eines oder mehrerer von: (1) Identifizieren eines Krankheitsstadiums, einer Prognose oder eines Verlaufs der Behandlung für einen Zustand, der mit der vernetzten biologischen Probe assoziiert ist; (2) Auswählen eines Arzneimittels auf Grundlage des Vorhandenseins der Variante; oder (3) Identifizieren einer Arzneimittelwirksamkeit für einen Zustand, der mit der vernetzten biologischen Probe assoziiert ist.

18. Verfahren nach einem der Ansprüche 1 bis 17, wobei die unspezifische Endonuklease an ein immunglobulinbindendes Protein oder ein Fragment davon gekoppelt oder fusioniert ist, wobei das immunglobulinbindende Protein optional aus einem Protein A, einem Protein G, einem Protein A/G und einem Protein L ausgewählt ist.

## Revendications

1. Procédé comprenant :
(a) l'obtention d'un échantillon biologique réticulé comprenant une molécule d'acide nucléique complexée avec au moins une protéine de liaison à un acide nucléique ;
(b) la mise en contact de l'échantillon biologique réticulé avec une endonucléase non spécifique pour cliver la molécule d'acide nucléique en une pluralité de segments ;
(c) la fixation d'un premier segment et d'un deuxième segment de la pluralité de segments au niveau d'une jonction pour ainsi obtenir un produit ;
(d) l'inversion de réticulations dans le produit de (c) pour obtenir un produit additionnel ; et
(e) la soumission du produit additionnel de (d) à une sélection par taille pour obtenir une pluralité de segments sélectionnés ayant une taille de 100 pb à 600 pb,
dans lequel la fixation comprend la mise en contact au moins du premier segment et du deuxième segment avec au moins un oligonucléotide de pontage.

2. Procédé selon la revendication 1, comprenant en outre, avant (d), la préparation d'une bibliothèque de séquençage à partir de la pluralité de segments et éventuellement la soumission de la bibliothèque de séquençage à une sélection par taille pour obtenir une bibliothèque sélectionnée par taille.

3. Procédé selon l'une quelconque de la revendication 1 ou de la revendication 2, dans lequel l'échantillon biologique réticulé comprend un lysat cellulaire réticulé, une cellule intacte réticulée, ou un noyau intact réticulé.

4. Procédé selon la revendication 3, dans lequel l'échantillon biologique réticulé comprend la cellule intacte ou le noyau intact, et dans lequel (b) est conduite avant une lyse de la cellule intacte ou du noyau intact.

5. Procédé selon la revendication 1, comprenant en outre, avant (c), la lyse de cellules ou de noyaux dans l'échantillon biologique réticulé.

6. Procédé selon l'une quelconque des revendications 1 à 5, dans lequel l'endonucléase non spécifique est sélectionnée parmi une ou plusieurs parmi l'ADNase I, l'ADNase II, et la nucléase micrococcique.

7. Procédé selon l'une quelconque des revendications 1 à 6, dans lequel (a) comprend le traitement d'un échantillon biologique avec un fixateur chimique ou de la lumière ultraviolette pour obtenir l'échantillon biologique réticulé.

8. Procédé selon l'une quelconque des revendications 1 à 7, comprenant en outre la mise en contact de la pluralité de segments sélectionnés avec un anticorps et éventuellement la conduite d'une immunoprécipitation sur la pluralité de segments.

9. Procédé selon l'une quelconque des revendications 1 à 8, dans lequel la fixation dans (c) comprend le remplissage d'extrémités cohésives à l'aide de nucléotides marqués à la biotine, le remplissage d'extrémités cohésives à l'aide de nucléotides non marqués, la ligature d'extrémités franches, l'ajout d'extensions, ou la mise en contact au moins du premier segment et du deuxième segment avec un code à barres.

10. Procédé selon la revendication 1, dans lequel l'oligonucléotide de pontage comprend une séquence de code à barres ou un marqueur d'affinité.

11. Procédé selon la revendication 1, dans lequel la fixation dans (c) comprend la mise en contact au moins du premier segment et du deuxième segment avec de multiples oligonucléotides de pontage en série.

12. Procédé selon l'une quelconque des revendications 1 à 11, dans lequel l'au moins un oligonucléotide de pontage est couplé à une protéine de liaison à l'immunoglobuline ou un fragment de celle-ci, dans lequel la protéine de liaison à l'immunoglobuline est éventuellement sélectionnée parmi une protéine A, une protéine G, une protéine A/G, et une protéine L.

13. Procédé selon la revendication 1, comprenant en outre :
(f) l'obtention d'au moins certaines séquences sur chaque côté de la jonction afin de générer une première paire de lecture.

14. Procédé selon la revendication 13, comprenant en outre :
(f) la mise en correspondance de la première paire de lecture avec un ensemble de contigs ; et
(g) la détermination d'un trajet à travers l'ensemble de contigs qui représente un ordre ou une orientation vers un génome.

15. Procédé selon la revendication 13, comprenant en outre :
(f) la mise en correspondance de la première paire de lecture avec un ensemble de contigs ; et
(g) la détermination, à partir de l'ensemble de contigs, d'une présence d'au variant structurel ou d'une perte d'hétérozygotie dans l'échantillon biologique réticulé.

16. Procédé selon la revendication 13, comprenant en outre :
(f) la mise en correspondance de la première paire de lecture avec un ensemble de contigs ; et
(g) l'attribution d'un variant dans l'ensemble de contigs à une phase,
dans lequel le variant est éventuellement sélectionné parmi un variant d'antigène de leucocyte humain (HLA) ou un variant de récepteur de type immunoglobuline de cellule tueuse (KIR).

17. Procédé selon la revendication 13, comprenant en outre :
(f) la mise en correspondance de la première paire de lecture avec un ensemble de contigs ;
(g) la détermination, à partir de l'ensemble de contigs, d'une présence d'un variant dans l'ensemble de contigs ; et
(h) la conduite d'une ou de plusieurs parmi : (1) l'identification d'un stade de maladie, d'un pronostic, ou d'une série de traitements pour une affection associée à l'échantillon biologique réticulé ; (2) la sélection d'un médicament sur la base de la présence du variant ; ou (3) l'identification d'une efficacité de médicament pour une affection associée à l'échantillon biologique réticulé.

18. Procédé selon l'une quelconque des revendications 1 à 17, dans lequel l'endonucléase non spécifique est couplée ou fusionnée à une protéine de liaison à l'immunoglobuline ou un fragment de celle-ci, dans lequel la protéine de liaison à l'immunoglobuline est éventuellement sélectionnée parmi une protéine A, une protéine G, une protéine A/G, et une protéine L.
